# EUROPEAN PATENT APPLICATION

(11) **EP 3 922 719 A1**
(43) Date of publication of application: **15.12.2021**
(21) Application number: 21179356.7
(22) Date of filing: 14.06.2021
(51) Int. Cl.: C12N 15/10

(54) **SPECIFIC DECOLONIZATION OF ANTIBIOTIC RESISTANT BACTERIA FOR PROPHYLACTIC PURPOSES**

(30) Priority: 12.06.2020 US 202063038386 P; 03.07.2020 US 202063048034 P; 30.12.2020 US 202063132190 P; 15.01.2021 US 202163137989 P; 11.02.2021 US 202163148479 P
(71) Applicant: Eligo Bioscience, 75014 Paris (FR)
(72) Inventor: DUPORTET, Xavier, 75014 PARIS (FR); STZEPOURGINSKI, Igor, 75014 PARIS (FR); FERNANDEZ RODRIGUEZ, Jesus, 75014 Paris (FR)
(74) Representative: Jan, Gaëlle

(57) **Abstract**

The invention relates to methods, kits, and compositions for reducing the level of or eliminating antimicrobial resistant (AMR) bacteria *in situ.* The invention encompasses compositions and methods for selectively eradicating antibiotic resistance in bacteria that carry antibiotic resistance genes in the microbiota using packaged phagemids. The microbiota can be intestinal and the packaged phagemids can be administered to a healthy subject or patient, for example, orally, rectally (e.g., in an enema), vaginally, nasally or to the skin. The phagemid encodes a nuclease or other enzyme that genetically modifies the DNA encoding the antibiotic resistance gene so that the bacteria can then be eliminated with the antibiotic.

## Description

### Background of the invention

Antimicrobial resistant (AMR) infections are poised to represent an increasing threat in the near future, particularly in the hospital setting where they are the most prevalent and put patients at risk as they increase the length and cost of hospitalization, but also more generally in immunocompromised patients, and dramatically impair prognosis.

In most cases the bacteria responsible for AMR infections in hospitalized and/or immunocompromised patients originate from the patients themselves. Indeed, microbial communities that colonize our body (the microbiota) can carry AMR bacteria that do not represent any threat under normal circumstances but can be responsible for difficult-to-treat infections when they disseminate in the body after various triggering events, for example in the hospital setting but also in immunocompromised patients. These triggering events can be invasive procedures (surgery, intubation, catheterization, etc.) or harsh conditioning procedures (immunosuppression, irradiation, etc.) that compromise epithelial barriers and promote dissemination of bacteria and infections in patients.

Indeed, it is now well accepted that asymptomatic carriage of AMR bacteria predisposes to difficult-to-treat infections with the same organism. Patients presenting skin/nasal or intestinal carriage of AMR bacteria (notably Methicillin resistant Staphylococcus aureus - MRSA), and Extended Spectrum beta lactamase (ESBL) and/or Carbapenemase-producing (CP) Enterobacteria, respectively) at the time of hospitalization and/or when suffering from another disease, are more prone to develop AMR infections with the same organism in various situations (ICU, surgery, dialysis, transplants, hematopoietic stem cell transplantation, chemotherapy, irradiation, immune checkpoint inhibitors therapy, etc.) (Sakr et al., 2019; Gargiullo et al., 2019; Stalin et al., 2018; Lambelet et al., 2017).

In this context, strategies aiming at decolonizing AMR bacteria from the microbiota of patients before their invasive or non-invasive medical procedures to reduce the risk of infection have been attempted. The benefit of prophylactic decolonization of MRSA from the skin/nose has already been demonstrated as it was shown to reduce the risk of MRSA infections later during hospitalization (Bode et al., 2010; Perl et al., 2002). The gold standard for such decolonization is topical treatment with mupirocin (Walker et al., 2003) but, more recently, other approaches using lysins have shown similar or better performance in preclinical models (Kokai-kun et al., 2003; Pastagia et al., 2011).

The situation is more complicated for the intestine where the microbiota is much more diverse and complex, and more difficult to access than the skin microbiota. Selective Digestive Decontamination (SDD) consists in treating a patient presenting intestinal carriage with antibiotic resistant bacteria with cocktails of non-absorbable wide spectrum antibiotics (colistin, tobramycin) to eradicate the antibiotic resistant bacteria from their intestines. SDD remains poorly efficient, not durable and presents important side effects (disruption of the microbiota - dysbiosis - and associated infections, selection of additional antibiotic resistance, impaired prognosis in bone marrow transplant patients) that prevent its adoption in the clinic (Rieg et al., 2015; Gargiullo et al., 2019). It was also proposed to administer a selective oral decontamination therapy of carbapenem-resistant *Klebsiella pneumoniae* with oral gentamicin in hematologic patients as a complementary approach for reducing the risk of severe infection with these antibiotic-resistant bacteria (Lambelet et al., 2017). However, such a therapy also presents important side effects (disruption of the microbiota - dysbiosis - and associated infections, selection of additional antibiotic resistance) that questions its use in the clinic.

Probiotics and live biological products have shown promising results for the elimination of gram positive bacteria (including MRSA and Vancomycin-Resistant Enterococci) in the intestine (Manley et al., 2007; Szachta et al., 2011), but have given no sign of efficacy for antibiotic resistant gram negative bacteria (Tannock et al., 2011; Salomão et al., 2016).

Fecal Material Transplant (FMT) has also been proposed as a potential way to eradicate carriage of antibiotic resistant bacteria as it showed some degree of efficacy in various studies in patients (Saha et al., 2019). However, intrinsic limitations (dependency on stool donors, standardization issues due to donor heterogeneity, limited amount of available material) as well as safety issues (transfer of cryptic pathogens and/or other antibiotic resistant organisms especially in weakened patients at risk of infections) prevents the widespread use of FMT as a drug in the clinic.

Approaches using bacteriophages or bacteriophage parts (lysins, pyocins, etc.) to selectively eradicate bacterial populations at the strain or species level are also considered as potential strategies to eradicate antibiotic resistant bacteria from a patient's intestine. However, these approaches have failed to be proven efficacious in the intestine so far. Moreover, wild-type bacteriophages and bacteriophage parts cannot selectively eradicate antibiotic resistant bacterial strains as their specificity is solely driven by the recognition of molecular patterns exposed at the surface of the target bacteria (outer membrane proteins, serotype, capsule type, etc.) and not on the genes they carry (De Jonge et al,. 2019). Antibiotic resistance genes are, for the most part, carried by mobile genetic elements and acquired by horizontal gene transfers: their presence in bacterial strains does not necessarily correlate with their serotype or capsule type (San Millan et al,. 2018). In complex ecosystems like the intestine, antibiotic resistant and antibiotic sensitive strains of a given species are thought to occupy the same ecological niche.

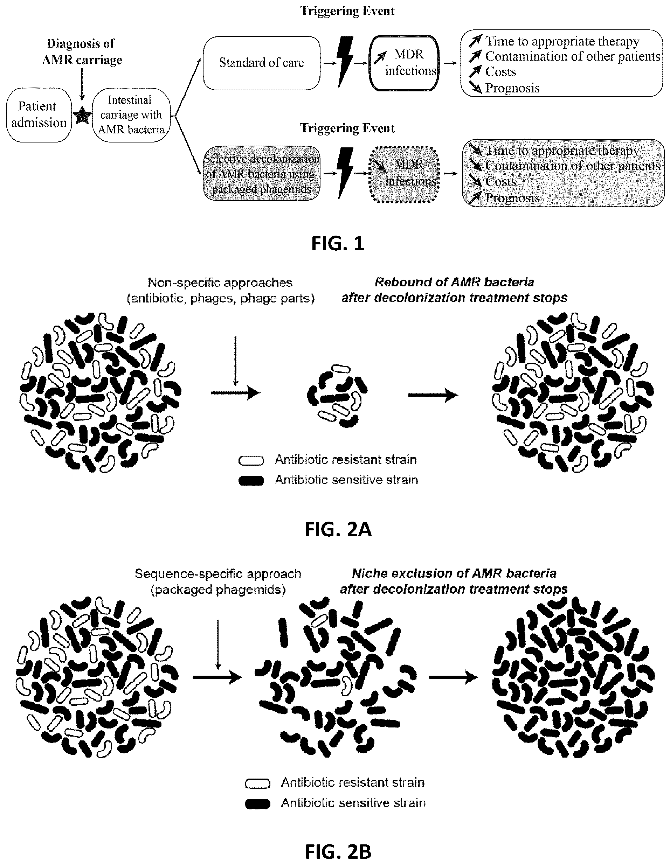

Therefore, targeting both antibiotic resistant and antibiotic sensitive strains irrespectively has little chance to lead to eradication of the antibiotic resistant strains as both antibiotic resistant and antibiotic sensitive strains are expected to come back at a similar ratio after bacterial cells that survived the treatment repopulate their niche **(****Fig.2A****).**

Attempts to decolonize antibiotic resistant bacteria from the intestine have failed to be proven efficient. Thus, compositions and methods for decolonizing antibiotic resistant bacteria from the microbiota, and particularly for decolonizing antibiotic resistant bacteria from the intestine, are needed. The present invention fulfills this need.

### Brief Summary of Invention

The invention encompasses compositions, kits, and methods for selectively eradicating bacteria that carry antibiotic resistance genes in the microbiota using packaged phagemids or engineered bacteriophages. Compared to wild-type phages used in the prior art, engineered bacteriophages used in the context of the invention can selectively eradicate antibiotic resistant bacterial strains because of their specificity which is not only driven by the recognition of molecular patterns exposed at the surface of the target bacteria (outer membrane proteins, serotype, capsule type, etc.) but also on the genes they carry after engineering, in particular the nucleic acid encoding a DNA modifying enzyme inactivating the antibiotic resistance gene(s) responsible for antibiotic-resistance of bacterial strain(s), as disclosed below.

Preferably, the microbiota is intestinal and the packaged phagemids or engineered bacteriophages are administered orally. Preferably, the phagemid or the genome of engineered bacteriophages encodes a nuclease or other enzyme that genetically modifies the DNA encoding the antibiotic resistance gene so that the bacteria can be eliminated with the antibiotic.

In one embodiment, the invention encompasses a method of selectively removing antibiotic resistance from an antibiotic resistant bacterial strain(s) in a healthy subject carrying antibiotic resistant bacterial strain(s) or in a patient carrying antibiotic resistant bacterial strain(s), wherein the method comprises administering to the subject or patient at least one vector (in particular at least 2, 3, 4, 5, 6, 7, 8, 9, 10 or more vectors), said vector encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said healthy subject or patient and preventing antibiotic resistance related infection in the subject or patient. In the context of the invention, said healthy subject and said patient do not suffer from a bacterial infection, in particular do not suffer from an antibiotic resistance related bacterial infection, i.e. said healthy subject and said patient are asymptomatic to a bacterial infection at the time of administration of said vector.

In one embodiment, the invention encompasses a method of selectively removing antibiotic resistance from an antibiotic resistant bacterial strain(s) in a healthy subject carrying antibiotic resistant bacterial strain(s) or in a patient carrying antibiotic resistant bacterial strain(s), wherein the method comprises administering to the subject or patient at least one recombinant phage, (in particular at least 2, 3, 4, 5, 6, 7, 8, 9, 10 or more recombinant phages), said recombinant phage comprising a nucleic acid encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said healthy subject or patient and preventing antibiotic resistance related infection in the subject or patient. In the context of the invention, said healthy subject and said patient do not suffer from a bacterial infection, in particular do not suffer from an antibiotic resistance related bacterial infection, i.e. said healthy subject and said patient are asymptomatic to a bacterial infection at the time of administration of said vector.

In one embodiment, the invention concerns at least one vector for use in a method of selectively removing antibiotic resistance from an antibiotic resistant bacterial strain(s) in a healthy subject carrying antibiotic resistant bacterial strain(s) or in a patient carrying antibiotic resistant bacterial strain(s), wherein said vector encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns at least one recombinant phage for use in a method of selectively removing antibiotic resistance from an antibiotic resistant bacterial strain(s) in a healthy subject carrying antibiotic resistant bacterial strain(s) or in a patient carrying antibiotic resistant bacterial strain(s), wherein said recombinant phage comprising a nucleic acid encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein the method comprises administering to the subject or patient at least one vector (in particular at least 2, 3, 4, 5, 6, 7, 8, 9, 10 or more vectors), said vector encoding a DNA modifying enzyme inactivating the antibiotic resistance gene (s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein the method comprises administering to the subject or patient at least one recombinant phage (in particular at least 2, 3, 4, 5, 6, 7, 8, 9, 10 or more recombinant phages), said recombinant phage comprising a nucleic acid encoding a DNA modifying enzyme inactivating the antibiotic resistance gene (s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns at least one vector for use in a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein said vector encodes a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns at least one recombinant phage for use in a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein said recombinant phage comprises a nucleic acid encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns the use of at least one vector (in particular at least 2, 3, 4, 5, 6, 7, 8, 9, 10 or more) for the manufacture of a medicament intended for the prevention of antibiotic resistance related infection, in particular infection due to antibiotic-resistant bacteria, in a healthy subject carrying antibiotic resistant bacterial strain(s) or in a patient carrying antibiotic resistant bacterial strain(s), wherein said vector encodes a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns the use of at least one recombinant phage (in particular at least 2, 3, 4, 5, 6, 7, 8, 9, 10 or more recombinant phages) for the manufacture of a medicament intended for the prevention of antibiotic resistance related infection, in particular infection due to antibiotic-resistant bacteria, in a healthy subject carrying antibiotic resistant bacterial strain(s) or in a patient carrying antibiotic resistant bacterial strain(s), wherein said recombinant phage comprises a nucleic acid encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In the context of the invention, all the embodiments disclosed below concerning vectors and their use also apply to recombinant phages and their use.

In one embodiment, said vector further comprises an origin of replication. In another embodiment, said vector further comprises a conditional origin of replication which is inactive in the targeted bacteria but is active in a donor bacterial cell. In a particular embodiment, said conditional origin of replication is an origin of replication, the replication of which depends upon the presence of a given protein, peptid, nucleic acid, RNA, molecule or any combination thereof. In a particular embodiment, said conditional origin of replication is active in said donor bacterial cell because said donor bacterial cell expresses said given protein, peptid, nucleic acid, RNA, molecule or any combination thereof. In a particular embodiment, said conditional origin of replication is an origin of replication derived from phage-inducible chromosomal islands (PICIs). In a particular embodiment, said conditional origin of replication is active in said donor bacterial cell because said donor bacterial cell expresses a rep protein, in particular a primase-helicase. In a particular embodiment, said conditional origin of replication is derived from the origin of replication from the PICI of the Escherichia coli strain CFT073. In a particular embodiment, said conditional origin of replication comprises or consists of the sequence SEQ ID NO: 1 or SEQ ID NO:2.

In one embodiment, said vector is devoid of any antibiotic resistance marker.

In one embodiment, the method further comprises obtaining a microbiota sample from the subject or patient and confirming the presence of said antibiotic resistant bacterial strain(s) in the sample, either before or after administration.

In one embodiment, the method comprises selectively removing antibiotic resistance from an antibiotic resistant bacterial strain(s) in a healthy subject carrying antibiotic resistant bacterial strain(s) or in a patient carrying antibiotic resistant bacterial strain(s), wherein the method comprises obtaining a microbiota sample from the subject or patient, confirming the presence of said antibiotic resistant bacterial strain(s) in the sample, administering to the subject or patient a payload/vector (comprised or not inside a bacterial delivery vehicle), said payload/vector encoding a DNA modifying enzyme, such as a nuclease or base editor, inactivating the antibiotic resistance gene(s) responsible for antibiotic-resistance of bacterial strain(s), thereby selectively removing antibiotic resistance from an antibiotic resistant bacterial strain(s) in the microbiota of said subject or patient and preventing antibiotic resistance related infection in the subject or patient.

In one embodiment, the invention concerns a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein the method comprises obtaining a microbiota sample from the subject or patient, confirming the presence of said antibiotic resistant bacterial strain(s) in the sample, administering to the subject or patient at least one payload/vector (comprised or not inside a bacterial delivery vehicle) (in particular at least 2, 3, 4, 5, 6, 7, 8, 9, 10 or more), said payload/vector encoding a DNA modifying enzyme, such as a nuclease or base editor, inactivating the antibiotic resistance gene(s) responsible for antibiotic-resistance of bacterial strain(s), thereby selectively removing antibiotic resistance from an antibiotic resistant bacterial strain(s) in the microbiota of the subject or patient and preventing antibiotic resistance related infection in the subject or patient.

In one embodiment, the invention concerns at least one payload/vector for use in a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), wherein said payload/vector encodes a DNA modifying enzyme, such as a nuclease or base editor, inactivating the antibiotic resistance gene(s) responsible for antibiotic-resistance of bacterial strain(s), thereby selectively removing antibiotic resistance from an antibiotic resistant bacterial strain(s) in the microbiota of the subject or patient and preventing antibiotic resistance related infection in the subject or patient, and wherein the method comprises obtaining a microbiota sample from the subject or patient, confirming the presence of said antibiotic resistant bacterial strain(s) in the sample, preferably before administration of said payload/vector.

In one embodiment, antibiotic resistance is removed through targeted elimination of the antibiotic resistant strains when the nuclease is programmed to perform a DNA cleavage, e.g. a double strand DNA break in an antibiotic resistance gene located on the chromosome of the target bacteria or on a plasmid with addictive systems (toxin/antitoxin). In another embodiment, antibiotic resistance is removed by resensitization of the target bacteria to the antibiotic when the nuclease is programmed to perform a DNA cleavage, e.g. a double strand DNA break in the antibiotic resistance gene located on a plasmid without addictive systems. In another embodiment, antibiotic resistance is removed by resensitization of the target bacteria to the antibiotic when the nuclease is programmed to inactivate the antibiotic resistance gene through base editing.

In one embodiment, the payload/vector is a conjugative plasmid not comprised inside a bacterial delivery vehicle but inside a donor cell, and is transferred into the antibiotic resistant bacterial strain(s) by conjugation from the donor cell carrying the conjugative machinery. Alternatively, the payload/vector can be transferred into the antibiotic resistant bacterial strain(s) by bacterial transformation as a naked DNA.

In one embodiment, said payload/vector further comprises a conditional origin of replication which is inactive in the targeted bacteria but is active in a donor bacterial cell. In a particular embodiment, said conditional origin of replication is an origin of replication, the replication of which depends upon the presence of a given protein, peptid, nucleic acid, RNA, molecule or any combination thereof. In a particular embodiment, said conditional origin of replication is active in said donor bacterial cell because said donor bacterial cell expresses said given protein, peptid, nucleic acid, RNA, molecule or any combination thereof. In a particular embodiment, said conditional origin of replication is an origin of replication derived from phage-inducible chromosomal islands (PICIs). In a particular embodiment, said conditional origin of replication is active in said donor bacterial cell because said donor bacterial cell expresses a rep protein, in particular a primase-helicase. In a particular embodiment, said conditional origin of replication is derived from the origin of replication from the PICI of the *Escherichia coli* strain CFT073. In a particular embodiment, said conditional origin of replication comprises or consists of the sequence SEQ ID NO: 1 or SEQ ID NO: 2.

In one embodiment, said payload/vector comprises an auxotrophic marker.

In one embodiment, antibiotic resistance is removed from the intestine of the healthy subject or of the patient. By "removal of antibiotic resistance" is meant herein that antibiotic resistance gene(s) is(are) removed or inactivated from bacterial strains carrying such gene(s) and/or antibiotic resistant bacterial cells are eliminated. Therefore, in one embodiment, antibiotic resistance gene(s) is(are) removed or inactivated from bacterial strains present in the intestine of the healthy subject or of the patient, and/or antibiotic resistant bacterial strains are eliminated from the intestine of the healthy subject or of the patient.

In one embodiment, antibiotic resistance is removed before the subject or the patient undergoes an invasive medical procedure, such as a surgery. In one embodiment, the surgery is a solid organ transplant surgery. Preferably, the organ transplant is a kidney or liver transplant. In one embodiment, the surgery is a bone marrow transplant or a hematopoietic stem cell transplantation. In one embodiment, antibiotic resistance is removed before the subject or the patient undergoes a non-invasive medical procedure conditioning procedure, such as immunosuppression, irradiation, or chemotherapy or other non-invasive medical procedure.

In one embodiment, the invention concerns a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), said subject or patient being intended to undergo an invasive medical procedure, such as a surgery, or non-invasive medical procedure, such as immunosuppression, irradiation, or chemotherapy, wherein said method comprises administering to the subject or patient, before said subject or patient undergoes an invasive or non-invasive medical procedure, at least one vector (in particular at least 2, 3, 4, 5, 6, 7, 8, 9, 10 or more vectors), said vector encoding a DNA modifying enzyme inactivating the antibiotic resistance gene(s) responsible for antibiotic-resistance of bacterial strain(s), thereby selectively removing antibiotic resistance from an antibiotic resistant bacterial strain(s) in the microbiota and preventing antibiotic resistance related infection in the subject or patient before said subject or patient undergoes said invasive or non-invasive medical procedure.

In one embodiment, the invention concerns at least one vector for use in a method of preventing antibiotic resistance related infection, in particular infection due to antibiotic-resistant bacteria, in a healthy subject carrying antibiotic resistant bacterial strain(s) or in a patient carrying antibiotic resistant bacterial strain(s), said subject or patient being intended to undergo an invasive medical procedure, such as a surgery, or non-invasive medical procedure, such as immunosuppression, irradiation, or chemotherapy, wherein said vector encodes a DNA modifying enzyme inactivating the antibiotic resistance gene(s) responsible for antibiotic-resistance of bacterial strain(s), and wherein said method comprises administering said vector to the subject or patient, before said subject or patient undergoes an invasive or non-invasive medical procedure, thereby selectively removing antibiotic resistance from an antibiotic resistant bacterial strain(s) in the microbiota and preventing antibiotic resistance related infection in the subject or patient before said subject or patient undergoes said invasive or non-invasive medical procedure.

The present invention also concerns the use of at least one vector (in particular at least 2, 3, 4, 5, 6, 7, 8, 9, 10 or more vectors) in the manufacture of a medicament intended for the prevention of antibiotic resistance related infection, in particular infection due to antibiotic-resistant bacteria, in a healthy subject carrying antibiotic resistant bacterial strain(s) or in a patient carrying antibiotic resistant bacterial strain(s), said subject or patient being intended to undergo an invasive medical procedure, such as a surgery, or non-invasive medical procedure, such as immunosuppression, irradiation, or chemotherapy, wherein said vector encodes a DNA modifying enzyme inactivating the antibiotic resistance gene(s) responsible for antibiotic-resistance of bacterial strain(s), and wherein said subject or patient is administered with said vector before undergoing an invasive or non-invasive medical procedure, thereby selectively removing antibiotic resistance from an antibiotic resistant bacterial strain(s) in the microbiota and preventing antibiotic resistance related infection in the subject or patient before said subject or patient undergoes said invasive or non-invasive medical procedure.

In one embodiment, the microbiota is the gut microbiota.

In one embodiment, the antibiotic is from the carbapenem class or beta-lactam class of antibiotics. In one embodiment, the antibiotic is methicillin or vancomycin.

In one embodiment, the nuclease is a CRISPR associated nuclease.

In one embodiment, the payload/vector is a conjugative plasmid not comprised inside a bacterial delivery vehicle but inside a donor cell, and is transferred into the antibiotic resistant bacterial strain(s) by conjugation from a donor cell carrying the conjugative machinery. Alternatively, the payload/vector can be transferred into the antibiotic resistant bacterial strain(s) by bacterial transformation as a naked DNA.

In a particular embodiment, at least two distinct vectors are used, in particular at least 3, 4, 5, 6, 7, 8, 9, 10 or more vectors, preferably four vectors, each vector targeting different bacteria strains or species, in particular different *E*. *coli* bacteria strains, and/or encoding a DNA modifying enzyme targeting a different nucleic acid sequence inside the targeted bacteria. In a particular embodiment, at least two recombinant phages are used, in particular at least 3, 4, 5, 6, 7, 8, 9, 10 or more recombinant phages, preferably four recombinant phages, each recombinant phage targeting a different bacteria strains or species, in particular different *E. coli* bacteria strains, and/or comprising nucleic acids encoding a DNA modifying enzyme targeting a different nucleic acid sequence inside the targeted bacteria.

### Brief Description of Drawings

**Fig. 1****.** Principle of selective intestinal decolonization of AMR bacteria using packaged phagemids for prophylactic purposes. After a patient is diagnosed with intestinal carriage with AMR bacteria (by culture dependent and/or independent methods from a rectal swab and/or a fecal sample) they receive an oral treatment of packaged phagemids that will selectively eradicate AMR bacteria from their intestine without altering the rest of their microbiota. Therefore, if the patient intestinal barrier is later compromised by an invasive or non invasive-associated triggering event (invasive procedure, immunosuppression...) the patient has a lower risk of developing a difficult-to-treat infection, such as multidrug resistant (MDR) infections, with an AMR bacteria they were carrying in their intestine.
**Fig. 2A-B**. Comparison of sequence-specific and non-specific approaches for the intestinal decolonization of AMR bacteria. (A.) Non-specific approaches do not discriminate between AMR and non-AMR strains from a given species: they lead to a reduction of the whole bacterial population during the treatment. When the treatment stops, in absence of negative selective pressure, both AMR and non-AMR strains repopulate their niche. (B.) On the contrary, sequence-specific approach using packaged phagemids only eliminates AMR strains and leaves the non-AMR strain population intact. In the absence of negative selective pressure against non-AMR strains, they populate the niche vacated by AMR strains, leading to a durable exclusion of AMR strains even after the treatment stops.

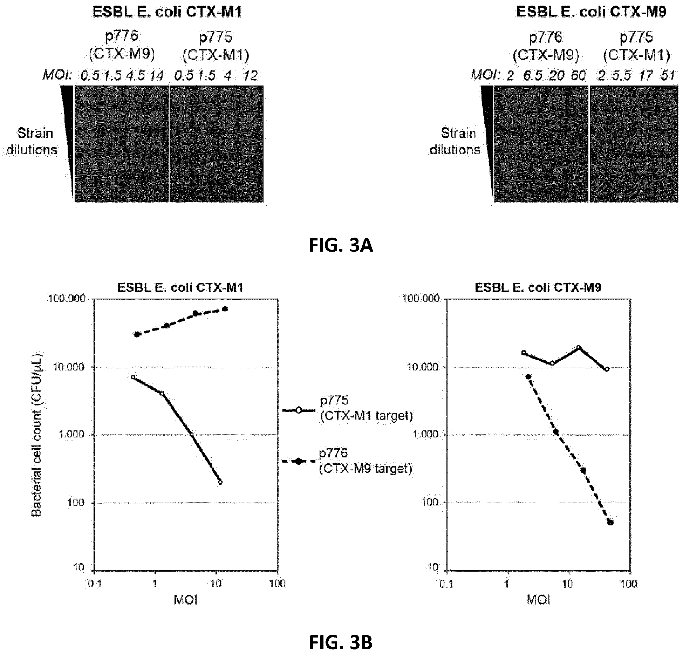

**Fig. 3A-B**. Sequence specific elimination of clinical strains of ESBL E. coli using packaged phagemids. Representation of (A.) a spot assay and (B.) associated cell counts for two clinical E. coli strains carrying different ESBL gene variants (CTX-M1 strain on the left, CTX-M9 strain on the right) transduced with packaged phagemids targeting either CTX-M1 or CTX-M9.The two packaged phagemids consisted in the same delivery vehicle (lambda-derived scaffold, 1A2 gpJ tip protein, stf27 tail fiber protein) containing either the p775 payload (FnCpf1 + crRNA against CTX-M1 ESBL gene variant) or the p776 payload (FnCpf1 + crRNA against CTX-M9 ESBL gene variant).

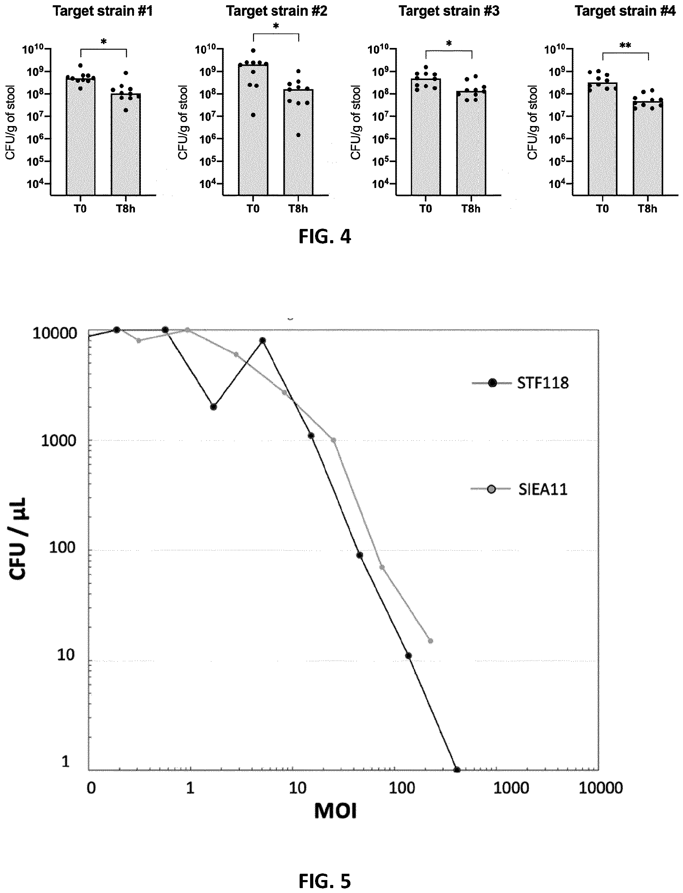

**Fig. 4****.** Variation in ESBL *E. coli* bacterial levels in the feces of mice between T0 (before the treatment) and T8h (8 hours after the treatment), expressed as CFU/g of stool. Between 0.5 log₁₀ and 1.0 log₁₀ decrease was observed in all groups.
**Fig. 5****.** Sequence-specific killing of *Klebsiella pneumoniae.* Black line, *K. pneumoniae* treated with different MOls of Lambda-derived particles harboring the chimeric STF118. Grey line, *K. pneumoniae* treated with different MOls of Lambda-derived particles harboring the chimeric STF SIEA11.

### Detailed Description of the Invention

The invention encompasses methods, kits, and compositions for selectively removing antibiotic resistance from a healthy subject carrying antibiotic resistant bacterial strain(s) or from a patient carrying antibiotic resistant bacterial strain(s). Phagemids, preferably encoding nucleases or other enzymes that genetically modify the DNA encoding an antibiotic resistance gene, can be used to eliminate nucleic acids encoding antibiotic resistance genes or inactivate antibiotic resistance genes. In this way, healthy subjects or patients can be prepared before their invasive or non-invasive medical procedures to reduce the risk of antibiotic resistant infection later on. Typically, after the invasive or non-invasive medical procedure, the bacteria can be eliminated with the antibiotic.

In the context of the invention, the healthy subject or the patient to be treated with the vector of the invention does not suffer from a bacterial infection. In other words, the methods of the invention do not aim at treating a bacterial infection nor at preventing the aggravation of a bacterial infection (for example preventing the onset of sepsis or septicemia). The methods of the present invention typically aim at decolonizing a healthy subject or a patient from antibiotic resistant bacterial strain(s), in particular to avoid the onset of a bacterial infection due to this(these) antibiotic resistant bacterial strain(s) typically when said subject or patient faces a potentially triggering event such as a invasive or non-invasive medical procedures. In the context of the invention, said decolonization can typically be obtained by specifically killing said antibiotic resistant bacterial strain(s), by removing the antibiotic resistance gene(s) from said antibiotic resistant bacterial strain(s) and/or by inactivating the antibiotic resistance gene(s) from said antibiotic resistant bacterial strain(s).

In one embodiment, the invention encompasses a method to selectively eradicate bacteria that carry antibiotic resistance gene(s) from the intestinal microbiota using vectors, in particular packaged phagemids or engineered/recombinant phages, administered to a healthy subject or patient, for example orally, rectally (e.g., in an enema), vaginally, nasally or to the skin. This prophylactic elimination of intestinal carriage of antibiotic resistant bacteria can reduce the risk of antibiotic resistant infections with that same bacteria after various triggering events, and their associated costs **(****Fig.1****).**

In one embodiment, the invention encompasses a method of selectively removing antibiotic resistance from an antibiotic resistant bacterial strain(s) in a healthy subject or patient carrying antibiotic resistant bacterial strain(s), wherein the method comprises administering to the subject or patient at least one vector (in particular at least 2, 3, 4, 5, 6, 7, 8, 9, 10 or more vectors), in particular a packaged phagemid or an engineered/recombinant phage, said vector encoding a DNA modifying enzyme inactivating the antibiotic resistance gene (s) responsible for antibiotic-resistance of bacterial strain(s), thereby selectively removing antibiotic resistance from an antibiotic resistant bacterial strain(s) in the microbiota and preventing antibiotic resistance related infection in the subject.

In one embodiment, the method further comprises obtaining a microbiota sample from the subject or patient and confirming the presence of said antibiotic resistant bacterial strain(s) in the sample, either before or after administration.

In one embodiment, after a healthy subject or patient is diagnosed with intestinal carriage with AMR bacteria (for example by culture dependent and/or independent methods from a rectal swab and/or a fecal sample) they can receive an oral treatment of the vectors of the invention, in particular the packaged phagemids or engineered/recombinant phages, that can selectively remove antibiotic resistance from AMR bacteria from their intestine, and without altering the rest of their microbiota in situation where AMR bacteria are eradicated. Therefore, if the subject or patient intestinal barrier is later compromised by an invasive or non-invasive medical procedure -associated triggering event, the subject or patient has a lower risk of developing a difficult-to-treat infection, such as multidrug resistant (MDR) infections, with an AMR bacteria they were carrying in their intestine.

In one embodiment, healthy subject or patient can optionally be tested for intestinal carriage with AMR bacteria (for example by culture dependent and/or independent methods from a rectal swab and/or a fecal sample) and can receive an administration, for example, an oral, rectal (e.g., in an enema), vaginal, nasal, or skin treatment, of vectors of the invention, in particular of packaged phagemids or engineered/recombinant phages, that can selectively remove antibiotic resistance from AMR bacteria from their intestine, in situation where AMR bacteria are eradicated. Therefore, if the subject or patient intestinal barrier is later compromised by an invasive or non-invasive medical procedure -associated triggering event, the subject or patient has a lower risk of developing a difficult-to-treat infection, such as multidrug resistant (MDR) infections, with an AMR bacteria they were carrying in their intestine.

Packaged phagemids can allow the transduction in the target bacteria of a synthetic DNA payload.

Preferably, the DNA payload encodes a sequence-specific RNA-guided nuclease complex (e.g., type I, II, III or V CRISPR-Cas system) programmed to generate DNA cleavage, e.g. double strand DNA breaks, in antibiotic resistance gene sequences located either on the bacterial chromosome or on a plasmid. When the antibiotic resistance gene is located in the chromosome or on a plasmid with addictive systems (toxin / antitoxin), cleavage of the DNA leads to bacterial death. When the antibiotic resistance gene is located on a plasmid without addiction systems, cleavage can occur either in the antibiotic resistance gene or anywhere else in the plasmid, leading in both situations to resensitization of the target bacteria that can then be eliminated in the presence of the corresponding antibiotic in case of infection.

Alternatively, a DNA modifying enzyme can be programmed to inactivate the antibiotic resistance gene through base editing, leading to the resensitization of the target bacteria to the antibiotic.

All these approaches lead to the same outcome: the elimination of AMR genes from bacterial populations with an unparalleled specificity. They allow the durable decolonization of antibiotic resistant bacteria from complex ecological ecosystems, such as the intestine. In the context where both antibiotic sensitive and resistant strains compete for the same niche/resources, the specific elimination of AMR strains typically leads to the outgrowth of the non-AMR strains that take over the niche and prevent AMR strains from growing back. Alternatively, in the case where all strains from a given species are resistant to the antibiotic, the removal of antibiotic resistance through plasmid curing or gene editing typically results in the majority of bacteria being resensitized to antibiotics. This majority of antibiotic sensitive bacteria typically competes with the remaining antibiotic resistant bacteria, takes over the niche and prevents antibiotic resistant bacteria to grow back to their initial level. In the context where there is a single strain of the target species and that strain is resistant, the specific elimination of the resistance of the target strain can typically lead to the outgrowth of the non-resistant bacteria that can take over the niche and prevent the resistant bacteria from growing back.

Preferably, the target, that is to say the antibiotic resistance gene(s), is on a plasmid and the plasmid is cured. Then, the cured bacteria will compete with the initial resistant bacteria and help replace them. Therefore, this approach providing the sequence-specific elimination of AMR strains allows the leverage of the competitive environment of the Gl (Gastro Intestinal) tract to favor their durable exclusion from the microbiota by antibiotic sensitive commensal strains (Fig. 2B).

To further favor the exclusion of AMR strains from their intestinal niche, AMR-colonized subjects or patients can be treated with a combination of vectors of the invention, in particular packaged phagemids, and a probiotic strain. The vector, in particular the packaged phagemid, can specifically eliminate AMR strains from the subject or patient's intestine and the probiotic strain can take over the ecological niche, further preventing AMR strains from bouncing back.

The invention encompasses methods of selectively removing antibiotic resistance bacteria from a healthy subject carrying antibiotic resistant bacterial strain(s) or from a patient carrying antibiotic resistant bacterial strain(s). In one embodiment, the method comprises administering to the subject or patient at least one payload/vector (in particular at least 2, 3, 4, 5, 6, 7, 8, 9, 10 or more), which can be inside one or more bacterial delivery vehicle(s). In one embodiment, the payload/vector encodes a nuclease that can cleave the nucleic acid encoding the antibiotic resistance gene(s) responsible for antibiotic-resistance of bacterial strain(s), thereby selectively removing antibiotic resistant bacteria from the microbiota of the subject or patient, when the antibiotic resistance gene is located on the bacterial chromosome or plasmid with addictive systems or removing antibiotic resistance from antibiotic resistant bacteria of the microbiota of the subject or patient, when the antibiotic resistance gene is located on a plasmid without addictive systems, and preventing antibiotic resistance related infection in the subject or patient.

In one embodiment, the method comprises obtaining a microbiota sample from the subject or patient confirming the presence of said antibiotic resistant bacterial strain(s) in the sample, preferably before the administration. The microbiota sample can also be obtained from the subject or patient after the administration to measure the effectiveness of the administration.

Optionally, the method comprises a step of confirming that the payload/vector works on the patient or subject sample *in vitro.* Thus a sample of microbiota taken from the patient or subject can be contacted with the payload/vector *in vitro* and the effect of the payload/vector on antibiotic resistance assessed by routine *in vitro* procedures.

In one embodiment, the antibiotic resistant bacteria are removed from the gut of the healthy subject or of the patient.

In one embodiment, the selective decolonization (or removal of antibiotic resistance bacteria) occurs before the subject or patient undergoes an invasive or non-invasive medical procedure, such as a surgical procedure or such as a treatment of cancer or such as an immunosuppressive treatment. In another embodiment, the selective decolonization occurs in patients more prone at developing AMR infection such as patients with hematological malignancy, cancer, chronic disease, autoimmune disease or under viral attack.

In one embodiment, the surgical procedure is a bone marrow transplant or a hematopoietic stem cell transplantation.

In one embodiment, the surgery is a solid organ transplant surgery, in particular a kidney transplant surgery and/or a liver transplant surgery.

In one embodiment, the treatment of cancer is selected from the group consisting of cytotoxic agents, chemotherapeutic agents, growth inhibitory agents, agents used in radiation therapy, anti-angiogenesis agents, apoptotic agents, anti-tubulin agents, and other agents to treat cancer, for example, anti-CD20 antibodies, platelet derived growth factor inhibitors (e.g., GLEEVEC^{™} (imatinib mesylate)), a COX-2 inhibitor (e.g., celecoxib), interferons, cytokines, antagonists (e.g., neutralizing antibodies) that bind to one or more of the following targets PDGFR-β, BlyS, APRIL, BCMA receptor(s), TRAIL/Apo2, other bioactive and organic chemical agents, and combinations thereof.

The term "cytotoxic agent" as used herein refers to a substance that inhibits or prevents the function of cells and/or causes destruction of cells. The term is intended to include radioactive isotopes (e.g., At211, 1131, 1125, Y90, Re186, Re188, Sm153, Bi212, P32, and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below.

A "chemotherapeutic agent" is a chemical compound useful in the treatment of cancer. Examples of chemotherapeutic agents include alkylating agents such as thiotepa and CYTOXAN^{®} cyclophosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins; delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γ1l and calicheamicin ω1l; dynemicin, including dynemicin A; an esperamicin; as well as neocarzinostatin chromophore and related chromoprotein enediyne antiobiotic chromophores, aclacinomysins, actinomycin, authramycin, azaserine, bleomycins, cactinomycin, carabicin, carminomycin, carzinophilin, chromomycin, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoids, for example taxanes including TAXOL^{®} paclitaxel (Bristol-Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE^{®} docetaxel (Rhône-Poulenc Rorer, Antony, France); chloranbucil; gemcitabine (GEMZAR^{®}); 6-thioguanine; mercaptopurine; methotrexate; platinum or platinum-based chemotherapy agents and platinum analogs, such as cisplatin, carboplatin, oxaliplatin (ELOXATIN^{™}), satraplatin, picoplatin, nedaplatin, triplatin, and lipoplatin; vinblastine (VELBAN^{®}); platinum; etoposide (VP-16); ifosfamide; mitoxantrone; vincristine (ONCOVIN^{®}); oxaliplatin; leucovovin; vinorelbine (NAVELBINE^{®}); novantrone; edatrexate; daunomycin; aminopterin; ibandronate; topoisomerase inhibitor RFS 2000; difluorometlhylornithine (DMFO); retinoids such as retinoic acid; capecitabine (XELODA^{®}); pharmaceutically acceptable salts or acids of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovorin. Additional chemotherapeutic agents include the cytotoxic agents useful as antibody drug conjugates, such as maytansinoids (DM1, for example) and the auristatins MMAE and MMAF, for example.

Chemotherapeutic agents also include "anti-hormonal agents" or "endocrine therapeutics" that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX^{®} tamoxifen), EVISTA^{®} raloxifene, droloxifene, 4-hydroxytamoxifen, trioxifene, keoxifene, LY117018, onapristone, and FARESTON^{®} toremifene; anti-progesterones; estrogen receptor down-regulators (ERDs); agents that function to suppress or shut down the ovaries, for example, leutinizing hormone-releasing hormone (LHRH) agonists such as LUPRON^{®} and ELIGARD^{®} leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE^{®} megestrol acetate, AROMASIN^{®} exemestane, formestanie, fadrozole, RIVISOR^{®} vorozole, FEMARA^{®} letrozole, and ARIMIDEX^{®} anastrozole. In addition, such definition of chemotherapeutic agents includes bisphosphonates such as clodronate (for example, BONEFOS^{®} or OSTAC^{®}), DIDROCAL^{®} etidronate, NE-58095, ZOMETA^{®} zoledronic acid/zoledronate, FOSAMAX^{®} alendronate, AREDIA^{®} pamidronate, SKELID^{®} tiludronate, or ACTONEL^{®} risedronate; as well as troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGFR); vaccines such as THERATOPE^{®} vaccine and gene therapy vaccines, for example, ALLOVECTIN^{®} vaccine, LEUVECTIN^{®} vaccine, and VAXID^{®} vaccine; LURTOTECAN^{®} topoisomerase 1 inhibitor; ABARELIX^{®} rmRH; lapatinib ditosylate (an ErbB-2 and EGFR dual tyrosine kinase small-molecule inhibitor also known as GW572016); and pharmaceutically acceptable salts or acids of any of the above.

Chemotherapeutic agents also include antibodies such as alemtuzumab (Campath), bevacizumab (AVASTIN^{®}, Genentech); cetuximab (ERBITUX^{®}, Imclone); panitumumab (VECTIBIX^{®}, Amgen), rituximab (RITUXAN^{®}, Genentech/Biogen Idec), pertuzumab (OMNITARG^{®}, 2C4, Genentech), trastuzumab (HERCEPTIN^{®}, Genentech), tositumomab (Bexxar, Corixia), and the antibody drug conjugate, gemtuzumab ozogamicin (MYLOTARG^{®}, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti- interleukin-12 (ABT-874/J695, Wyeth Research and Abbott Laboratories), which is a recombinant exclusively human-sequence, full-length IgG1 λ antibody genetically modified to recognize interleukin-12 p40 protein.

Chemotherapeutic agents also include "EGFR inhibitors", which refers to compounds that bind to or otherwise interact directly with EGFR and prevent or reduce its signaling activity, and is alternatively referred to as an "EGFR antagonist". Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No.4,943, 533, Mendelsohn et al.) and variants thereof, such as chimerized 225 (C225 or Cetuximab; ERBUTIX) and reshaped human 225 (H225); IMC-11 F8, a fully human, EGFR-targeted antibody (Imclone); antibodies that bind type II mutant EGFR; humanized and chimeric antibodies that bind EGFR; and human antibodies that bind EGFR, such as ABX-EGF or Panitumumab; EMD 55900; EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6.3, and E7.6.3; MDX-447 (Medarex Inc); and mAb 806 or humanized mAb 806. The anti-EGFR antibody may be conjugated with a cytotoxic agent, thus generating an immunoconjugate. EGFR antagonists include small molecules such as OSI-774 (CP-358774, erlotinib, TARCEVA Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA^{®}) 4-(3'-Chloro-4'-fluoroanilino)-7-methoxy-6-(3-morpholinopropoxy)quinazoline, AstraZeneca); ZM 105180 ((6-amino-4-(3-methylphenylamino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); and dual EGFR/HER2 tyrosine kinase inhibitors such as lapatinib (TYKERB^{®}, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitors such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (Cl-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC^{®}, available from Glaxo SmithKline); multi-targeted tyrosine kinase inhibitors such as sunitinib (SUTENT^{®}, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g., those that bind to HER-encoding nucleic acid); quinoxalines; tryphostins; ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC^{®}); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE^{®}).

Chemotherapeutic agents also include immune checkpoint inhibitors such as human programmed death receptor-1 (PD-1) blocking antibodies and human cytotoxic T-lymphocyte antigen 4 (CTLA-4)-blocking antibodies. Checkpoint inhibitors include, but are not limited to nivolumab, ipilimumab, pembrolizumab, tremelimumab, and pidilizumab.

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFα) blockers such as etanercept (ENBREL^{®}), infliximab (REMICADE^{®}), adalimumab (HUMIRA^{®}), certolizumab pegol (CIMZIA^{®}), golimumab (SIMPONI^{®}), Interleukin 1 (IL-1) blockers such as anakinra (KINERET^{®}), T-cell co-stimulation blockers such as abatacept (ORENCIA^{®}), Interleukin 6 (IL-6) blockers such as tocilizumab (ACTEMERA^{®}); Interleukin 13 (IL-13) blockers such as lebrikizumab; Interferon alpha (IFN) blockers such as rontalizumab; beta 7 integrin blockers such as rhuMAb Beta7; IgE pathway blockers such as Anti-M1 prime; Secreted homotrimeric LTa3 and membrane bound heterotrimer LTal/β2 blockers such as Anti-lymphotoxin alpha (LTa); miscellaneous investigational agents such as thioplatin, PS-341, phenylbutyrate, ET-18-OCH3, and farnesyl transferase inhibitors (L-739749, L-744832); polyphenols such as quercetin, resveratrol, piceatannol, epigallocatechine gallate, theaflavins, flavanols, procyanidins, betulinic acid; autophagy inhibitors such as chloroquine; delta-9-tetrahydrocannabinol (dronabinol, MARINOL^{®}); beta-lapachone; lapachol; colchicines; betulinic acid; acetylcamptothecin, scopolectin, and 9-aminocamptothecin); podophyllotoxin; tegafur (UFTORAL^{®}); bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTONEL^{®}); and epidermal growth factor receptor (EGF-R); vaccines such as THERATOPE^{®} vaccine; perifosine, COX-2 inhibitor (e.g., celecoxib or etoricoxib), proteosome inhibitor (e.g., PS341); CCI-779; tipifarnib (R11577); orafenib, ABT510; Bcl-2 inhibitor such as oblimersen sodium (GENASENSE^{®}); pixantrone; farnesyltransferase inhibitors such as lonafarnib (SCH 6636, SARASAR^{™}); and pharmaceutically acceptable salts or acids of any of the above; as well as combinations of two or more of the above.

A "growth inhibitory agent" when used herein refers to a compound or composition which inhibits growth and/or proliferation of a cell either in vitro or in vivo. Thus, the growth inhibitory agent may be one that significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as the anthracycline antibiotic doxorubicin ((8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione), epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

In one embodiment, the hematological malignancy is selected from leukemia, lymphoma, and myeloma.

Leukemias include acute lymphoblastic leukemia (ALL), acute myelogenous leukemia (AML), chronic lymphocytic leukemia (CLL), small lymphocytic lymphoma (SLL), chronic myelogenous leukemia (CML), and acute monocytic leukemia (AMOL).

Lymphomas include Hodgkin's lymphomas and Non-Hodgkin's lymphomas. NHL can be formed from either B-cells or T-cells. B-cell non-Hodgkin lymphomas include Burkitt lymphoma, chronic lymphocytic leukemia/small lymphocytic lymphoma (CLL/SLL), diffuse large B-cell lymphoma, follicular lymphoma, immunoblastic large cell lymphoma, precursor B-lymphoblastic lymphoma, and mantle cell lymphoma. T-cell non-Hodgkin lymphomas include mycosis fungoides, anaplastic large cell lymphoma, and precursor T-lymphoblastic lymphoma.

By "chronic disease" is meant herein conditions that last 1 year or more and require ongoing medical attention or limit activities of daily living or both.

In one embodiment, the chronic disease is selected from arthritis, asthma, cancer, chronic obstructive pulmonary disease, diabetes, Lyme disease, autoimmune diseases, genetic disorders and some viral diseases such as hepatitis C and acquired immunodeficiency syndrome, Alzheimer's Disease and other Dementias, eating disorders, Osteoporosis, Reflex Sympathetic Dystrophy (RSD) Syndrome and tobacco use-related conditions.

In one embodiment, the autoimmune disease is selected from alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune parotitis, autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigoid, cold agglutinin disease, crest syndrome, Crohn's disease, Degos' disease, dermatomyositis, diabetes mellitus, discoid lupus, essential mixed cryoglobulinemia, dystrophic epidermolysis bullosa, epididymitis, fibromyalgia-fibromyositis, glomerulonephritis, Graves' disease, Guillain-Barre syndrome, Hashimoto's thyroiditis, hemolytic anemia, idiopathic pulmonary fibrosis, idiopathic thrombocytopenia purpura (ITP), IgA nephropathy, juvenile chronic arthritis (Still's disease), juvenile rheumatoid arthritis, Meniere's disease, mixed connective tissue disease, multiple sclerosis, myasthenia gravis, myxedema, pemphigus vulgaris, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, spondyloarthropathies, stiff-man syndrome, systemic lupus erythematosus, Takayasu arteritis, temporal arteritis/giant cell arteritis, thyroiditis, ulcerative colitis, uveitis, vasculitis, vitiligo and Wegener's granulomatosis.

In one embodiment, the viral attack is an attack from a virus selected from the group consisting of Poxviridae including Chordopoxvirinae in particular Orthopox viruses (e.g. cowpox virus, monkey pox virus, vaccinia virus, variola virus, smallpox virus), Parapox viruses (e.g. bovine papular stomatitis virus, Orf virus, pseudocowpox virus), Molluscipox viruses (e.g. Molluscum contagiosum virus) and Yatapox viruses (e.g. tanapox virus, yaba monkey tumor virus); Herpesviridae including Alphaherpesvirinae in particular simplexviruses (e.g. human herpesvirus 1 (Herpes simplex virus 1), human herpesvirus 2 (Herpes simplex virus 2)) and varicelloviruses (e.g. human herpesvirus 3 (Varicella-zoster virus)), Betaherpesvirinae in particular cytomegaloviruses (e.g. human herpesvirus 5 (human cytomegalovirus)) and roseoloviruses (e.g. human herpesvirus 6, human herpesvirus 7), and Gammaherpesvirinae, in particular lymphocryptoviruses (e.g. human herpesvirus 4 (Epstein-Barr virus)) and rhadinoviruses (e.g. human herpesvirus 8 (Kaposi's sarcoma-associated herpesvirus)); Adenoviridae including mastadenoviruses (e.g. human adenovirus A, human adenovirus B, human adenovirus C, human adenovirus D, human adenovirus E and human adenovirus F); Polyomaviridae including polyomaviruses (e.g. BK polyomavirus, human polyomavirus, JC polyomavirus, Kl polyomavirus, WU polyomavirus, Merkel cell polyomavirus); Papillomaviridae including alphapapillomaviruses (e.g. human papillomavirus 6, human papillomavirus 11, human papillomavirus 16, human papillomavirus 18, human papillomavirus 2, human papillomavirus 10, human papillomavirus 61, human papillomavirus 71, human papillomavirus cand90, human papillomavirus 26, human papillomavirus 30, human papillomavirus 32, human papillomavirus 54, human papillomavirus 7, human papillomavirus 34), betapapillomaviruses (e.g. human papillomavirus 5, human papillomavirus cand92, human papillomavirus cand96, human papillomavirus 9, human papillomavirus 49), gammapapillomaviruses (e.g. human papillomavirus 4, human papillomavirus 50, human papillomavirus 48, human papillomavirus 60, human papillomavirus 88) and mupapillomaviruses (e.g. human papillomavirus 1); Parvoviridae including Parvovirinae in particular dependoviruses (e.g. adeno-associated virus), erythroviruses (e.g. B19 virus) and bocaviruses (e.g. human bocavirus); Hepadnaviridae including orthohepadnaviruses (e.g. hepatitis B virus); Retroviridae including Orthoretrovirinae in particular deltaretroviruses (e.g. human T-lymphotropic virus 1 (HTLV-1), human T-lymphotropic virus 2 (HTLV-2)), and lentiviruses (e.g. human immunodeficiency virus 1, human immunodeficiency virus 2); Reoviridae including coltiviruses (e.g. Colorado tick fever virus), orbiviruses (e.g. African horse sickness virus, Changuinola virus, Corriparta virus, Orungo virus), orthoreoviruses (e.g. mammalian orthoreovirus), rotaviruses (e.g. Rotavirus A, Rotavirus B, Rotavirus C); Mononegavirales including Filoviridae in particular ebolaviruses (e.g. Ivory Coast ebolavirus, Zaire ebolavirus, Sudan ebolavirus, Reston ebolavirus, Bundibugyo ebolavirus) and marburgviruses (e.g. Lake Victoria marburgvirus), Paramyxoviridae in particular Paramyxovirinae comprising henipaviruses (e.g. Hendra virus, Nipah virus), morbilliviruses (e.g. measles virus (Edmonston virus)), respiroviruses (e.g. human parainfluenza virus 1, human parainfluenza virus 3) and rubulaviruses (e.g. mumps virus, human parainfluenza virus 2, human parainfluenza virus 4), Pneumovirinae comprising pneumoviruses (e.g. human respiratory syncytial virus) and metapneumoviruses (e.g. human metapneumovirus), and Rhabdoviridae in particular vesiculoviruses (e.g. vesicular stomatitis Alagoas virus, vesicular stomatitis Indiana virus, vesicular stomatitis New Jersey virus, Chandipura virus, Cocal virus, Isfahan virus, Piry virus) and lyssaviruses (e.g. australian bat lyssavirus, rabies virus); Orthomyxoviridae including influenzaviruses A (e.g. Influenza A virus), influenzaviruses B (e.g. Influenza B virus) and influenzaviruses C (e.g. Influenza C virus); Bunyaviridae including hantaviruses (e.g. Andes virus, Bayou virus, Black Creek Canal virus, Dobrava-Belgrade virus, Hantaan virus, Seoul virus, Sin Nombre virus, New York virus, Puumala virus), nairoviruses (e.g. Crimean-Congo hemorrhagic fever virus, Nairobi sheep disease virus), orthobunyaviruses (e.g. Bunyamwera virus, Bwamba virus, California encephalitis virus, Guama virus, Oriboca virus, Oropouche virus) and phleboviruses (e.g. Rift Valley fever virus, Sandfly fever Naples virus); Arenaviridae including arenaviruses (e.g. Guanarito virus, Junín virus, Lassa virus, lymphocytic choriomeningitis virus, Machupo virus, Sabiá virus); Deltavirus (e.g. Hepatitis delta virus); Nidovirales including Coronaviridae, in particular coronaviruses (e.g. human coronavirus 229E, human coronavirus OC43, human enteric coronavirus, severe acute respiratory syndrom coronavirus) and toroviruses (e.g. human torovirus); Picornaviridae including enteroviruses (e.g. human enterovirus A, human enterovirus B, human enterovirus C, human enterovirus D, poliovirus), rhinoviruses (e.g. human rhinovirus A, human rhinovirus B, human rhinovirus C), hepatoviruses (e.g. Hepatitis A virus), parechoviruses (e.g. human parechovirus), cardioviruses (e.g. Vilyuisk human encephalomyelitis virus), aphtoviruses (e.g. Foot-and-mouth disease virus) and kobuviruses (e.g. Aichi virus); Caliciviridae including noroviruses (e.g. Norwalk virus) and sapoviruses (e.g. Sapporo virus); Hepevirus including Hepatitis E virus; Astroviridae including mamastroviruses (e.g. human astrovirus); Togaviridae including alphaviruses (e.g. Chikungunya virus, O'nyong-nyong virus, Mayaro virus, Ross River virus, Barmah Forest virus, Sindbis virus, venezuelan equine encephalitis virus, western equine encephalitis virus, eastern equine encephalitis virus, Ockelbo virus) and rubiviruses (e.g. rubella virus); Flaviviridae including flaviviruses (e.g. Kyasanur Forest disease virus, Omsk hemorrhagic fever virus, Powassan virus, Louping ill virus, tick-borne encephalitis virus, Dengue virus, japanese encephalitis virus, Murray Valley encephalitis virus, St. Louis encephalitis virus, West Nile virus, IIheus virus, yellow fever virus, Apoi virus), hepaciviruses (e.g. Hepatitis C virus), GB virus B and GB virus A; and Birnaviridae (e.g. Infectious bursal disease virus).

In one embodiment, the microbiota is the gut microbiota.

In one embodiment, the antibiotic is methicillin, vancomycin, or of the carbapenem class or beta-lactam class of antibiotics.

In one embodiment, the nuclease is a CRISPR associated nuclease.

The invention encompasses compositions, kits and methods for reducing or eliminating an AMR bacteria *in situ.* The compositions, kits and methods of the invention reduce or eliminate deleterious AMR bacteria within the host microbiome by eliminating or reducing the antibiotic resistance of these AMR bacteria. Preferably, the elimination of AMR bacteria *in situ* involves the use of phages, recombinant phage, packaged phagemid, introducing a DNA cleavage, e.g. a double strand break in the DNA sequence, with or without the use of antibiotics.

The invention encompasses methods of selectively removing antibiotic resistance from antibiotic resistant bacterial strain(s) in a healthy subject carrying antibiotic resistant bacterial strain(s) or a patient carrying antibiotic resistant bacterial strain(s). In one embodiment, the method comprises administering to the subject or patient at least payload/vector (in particular at least 2, 3, 4, 5, 6, 7, 8, 9, 10 or more), which can be inside a bacterial delivery vehicle. In one embodiment, the payload/vector encodes a DNA modifying enzyme that can modify and inactivate the nucleic acid encoding the antibiotic resistance gene(s) responsible for antibiotic-resistance of bacterial strain(s), thereby selectively removing antibiotic resistance from antibiotic resistant bacteria of the microbiota of the subject or patient, and preventing antibiotic resistance related infection in the subject or patient.

In one embodiment, the method comprises obtaining a microbiota sample from the subject or patient confirming the presence of said antibiotic resistant bacterial strain(s) in the sample, preferably before the administration. The microbiota sample can also be obtained from the subject or patient after the administration to measure the effectiveness of the administration.

Optionally, the method comprises a step of confirming that the payload(s)/vector(s) work(s) on the patient or subject sample *in vitro.* Thus a sample of microbiota taken from the patient or subject can be contacted with the payload/vector *in vitro* and the effect of the payload/vector on antibiotic resistance assessed by routine *in vitro* procedures.

In one embodiment, the antibiotic resistance is removed from antibiotic resistant bacteria of the gut of the healthy subject or of the patient.

In one embodiment, the selective removal of antibiotic resistance occurs before the subject or patient undergoes an invasive or non-invasive medical procedure, such as a surgical procedure.

In one embodiment, the surgical procedure is a bone marrow transplant or a hematopoietic stem cell transplantation.

In one embodiment, the surgery is a solid organ transplant surgery, in particular a kidney transplant surgery and/or a liver transplant surgery.

In one embodiment, the microbiota is the gut microbiota.

In one embodiment, the antibiotic is methicillin, vancomycin, or of the carbapenem class or beta-lactam class of antibiotics.

In one embodiment, the DNA modifying enzyme is a base editor.

The invention encompasses the use of at least one vector (in particular at least 2, 3, 4, 5, 6, 7, 8, 9, 10 or more) that can transfer with high efficiency a nucleic acid, preferably a plasmid, into a bacterial population within the microbiome that allows the expression of an exogenous enzyme that will modify a DNA encoding an antibiotic resistance gene in an AMR bacteria.

### Methods of prophylactic treatment

The invention encompasses methods of reducing or eliminating an AMR bacteria *in situ.* The AMR bacteria can be contacted with a vector *in situ.* Preferably, the bacteria is a gut bacteria, a skin bacteria, an oral bacteria, or a bacteria on a mucosal surface.

Preferably, the AMR bacteria is reduced or eliminated by eliminating an antibiotic resistance gene. Subsequently, the bacteria will be sensitive to the antibiotic and the bacteria can be killed by contact with the antibiotic.

In one embodiment, the AMR bacteria is genetically modified to modify, reduce or eliminate expression of an antibiotic resistance gene. Subsequent contact of the bacteria with the antibiotic will lead to the death or reduction in growth of the modified bacteria, for example, as described in Bikard et al., Cell Host Microbe, Vol. 12, 177-186 (2012) and Bikard et al., Nature Biotechnology Vol. 32 (11) 1146-51 (2014).

In one embodiment, the method comprises contacting the AMR bacteria with an effective amount of at least one antibiotic, phage, recombinant phage, packaged phagemid, plasmid, or combination thereof.

In one embodiment, bacteria producing the phage, recombinant phage, packaged phagemid or plasmid can be administered. In one embodiment, the phage, recombinant phage, packaged phagemid or plasmid can be directly administered.

In one embodiment, the antibiotic (and corresponding antibiotic resistance gene) is selected from methicillin, streptomycin, vancomycin, clindamycin, metronidazole, sulphadoxine, trimethoprim, or any combination of 1, 2, 3, 4, 5, 6, or 7 of these antibiotics.

In one embodiment, the phage, recombinant phage, packaged phagemid or plasmid, encodes a nuclease selected from CRISPR-Cas and variants, TALENs and variants, zinc finger nuclease (ZFN) and ZFN variants, natural, evolved or engineered meganuclease or recombinase variants.

In a preferred embodiment, the AMR bacteria are contacted *in situ with* at least one vector (in particular at least 2, 3, 4, 5, 6, 7, 8, 9, 10 or more) that can transfer with high efficiency a nucleic acid into the bacteria to express (i) an exogenous enzyme (such as Cas9 or Cpf1 also known as Cas12a, such as Mad4 or such as Cms1) and/or (ii) crRNA and/or tracrRNA, in the bacteria, that genetically modifies a DNA encoding an antibiotic resistance gene. Alternatively, the vector can transfer with high efficiency a nucleic acid into the bacteria to express crRNA and/or tracrRNA in the bacteria and program an endogenous enzyme (such as a Type I CRISPR nuclease, e.g.a Cas3 nuclease) of the bacteria to genetically modify a DNA encoding an antibiotic resistance gene.

The antibiotic resistance gene can be targeted directly. Alternatively, or in addition, if the antibiotic resistance gene is encoded by a plasmid, plasmid sequences outside of the antibiotic resistance gene can be targeted. For example, a plasmid origin of replication can be targeted.

In a particular embodiment, the exogenous enzyme can result in a genetic modification where a Cas nuclease, such as a Cas9 nuclease, is used to make the desired modification. Thus, the invention contemplates introducing a cleavage, e.g. a double strand break, in the DNA of the bacterial DNA at a specific sequence, for example with a CRISPR/Cas system, together with non-homologous end joining (NHEJ) or homologous recombination (HR) to generate the desired genetic modification. In one embodiment, the DNA cleavage, e.g. a double strand break, is generated in the presence of an editing template comprising homologous region with DNA regions located around the specific sequence located in the bacterial DNA. In a particular embodiment, the genetic modification involves either NHEJ or HR endogenous repair mechanism of the host bacteria.

In a particular embodiment, the genetic modification involves a base editor or a prime editor, in particular a base editor as disclosed in the section *"Enzymes*/*Systems for Inducing Modifications"* below or a prime editor as disclosed in the section *"Enzymes*/*Systems for Inducing Modifications"* below.

The genetic modification can be a point mutation(s), a deletion(s), insertion(s) or any combination thereof. Preferably, the genetic modification is a point mutation, an insertion or a deletion inside a coding sequence leading to a frameshift mutation or a deletion mutation, preferably in an antibiotic resistance gene. The genetic modification preferably eliminates or reduces the expression of an antibiotic resistance gene. The genetic modification can be in the translated or untranslated regions of a gene. The genetic modification can be in the promoter region of a gene or within any other region involved in gene regulation. In one embodiment, the genetic modification integrates a phage genome or exogenous DNA into the host bacterial chromosome or endogenous plasmid(s). In one embodiment, the genetic modification results in expression of an exogenous protein from an integrated exogenous DNA in the host bacterial chromosome or endogenous plasmid(s).

In some embodiments, the genetic modification results in the change in at least 1, 2, 3, 4, 5 , 6, 7, 8, 9, 10, 11, 12, 13, 14, 15, 20, 25, 30, 35, 40, 45, 50, 100, 200, 500, etc. amino acids to a different amino acid. In some embodiments, the genetic modification introduces a stop codon. In some embodiments, the genetic modification is outside protein coding sequences, within RNA, or within regulatory sequences.

### Production of packaged phagemids and bacterial virus particles

Generation of packaged phagemids or bacterial virus particles are routine techniques well-known to one skilled in the art. A satellite phage and/or helper phage may be used to promote the packaging of the payload in delivery vehicles of the present invention. Helper phages provide functions *in trans* and are well known to the man skilled in the art. The helper phage comprises all the genes coding for the structural and functional proteins that are indispensable for the payload to be packaged, according to the invention (i.e. the helper phage provides all the necessary gene products for the assembly of the delivery vehicle). The helper phage may contain a defective origin of replication or packaging signal, or completely lack the latter, and hence it is incapable of self-packaging, thus only bacterial delivery particles carrying the payload or plasmid will be produced. Helper phages may be chosen so that they cannot induce lysis of the host used for the delivery particle production. One skilled in the art would understand that some bacteriophages are defective and need a helper phage for payload packaging. Thus, depending on the bacteriophage chosen in connection with the present invention to prepare the bacterial delivery particles, the person skilled in the art would know if a helper phage is required. Sequences coding for one or more proteins or regulatory processes necessary for the assembly or production of packaged payloads may be supplied *in trans.* For example, the stf, gpJ and gpH proteins may be provided in a plasmid under the control of an inducible promoter or expressed constitutively. In this case, the phage wild-type sequence may or not contain a deletion of the gene or sequence supplied *in trans.* Additionally, chimeric or modified phage sequences encoding a new function, like an engineered stf, gpJ or gpH protein, may be directly inserted into the desired position in the genome of the helper phage, hence bypassing the necessity of providing the modified sequence *in trans.* Methods for both supplying a sequence or protein *in trans* in the form of a plasmid, as well as methods to generate direct genomic insertions, modifications and mutations are well known to those skilled in the art.

### Bacterial elimination with antibiotics

Particular bacteria or groups of bacteria such as AMR bacteria that have had the expression of their antibiotic resistance genes modified can be eliminated by treatment with antibiotic(s). Thus, the invention encompasses methods of treating a subject with an antibiotic after treatment to modify an antibiotic resistance gene. Preferably, the level of the AMR bacteria is measured before and after the treatment.

Preferably, the treatment is after the subject has undergone an invasive procedures (surgery, intubation, catheterization, etc.) or harsh conditioning procedures (immunosuppression, irradiation, etc.). In one embodiment, the antibiotic treatment occurs after the subject undergoes surgery. In a preferred embodiment, the surgery is a solid organ transplant surgery of the kidney or liver. In some embodiments, the subject is in the ICU and/or has undergone surgery, dialysis, or a transplant.

In one embodiment, the invention encompasses a method comprising measuring the level of an AMR bacteria, subsequently administering a phage, phagemid, and/or an antibiotic, and measuring the level of the AMR bacteria after the administration(s).

In one embodiment, the method comprises a step of confirming that the payload(s)/vector(s) work(s) on the patient or subject sample *in vitro.* Thus a sample of microbiota taken from the patient or subject can be contacted with the payload/vector *in vitro* and the effect of the payload/vector on antibiotic resistance assessed by routine *in vitro* procedures.

In some embodiments, the antibiotic is methicillin, streptomycin, vancomycin, clindamycin, or metronidazole, alone or in any possible combination. In some embodiments, the antibiotic is sulphadoxine, trimethoprim, or metronidazole, alone or in any possible combination. In some embodiments, the antibiotic is selected from methicillin, streptomycin, vancomycin, clindamycin, metronidazole, sulphadoxine, trimethoprim, or any combination of 1, 2, 3, 4, 5, 6, or 7 of these antibiotics.

In some embodiments, the antibiotic is selected from the group consisting of penicillins such as penicillin G, penicillin K, penicillin N, penicillin O, penicillin V, methicillin, benzylpenicillin, nafcillin, oxacillin, cloxacillin, dicloxacillin, ampicillin, amoxicillin, pivampicillin, hetacillin, bacampicillin, metampicillin, talampicillin, epicillin, carbenicillin, ticarcillin, temocillin, mezlocillin, and piperacillin; cephalosporins such as cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, cefoxitin, loracarbef, cefbuperazone, cefminox, cefotetan, cefoxitin, cefotiam, cefcapene, cefdaloxime, cefdinir, cefditoren, cefetamet, cefixime, cefmenoxime, cefodizime, cefotaxime, cefovecin, cefpimizole, cefpodoxime, cefteram, ceftamere, ceftibuten, ceftiofur, ceftiolene, ceftizoxime, ceftriaxone, cefoperazone, ceftazidime, latamoxef, cefclidine, cefepime, cefluprenam, cefoselis, cefozopran, cefpirome, cefquinome, flomoxef, ceftobiprole, ceftaroline, ceftolozane, cefaloram, cefaparole, cefcanel, cefedrolor, cefempidone, cefetrizole, cefivitril, cefmatilen, cefmepidium, cefoxazole, cefrotil, cefsumide, ceftioxide, cefuracetime, and nitrocefin; polymyxins such as polysporin, neosporin, polymyxin B, and polymyxin E, rifampicins such as rifampicin, rifapentine, and rifaximin; Fidaxomicin; quinolones such as cinoxacin, nalidixic acid, oxolinic acid, piromidic acid, pipemidic acid, rosoxacin, ciprofloxacin, enoxacin, fleroxacin, lomefloxacin, nadifloxacin, norfloxacin, ofloxacin, pefloxacin, rufloxacin, balofloxacin, grepafloxacin, levofloxacin, pazufloxacin, temafloxacin, tosufloxacin, clinafloxacin, gatifloxacin, gemifloxacin, moxifloxacin, sitafloxacin, trovafloxacin, prulifloxacin, delafloxacin, nemonoxacin, and zabofloxacin; sulfonamides such as sulfafurazole, sulfacetamide, sulfadiazine, sulfadimidine, sulfafurazole, sulfisomidine, sulfadoxine, sulfamethoxazole, sulfamoxole, sulfanitran, sulfadimethoxine, sulfametho-xypyridazine, sulfametoxydiazine, sulfadoxine, sulfametopyrazine, and terephtyl; macrolides such as azithromycin, clarithromycin, erythromycin, fidaxomicin, telithromycin, carbomycin A, josamycin, kitasamycin, midecamycin, oleandomycin, solithromycin, spiramycin, troleandomycin, tylosin, and roxithromycin; ketolides such as telithromycin, and cethromycin; fluoroketolides such as solithromycin; lincosamides such as lincomycin, clindamycin, and pirlimycin; tetracyclines such as demeclocycline, doxycycline, minocycline, oxytetracycline, and tetracycline; aminoglycosides such as amikacin, dibekacin, gentamicin, kanamycin, neomycin, netilmicin, sisomicin, tobramycin, paromomycin, and streptomycin; ansamycins such as geldanamycin, herbimycin, and rifaximin; carbacephems such as loracarbef; carbapenems such as ertapenem, doripenem, imipenem (or cilastatin), and meropenem; glycopeptides such as teicoplanin, vancomycin, telavancin, dalbavancin, and oritavancin; lincosamides such as clindamycin and lincomycin; lipopeptides such as daptomycin; monobactams such as aztreonam; nitrofurans such as furazolidone, and nitrofurantoin; oxazolidinones such as linezolid, posizolid, radezolid, and torezolid; teixobactin, clofazimine, dapsone, capreomycin, cycloserine, ethambutol, ethionamide, isoniazid, pyrazinamide, rifabutin, arsphenamine,chloramphenicol, fosfomycin, fusidic acid, metronidazole, mupirocin, platensimycin, quinupristin (or dalfopristin), thiamphenicol, tigecycline, tinidazole, trimethoprim, alatrofloxacin, fidaxomycin, nalidixic acid, rifampin, derivatives and combination thereof.

In some embodiments, the AMR bacteria is resistant to β-lactams, aminoglycosides, erythromycin or tetracycline. In these cases, the gene encoding the resistance gene for that antibiotic within the AMR bacteria can be modified to make the AMR bacteria susceptible to the antibiotic. In a further embodiment, the modified AMR bacteria is then treated with the specific antibiotic.

### Measurement of bacterial elimination

The elimination of AMR bacteria can be assessed by comparison with and without (control sample) the genetic modification treatment either *in vitro* or *in vivo.* Untreated samples can serve as control samples. The comparison is preferably performed by assessing the percentage of bacteria before and after the genetic modification treatment at least two timepoints and determining a reduced amount of the targeted AMR bacteria at a later timepoint.

Comparison *in vitro* can be performed by growing the bacteria in solid or liquid culture and determining the percentages or levels of an AMR bacteria over time. The percentages or levels can be determined by routine diagnostic procedures including antibiotic resistance/sensitivity, ELISA, PCR, High Resolution Melting, and nucleic acid sequencing.

Comparison *in vivo* can be performed by collecting samples (e.g., stool or swab) over time and determining the percentages or levels of a bacteria over time. The percentages can be determined by routine diagnostic procedures employing immunodetection (e.g. ELISA), nucleic acid amplification (e.g., PCR), High Resolution Melting, and nucleic acid sequencing.

Preferred levels of elimination of AMR bacteria are at least 70%, 80%, 90%, 95%, 97%, 98%, 99%, 99.9%, 99.99%, and 100% of the starting levels of an AMR bacteria. The "elimination" of the AMR bacteria can be either by killing of the bacteria or modification of the antibiotic resistant bacteria to an antibiotic sensitive bacteria. That is, an antibiotic sensitive bacteria is no longer an AMR bacteria.

### Enzymes/Systems for Inducing Modifications

In some embodiments, the genetic modification is made with one or more of the following enzymes/systems. Such base editing approach for modulation of the microbiota is further described in international application PCT/EP2021/059229.

Cytosine base editors (CBE) and Adenosine base editors (ABE), as described in Rees et al. Nat Rev Genet 19, 770-788 (2018) which is hereby incorporated by reference.

So far there are seven types of DNA base editors described:
- Cytosine Base Editor (CBE) that convert C:G into T:A (Komor, A et al. Programmable editing of a target base in genomic DNA without double-stranded DNA cleavage. Nature 533:420-4. (2016))
- Adenine Base Editor (ABE) that convert A:T into G:C (Gaudelli, N. M. et al. Programmable base editing of A·T to G·C in genomic DNA without DNA cleavage. Nature 551(7681) 464-471 (2017))
- Cytosine Guanine Base Editor (CGBE) that convert C:G into G:C (Chen, L et al. Precise and programmable C:G to G:C base editing in genomic DNA. Biorxiv (2020); Kurt, I et al. CRISPR C-to-G base editors for inducing targeted DNA transversions in human cells. Nature Biotechnology (2020))
- Cytosine Adenine Base Editor (CABE) that convert C:G into A:T (Zhao, D et al. New base editors change C to A in bacteria and C to G in mammalian cells. Nature Biotechnology (2020) )
- Adenine Cytosine Base Editor (ACBE) that convert A:T into C:G (WO2020181180)
- Adenine Thymine Base Editor (ATBE) that convert A:T into T:A (WO2020181202)
- Thymine Adenine Base Editor (TABE) that convert T:A into A:T (WO2020181193; WO2020181178; WO2020181195).

Base editors differ in the base modification enzymes. CBE rely on ssDNA cytidine deaminase among which: APOBEC1, rAPOBEC1, APOBEC1 mutant or evolved version (evoAPOBEC1), and APOBEC homologs (APOBEC3A (eA3A), Anc689), Cytidine deaminase 1 (CDA1), evoCDA1, FERNY, evoFERNY.

ABE rely on deoxyadenosine deaminase activity of a tandem fusion TadA-TadA* where TadA* is an evolved version of TadA, an *E. coli* tRNA adenosine deaminase enzyme, able to convert adenosine into Inosine on ssDNA. TadA* include TadA-8a-e and TadA-7.10.

Except from base modification enzyme there has been also modifications implemented to base editor to increase editing efficacy, precision and modularity:
- the addition of one or two uracil DNA glycosylase inhibitor domain (UGI) to prevent base excision repair mechanism to revert base edition
- the addition of Mu-GAM that decrease insertion-deletion rate by inhibiting Non-homologous end joining mechanism in the cell (NHEJ)
- the use of nickase active Cas9 (nCas9 D10A) that, by creating nicks on the non-edited strand favors its repair and consequently the fixation of the edited base.
- the use of diverse Cas proteins from for example different organisms, mutants with different PAM motifs or different fidelity or different family (e.g. Cas12a).

Non-limiting examples of DNA-based editor proteins include BE1, BE2, BE3, BE4, BE4-GAM, HF-BE3, Sniper-BE3, Target-AID, Target-AID-NG, ABE, EE-BE3, YE1-BE3, YE2-BE3, YEE-BE3, BE-PLUS, SaBE3, SaBE4, SaBE4-GAM, Sa(KKH)-BE3, VQR-BE3, VRER-BE3, EQR-BE3, xBE3, Cas12a-BE, Ea3A-BE3, A3A-BE3, TAM, CRISPR-X, ABE7.9, ABE7.10, ABE7.10*, xABE, ABESa, VQR-ABE, VRER-ABE, Sa(KKH)-ABE, ABE8e, SpRY-ABE, SpRY-CBE, SpG-CBE4, SpG-ABE, SpRY-CBE4, SpCas9-NG-ABE, SpCas9-NG-CBE4, enAsBE1.1, enAsBE1.2, enAsBE1.3, enAsBE1.4, AsBE1.1, AsBE1.4, CRISPR-Abest, CRISPR-Cbest, eA3A-BE3, AncBE4.

Cytosine Guanine Base Editors (CGBE) consist of a nickase CRISPR fused to:
a. A cytosine deaminase (rAPOBEC) and base excision repair proteins (e.g. rXRCC1) (Chen, L et al. Precise and programmable C:G to G:C base editing in genomic DNA. Biorxiv (2020)).
b. A rat APOBEC1 variant (R33A) protein and an *E.* coli-derived uracil DNA N-glycosylase (eUNG) (Kurt, I et al. CRISPR C-to-G base editors for inducing targeted DNA transversions in human cells. Nature Biotechnology (2020)).

Cytosine Adenine Base Editors (CABE) consist of a Cas9 nickase, a cytidine deaminase (e.g. AID), and a uracil-DNA glycosylase (Ung) (Zhao, D et al. New base editors change C to A in bacteria and C to G in mammalian cells. Nature Biotechnology (2020)).

ACBE include a nucleic acid programmable DNA-binding protein and an adenine oxidase (WO2020181180).

ATBE consist of a Cas9 nickase and one or more adenosine deaminase or an oxidase domain (WO2020181202).

TABE consist of a Cas9 nickase and an adenosine methyltransferase, a thymine alkyltransferase, or an adenosine deaminase domain (WO2020181193; WO2020181178; WO2020181195).

Base editor molecules can also consist of two or more of the above listed editor enzymes fused to a Cas protein (e.g. combination of an ABE and CBE). These biomolecules are named dual base editors and enable the editing of two different bases (Grunewald, J et al. A dual-deaminase CRISPR base editor enables concurrent adenine and cytosine editing, Nature Biotechnology (2020); Li, C et al. Targeted, random mutagenesis of plant genes with dual cytosine and adenine base editors, Nature Biotechnology (2020)).

Prime editors (PE), as described in Anzalone et al., Nature volume 576, pages 149-157(2019) , which is hereby incorporated by reference, consist of a nCas9 fused to a reverse transcriptase used in combination with a prime editing RNA (pegRNA, a guide RNA that includes a template region for the reverse transcription).

Prime Editing allows introduction of insertions, deletions (indels), and 12 base-to-base conversions. Prime editing relies on the ability of a reverse transcriptase (RT), fused to a Cas nickase variant, to convert RNA sequence brought by a prime editing guide RNA (pegRNA) into DNA at the nick site generated by the Cas protein. The DNA flap generated from this process is then included or not in the targeted DNA sequence.

Prime editing systems include:
- a Cas nickase variant such as Cas9-H840A fused to a reverse transcriptase domain such as M-MLV RT or its mutant version (M-MLV RT(D200N), M-MLV RT(D200N/L603W), M-MLV RT(D200N/L603W/T330P/ T306K/W313F)
- a prime editing guide RNA (pegRNA)

To favor editing, the prime editing system can include the expression of an additional sgRNA targeting the Cas nickase activity towards the non-edited DNA strand ideally only after the resolution of the edited strand flap by designing the sgRNA to anneal with the edited strand but not with the original strand.

Non-limiting examples of prime editing systems include PE1, PE1-M1, PE1-M2, PE1-M3, PE1-M6, PE1-M15, PE1-M3inv, PE2, PE3, PE3b.

Cas9 Retron preclSe Parallel Editing via homologY ('CRISPEY'), a retron RNA fused to the sgRNA and expressed together with Cas9 and the retron proteins including at least the reverse transcriptase (Sharon, E. et al. Functional Genetic Variants Revealed by Massively Parallel Precise Genome Editing. Cell 175, 544-557.e16 (2018)).

The SCRIBE strategy: a retron system expressed in combination with a recombinase promoting the recombination of single stranded DNA, also known as single stranded annealing proteins (SSAPs) (Farzadfard, F. & Lu, T. K. Genomically encoded analog memory with precise in vivo DNA writing in living cell populations. Science 346, 1256272 (2014)) . Such recombinases include but are not limited to phage recombinases such as lambda red, recET, Sak, Sak4, and newly described SSAPs described in Wannier et al., (bioRxiv 2020.01.14.906594), which is hereby incorporated by reference.

The targetron system based on group II introns described in Karberg et al., Nature Biotechnology volume 19, pages 1162-1167(2001), which is hereby incorporated by reference, and which has been adapted to many bacterial species.

Other retron based gene targeting approaches, as described in Simon et al., Nucleic Acids Research, Volume 47, Issue 21, 02 December 2019, Pages 11007-11019, which is hereby incorporated by reference.

CRISPR-Cas. The CRISPR system contains two distinct elements, i.e. i) an endonuclease, in this case the CRISPR associated nuclease (Cas or "CRISPR associated protein") and ii) a guide RNA. Depending on the type of CRISPR system, the guide RNA may be in the form of a chimeric RNA which consists of the combination of a CRISPR (crRNA) bacterial RNA and a tracrRNA (trans-activating RNA CRISPR) (Jinek et al., Science 2012). The guide RNA combines the targeting specificity of the crRNA corresponding to the "spacing sequences" that serve as guides to the Cas proteins, and the conformational properties of the tracrRNA in a single transcript. When the guide RNA and the Cas protein are expressed simultaneously in the cell, the target genomic sequence can be permanently interrupted (and causing disappearance of the targeted and surrounding sequences and/or cell death, depending on the location) or modified. The modification may be guided by a repair matrix.

The CRISPR system includes two main classes depending on the nuclease mechanism of action:
- Class 1 is made of multi-subunit effector complexes and includes type I, III and IV
- Class 2 is made of single-unit effector modules, like Cas9 nuclease, and includes type II (II-A,II-B,II-C,II-C variant), V (V-A,V-B,V-C,V-D,V-E,V- U1,V-U2,V-U3,V-U4,V-U5) and VI (VI-A,VI-B1,VI-B2,VI-C,VI-D)

The sequence of interest according to the present invention comprises a nucleic acid sequence encoding Cas protein. A variety of CRISPR enzymes are available for use as a sequence of interest on the plasmid according to the present invention. In some embodiments, the CRISPR enzyme is a Type II CRISPR enzyme, a Type II-A or Type II-B CRISPR enzyme. In another embodiment, the CRISPR enzyme is a Type I CRISPR enzyme such as a Cas3 nuclease or a Type III CRISPR enzyme. In some embodiments, the CRISPR enzyme catalyzes DNA modification. In some other embodiments, the CRISPR enzyme catalyzes RNA modification. For instance, Cas13- deaminase fusions have been used for RNA base editing thus modifying RNA (David BT Cox et al, Science, 358 (6366) p.1019-1027, 2017 Nov 24). In one embodiment, the CRISPR enzymes may be coupled to a guide RNA or single guide RNA (sgRNA). In preferred embodiment, the guide RNA or sgRNA targets an antibiotic resistance gene. In certain embodiments, the guide RNA or sgRNA targets or further targets a gene selected from the group consisting of an antibiotic resistance gene, virulence protein or factor gene, toxin protein or factor gene, a bacterial receptor gene, a membrane protein gene, a structural protein gene, a secreted protein gene, a gene expressing resistance to a drug in general and a gene causing a deleterious effect to the host. Preferably, the CRISPR enzyme makes a double strand break. In some embodiments, the CRISPR enzyme makes a single strand break or nicks. In some embodiments, the CRISPR enzyme does not make any break in the DNA or RNA.

In a particular embodiment, at least two distinct vectors are used, in particular at least 3, 4, 5, 6, 7, 8, 9, 10 or more, each vector comprising guide RNA or sgRNA which targets a different location in the targeted bacteria, in particular targeting different locations within an antibiotic resistance gene, or targeting different antibiotic resistant genes, or targeting different antibiotic resistant genes and other genes of interest, such as virulence protein or factor gene, toxin protein or factor gene, a bacterial receptor gene, a membrane protein gene, a structural protein gene, a secreted protein gene, a gene expressing resistance to a drug in general and a gene causing a deleterious effect to the host.

In another particular embodiment, at least two distinct vectors are used, in particular at least 3, 4, 5, 6, 7, 8, 9, 10 or more, each vector comprising guide RNA or sgRNA which targets different types of targeted bacteria, in particular bacteria with different antibiotic resistant genes.

The sequence of interest may comprise a nucleic acid sequence encoding a guide RNA or sgRNA to guide the Cas protein endogenous to the targeted bacteria, alone or in combination with a Cas protein and/or a guide RNA encoded by the payload.

Non-limiting examples of Cas proteins as part of a multi-subunit effector or as a single-unit effector include Cas1, Cas1B, Cas2, Cas3, Cas4, Cas5, Cas6, Cas7, Cas8, Cas9 (also known as Csn1 and Csx12), Cas10, Cas11 (SS), Cas12a (Cpf1), Cas12b (C2c1), Cas12c (C2c3), Cas12d (CasY), Cas12e (CasX), C2c4, C2c8, C2c5, C2c10, C2c9, Cas13a (C2c2), Cas13b (C2c6), Cas13c (C2c7), Cas13d, Csa5, Csc1, Csc2, Cse1, Cse2, Csy1, Csy2, Csy3, Csf1, Csf2, Csf3, Csf4, Csm1, Csm2, Csm3, Csm4, Csm5, Csm6, Cmr1, Cmr3, Cmr4, Cmr5, Cmr6, Csn2, Csb1, Csb2, Csb3, Csx17, Csx14, Csx10, Csx16, CsaX, Csx13, Csx1, Csx15, SdCpf1, CmtCpf1, TsCpf1, CmaCpf1, PcCpf1, ErCpf1, FbCpf1, UbcCpf1, AsCpf1, LbCpf1, Mad4, Mad7, Cms1, homologues thereof, orthologues thereof, variants thereof, or modified versions thereof. In some embodiments, the CRISPR enzyme cleaves both strands of the target nucleic acid at the Protospacer Adjacent Motif (PAM) site.

In various embodiments, the invention encompasses fusion proteins comprising a Cas9 (e.g., a Cas9 nickase) domain and a deaminase domain. In some embodiments, the fusion protein comprises Cas9 and a cytosine deaminase enzyme, such as APOBEC enzymes, or adenosine deaminase enzymes, such as ADAT enzymes, for example as disclosed in U.S. Patent Publ. 2015/0166980, which is hereby incorporated by reference. In one embodiment, the deaminase is an ACF1/ASE deaminase.

In various embodiments, the APOBEC deaminase is selected from the group consisting of APOBEC1 deaminase, APOBEC2 deaminase, APOBEC3A deaminase, APOBEC3B deaminase, APOBEC3C deaminase, APOBEC3D deaminase, APOBEC3F deaminase, APOBEC3G deaminase, and APOBEC3H deaminase. In various embodiments, the fusion protein comprises a Cas9 domain, a cytosine deaminase domain, and a uracil glycosylase inhibitor (UGI) domain.

In one embodiment, the deaminase is an adenosine deaminases that deaminate adenosine in DNA, for example as disclosed in U.S. Patent 10,113,163, which is hereby incorporated by reference. In some embodiments, the fusion proteins further comprise a nuclear localization sequence (NLS), and/or an inhibitor of base repair, such as, a nuclease dead inosine specific nuclease (dISN), for example as disclosed in U.S. Patent 10,113,163. In various embodiments, the invention encompasses fusion proteins comprising a catalytically impaired Cas9 endonuclease fused to an engineered reverse transcriptase, programmed with a prime editing guide RNA (pegRNA) that both specifies the target site and encodes the desired edit, for example as described in Anzalone et al., Nature, Vol. 576, pages 149-157 (2019), which is hereby incorporated by reference.

In a particular embodiment, the CRISPR enzyme is any Cas9 protein, for instance any naturally-occurring bacterial Cas9 as well as any variants, homologs or orthologs thereof.

By "Cas9" is meant a protein Cas9 (also called Csn1 or Csx12) or a functional protein, peptide or polypeptide fragment thereof, i.e. capable of interacting with the guide RNA(s) and of exerting the enzymatic activity (nuclease) which allows it to perform the double-strand cleavage of the DNA of the target genome. "Cas9" can thus denote a modified protein, for example truncated to remove domains of the protein that are not essential for the predefined functions of the protein, in particular the domains that are not necessary for interaction with the gRNA (s). In some embodiments, the CAS9 is a dCas9 (dead-Cas9) or nCas9 (nickase Cas9) lacking double stranded DNA cleavage activity.

The sequence encoding Cas9 (the entire protein or a fragment thereof) as used in the context of the invention can be obtained from any known Cas9 protein (Fonfara *et al.,* 2014; Koonin *et al.,* 2017). Examples of Cas9 proteins useful in the present invention include, but are not limited to, Cas9 proteins of *Streptococcus pyogenes* (SpCas9), *Streptococcus thermophiles* (St1Cas9, St3Cas9), *Streptococcus mutans, Staphylococcus aureus* (SaCas9), *Campylobacter jejuni* (CjCas9), *Francisella novicida* (FnCas9) and *Neisseria meningitides* (NmCas9).

The sequence encoding Cpf1 (Cas12a) (the entire protein or a fragment thereof) as used in the context of the invention can be obtained from any known Cpf1 (Cas12a) protein (Koonin *et al.,* 2017). Examples of Cpf1(Cas12a) proteins useful in the present invention include, but are not limited to, Cpf1 (Cas12a) proteins of *Acidaminococcus sp,* Lachnospiraceae bacteriu and *Francisella novicida.*

The sequence encoding Cas13a (the entire protein or a fragment thereof) as used in the context of the invention can be obtained from any known Cas13a (C2c2) protein (Abudayyeh *et al.,* 2017) . Examples of Cas13a (C2c2) proteins useful in the present invention include, but are not limited to, Cas13a (C2c2) proteins of *Leptotrichia wadei* (LwaCas13a).

The sequence encoding Cas13d (the entire protein or a fragment thereof) as used in the context of the invention can be obtained from any known Cas13d protein (Yan *et al.,* 2018) .Examples of Cas13d proteins useful in the present invention include, but are not limited to, Cas13d proteins of *Eubacterium siraeum* and *Ruminococcus sp.*

The sequence encoding Mad4 (the entire protein or a fragment thereof) as used in the context of the invention is disclosed in international application WO2018/236548.

The sequence encoding Mad7 (the entire protein or a fragment thereof) as used in the context of the invention is disclosed in international application WO2018/236548.

The sequence encoding Cms1 (the entire protein or a fragment thereof) as used in the context of the invention is disclosed in international patent application WO2017/141173.

In some embodiments, other programmable nucleases can be used. These include an engineered TALEN (Transcription Activator-Like Effector Nuclease) and variants, engineered zinc finger nuclease (ZFN) variants, natural, evolved or engineered meganuclease or recombinase variants, and any combination or hybrids of programmable nucleases. Thus, the programmable nucleases provided herein may be used to selectively modify a DNA encoding a gene of interest, such as an antibiotic resistance gene.

In some embodiments, the genetic modification is made at the RNA level. RNA base editing is based on the same principle as DNA base editing: an enzyme catalyzing the conversion of a RNA base into another must be brought close to the target base to perform its conversion locally. In one embodiment, the enzyme used for RNA editing is an adenosine deaminase from ADAR family that converts Adenosine into Inosine in dsRNA structure. Several seminal studies used this specificity for dsRNA and fused the ADAR deaminase domain (ADAR_{DD}) to an antisense oligo in order to program local RNA base editing. More recently the ability of some CRISPR-Cas systems to bind RNA molecules was repurposed into RNA editing. Using catalytically dead Cas13b enzyme (dPspCas13b) fused to a hyperactive mutant of ADAR2 deaminase domain (ADAR2_{DD}-E488Q for REPAIRv1 and ADAR2_{DD}-E488Q-T375G for REPAIRv2) Cox et al improved specificity and efficiency compare to previous RNA editing strategies.

Non-limiting examples of RNA based editor proteins include REPAIRv1, REPAIRv2.

### Vectors for Inducing Modifications

In various embodiments, one or more of the following vectors can be used to introduce the exogenous enzyme that results in a genetic modification:
- engineered phages (in particular with engineered genome and optionally engineered capsid)
- plasmid (e.g., a conjugative plasmid capable of transfer into a host cell), phage genome, phagemid or prophage.
- Each vector may be as described above, eg, a phage capable of infecting a host cell or conjugative plasmid capable of introduction into a host cell, which can be introduced either by a phage particle via transduction or by a donor bacteria via conjugation. In an example, the vectors are in combination with an antibiotic agent (eg, a beta-lactam antibiotic) and/or with any other agent.
- A bacteriophage whose genome has been genetically engineered for modifying a naturally occurring bacteria *in situ* and comprising a nucleic acid encoding a gene editing enzyme/system for transduction of a target bacteria in a mixed bacterial population wherein said gene editing enzyme/system modifies the genome of said target bacteria, but does not lead to the death of the target bacteria.

Vectors usable in the context of the invention, inside a bacterial delivery vehicle or not, include without limitation plasmid (e.g. conjugative plasmid), carrier bacteria comprising a plasmid such as conjugative plasmid, phagemid, packaged phagemid, and engineered (or recombinant) phage (with an engineered genome and/or capsid).

In a particular embodiment, the vector(s) used in the context of the invention is(are) inside a bacterial delivery vehicle.

The invention encompasses the use of these vectors wherein the gene modifying/editing enzyme/system targets a DNA within the target bacteria encoding an antibiotic resistance gene.

### Origin of replication

In particular embodiments, the vector of the invention comprises an origin of replication. Origins of replication known in the art have been identified from species-specific plasmid DNAs (e.g. ColE1, RI, pT181, pSC101, pMB1, R6K, RK2, p15a and the like), from bacterial virus (e.g. ϕX174, M13, F1 and P4) and from bacterial chromosomal origins of replication (e.g. oriC).

In one embodiment, the vector of the invention does not comprise any functional bacterial origin of replication or contain an origin of replication that is inactive in the targeted bacteria. Thus, the vector of the invention cannot replicate by itself once it has been introduced into a bacterium.

In one embodiment, the origin of replication on the vector to be packaged is inactive in the targeted bacteria, meaning that this origin of replication is not functional in the bacteria transformed / transduced by the vector or in the bacteria being the receiver bacteria of a conjugative vector, thus preventing unwanted vector replication.

In one embodiment, the vector comprises a bacterial origin of replication that is functional in the bacteria used for the production of the vector.

### Bacteria-specific origins of replication

Plasmid replication depends on host enzymes and on plasmid-controlled cis and trans determinants. For example, some plasmids have determinants that are recognized in almost all gram-negative bacteria and act correctly in each host during replication initiation and regulation. Other plasmids possess this ability only in some bacteria (Kues, U and Stahl, U 1989 Microbiol Rev 53:491-516).

Plasmids are replicated by three general mechanisms, namely theta type, strand displacement, and rolling circle (reviewed by Del Solar et al. 1998 Microhio and Molec Biol. Rev 62:434-464) that start at the origin of replication. These replication origins contain sites that are required for interactions of plasmid and/or host encoded proteins.

Origins of replication may be moderate copy number, such as ColE1 ori from pBR322 (15-20 copies per cell) or the R6K plasmid (15-20 copies per cell) or may be high copy number, e.g. pUC oris (500-700 copies per cell), pGEM oris (300-400 copies per cell), pTZ oris (>1000 copies per cell) or pBluescript oris (300-500 copies per cell).

Examples of bacterial origins of replication include bacterial origins of replication selected in the group consisting of ColE1, pMB1 and variants (pBR322, pET, pUC, etc), p15a, ColA, ColE2, pOSAK, pSC101, R6K, IncW (pSa etc), IncFII, pT181, P1, F IncP, IncC, IncJ, IncN, IncP1, IncP4, IncQ, IncH11, RSF1010, CloDF13, NTP16, R1, f5, pPS10, pC194, pE194, BBR1, pBC1, pEP2, pWVO1, pLF1311, pAP1, pWKS1, pLS1, pLS11, pUB6060, pJD4, pIJ101, pSN22, pAMbeta1, pIP501, pIP407, ZM6100(Sa), pCU1, RA3, pMOL98, RK2/RP4/RP1/R68, pB10, R300B, pRO1614, pRO1600, pECB2, pCM1, pFA3, RepFIA, RepFIB, RepFIC, pYVE439-80, R387, phasyl, RA1, TF-FC2, pMV158 and pUB113.

The bacterial origin of replication may be an *E. coli* origin of replication selected in the group consisting of ColE1, pMB1 and variants (pBR322, pET, pUC, etc), p15a, ColA, ColE2, pOSAK, pSC101, R6K, IncW (pSa etc), IncFII, pT181, P1, F IncP, IncC, IncJ, IncN, IncP1, IncP4, IncQ, IncH11, RSF1010, CloDF13, NTP16, R1, f5, pPS10.

The bacterial origin of replication may be selected in the group consisting of pC194, pE194, BBR1, pBC1, pEP2, pWVO1, pLF1311, pAP1, pWKS1, pLS1, pLS11, pUB6060, pJD4, pIJ101, pSN22, pAMbeta1, pIP501, pIP407, ZM6100(Sa), pCU1, RA3, pMOL98, RK2/RP4/RP1/R68, pB10, R300B, pRO1614, pRO1600, pECB2, pCM1, pFA3, RepFIA, RepFIB, RepFIC, pYVE439-80, R387, phasyl, RA1, TF-FC2, pMV158 and pUB113.

More particularly, the bacterial origins of replication may be ColE1 and p15a.

Alternatively, the bacterial origin of replication may be functional in *Propionibacterium* and *Cutibacterium* more specifically in *Propionibacterium freudenreichii* and *Cutibacterium acnes* and may be selected from the group consisting of pLME108, pLME106, p545, pRGO1, pZGX01, pPG01, pYS1, FRJS12-3, FRJS25-1, pIMPLE-HL096PA1,A_15_1_R1.

### Phage origin of replication

The vector according to the invention may comprise a phage replication origin which can initiate, with complementation in cis or in trans of a complete or modified phage genome, the replication of the payload for later encapsulation into the different capsids.

The phage origin can also be engineered to act as a bacterial origin of replication without the need to package any phage particles.

A phage origin of replication comprised in the payload/vector of the invention can be any origin of replication found in a phage.

Preferably, the phage origin of replication can be the wild-type or non-wildtype sequence of the M13, f1, ϕX174, P4, Lambda, P2, 186, Lambda-like, HK022, mEP237, HK97, HK629, HK630, mEP043, mEP213, mEP234, mEP390, mEP460, mEPx1, mEPx2, phi80, mEP234, T2, T4, T5, T7, RB49, phiX174, R17, PRD1 PI-like, P2-like, P22, P22-like, N15 and N15-like bacteriophages.

More preferably, the phage origin of replication is selected in the group consisting of phage origins of replication of M13, f1, ϕX174, P4, and Lambda.

In a particular embodiment, the phage origin of replication is the P4 origin of replication.

In a particular embodiment, the phage origin of replication is from Propionibacterium phages: BW-like phages such as Doucette, B22, E6, G4, BV-like phages such as Anatole, E1, B3, BX-like phages such as PFR1 and PFR2, filamentous B5 phage, BU-like phages (Cutibacterium acnes phages).

### Conditional origin of replication

In a particular embodiment, the vector of the invention comprises a conditional origin of replication which is inactive in the targeted bacteria but is active in a donor bacterial cell.

In the context of the invention, a "conditional origin of replication" refers to an origin of replication whose functionality may be controlled by the presence of a specific molecule.

In a particular embodiment, the conditional origin of replication is an origin of replication, the replication of which depends upon the presence of one or more given protein, peptid, RNA, nucleic acid, molecule or any combination thereof.

In a particular embodiment, the replication of the vector comprising said origin of replication may further depend on a process, such as transcription, to activate said replication.

In the context of the invention, said conditional origin of replication is inactive in the targeted bacteria because of the absence of said given protein, peptid, RNA, nucleic acid, molecule or any combination thereof in said targeted bacteria.

In a particular embodiment, said conditional origin of replication is active in said donor bacterial cell because said donor bacterial cell expresses said given protein, peptid, RNA, nucleic acid, molecule or any combination thereof. In a particular embodiment, said protein, peptid, RNA nucleic acid, molecule or any combination thereof is expressed in trans in said donor bacterial cell.

By "in trans" is meant herein that said protein, peptid, RNA, nucleic acid, molecule or any combination thereof is not encoded on the same nucleic acid molecule as the one comprising the origin of replication. In a particular embodiment, said protein, peptid, RNA, nucleic acid, molecule or any combination thereof is encoded on a chromosome or on a plasmid. In a particular embodiment, said plasmid comprises an antibiotic resistance marker or an auxotrophic resistance marker. In an alternative embodiment, said plasmid is devoid of antibiotic resistance marker.

Since said conditional origin of replication is inactive in the targeted bacteria because of the absence of said given protein, peptid, RNA, nucleic acid, molecule or any combination thereof in said targeted bacteria, said conditional origin of replication may be selected depending on the specific bacteria to be targeted.

The conditional origin of replication disclosed herein may originate from plasmids, bacteriophages or PICIs which preferably share the following characteristics: they contain in their origin of replication repeat sequences, or iterons, and they code for at least one protein interacting with said origin of replication (i.e. Rep, protein O, protein P, pri) which is specific to them.

By way of example, mention may be made of the conditional replication systems of the following plasmids and bacteriophages: RK2, R1, pSC101, F, Rts1, RSF1010, P1, P4, lambda, phi82, phi80.

In a particular embodiment, said conditional origin of replication is selected from the group consisting of the R6Kλ DNA replication origin and derivatives thereof, the IncPα oriV origin of replication and derivatives thereof, ColE1 origins of replication modified to be under an inducible promoter, and origins of replication from phage-inducible chromosomal islands (PICIs) and derivatives thereof.

In a particular embodiment, said conditional origin of replication is an origin of replication present in less than 50%, or less than 40%, less than 30%, less than 20%, less than 10% or less than 5% of the bacteria of the host microbiome.

In another particular embodiment, said conditional origin of replication comprises or consists of a sequence less than 80% identical, in particular less than 70%, less than 60%, less than 50%, less than 40%, less than 30%, less than 20%, less than 10%, less than 5% or less than 1% identical to the sequences of the origins of replication of the bacteria of the host microbiome, in particular of the bacteria representing more than 50%, more particularly more than 60%, more than 70%, more than 80%, more than 90% or more than 95% of the host microbiome.

As used herein, the term "phage-inducible chromosomal islands" or "PICIs" refers to mobile genetic elements having a conserved gene organization, and encoding a pair of divergent regulatory genes, including a PICI master repressor. Typically, in Gram-positive bacteria, left of rpr, and transcribed in the same direction, PICIs encode a small set of genes including an integrase (int) gene; right of rpr, and transcribed in the opposite direction, the PICIs encode an excision function (xis), and a replication module consisting of a primase homolog (pri) and optionally a replication initiator (rep), which are sometimes fused, followed by a replication origin (ori), next to these genes, and also transcribed in the same direction, PICIs encode genes involved in phage interference, and optionally, a terminase small subunit homolog (terS).

In a particular embodiment, said conditional origin of replication is an origin of replication derived from phage-inducible chromosomal islands (PICIs).

A particular conditional origin of replication has indeed been derived from PICIs.

It was shown that it is possible to derive novel conditionally replicative plasmids, in particular based on the primase-helicase and origin of replication from PICIs. These origins may be relatively rare in target strains, and more advantageously the primase-ori pair may be unique for each PICI, significantly reducing the possibility of undesired recombination or payload spread events. They can further be modified to further limit recombination chances and remove restriction sites to bypass target bacteria defense systems.

In a particular embodiment, said conditional origin of replication is derived from the origin of replication from the PICI of the *Escherichia coli* strain CFT073, disclosed in Fillol-Salom et al. (2018) The ISME Journal 12:2114-2128.

In a particular embodiment, said conditional origin of replication is the primase ori from the PICI of the *Escherichia coli* strain CFT073, typically of sequence SEQ ID NO: 3.

In another particular embodiment, said conditional origin of replication is the primase ori from the PICI of the *Escherichia coli* strain CFT073, devoid of at least 1, at least 2, at least 3, at least 4, at least 5, at least 6, at least 7, at least 8, at least 9, at least 10, at least 11, at least 12, at least 13, at least 14, at least 15 or at least 16 restriction site(s) selected from the group consisting of GAAABCC, GCCGGC, RCCGGY, GCNGC, TWCANNNNNNTGG (SEQ ID NO: 8), TGGCCA, ACCYAC, YGGCCR, AGACC, GCWGC, GGGANGC, GKAGATD, GCCGGYYD, GGCYAC, RGCCGGYYD, and VGCCGGYBD.

In a particular embodiment, said conditional origin of replication is the primase ori from the PICI of the *Escherichia coli* strain CFT073, devoid of the restriction site GAAABCC (SEQ ID NO: 4). Preferably, said conditional origin of replication is of sequence SEQ ID NO: 1.

In another particular embodiment, said conditional origin of replication is the primase ori from the PICI of the *Escherichia coli* strain CFT073 devoid of the restriction sites GAAABCC, GCCGGC, RCCGGY, GCNGC, TWCANNNNNNTGG (SEQ ID NO: 8), TGGCCA, ACCYAC, YGGCCR, AGACC, GCWGC, GGGANGC, GKAGATD, GCCGGYYD, GGCYAC, RGCCGGYYD, and VGCCGGYBD. Preferably, said conditional origin of replication is of sequence SEQ ID NO: 2.

In a particular embodiment, wherein said origin of replication is derived from phage-inducible chromosomal islands (PICIs), said conditional origin of replication is active in said donor bacterial cell because said donor bacterial cell expresses a rep protein, in particular a primase-helicase, in particular a primase-helicase of sequence SEQ ID NO: 20, typically encoded by a nucleic acid comprising or consisting of the sequence SEQ ID NO: 21.

It was demonstrated that these specific conditional origins of replication were particularly compatible with lambda-based packaging, leading to sufficiently high titers (>10¹⁰/mL) required for microbiota-related applications.

In a particular embodiment, when said vector is a phagemid, said origin of replication may be derived from a microorganism which is different from the one that is used to encode the structural elements of the capsid packaging said phagemid.

By "donor bacterial cell" is meant herein a bacterium that is capable of hosting a vector as defined above, of producing a vector as defined above and/or which is capable of transferring said vector as defined above to another bacterium. In a particular embodiment, said vector may be a phagemid, and said donor bacterial cell may then be a bacterial cell able to produce said phagemid, more particularly in the form of a packaged phagemid. In an alternative embodiment, said vector may be a plasmid, more particularly a conjugative plasmid, and said donor bacterial cell may then be a bacterium that is capable of transferring said conjugative plasmid to another bacterium, in particular by conjugation.

Preferably, said donor bacterial cell stably comprises said vector and is able to replicate said vector.

In a particular embodiment, when the conditional origin of replication of said vector is an origin of replication, the replication of which depends upon the presence of a given protein, peptid, nucleic acid, RNA, molecule or any combination thereof, said donor bacterial cell expresses said protein, peptid, nucleic acid, RNA, molecule or any combination thereof. Preferably, said protein, peptid, nucleic acid, RNA, molecule or any combination thereof is expressed in trans, as defined above.

In a particular embodiment, said donor bacterial cell stably comprises a nucleic acid encoding said protein, peptid, nucleic acid, RNA, molecule or any combination thereof.

In a particular embodiment, when said origin of replication is derived from phage-inducible chromosomal islands (PICIs), said conditional origin of replication is active in said donor bacterial cell because said donor bacterial cell expresses a rep protein, in particular a primase-helicase, in particular a primase-helicase of sequence SEQ ID NO: 20.

In a particular embodiment, said donor bacterial cell stably comprises a nucleic acid encoding said rep protein, in particular said primase-helicase, said nucleic acid typically comprising or consisting of the sequence SEQ ID NO: 21.

In a particular embodiment, said donor bacterial cell is a production cell line, in particular a cell line producing packaged phagemids including the vector of the invention.

### Delivery vehicle incapable of self-reproduction

In a particular embodiment, the delivery vehicle, in particular the bacteriophage, bacterial virus particle or packaged phagemid, comprising the vector of the invention is incapable of self-reproduction.

In the context of the present invention, "self-reproduction" is different from "self-replication", "self-replication" referring to the capability of replicating a nucleic acid, whereas "self-reproduction" refers to the capability of having a progeny, in particular of producing new delivery vehicles, said delivery vehicles being either produced empty or with a nucleic acid of interest packaged.

By "delivery vehicle incapable of self-reproduction" is meant herein that at least one, several or all functional gene(s) necessary to produce said delivery vehicle is(are) absent from said delivery vehicle (and from said vector included in said delivery vehicle). In a preferred embodiment, said at least one, several or all functional gene(s) necessary to produce said delivery vehicle is(are) present in the donor cell as defined above, preferably in a plasmid, in the chromosome or in a helper phage present in the donor cell as defined above, enabling the production of said delivery vehicle in said donor cell.

In the context of the invention, said functional gene necessary to produce a delivery vehicle may be absent through (i) the absence of the corresponding gene or (ii) the presence of the corresponding gene but in a non-functional form.

In an embodiment, the sequence of said gene necessary to produce said delivery vehicle is absent from said delivery vehicle. In a preferred embodiment, the sequence of said gene necessary to produce said delivery vehicle has been replaced by a nucleic acid sequence of interest, in particular by a nucleic acid sequence encoding enzymes or systems for inducing genetic modifications, as defined above.

Alternatively, said gene necessary to produce said delivery vehicle is present in said delivery vehicle in a non-functional form, for example in a mutant non-functional form, or in a non-expressible form, for example with deleted or mutated non-functional regulators. In a preferred embodiment, said gene necessary to produce said delivery vehicle is present in said delivery vehicle in a mutated form which renders it non-functional in the target cell, while remaining functional in the donor cell.

In the context of the invention, genes necessary to produce said delivery vehicle encompass any coding or non-coding nucleic acid required for the production of said delivery vehicle.

Examples of genes necessary to produce said delivery vehicle include genes encoding phage structural proteins; phage genes involved in the control of genetic expression; phage genes involved in transcription and/or translation regulation; phage genes involved in phage DNA replication; phage genes involved in production of phage proteins; phage genes involved in phage proteins folding; phage genes involved in phage DNA packaging; and phage genes encoding proteins involved in bacterial cell lysis.

### Controlled self-inactivation or self-destruction of the vector

In a particular embodiment, the vector of the invention can self-inactivate or self-destroy in a controlled way. In a particular embodiment, the vector of the invention comprises a nucleic acid sequence comprising a gene encoding a DNA modifying enzyme, e.g. a nuclease, which can be expressed in the targeted bacterial cell, wherein the DNA modifying enzyme when expressed from the nucleic acid sequence modifies said nucleic acid at one or multiple locations in the nucleic acid sequence. In some embodiments, modification by the DNA modifying enzyme occurs after the nucleic acid sequence of interest, as defined below, comprised by the vector of the invention, has been transcribed and translated. In some embodiments, the DNA modifying enzyme is a nuclease which cleaves the nucleic acid sequence, and the cleavage occurs after the nucleic acid sequence of interest comprised by the vector has been transcribed and translated.

In a particular embodiment, said controlled self-inactivation or self-destruction is carried out by preventing the transcription or translation of said gene encoding a DNA modifying enzyme for a certain amount of time, allowing transcription or translation of other sequence(s), in particular of the sequence of interest comprised by the vector, during this amount of time, and delaying expression of said DNA modifying enzyme during this amount of time, therefore delaying inactivation or loss of the nucleic acid vector.

Said controlled self-inactivation or self-destruction of the vector of the invention relies on the use of a system, which can be encoded on the nucleic acid vector, that is programmed to lead a DNA modification or cleavage at one or multiple location on the nucleic acid vector, which leads to the inactivation or degradation of the nucleic acid vector. Preferably, the system is independent of any induction system based on external stimuli or molecule and is contained within the same vector as the one carrying the nucleic acid of interest.

In some embodiments, the delayed destruction or inactivation system relies on the expression of the DNA modifying enzyme that is programmed to modify, e.g. cleave, one or multiple sequences that have been added or engineered at specific locations on the nucleic acid vector. In some embodiments, the DNA modifying enzyme is a nuclease. The nuclease can be a restriction enzyme, a meganuclease, a zinc finger or a TALEN. In some embodiments, the nuclease can be an RNA-guided nuclease and can target one or multiple sequences to be cleaved by encoding one or multiple crRNA, and optionally a tracrRNA on the vector.

The targeted sequences can be located at one or multiple locations in the nucleic acid vector. In one embodiment, the targeted sequences are within the nucleic acid of interest carried by the nucleic acid vector. In one embodiment, the targeted sequences are within a nuclease, or other modification enzyme, carried by the nucleic acid vector.

The targeted sequences to be inactivated can be within an origin of replication if an origin of replication is present, within the nucleic acid of interest or at any other essential locations of the nucleic acid vector. If the desired outcome does not rely on nor necessitate the death of the bacteria, the targeted sequences on the nucleic acid vector should be engineered to not be homologous to any sequences from the target bacterial cell chromosome.

To incorporate a delay between the nucleic acid of interest expression and the destruction of the nucleic acid vector, it is herein provided engineered mechanisms to ensure that the production of a correctly folded DNA modifying protein will take longer than the time required for the nucleic acid(s) of interest to be expressed and exercise its/their intended function(s). In some embodiments, this is achieved by engineering a weak promoter for the DNA modifying protein. In some embodiments, this is achieved by engineering a weak RBS for the DNA modifying protein. In some embodiments, this is achieved by recoding the DNA modifying protein sequence leading to a slower translation rate of its corresponding transcribed RNA. In some embodiments, this is achieved by adding a proteolytic degradation tag to the DNA modifying protein. These engineering approaches can be combined to achieve the desired delayed inactivation or cleavage of the nucleic acid vector.

In some embodiments, the sequence targeted by the DNA modifying enzyme on the nucleic acid vector carries mutations reducing the DNA modifying enzyme activity and delaying the loss of the vector. In the case where the DNA modifying enzyme is a nuclease, and more particularly an RNA guided nuclease, mismatches can be introduced between the guide RNA and the target to reduce the nuclease activity and delay the loss of the nucleic acid vector. The nature and position of mismatches that reduce the activity of Cas nucleases has been well characterized in the literature, for example in Jung et al., Cell 170, 35-47, 2017, and Jones et al. , biorxiv.org/content/ 10.1101/696393v1, which are hereby incorporated by reference. Alternatively, the length of the guide RNA can be modulated to reduce the activity of the nuclease, e.g. a shorter or longer guide RNA can be used in comparison to the optimal guide RNA size. Any of these approaches can be combined to achieve the desired delayed cleavage of the nucleic acid vector.

In some embodiment, it is the transcription itself of the DNA modifying enzyme that can be delayed by engineering a genetic cascade where the transcription of the DNA modifying enzyme gene is activated by a protein or a molecule encoded via a gene constitutively expressed on the nucleic acid vector, or whose transcription can itself be controlled by another gene on the nucleic acid vector which can itself be constitutively expressed or controlled via another gene, etc.

In some embodiments, this control can be activated by the displacement, inversion or transposition of a sequence, required for the transcription, in frame with the nuclease gene itself. The displacement, inversion or transposition can be mediated via an exogenous recombinase.

In some embodiments, the nuclease is an endogenous nuclease, naturally found in the target bacteria or not, and not carried by the delivered nucleic acid vector.

Bacterial viruses (also called bacteriophages or phages) are small viruses displaying the ability to infect and kill bacteria while they do not affect cells from other organisms. Initially described almost a century ago by William Twort, and independently discovered shortly thereafter by Félix d'Herelle, more than 6000 different bacterial viruses have been discovered so far and described morphologically. The vast majority of these viruses are tailed while a small proportion are polyhedral, filamentous or pleomorphic. They may be classified according to their morphology, their genetic content (DNA vs. RNA), their specific host, the place where they live (marine virus vs. other habitats), and their life cycle. As intracellular parasites of bacterial cells, phages display different life cycles within the bacterial host: lytic, lysogenic, pseudo-lysogenic, and chronic infection. Lytic phages, once their DNA injected into their host, replicate their own genome and produce new viral particles at the expense of the host. Indeed, they cause lysis of the host bacterial cell as a normal part of the final stage of their life cycles to liberate viral particles. Temperate phages can either replicate by means of the lytic life cycle and cause lysis of the host bacterium, or they can incorporate their DNA into the host bacterial DNA and become non-infectious prophages (lysogenic cycle). In some embodiments, lytic phages are used.

Unlike classical chemically-based antibiotics that are active against a broad spectrum of bacterial species, a bacteriophage can infect and kill only a small number of different closely-related bacteria.

The use of packaged phagemids allows to have a defined and control way of killing the host. Example of packaged phagemids encoding CRISPR-Cas9 or toxins have shown promising results in killing targeted bacterial population (Bikard et al., 2012, Cell Host &Microbe 12, 177-186; Jiang et al., 2013, Nat Biotechnol31, 233-239 ; Krom et al., 2015, Nano Letters 15, 4808-4813; Bikard et al, 2014, Nat Biotech 11, Vol. 32, Citorik, R et al, 2014, Nat Biotech 11,Vol. 32).

### Sequence of interest under the control of the promoter

The vector can comprise a sequence of interest under the control of a promoter.

In one embodiment, the sequence of interest is a programmable nuclease circuit to be delivered to the targeted bacteria, in particular as defined above. This programmable nuclease circuit may be able to mediate in vivo sequence-specific elimination of bacteria that contain a target gene of interest. Some embodiments of the present disclosure relate to engineered variants of the Type II CRISPR-Cas (Clustered Regularly Interspaced Short Palindromic Repeats-CRISPR-associated) system of *Streptococcus pyogenes.* Other programmable nucleases that can be used include other CRISPR-Cas systems, engineered TALEN (Transcription Activator-Like Effector Nuclease) variants, engineered zinc finger nuclease (ZFN) variants, natural, evolved or engineered meganuclease or recombinase variants, and any combination or hybrids of programmable nucleases.

Other sequences of interest, preferably programmable, can be added to the payload/vector so as to be delivered to targeted bacteria.

Preferably, the sequence of interest circuit added to the payload/vector leads to the reduction or elimination of expression of an antibiotic resistance gene.

In a particular embodiment, the nucleic acid sequence of interest is selected from the group consisting of a sequence encoding a Cas nuclease, a guide RNA, a single guide RNA (sgRNA), a CRISPR locus, a gene encoding an enzyme such as a nuclease, a kinase, a TALEN, a ZFN, a meganuclease, a recombinase, a base editor or a prime editor. These proteins can also be modified or engineered to include extra features, like the addition or removal of a function (e.g. dCas9).

### Targeted Bacteria

The AMR bacteria targeted by a composition of the invention can be present *in vivo,* in a mammalian organism, or *in vitro,* for example in liquid or solid culture.

A microbiome may comprise a variety of endogenous AMR bacteria species, any of which may be targeted in accordance with the present disclosure. In some embodiments, the species of targeted AMR bacterial cells may depend on the type of bacteriophages being used for preparing the bacterial virus particles. For example, some bacteriophages exhibit tropism for, or preferentially target, specific host species of bacteria. Other bacteriophages do not exhibit such tropism and may be used to target a number of different genus and/or species of endogenous bacterial cells.

Examples of bacterial cells include, without limitation, cells from bacteria of the genus *Yersinia spp., Escherichia spp., Klebsiella spp., Acinetobacter spp., Bordetella spp., Neisseria spp., Aeromonas spp., Franciesella spp., Corynebacterium spp., Citrobacter spp., Chlamydia spp., Hemophilus spp., Brucella spp., Mycobacterium spp., Legionella spp., Rhodococcus spp., Pseudomonas spp., Helicobacter spp., Vibrio spp., Bacillus spp., Erysipelothrix spp., Salmonella spp., Streptomyces spp., Streptococcus spp., Staphylococcus spp., Bacteroides spp., Prevotella spp., Clostridium spp., Bifidobacterium spp., Clostridium spp., Brevibacterium spp., Lactococcus spp., Leuconostoc spp., Actinobacillus spp., Selnomonas spp., Shigella spp., Zymonas spp., Mycoplasma spp., Treponema spp., Leuconostoc spp., Corynebacterium spp., Enterococcus spp., Enterobacter spp., Pyrococcus spp., Serratia spp., Morganella spp., Parvimonas spp., Fusobacterium spp., Actinomyces spp., Porphyromonas spp., Micrococcus spp., Bartonella spp., Borrelia spp., Brucelia spp., Campylobacter spp., Chlamydophilia spp., Cutibacterium spp., Propionibacterium spp., Gardnerella spp., Ehrlichia spp., Haemophilus spp., Leptospira spp., Listeria spp., Mycoplasma spp., Nocardia spp., Rickettsia spp., Ureaplasma spp., Lactobacillus spp. and a mixture thereof.*

Thus, bacterial virus particles may target (e.g., specifically target) a bacterial cell from any one or more of the foregoing genus of bacteria to specifically deliver the plasmid according to the invention.

Preferably, the targeted bacteria can be selected from the group consisting of *Yersinia spp., Escherichia spp., Klebsiella spp., Acinetobacter spp., Pseudomonas spp., Helicobacter spp., Vibrio spp, Salmonella spp., Streptococcus spp., Staphylococcus spp., Bacteroides spp., Clostridium spp., Shigella spp., Enterococcus spp., Enterobacter spp., Listeria spp., Cutibacterium spp., Propionibacterium spp., Fusobacterium spp., Porphyromonas spp. and Gardnerella spp.*

In some embodiments, bacterial cells of the present invention are anaerobic bacterial cells (e.g., cells that do not require oxygen for growth). Anaerobic bacterial cells include facultative anaerobic cells such as but not limited to *Escherichia coli, Shewanella oneidensi, Gardnerella vaginalis* and *Listeria.* Anaerobic bacterial cells also include obligate anaerobic cells such as, for example, *Bacteroides, Clostridium, Cutibacterium, Propionibacterium, Fusobacterium and Porphyromonas* species. In humans, anaerobic bacteria are most commonly found in the gastrointestinal tract. In some particular embodiment, the targeted bacteria are thus bacteria most commonly found in the gastrointestinal tract. Bacteriophages used for preparing the bacterial virus particles, and then the bacterial virus particles, may target (e.g., to specifically target) anaerobic bacterial cells according to their specific spectra known by the person skilled in the art to specifically deliver the plasmid.

In some embodiments, the targeted bacterial cells are, without limitation, *Bacteroides faecis, Bacteroides thetaiotaomicron, Bacteroides fragilis, Bacteroides distasonis, Bacteroides vulgatus, Clostridium leptum, Clostridium coccoides, Staphylococcus aureus, Bacillus subtilis, Clostridium butyricum, Brevibacterium lactofermentum, Streptococcus agalactiae, Lactococcus lactis, Leuconostoc lactis, Actinobacillus actinobycetemcomitans, cyanobacteria, Escherichia coli, Helicobacter pylori, Selnomonas ruminatium, Shigella sonnei, Zymomonas mobilis, Mycoplasma mycoides, Treponema denticola, Bacillus thuringiensis, Staphilococcus lugdunensis, Leuconostoc oenos, Corynebacterium xerosis, Lactobacillus plantarum, Lactobacillus rhamnosus, Lactobacillus casei, Lactobacillus acidophilus, Enterococcus faecalis, Bacillus coagulans, Bacillus cereus, Bacillus popillae, Synechocystis strain PCC6803, Bacillus liquefaciens, Pyrococcus abyssi, Selenomonas nominantium, Lactobacillus hilgardii, Streptococcus ferus, Lactobacillus pentosus, Bacteroides fragilis, Staphylococcus epidermidis, Streptomyces phaechromogenes, Streptomyces ghanaenis, Klebsiella pneumoniae, Enterobacter cloacae, Enterobacter aerogenes, Serratia marcescens, Morganella morganii, Citrobacter freundii, Propionibacterium freudenreichii, Pseudomonas aerigunosa, Parvimonas micra, Prevotella intermedia, Fusobacterium nucleatum, Prevotella nigrescens, Actinomyces israelii, Porphyromonas endodontalis, Porphyromonas gingivalis Micrococcus luteus, Bacillus megaterium, Aeromonas hydrophila, Aeromonas caviae, Bacillus anthracis, Bartonella henselae, Bartonella Quintana, Bordetella pertussis, Borrelia burgdorferi, Borrelia garinii, Borrelia afzelii, Borrelia recurrentis, Brucella abortus, Brucella canis, Brucella melitensis, Brucella suis, Campylobacter jejuni, Campylobacter coli, Campylobacter fetus, Chlamydia pneumoniae, Chlamydia trachomatis, Chlamydophila psittaci, Clostridium botulinum, Clostridium difficile, Clostridium perfringens, Clostridium tetani, Corynebacterium diphtheria, Cutibacterium acnes (formerly Propionibacterium acnes), Ehrlichia canis, Ehrlichia chaffeensis, Enterococcus faecium, Francisella tularensis, Haemophilus influenza, Legionella pneumophila, Leptospira interrogans, Leptospira santarosai, Leptospira weilii, Leptospira noguchii, Listeria monocytogenes, Mycobacterium leprae, Mycobacterium tuberculosis, Mycobacterium ulcerans, Mycoplasma pneumonia, Neisseria gonorrhoeae, Neisseria meningitides, Nocardia asteroids, Rickettsia rickettsia, Salmonella enteritidis, Salmonella typhi, Salmonella paratyphi, Salmonella typhimurium, Shigella flexnerii, Shigella dysenteriae, Staphylococcus saprophyticus, Streptococcus pneumoniae, Streptococcus pyogenes, Gardnerella vaginalis, Streptococcus viridans, Treponema pallidum, Ureaplasma urealyticum, Vibrio cholera, Vibrio parahaemolyticus, Yersinia pestis, Yersinia enterocolitica, Yersinia pseudotuberculosis, Actinobacter baumanii, Pseudomonas aerigunosa, and a mixture thereof, preferably the bacteria of interest are selected from the group consisting of Escherichia coli, Enterococcus faecium, Staphylococcus aureus, Klebsiella pneumoniae, Acinetobacter baumanii, Pseudomonas aeruginosa, Enterobacter cloacae, and Enterobacter aerogenes, and a mixture thereof.*

In some embodiments, the targeted bacterial cells are, without limitation, *Anaerotruncus, Acetanaerobacterium, Acetitomaculum, Acetivibrio, Anaerococcus, Anaerofilum, Anaerosinus, Anaerostipes, Anaerovorax, Butyrivibrio, Clostridium, Capracoccus, Dehalobacter, Dialister, Dorea, Enterococcus, Ethanoligenens, Faecalibacterium, Fusobacterium, Gracilibacter, Guggenheimella, Hespellia, Lachnobacterium, Lachnospira, Lactobacillus, Leuconostoc, Megamonas, Moryella, Mitsuokella, Oribacterium, Oxobacter, Papillibacter, Proprionispira, Pseudobutyrivibrio, Pseudoramibacter, Roseburia, Ruminococcus, Sarcina, Seinonella, Shuttleworthia, Sporobacter, Sporobacterium, Streptococcus, Subdoligranulum, Syntrophococcus, Thermobacillus, Turibacter, Weisella, Clostridium, Bacteroides, Ruminococcus, Faecalibacterium, Treponema, Phascolarctobacterium, Megasphaera, Faecalibacterium, Bifidobacterium, Lactobacillus, Sutterella, and*/*or Prevotella.*

*In* other *embodiments, the targeted bacteria cells are, without limitation, Achromobacter xylosoxidans, Acidaminococcus fermentans, Acidaminococcus intestini, Acidaminococcus sp., Acinetobacter baumannii, Acinetobacter junii, Acinetobacter Iwoffii, Actinobacillus capsulatus, Actinomyces naeslundii, Actinomyces neuii, Actinomyces odontolyticus, Actinomyces radingae, Adlercreutzia equolifaciens, Aeromicrobium massiliense, Aggregatibacter actinomycetemcomitans, Akkermansia muciniphila, Aliagarivorans marinus, Alistipes finegoldii, Alistipes indistinctus, Alistipes inops, Alistipes onderdonkii, Alistipes putredinis, Alistipes senegalensis, Alistipes shahii, Alistipes timonensis, Alloscardovia omnicolens, Anaerobacter polyendosporus, Anaerobaculum hydrogeniformans, Anaerococcus hydrogenalis, Anaerococcus prevotii, Anaerococcus senegalensis, Anaerofustis stercorihominis, Anaerostipes caccae, Anaerostipes hadrus, Anaerotruncus colihominis, Aneurinibacillus aneurinilyticus, Bacillus licheniformis, Bacillus massilioanorexius, Bacillus massiliosenegalensis, Bacillus simplex, Bacillus smithii, Bacillus subtilis, Bacillus thuringiensis, Bacillus timonensis, Bacteriodes xylanisolvens, Bacteroides acidifaciens, Bacteroides caccae, Bacteroides capillosus, Bacteroides cellulosilyticus, Bacteroides clarus, Bacteroides coprocola, Bacteroides coprophilus, Bacteroides dorei, Bacteroides eggerthii, Bacteroides faecis, Bacteroides finegoldii, Bacteroides fluxus, Bacteroides fragilis, Bacteroides gallinarum, Bacteroides intestinalis, Bacteroides nordii, Bacteroides oleiciplenus, Bacteroides ovatus, Bacteroides pectinophilus, Bacteroides plebeius, Bacteroides salanitronis, Bacteroides salyersiae, Bacteroides sp., Bacteroides stercoris, Bacteroides thetaiotaomicron, Bacteroides uniformis, Bacteroides vulgatus, Bacteroides xylanisolvens, Bacteroidespectinophilus ATCC, Barnesiella intestinihominis, Bavariicoccus seileri, Bifidobacterium adolescentis, Bifidobacterium angulatum, Bifidobacterium animalis, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Bifidobacterium stercoris, Bilophila wadsworthia, Blautia faecis, Blautia hansenii, Blautia hydrogenotrophica, Blautia luti, Blautia obeum, Blautia producta, Blautia wexlerae, Brachymonas chironomi, Brevibacterium senegalense, Bryantella formatexigens, butyrate-producing bacterium, Butyricicoccus pullicaecorum, Butyricimonas virosa, Butyrivibrio crossotus, Butyrivibrio fibrisolvens, Caldicoprobacter faecalis, Campylobacter concisus, Campylobacter jejuni, Campylobacter upsaliensis, Catenibacterium mitsuokai, Cedecea davisae, Cellulomonas massiliensis, Cetobacterium somerae, Citrobacter braakii, Citrobacter freundii, Citrobacter pasteurii, Citrobacter sp., Citrobacter youngae, Cloacibacillus evryensis, Clostridiales bacterium, Clostridioides difficile, Clostridium asparagiforme, Clostridium bartlettii, Clostridium boliviensis, Clostridium bolteae, Clostridium hathewayi, Clostridium hiranonis, Clostridium hylemonae, Clostridium leptum, Clostridium methylpentosum, Clostridium nexile, Clostridium orbiscindens, Clostridium ramosum, Clostridium scindens, Clostridium sp, Clostridium sp., Clostridium spiroforme, Clostridium sporogenes, Clostridium symbiosum, Collinsella aerofaciens, Collinsella intestinalis, Collinsella stercoris, Collinsella tanakaei, Coprobacillus cateniformis, Coprobacter fastidiosus, Coprococcus catus, Coprococcus comes, Coprococcus eutactus, Corynebacterium ammoniagenes, Corynebacterium amycolatum, Corynebacterium pseudodiphtheriticum, Cutibacterium acnes, Dermabacter hominis, Desulfitobacterium hafniense, Desulfovibrio fairfieldensis, Desulfovibrio piger, Dialister succinatiphilus, Dielma fastidiosa, Dorea formicigenerans, Dorea longicatena, Dysgonomonas capnocytophagoides, Dysgonomonas gadei, Dysgonomonas mossii, Edwardsiella tarda, Eggerthella lenta, Eisenbergiella tayi, Enorma massiliensis, Enterobacter aerogenes, Enterobacter asburiae, Enterobacter cancerogenus, Enterobacter cloacae, Enterobacter massiliensis, Enterococcus casseliflavus, Enterococcus durans, Enterococcus faecalis, Enterococcus faecium, Enterococcus flavescens, Enterococcus gallinarum, Enterococcus sp., Enterovibrio nigricans, Erysipelatoclostridium ramosum, Escherichia coli, Escherichia sp., Eubacterium biforme, Eubacterium dolichum, Eubacterium hallii, Eubacterium limosum, Eubacterium ramulus, Eubacterium rectale, Eubacterium siraeum, Eubacterium ventriosum, Exiguobacterium marinum, Exiguobacterium undae, Faecalibacterium cf, Faecalibacterium prausnitzii, Faecalitalea cylindroides, Ferrimonas balearica, Finegoldia magna, Flavobacterium daejeonense, Flavonifractor plautii, Fusicatenibacter saccharivorans, Fusobacterium gonidiaformans, Fusobacterium mortiferum, Fusobacterium necrophorum, Fusobacterium nucleatum, Fusobacterium periodonticum, Fusobacterium sp., Fusobacterium ulcerans, Fusobacterium varium, Gallibacterium anatis, Gemmiger formicilis, Gordonibacter pamelaeae, Hafnia alvei, Helicobacter bilis, Helicobacter bills, Helicobacter canadensis, Helicobacter canis, Helicobacter cinaedi, Helicobacter macacae, Helicobacter pametensis, Helicobacter pullorum, Helicobacter pylori, Helicobacter rodentium, Helicobacter winghamensis, Herbaspirillum massiliense, Holdemanella biformis, Holdemania fdiformis, Holdemania filiformis, Holdemania massiliensis, Holdemaniafiliformis, Hungatella hathewayi, Intestinibacter bartlettii, Intestinimonas butyriciproducens, Klebsiella oxytoca, Klebsiella pneumoniae, Kurthia massiliensis, Lachnospira pectinoschiza, Lactobacillus acidophilus, Lactobacillus amylolyticus, Lactobacillus animalis, Lactobacillus antri, Lactobacillus brevis, Lactobacillus buchneri, Lactobacillus casei, Lactobacillus curvatus, Lactobacillus delbrueckii, Lactobacillus fermentum, Lactobacillus gasseri, Lactobacillus helveticus, Lactobacillus hilgardii, Lactobacillus iners, Lactobacillus intestinalis, Lactobacillus johnsonii, Lactobacillus murinus, Lactobacillus paracasei, Lactobacillus plantarum, Lactobacillus reuteri, Lactobacillus rhamnosus, Lactobacillus ruminis, Lactobacillus sakei, Lactobacillus salivarius, Lactobacillus ultunensis, Lactobacillus vaginalis, Lactobacillusplantarum subsp., Leuconostoc mesenteroides, Leuconostoc pseudomesenteroides, Listeria grayi, Listeria innocua, Mannheimia granulomatis, Marvinbryantia formatexigens, Megamonas funiformis, Megamonas hypermegale, Methanobrevibacter smithii, Methanobrevibacter smithiiFI, Micrococcus luteus, Microvirgula aerodenitrificans, Mitsuokella jalaludinii, Mitsuokella multacida, Mollicutes bacterium, Murimonas intestini, Neisseria macacae, Nitriliruptor alkaliphilus, Oceanobacillus massiliensis, Odoribacter laneus, Odoribacter splanchnicus, Ornithobacterium rhinotracheale, Oxalobacter formigenes, Paenibacillus barengoltzii, Paenibacillus chitinolyticus, Paenibacillus lautus, Paenibacillus motobuensis, Paenibacillus senegalensis, Paenisporosarcina quisquiliarum, Parabacteroides distasonis, Parabacteroides goldsteinii, Parabacteroides gordonii, Parabacteroides johnsonii, Parabacteroides merdae, Paraprevotella xylaniphila, Parasutterella excrementihominis, Parvimonas micra, Pediococcus acidilactici, Peptoclostridium difficile, Peptoniphilus harei, Peptoniphilus obesi, Peptoniphilus senegalensis, Peptoniphilus timonensis, Phascolarctobacterium succinatutens, Porphyromonas asaccharolytica, Porphyromonas uenonis, Prevotella baroniae, Prevotella bivia, Prevotella copri, Prevotella dentalis, Prevotella micans, Prevotella multisaccharivorax, Prevotella oralis, Prevotella salivae, Prevotella stercorea, Prevotella veroralis, Propionibacterium acnes, Propionibacterium avidum, Propionibacterium freudenreichii, Propionimicrobium lymphophilum, Proteus mirabilis, Proteuspenneri ATCC, Providencia alcalifaciens, Providencia rettgeri, Providencia rustigianii, Providencia stuartii, Pseudoflavonifractor capillosus, Pseudomonas aeruginosa, Pseudomonas luteola, Ralstonia pickettii, Rheinheimera perlucida, Rheinheimera texasensis, Riemerella columbina, Romboutsia lituseburensis, Roseburia faecis, Roseburia intestinalis, Roseburia inulinivorans, Ruminococcus bicirculans, Ruminococcus bromii, Ruminococcus callidus, Ruminococcus champanellensis, Ruminococcus faecis, Ruminococcus gnavus, Ruminococcus lactaris, Ruminococcus obeum, Ruminococcus sp, Ruminococcus sp., Ruminococcus torques, Sarcina ventriculi, Sellimonas intestinalis, Senegalimassilia anaerobia, Shigella sonnei, Slackia piriformis, Staphylococcus epidermidis, Staphylococcus lentus, Staphylococcus nepalensis, Staphylococcus pseudintermedius, Staphylococcus xylosus, Stenotrophomonas maltophilia, Streptococcus agalactiae, Streptococcus anginosus, Streptococcus australis, Streptococcus caballi, Streptococcus castoreus, Streptococcus didelphis, Streptococcus equinus, Streptococcus gordonii, Streptococcus henryi, Streptococcus hyovaginalis, Streptococcus infantarius, Streptococcus infantis, Streptococcus lutetiensis, Streptococcus merionis, Streptococcus mitis, Streptococcus mutans, Streptococcus oralis, Streptococcus ovis, Streptococcus parasanguinis, Streptococcus plurextorum, Streptococcus porci, Streptococcus pyogenes, Streptococcus salivarius, Streptococcus sobrinus, Streptococcus thermophilus, Streptococcus thoraltensis, Streptomyces albus, Subdoligranulum variabile, Succinatimonas hippei, Sutterella parvirubra, Sutterella wadsworthensis, Terrisporobacter glycolicus, Terrisporobacter mayombei, Thalassobacillus devorans, Timonella senegalensis, Turicibacter sanguinis, unknown sp, unknown sp., Varibaculum cambriense, Veillonella atypica, Veillonella dispar, Veillonella parvula, Vibrio cincinnatiensis, Virgibacillus salexigens, Weissella paramesenteroides, Weissellaparamesenteroides ATCC,*

In other embodiments, the targeted bacteria cells are those commonly found on the skin microbiota and are without limitation *Acetobacter farinalis, Acetobacter malorum, Acetobacter orleanensis, Acetobacter sicerae, Achromobacter anxifer, Achromobacter denitrificans, Achromobacter marplatensis, Achromobacter spanius, Achromobacter xylosoxidans subsp. xylosoxidans, Acidovorax konjaci, Acidovorax radicis, Acinetobacter johnsonii, Actinomadura citrea, Actinomadura coerulea, Actinomadura fibrosa, Actinomadura fulvescens, Actinomadura jiaoheensis, Actinomadura luteofluorescens, Actinomadura mexicana, Actinomadura nitritigenes, Actinomadura verrucosospora, Actinomadura yumaensis, Actinomyces odontolyticus, Actinomycetospora atypica, Actinomycetospora corticicola, Actinomycetospora rhizophila, Actinomycetospora rishiriensis, Aeromonas australiensis, Aeromonas bestiarum, Aeromonas bivalvium, Aeromonas encheleia, Aeromonas eucrenophila, Aeromonas hydrophila subsp. hydrophila, Aeromonas piscicola, Aeromonas popoffii, Aeromonas rivuli, Aeromonas salmonicida subsp. pectinolytica, Aeromonas salmonicida subsp. smithia, Amaricoccus kaplicensis, Amaricoccus veronensis, Aminobacter aganoensis, Aminobacter ciceronei, Aminobacter lissarensis, Aminobacter niigataensis, Ancylobacter polymorphus, Anoxybacillus flavithermus subsp. yunnanensis, Aquamicrobium aerolatum, Archangium gephyra, Archangium gephyra, Archangium minus, Archangium violaceum, Arthrobacter viscosus, Bacillus anthracis, Bacillus australimaris, Bacillus drentensis, Bacillus mycoides, Bacillus pseudomycoides, Bacillus pumilus, Bacillus safensis, Bacillus vallismortis, Bosea thiooxidans, Bradyrhizobium huanghuaihaiense, Bradyrhizobium japonicum, Brevundimonas aurantiaca, Brevundimonas intermedia, Burkholderia aspalathi, Burkholderia choica, Burkholderia cordobensis, Burkholderia diffusa, Burkholderia insulsa, Burkholderia rhynchosiae, Burkholderia terrestris, Burkholderia udeis, Buttiauxella gaviniae, Caenimonas terrae, Capnocytophaga gingivalis, Chitinophaga dinghuensis, Chryseobacterium gleum, Chryseobacterium greenlandense, Chryseobacterium jejuense, Chryseobacterium piscium, Chryseobacterium sediminis, Chryseobacterium tructae, Chryseobacterium ureilyticum, Chryseobacterium vietnamense, Corynebacterium accolens, Corynebacterium afermentans subsp. lipophilum, Corynebacterium minutissimum, Corynebacterium sundsvallense, Cupriavidus metallidurans, Cupriavidus nantongensis, Cupriavidus necator, Cupriavidus pampae, Cupriavidus yeoncheonensis, Curtobacterium flaccumfaciens, Devosia epidermidihirudinis, Devosia riboflavina, Devosia riboflavina, Diaphorobacter oryzae, Dietzia psychralcaliphila, Ensifer adhaerens, Ensifer americanus, Enterococcus malodoratus, Enterococcus pseudoavium, Enterococcus viikkiensis, Enterococcus xiangfangensis, Erwinia rhapontici, Falsirhodobacter halotolerans, Flavobacterium araucananum, Flavobacterium frigidimaris, Gluconobacter frateurii, Gluconobacter thailandicus, Gordonia alkanivorans, Halomonas aquamarina, Halomonas axialensis, Halomonas meridiana, Halomonas olivaria, Halomonas songnenensis, Halomonas variabilis, Herbaspirillum chlorophenolicum, Herbaspirillum frisingense, Herbaspirillum hiltneri, Herbaspirillum huttiense subsp. putei, Herbaspirillum lusitanum, Herminiimonas fonticola, Hydrogenophaga intermedia, Hydrogenophaga pseudoflava, Klebsiella oxytoca, Kosakonia sacchari, Lactobacillus delbrueckii subsp. bulgaricus, Lactobacillus modestisalitolerans, Lactobacillus plantarum subsp. argentoratensis, Lactobacillus xiangfangensis, Lechevalieria roselyniae, Lentzea albida, Lentzea californiensis, Leuconostoc carnosum, Leuconostoc citreum, Leuconostoc gelidum subsp. gasicomitatum, Leuconostoc mesenteroides subsp. suionicum, Luteimonas aestuarii, Lysobacter antibioticus, Lysobacter koreensis, Lysobacter oryzae, Magnetospirillum moscoviense, Marinomonas alcarazii, Marinomonas primoryensis, Massilia aurea, Massilia jejuensis, Massilia kyonggiensis, Massilia timonae, Mesorhizobium acaciae, Mesorhizobium qingshengii, Mesorhizobium shonense, Methylobacterium haplocladii, Methylobacterium platani, Methylobacterium pseudosasicola, Methylobacterium zatmanii, Microbacterium oxydan, Micromonospora chaiyaphumensis, Micromonospora chalcea, Micromonospora citrea, Micromonospora coxensis, Micromonospora echinofusca, Micromonospora halophytica, Micromonospora kangleipakensis, Micromonospora maritima, Micromonospora nigra, Micromonospora purpureochromogene, Micromonospora rhizosphaerae, Micromonospora saelicesensis, Microvirga subterranea, Microvirga zambiensis, Mycobacterium alvei, Mycobacterium avium subsp. silvaticum, Mycobacterium colombiense, Mycobacterium conceptionense, Mycobacterium conceptionense, Mycobacterium farcinogenes, Mycobacterium fortuitum subsp. fortuitum, Mycobacterium goodii, Mycobacterium insubricum, Mycobacterium Ilatzerense, Mycobacterium neoaurum, Mycobacterium neworleansense, Mycobacterium obuense, Mycobacterium peregrinum, Mycobacterium saopaulense, Mycobacterium septicum, Mycobacterium setense, Mycobacterium smegmatis, Neisseria subflava, Nocardia lijiangensis, Nocardia thailandica, Novosphingobium barchaimii, Novosphingobium lindaniclasticum, Novosphingobium lindaniclasticum, Novosphingobium mathurense, Ochrobactrum pseudogrignonense, Oxalicibacterium solurbis, Paraburkholderia glathei, Paraburkholderia humi, Paraburkholderia phenazinium, Paraburkholderia phytofirmans, Paraburkholderia sordidicola, Paraburkholderia terricola, Paraburkholderia xenovorans, Paracoccus laeviglucosivorans, Patulibacter ginsengiterrae, Polymorphospora rubra, Porphyrobacter colymbi, Prevotella jejuni, Prevotella melaninogenica, Propionibacterium acnes subsp. elongatum, Proteus vulgaris, Providencia rustigianii, Pseudoalteromonas agarivorans, Pseudoalteromonas atlantica, Pseudoalteromonas paragorgicola, Pseudomonas asplenii, Pseudomonas asuensis, Pseudomonas benzenivorans, Pseudomonas cannabina, Pseudomonas cissicola, Pseudomonas congelans, Pseudomonas costantinii, Pseudomonas ficuserectae, Pseudomonas frederiksbergensis, Pseudomonas graminis, Pseudomonas jessenii, Pseudomonas koreensis, Pseudomonas koreensis, Pseudomonas kunmingensis, Pseudomonas marginalis, Pseudomonas mucidolens, Pseudomonas panacis, Pseudomonas plecoglossicida, Pseudomonas poae, Pseudomonas pseudoalcaligenes, Pseudomonas putida, Pseudomonas reinekei, Pseudomonas rhizosphaerae, Pseudomonas seleniipraecipitans, Pseudomonas umsongensis, Pseudomonas zhaodongensis, Pseudonocardia alaniniphila, Pseudonocardia ammonioxydans, Pseudonocardia autotrophica, Pseudonocardia kongjuensis, Pseudonocardia yunnanensis, Pseudorhodoferax soli, Pseudoxanthomonas daejeonensis, Pseudoxanthomonas indica, Pseudoxanthomonas kaohsiungensis, Psychrobacter aquaticus, Psychrobacter arcticus, Psychrobacter celer, Psychrobacter marincola, Psychrobacter nivimaris, Psychrobacter okhotskensis, Psychrobacter okhotskensis, Psychrobacter piscatorii, Psychrobacter pulmonis, Ramlibacter ginsenosidimutans, Rheinheimera japonica, Rheinheimera muenzenbergensis, Rheinheimera soli, Rheinheimera tangshanensis, Rheinheimera texasensis, Rheinheimera tilapiae, Rhizobium alamii, Rhizobium azibense, Rhizobium binae, Rhizobium daejeonense, Rhizobium endophyticum, Rhizobium etli, Rhizobium fabae, Rhizobium freirei, Rhizobium gallicum, Rhizobium loessense, Rhizobium sophoriradicis, Rhizobium taibaishanense, Rhizobium vallis, Rhizobium vignae, Rhizobium vignae, Rhizobium yanglingense, Rhodococcus baikonurensis, Rhodococcus enclensis, Rhodoferax saidenbachensis, Rickettsia canadensis, Rickettsia heilongjiangensis, Rickettsia honei, Rickettsia raoultii, Roseateles aquatilis, Roseateles aquatilis, Salmonella enterica subsp. salamae, Serratia ficaria, Serratia myotis, Serratia vespertilionis, Shewanella aestuarii, Shewanella decolorationis, Sphingobium amiense, Sphingobium baderi, Sphingobium barthaii, Sphingobium chlorophenolicum, Sphingobium cupriresistens, Sphingobium czechense, Sphingobium fuliginis, Sphingobium indicum, Sphingobium indicum, Sphingobium japonicum, Sphingobium lactosutens, Sphingomonas dokdonensis, Sphingomonas pseudosanguinis, Sphingopyxis chilensis, Sphingopyxis fribergensis, Sphingopyxis granuli, Sphingopyxis indica, Sphingopyxis witflariensis, Staphylococcus agnetis, Staphylococcus aureus subsp. aureus, Staphylococcus epidermidis, Staphylococcus hominis subsp. novobiosepticus, Staphylococcus nepalensis, Staphylococcus saprophyticus subsp. bovis, Staphylococcus sciuri subsp. carnaticus, Streptomyces caeruleatus, Streptomyces canarius, Streptomyces capoamus, Streptomyces ciscaucasicus, Streptomyces griseorubiginosus, Streptomyces olivaceoviridis, Streptomyces panaciradicis, Streptomyces phaeopurpureus, Streptomyces pseudovenezuelae, Streptomyces resistomycificus, Tianweitania sediminis, Tsukamurella paurometabola, Variovorax guangxiensis, Vogesella alkaliphila, Xanthomonas arboricola, Xanthomonas axonopodis, Xanthomonas cassavae, Xanthomonas cucurbitae, Xanthomonas cynarae, Xanthomonas euvesicatoria, Xanthomonas fragariae, Xanthomonas gardneri, Xanthomonas perforans, Xanthomonas pisi, Xanthomonas populi, Xanthomonas vasicola, Xenophilus aerolatus, Yersinia nurmii, Abiotrophia defectiva, Acidocella aminolytica, Acinetobacter guangdongensis, Acinetobacter parvus, Acinetobacter radioresistens, Acinetobacter soli, Acinetobacter variabilis, Actinomyces cardiffensis, Actinomyces dentalis, Actinomyces europaeus, Actinomyces gerencseriae, Actinomyces graevenitzii, Actinomyces haliotis, Actinomyces johnsonii, Actinomyces massiliensis, Actinomyces meyeri, Actinomyces meyeri, Actinomyces naeslundii, Actinomyces neuii subsp. anitratus, Actinomyces odontolyticus, Actinomyces oris, Actinomyces turicensis, Actinomycetospora corticicola, Actinotignum schaalii, Aerococcus christensenii, Aerococcus urinae, Aeromicrobium flavum, Aeromicrobium massiliense, Aeromicrobium tamlense, Aeromonas sharmana, Aggregatibacter aphrophilus, Aggregatibacter segnis, Agrococcus baldri, Albibacter methylovorans, Alcaligenes faecalis subsp. faecalis, Algoriphagus ratkowskyi, Alkalibacterium olivapovliticus, Alkalibacterium pelagium, Alkalibacterium pelagium, Alloprevotella rava, Alsobacter metallidurans, Amaricoccus kaplicensis, Amaricoccus veronensis, Anaerococcus hydrogenalis, Anaerococcus lactolyticus, Anaerococcus murdochii, Anaerococcus octavius, Anaerococcus prevotii, Anaerococcus vaginalis, Aquabacterium citratiphilum, Aquabacterium olei, Aquabacterium olei, Aquabacterium parvum, Aquincola tertiaricarbonis, Arcobacter venerupis, Arsenicicoccus bolidensis, Arthrobacter russicus, Asticcacaulis excentricus, Atopobium deltae, Atopobium parvulum, Atopobium rimae, Atopobium vaginae, Aureimonas altamirensis, Aureimonas rubiginis, Azospira oryzae, Azospirillum oryzae, Bacillus circulans, Bacillus drentensis, Bacillus fastidiosus, Bacillus lehensis, Bacillus oceanisediminis, Bacillus rhizosphaerae, Bacteriovorax stolpii, Bacteroides coagulans, Bacteroides dorei, Bacteroides fragilis, Bacteroides ovatus, Bacteroides stercoris, Bacteroides uniformis, Bacteroides vulgatus, Bdellovibrio bacteriovorus, Bdellovibrio exovorus, Belnapia moabensis, Belnapia soli, Blautia hansenii, Blautia obeum, Blautia wexlerae, Bosea lathyri, Brachybacterium fresconis, Brachybacterium muris, Brevibacterium ammoniilyticum, Brevibacterium casei, Brevibacterium epidermidis, Brevibacterium iodinum, Brevibacterium luteolum, Brevibacterium paucivorans, Brevibacterium pityocampae, Brevibacterium sanguinis, Brevundimonas albigilva, Brevundimonas diminuta, Brevundimonas vancanneytii, Caenimonas terrae, Calidifontibacter indicus, Campylobacter concisus, Campylobacter gracilis, Campylobacter hominis, Campylobacter rectus, Campylobacter showae, Campylobacter ureolyticus, Capnocytophaga gingivalis, Capnocytophaga leadbetteri, Capnocytophaga ochracea, Capnocytophaga sputigena, Cardiobacterium hominis, Cardiobacterium valvarum, Carnobacterium divergens, Catonella morbi, Caulobacter henricii, Cavicella subterranea, Cellulomonas xylanilytica, Cellvibrio vulgaris, Chitinimonas taiwanensis, Chryseobacterium arachidis, Chryseobacterium daecheongense, Chryseobacterium formosense, Chryseobacterium formosense, Chryseobacterium greenlandense, Chryseobacterium indologenes, Chryseobacterium piscium, Chryseobacterium rigui, Chryseobacterium solani, Chryseobacterium taklimakanense, Chryseobacterium ureilyticum, Chryseobacterium ureilyticum, Chryseobacterium zeae, Chryseomicrobium aureum, Cloacibacterium haliotis, Cloacibacterium normanense, Cloacibacterium normanense, Collinsella aerofaciens, Comamonas denitrificans, Comamonas terrigena, Corynebacterium accolens, Corynebacterium afermentans subsp. lipophilum, Corynebacterium ammoniagenes, Corynebacterium amycolatum, Corynebacterium aurimucosum, Corynebacterium aurimucosum, Corynebacterium coyleae, Corynebacterium durum, Corynebacterium freiburgense, Corynebacterium glaucum, Corynebacterium glyciniphilum, Corynebacterium imitans, Corynebacterium jeikeium, Corynebacterium jeikeium, Corynebacterium kroppenstedtii, Corynebacterium lipophiloflavum, Corynebacterium massiliense, Corynebacterium mastitidis, Corynebacterium matruchotii, Corynebacterium minutissimum, Corynebacterium mucifaciens, Corynebacterium mustelae, Corynebacterium mycetoides, Corynebacterium pyruviciproducens, Corynebacterium simulans, Corynebacterium singulare, Corynebacterium sputi, Corynebacterium suicordis, Corynebacterium tuberculostearicum, Corynebacterium tuberculostearicum, Corynebacterium ureicelerivorans, Corynebacterium variabile, Couchioplanes caeruleus subsp. caeruleus, Cupriavidus metallidurans, Curtobacterium herbarum, Dechloromonas agitata, Deinococcus actinosclerus, Deinococcus antarcticus, Deinococcus caeni, Deinococcus ficus, Deinococcus geothermalis, Deinococcus radiodurans, Deinococcus wulumuqiensis, Deinococcus xinjiangensis, Dermabacter hominis, Dermabacter vaginalis, Dermacoccus nishinomiyaensis, Desemzia incerta, Desertibacter roseus, Dialister invisus, Dialister micraerophilus, Dialister propionicifaciens, Dietzia aurantiaca, Dietzia cercidiphylli, Dietzia timorensis, Dietzia timorensis, Dokdonella koreensis, Dokdonella koreensis, Dolosigranulum pigrum, Eikenella corrodens, Elizabethkingia miricola, Elstera litoralis, Empedobacter brevis, Enhydrobacter aerosaccus, Enterobacter xiangfangensis, Enterococcus aquimarinus, Enterococcus faecalis, Enterococcus olivae, Erwinia rhapontici, Eubacterium eligens, Eubacterium infirmum, Eubacterium rectale, Eubacterium saphenum, Eubacterium sulci, Exiguobacterium mexicanum, Facklamia tabacinasalis, Falsirhodobacter halotolerans, Finegoldia magna, Flavobacterium cutihirudinis, Flavobacterium lindanitolerans, Flavobacterium resistens, Friedmanniella capsulata, Fusobacterium nucleatum subsp. polymorphum, Gemella haemolysans, Gemella morbillorum, Gemella palaticanis, Gemella sanguinis, Gemmobacter aquaticus, Gemmobacter caeni, Gordonia jinhuaensis, Gordonia kroppenstedtii, Gordonia polyisoprenivorans, Gordonia polyisoprenivorans, Granulicatella adiacens, Granulicatella elegans, Haemophilus parainfluenzae, Haemophilus sputorum, Halomonas sulfidaeris, Herpetosiphon aurantiacus, Hydrocarboniphaga effusa, Idiomarina maris, Janibacter anophelis, Janibacter hoylei, Janibacter indicus, Janibacter limosus, Janibacter melonis, Jeotgalicoccus halophilus, Jonquetella anthropi, Kaistia geumhonensis, Kingella denitrificans, Kingella oralis, Klebsiella oxytoca, Knoellia aerolata, Knoellia locipacati, Kocuria atrinae, Kocuria carniphila, Kocuria kristinae, Kocuria palustris, Kocuria turfanensis, Lachnoanaerobaculum saburreum, Lachnoanaerobaculum saburreum, Lactobacillus crispatus, Lactobacillus iners, Lactococcus lactis subsp. lactis, Lactococcus lactis subsp. lactis, Lactococcus piscium, Lapillicoccus jejuensis, Lautropia mirabilis, Legionella beliardensis, Leptotrichia buccalis, Leptotrichia goodfellowii, Leptotrichia hofstadii, Leptotrichia hongkongensis, Leptotrichia shahii, Leptotrichia trevisanii, Leptotrichia wadei, Luteimonas terricola, Lysinibacillus fusiformis, Lysobacter spongiicola, Lysobacter xinjiangensis, Macrococcus caseolyticus, Marmoricola pocheonensis, Marmoricola scoriae, Massilia alkalitolerans, Massilia alkalitolerans, Massilia aurea, Massilia plicata, Massilia timonae, Megamonas rupellensis, Meiothermus silvanus, Methylobacterium dankookense, Methylobacterium goesingense, Methylobacterium goesingense, Methylobacterium isbiliense, Methylobacterium jeotgali, Methylobacterium oxalidis, Methylobacterium platani, Methylobacterium pseudosasicola, Methyloversatilis universalis, Microbacterium foliorum, Microbacterium hydrothermale, Microbacterium hydrothermale, Microbacterium lacticum, Microbacterium lacticum, Microbacterium laevaniformans, Microbacterium paludicola, Microbacterium petrolearium, Microbacterium phyllosphaerae, Microbacterium resistens, Micrococcus antarcticus, Micrococcus cohnii, Micrococcus flavus, Micrococcus lylae, Micrococcus terreus, Microlunatus aurantiacus, Micropruina glycogenica, Microvirga aerilata, Microvirga aerilata, Microvirga subterranea, Microvirga vignae, Microvirga zambiensis, Microvirgula aerodenitrificans, Mogibacterium timidum, Moraxella atlantae, Moraxella catarrhalis, Morganella morganii subsp. morganii, Morganella psychrotolerans, Murdochiella asaccharolytica, Mycobacterium asiaticum, Mycobacterium chubuense, Mycobacterium crocinum, Mycobacterium gadium, Mycobacterium holsaticum, Mycobacterium iranicum, Mycobacterium longobardum, Mycobacterium neoaurum, Mycobacterium neoaurum, Mycobacterium obuense, Negativicoccus succinicivorans, Neisseria bacilliformis, Neisseria oralis, Neisseria sicca, Neisseria subflava, Nesterenkonia lacusekhoensis, Nesterenkonia rhizosphaerae, Nevskia persephonica, Nevskia ramosa, Niabella yanshanensis, Niveibacterium umoris, Nocardia niwae, Nocardia thailandica, Nocardioides agariphilus, Nocardioides dilutus, Nocardioides ganghwensis, Nocardioides hwasunensis, Nocardioides nanhaiensis, Nocardioides sediminis, Nosocomiicoccus ampullae, Noviherbaspirillum malthae, Novosphingobium lindaniclasticum, Novosphingobium rosa, Ochrobactrum rhizosphaerae, Olsenella uli, Ornithinimicrobium murale, Ornithinimicrobium tianjinense, Oryzobacter terrae, Ottowia beijingensis, Paenalcaligenes suwonensis, Paenibacillus agaridevorans, Paenibacillus phoenicis, Paenibacillus xylanexedens, Paludibacterium yongneupense, Pantoea cypripedii, Parabacteroides distasonis, Paraburkholderia andropogonis, Paracoccus alcaliphilus, Paracoccus angustae, Paracoccus kocurii, Paracoccus laeviglucosivorans, Paracoccus sediminis, Paracoccus sphaerophysae, Paracoccus yeei, Parvimonas micra, Parviterribacter multiflagellatus, Patulibacter ginsengiterrae, Pedobacter aquatilis, Pedobacter ginsengisoli, Pedobacter xixiisoli, Peptococcus niger, Peptoniphilus coxii, Peptoniphilus gorbachii, Peptoniphilus harei, Peptoniphilus koenoeneniae, Peptoniphilus lacrimalis, Peptostreptococcus anaerobius, Peptostreptococcus stomatis, Phascolarctobacterium faecium, Phenylobacterium haematophilum, Phenylobacterium kunshanense, Pluralibacter gergoviae, Polymorphobacter multimanifer, Porphyromonas bennonis, Porphyromonas endodontalis, Porphyromonas gingivalis, Porphyromonas gingivicanis, Porphyromonas pasteri, Porphyromonas pogonae, Porphyromonas somerae, Povalibacter uvarum, Prevotella aurantiaca, Prevotella baroniae, Prevotella bivia, Prevotella buccae, Prevotella buccalis, Prevotella copri, Prevotella corporis, Prevotella denticola, Prevotella enoeca, Prevotella histicola, Prevotella intermedia, Prevotella jejuni, Prevotella jejuni, Prevotella maculosa, Prevotella melaninogenica, Prevotella melaninogenica, Prevotella micans, Prevotella multiformis, Prevotella nanceiensis, Prevotella nigrescens, Prevotella oris, Prevotella oulorum, Prevotella pallens, Prevotella pleuritidis, Prevotella saccharolytica, Prevotella salivae, Prevotella shahii, Prevotella timonensis, Prevotella veroralis, Propionibacterium acidifaciens, Propionibacterium acnes subsp. acnes, Propionibacterium acnes subsp. acnes, Propionibacterium acnes subsp. elongatum, Propionibacterium granulosum, Propionimicrobium lymphophilum, Propionispira arcuata, Pseudokineococcus lusitanus, Pseudomonas aeruginosa, Pseudomonas chengduensis, Pseudonocardia benzenivorans, Pseudorhodoplanes sinuspersici, Psychrobacter sanguinis, Ramlibacter ginsenosidimutans, Rheinheimera aquimaris, Rhizobium alvei, Rhizobium daejeonense, Rhizobium larrymoorei, Rhizobium rhizoryzae, Rhizobium soli, Rhizobium taibaishanense, Rhizobium vignae, Rhodanobacter glycinis, Rhodobacter veldkampii, Rhodococcus enclensis, Rhodococcus fascians, Rhodococcus fascians, Rhodovarius lipocyclicus, Rivicola pingtungensis, Roseburia inulinivorans, Rosenbergiella nectarea, Roseomonas aerilata, Roseomonas aquatica, Roseomonas mucosa, Roseomonas rosea, Roseomonas vinacea, Rothia aeria, Rothia amarae, Rothia dentocariosa, Rothia endophytica, Rothia mucilaginosa, Rothia nasimurium, Rubellimicrobium mesophilum, Rubellimicrobium roseum, Rubrobacter bracarensis, Rudaea cellulosilytica, Ruminococcus gnavus, Runella zeae, Saccharopolyspora rectivirgula, Salinicoccus qingdaonensis, Scardovia wiggsiae, Sediminibacterium ginsengisoli, Selenomonas artemidis, Selenomonas infelix, Selenomonas noxia, Selenomonas sputigena, Shewanella aestuarii, Shuttleworthia satelles, Simonsiella muelleri, Skermanella aerolata, Skermanella stibiiresistens, Slackia exigua, Smaragdicoccus niigatensis, Sneathia sanguinegens, Solirubrobacter soli, Sphingobacterium caeni, Sphingobacterium daejeonense, Sphingobacterium hotanense, Sphingobacterium kyonggiense, Sphingobacterium multivorum, Sphingobacterium nematocida, Sphingobacterium spiritivorum, Sphingobium amiense, Sphingobium indicum, Sphingobium lactosutens, Sphingobium subterraneum, Sphingomonas abaci, Sphingomonas aestuarii, Sphingomonas canadensis, Sphingomonas daechungensis, Sphingomonas dokdonensis, Sphingomonas echinoides, Sphingomonas fonticola, Sphingomonas fonticola, Sphingomonas formosensis, Sphingomonas gei, Sphingomonas hankookensis, Sphingomonas hankookensis, Sphingomonas koreensis, Sphingomonas kyeonggiensis, Sphingomonas laterariae, Sphingomonas mucosissima, Sphingomonas oligophenolica, Sphingomonas pseudosanguinis, Sphingomonas sediminicola, Sphingomonas yantingensis, Sphingomonas yunnanensis, Sphingopyxis indica, Spirosoma rigui, Sporacetigenium mesophilum, Sporocytophaga myxococcoides, Staphylococcus auricularis, Staphylococcus epidermidis, Staphylococcus epidermidis, Staphylococcus hominis subsp. novobiosepticus, Staphylococcus lugdunensis, Staphylococcus pettenkoferi, Stenotrophomonas koreensis, Stenotrophomonas rhizophila, Stenotrophomonas rhizophila, Streptococcus agalactiae, Streptococcus canis, Streptococcus cristatus, Streptococcus gordonii, Streptococcus infantis, Streptococcus intermedius, Streptococcus mutans, Streptococcus oligofermentans, Streptococcus oralis, Streptococcus sanguinis, Streptomyces iconiensis, Streptomyces yanglinensis, Tabrizicola aquatica, Tahibacter caeni, Tannerella forsythia, Tepidicella xavieri, Tepidimonas fonticaldi, Terracoccus luteus, Tessaracoccus flavescens, Thermus thermophilus, Tianweitania sediminis, Tianweitania sediminis, Treponema amylovorum, Treponema denticola, Treponema lecithinolyticum, Treponema medium, Turicella otitidis, Turicibacter sanguinis, Undibacterium oligocarboniphilum, Undibacterium squillarum, Vagococcus salmoninarum, Varibaculum cambriense, Vibrio metschnikovii, Xanthobacter tagetidis, Xenophilus aerolatus, Xenophilus arseniciresistens, Yimella lutea, Zimmermannella alba, Zimmermannella bifida, Zoogloea caeni,*

In other embodiments, the targeted bacteria cells are those commonly found in the vaginal microbiota and are, *without limitation, Acinetobacter antiviralis, Acinetobacter baumannii, Acinetobacter calcoaceticus, Acinetobacter johnsonii, Actinobaculum massiliense, Actinobaculum schaalii, Actinomyces europaeus, Actinomyces graevenitzii, Actinomyces israelii, Actinomyces meyeri, Actinomyces naeslundii, Actinomyces neuii, Actinomyces odontolyticus, Actinomyces turicensis, Actinomyces urogenitalis, Actinomyces viscosus, Aerococcus christensenii, Aerococcus urinae, Aerococcus viridans, Aeromonas encheleia, Aeromonas salmonicida, Afipia massiliensis, Agrobacterium tumefaciens, Algoriphagus aquatilis, Aliivibrio wodanis, Alistipes finegoldii, Alloiococcus otitis, Alloprevotella tannerae, Alloscardovia omnicolens, Altererythrobacter epoxidivorans, Ammoniphilus oxalaticus, Amnibacterium kyonggiense, Anaerococcus hydrogenalis, Anaerococcus lactolyticus, Anaerococcus murdochii, Anaerococcus obesiensis, Anaerococcus prevotii, Anaerococcus tetradius, Anaerococcus vaginalis, Anaeroglobus geminatus, Anoxybacillus pushchinoensis, Aquabacterium parvum, Arcanobacterium phocae, Arthrobacter aurescens, Asticcacaulis excentricus, Atopobium minutum, Atopobium parvulum, Atopobium rimae, Atopobium vaginae, Avibacterium gallinarum, Bacillus acidicola, Bacillus atrophaeus, Bacillus cereus, Bacillus cibi, Bacillus coahuilensis, Bacillus gaemokensis, Bacillus methanolicus, Bacillus oleronius, Bacillus pumilus, Bacillus shackletonii, Bacillus sporothermodurans, Bacillus subtilis, Bacillus wakoensis, Bacillus weihenstephanensis, Bacteroides barnesiae, Bacteroides coagulans, Bacteroides dorei, Bacteroides faecis, Bacteroides forsythus, Bacteroides fragilis, Bacteroides nordii, Bacteroides ovatus, Bacteroides salyersiae, Bacteroides stercoris, Bacteroides uniformis, Bacteroides vulgatus, Bacteroides xylanisolvens, Bacteroides zoogleoformans, Barnesiella viscericola, Bhargavaea cecembensis, Bifidobacterium adolescentis, Bifidobacterium bifidum, Bifidobacterium breve, Bifidobacterium dentium, Bifidobacterium logum subsp. infantis, Bifidobacterium longum, Bifidobacterium pseudocatenulatum, Bifidobacterium scardovii, Bilophila wadsworthia, Blautia hydrogenotrophica, Blautia obeum, Blautia producta, Brachybacterium faecium, Bradyrhizobium japonicum, Brevibacterium mcbrellneri, Brevibacterium otitidis, Brevibacterium paucivorans, Bulleidia extructa, Burkholderia fungorum, Burkholderia phenoliruptix, Caldicellulosiruptor saccharolyticus, Caldimonas taiwanensis, Campylobacter gracilis, Campylobacter hominis, Campylobacter sputorum, Campylobacter ureolyticus, Capnocytophaga ochracea, Cardiobacterium hominis, Catonella morbi, Chlamydia trachomatis, Chlamydophila abortus, Chondromyces robustus, Chryseobacterium aquaticum, Citrobacter youngae, Cloacibacterium normanense, Clostridium cavendishii, Clostridium colicanis, Clostridium jejuense, Clostridium perfringens, Clostridium ramosum, Clostridium sordellii, Clostridium viride, Comamonas terrigena, Corynebacterium accolens, Corynebacterium appendicis, Corynebacterium coyleae, Corynebacterium glucuronolyticum, Corynebacterium glutamicum, Corynebacterium jeikeium, Corynebacterium kroppenstedtii, Corynebacterium lipophiloflavum, Corynebacterium minutissimum, Corynebacterium mucifaciens, Corynebacterium nuruki, Corynebacterium pseudogenitalium, Corynebacterium pyruviciproducens, Corynebacterium singulare, Corynebacterium striatum, Corynebacterium tuberculostearicum, Corynebacterium xerosis, Cryobacterium psychrophilum, Curtobacterium flaccumfaciens, Cutibacterium acnes, Cutibacterium avidum, Cytophaga xylanolytica, Deinococcus radiophilus, Delftia tsuruhatensis, Desulfovibrio desulfuricans, Dialister invisus, Dialister micraerophilus, Dialister pneumosintes, Dialister propionicifaciens, Dickeya chrysanthemi, Dorea longicatena, Eggerthella lenta, Eggerthia catenaformis, Eikenella corrodens, Enhydrobacter aerosaccus, Enterobacter asburiae, Enterobacter cloacae, Enterococcus avium, Enterococcus durans, Enterococcus faecalis, Enterococcus faecium, Enterococcus hirae, Erwinia persicina, Erwinia rhapontici, Erwinia toletana, Escherichia coli, Escherichia fergusonii, Eubacterium brachy, Eubacterium eligens, Eubacterium nodatum, Eubacterium rectale, Eubacterium saphenum, Eubacterium siraeum, Eubacterium sulci, Eubacterium yurii, Exiguobacterium acetylicum, Facklamia ignava, Faecalibacterium prausnitzii, Filifactor alocis, Finegoldia magna, Fusobacterium gonidiaformans, Fusobacterium nucleatum, Fusobacterium periodonticum, Gardnerella vaginalis, Gemella asaccharolytica, Gemella bergeri, Gemella haemolysans, Gemella sanguinis, Geobacillus stearothermophilus, Geobacillus thermocatenulatus, Geobacillus thermoglucosidasius, Geobacter grbiciae, Granulicatella elegans, Haemophilus ducreyi, Haemophilus haemolyticus, Haemophilus parahaemolyticus, Haemophilus parainfluenzae, Hafnia alvei, Halomonas meridiana, Halomonas phoceae, Halomonas venusta, Herbaspirillum seropedicae, Janthinobacterium lividum, Jonquetella anthropi, Klebsiella granulomatis, Klebsiella oxytoca, Klebsiella pneumoniae, Lactobacillus acidophilus, Lactobacillus amylovorus, Lactobacillus brevis, Lactobacillus coleohominis, Lactobacillus crispatus, Lactobacillus curvatus, Lactobacillus delbrueckii, Lactobacillus fermentum, Lactobacillus gasseri, Lactobacillus helveticus, Lactobacillus iners, Lactobacillus jensenii, Lactobacillus johnsonii, Lactobacillus kalixensis, Lactobacillus kefiranofaciens, Lactobacillus kimchicus, Lactobacillus kitasatonis, Lactobacillus mucosae, Lactobacillus panis, Lactobacillus paracasei, Lactobacillus plantarum, Lactobacillus pontis, Lactobacillus reuteri, Lactobacillus rhamnosus, Lactobacillus salivarius, Lactobacillus ultunensis, Lactobacillus vaginalis, Lactococcus lactis, Leptotrichia buccalis, Leuconostoc carnosum, Leuconostoc citreum, Leuconostoc garlicum, Leuconostoc lactis, Leuconostoc mesenteroides, Lysinimonas kribbensis, Mageeibacillus indolicus, Maribacter orientalis, Marinomonas protea, Marinospirillum insulare, Massilia timonae, Megasphaera elsdenii, Megasphaera micronuciformis, Mesorhizobium amorphae, Methylobacterium radiotolerans, Methylotenera versatilis, Microbacterium halophilum, Micrococcus luteus, Microterricola viridarii, Mobiluncus curtisii, Mobiluncus mulieris, Mogibacterium timidum, Moorella glycerini, Moraxella osloensis, Morganella morganii, Moryella indoligenes, Murdochiella asaccharolytica, Mycoplasma alvi, Mycoplasma genitalium, Mycoplasma hominis, Mycoplasma muris, Mycoplasma salivarium, Negativicoccus succinicivorans, Neisseria flava, Neisseria gonorrhoeae, Neisseria mucosa, Neisseria subflava, Nevskia ramosa, Nevskia soli, Nitriliruptor alkaliphilus, Odoribacter splanchnicus, Oligella urethralis, Olsenella uli, Paenibacillus amylolyticus, Paenibacillus humicus, Paenibacillus pabuli, Paenibacillus pasadenensis, Paenibacillus pini, Paenibacillus validus, Pantoea agglomerans, Parabacteroides merdae, Paraburkholderia caryophylli, Paracoccus yeei, Parastreptomyces abscessus, Parvimonas micra, Pectobacterium betavasculorum, Pectobacterium carotovorum, Pediococcus acidilactici, Pediococcus ethanolidurans, Pedobacter alluvionis, Pedobacter wanjuense, Pelomonas aquatica, Peptococcus niger, Peptoniphilus asaccharolyticus, Peptoniphilus gorbachii, Peptoniphilus harei, Peptoniphilus indolicus, Peptoniphilus lacrimalis, Peptoniphilus massiliensis, Peptostreptococcus anaerobius, Peptostreptococcus massiliae, Peptostreptococcus stomatis, Photobacterium angustum, Photobacterium frigidiphilum, Photobacterium phosphoreum, Porphyromonas asaccharolytica, Porphyromonas bennonis, Porphyromonas catoniae, Porphyromonas endodontalis, Porphyromonas gingivalis, Porphyromonas somerae, Porphyromonas uenonis, Prevotella amnii, Prevotella baroniae, Prevotella bergensis, Prevotella bivia, Prevotella buccae, Prevotella buccalis, Prevotella colorans, Prevotella copri, Prevotella corporis, Prevotella dentalis, Prevotella denticola, Prevotella disiens, Prevotella intermedia, Prevotella loescheii, Prevotella marshii, Prevotella melaninogenica, Prevotella micans, Prevotella nigrescens, Prevotella oris, Prevotella pleuritidis, Prevotella ruminicola, Prevotella shahii, Prevotella stercorea, Prevotella timonensis, Prevotella veroralis, Propionimicrobium lymphophilum, Proteus mirabilis, Pseudomonas abietaniphila, Pseudomonas aeruginosa, Pseudomonas amygdali, Pseudomonas azotoformans, Pseudomonas chlororaphis, Pseudomonas cuatrocienegasensis, Pseudomonas fluorescens, Pseudomonas fulva, Pseudomonas lutea, Pseudomonas mucidolens, Pseudomonas oleovorans, Pseudomonas orientalis, Pseudomonas pseudoalcaligenes, Pseudomonas psychrophila, Pseudomonas putida, Pseudomonas synxantha, Pseudomonas syringae, Pseudomonas tolaasii, Pseudopropionibacterium propionicum, Rahnella aquatilis, Ralstonia pickettii, Ralstonia solanacearum, Raoultella planticola, Rhizobacter dauci, Rhizobium etli, Rhodococcus fascians, Rhodopseudomonas palustris, Roseburia intestinalis, Roseburia inulinivorans, Rothia mucilaginosa, Ruminococcus bromii, Ruminococcus gnavus, Ruminococcus torques, Sanguibacter keddieii, Sediminibacterium salmoneum, Selenomonas bovis, Serratia fonticola, Serratia liquefaciens, Serratia marcescens, Shewanella algae, Shewanella amazonensis, Shigella boydii, Shigella sonnei, Slackia exigua, Sneathia amnii, Sneathia sanguinegens, Solobacterium moorei, Sorangium cellulosum, Sphingobium amiense, Sphingobium japonicum, Sphingobium yanoikuyae, Sphingomonas wittichii, Sporosarcina aquimarina, Staphylococcus aureus, Staphylococcus auricularis, Staphylococcus capitis, Staphylococcus epidermidis, Staphylococcus haemolyticus, Staphylococcus hominis, Staphylococcus lugdunensis, Staphylococcus saprophyticus, Staphylococcus schleiferi, Staphylococcus simiae, Staphylococcus simulans, Staphylococcus warneri, Stenotrophomonas maltophilia, Stenoxybacter acetivorans, Streptococcus agalactiae, Streptococcus anginosus, Streptococcus australis, Streptococcus equinus, Streptococcus gallolyticus, Streptococcus infantis, Streptococcus intermedius, Streptococcus lutetiensis, Streptococcus marimammalium, Streptococcus mitis, Streptococcus mutans, Streptococcus oralis, Streptococcus parasanguinis, Streptococcus phocae, Streptococcus pseudopneumoniae, Streptococcus salivarius, Streptococcus sanguinis, Streptococcus thermophilus, Sutterella wadsworthensis, Tannerella forsythia, Terrahaemophilus aromaticivorans, Treponema denticola, Treponema maltophilum, Treponema parvum, Treponema vincentii, Trueperella bernardiae, Turicella otitidis, Ureaplasma parvum, Ureaplasma urealyticum, Varibaculum cambriense, Variovorax paradoxus, Veillonella atypica, Veillonella dispar, Veillonella montpellierensis, Veillonella parvula, Virgibacillus proomii, Viridibacillus arenosi, Viridibacillus arvi, Weissella cibaria, Weissella soli, Xanthomonas campestris, Xanthomonas vesicatoria, Zobellia laminariae, Zoogloea ramigera,*

In one embodiment, the targeted bacteria are *Escherichia coli.*

In one embodiment, the targeted bacteria are *Klebsiella pneumoniae.*

In one embodiment, the targeted bacteria are *Escherichia coli* and/or *Klebsiella pneumoniae.*

In one embodiment, the targeted bacteria are *Klebsiella pneumoniae* of the K-type such as K2, K1, K62, K64, K23, K28, K54, K16, K5, K27 or combinations thereof. In one embodiment, the targeted bacteria are *Klebsiella pneumoniae* of the K-type K2, K1, K62, K64 or combinations thereof. In one embodiment, the targeted bacteria are *Klebsiella pneumoniae* of the K-type K23, K28, K54, K16, or combinations thereof. In one embodiment, the targeted bacteria are *Klebsiella pneumoniae* of the K-type K5, K27, or combinations thereof. In one embodiment, the targeted bacteria are *Klebsiella pneumoniae* of the K-type K24, K2, K23, K71, K62, K60, K41, K28, K22, K37, K17, K16, K64, K1, K15, K50, K51, K58, or combinations thereof. In one embodiment, the targeted bacteria are *Klebsiella pneumoniae* of the K-type K24, K2, K23, K71, or combinations thereof. In one embodiment, the targeted bacteria are *Klebsiella pneumoniae* of the K-type K62, K60, K41, K28, K22, K37, K17, K16, K64, K1, K15, K50, K51, K58, or combinations thereof. In one embodiment, the targeted bacteria are *Klebsiella pneumoniae* of the O-type such as O1, O2, O3, O5, O4, 012, OL101, OL104 or combinations thereof. In one embodiment, the targeted bacteria are *Klebsiella pneumoniae* of the O-type O1, O2, O3, O5, or combinations thereof. In one embodiment, the targeted bacteria are *Klebsiella pneumoniae* of the O-type O4, O12, OL101, OL104 or combinations thereof. In one embodiment, the targeted bacteria are *Klebsiella pneumoniae* strain MLST258.

In one embodiment, the targeted bacteria are *Escherichia coli* of the K type K1, K5 or combinations thereof. In one embodiment, the targeted bacteria are *Escherichia coli* of the O-type O1, O2, O6, O25, 018, or combinations thereof. In one embodiment, the targeted bacteria are *Escherichia coli* of the O-type O1, O2? O4, O6, O7, O8, 016, O25, O75, or combinations thereof. In one embodiment, the targeted bacteria are selected from the group consisting of *Escherichia coli* strains MLST131, ST73, ST95, ST12, ST10, ST14, ST2278, ST69, and combinations thereof.

In one embodiment, the targeted bacteria are *Cutibacterium acnes* more specifically the acne related *Cutibacterium acnes* from the phylogroup IA1 or RT4, RT5, RT8, RT9, RT10 or Clonal Complex(CC) CC1, CC3, CC4, more specifically the ST1, ST3, ST4.

Bacteriophages used for preparing bacterial virus particles such as packaged phagemids, may target (e.g., specifically target) a bacterial cell from any one or more of the disclosed genus and/or species of bacteria to specifically deliver the plasmid.

In one embodiment, the targeted bacteria are pathogenic bacteria. The targeted bacteria can be virulent bacteria.

The targeted bacteria can be antibiotic resistant bacteria, preferably selected from the group consisting of extended-spectrum beta-lactamase-producing (ESBL) *Escherichia coli,* ESBL *Klebsiella pneumoniae,* carbapenem-resistant (CR) *Escherichia coli,* CR *Klebsiella pneumoniae,* vancomycin-resistant *Enterococcus* (VRE), methicillin-resistant *Staphylococcus aureus* (MRSA), multidrug-resistant (MDR) *Acinetobacter baumannii,* MDR *Enterobacter spp.,* and any combination thereof. Preferably, the targeted bacteria can be selected from the group consisting of extended-spectrum beta-lactamase-producing (ESBL) *Escherichia coli* strains. Still preferably, the targeted bacteria can be selected from the group consisting of extended-spectrum beta-lactamase-producing (ESBL) *Klebsiella pneumoniae* strains. Still preferably, the targeted bacteria can be selected from the group consisting of extended-spectrum beta-lactamase-producing (ESBL) *Escherichia coli* strains, extended-spectrum beta-lactamase-producing (ESBL) *Klebsiella pneumoniae* strains, and mixtures thereof. Still preferably, the targeted bacteria can be selected from the group consisting of CR *Escherichia coli* strains. Still preferably, the targeted bacteria can be selected from the group consisting of CR *Klebsiella pneumoniae* strains. Still preferably, the targeted bacteria can be selected from the group consisting of CR *Escherichia coli* strains, CR *Klebsiella pneumoniae* strains, and mixtures thereof.

Alternatively, the targeted bacterium can be a bacterium of the microbiome of a given species, preferably a bacterium of the human microbiota.

Thus, bacterial virus particles may target (e.g., specifically target) a bacterial cell from any one or more of the foregoing species of bacteria to specifically deliver the plasmid/vector/genetic modification according to the invention.

In another particular embodiment, at least two distinct vectors are used, in particular at least 3, 4, 5, 6, 7, 8, 9, 10 or more, each vector targeting a different bacteria as defined above, in particular targeting different bacterial species, different bacterial K-type, different bacterial O-type, and/or different bacterial strains.

In a particularly preferred embodiment, at least two distinct vectors are used, in particular at least 3, 4, 5, 6, 7, 8, 9, 10 or more, each vector targeting a different *E*. *coli* bacterium, in particular different *E. coli* K-type, different *E. coli* O-type, and/or different *E*. *coli* strains.

### Bacterial viruses

The bacterial virus particles are prepared from bacterial virus. The bacterial viruses are chosen in order to be able to introduce the plasmid into the targeted bacteria.

Bacterial viruses are preferably bacteriophages. Bacteriophages are obligate intracellular parasites that multiply inside bacteria by co-opting some or all of the host biosynthetic machinery. Phage genomes come in a variety of sizes and shapes (e.g., linear or circular). Most phages range in size from 24-200 nm in diameter. Phages contain nucleic acid (i.e., genome) and proteins, and may be enveloped by a lipid membrane. Depending upon the phage, the nucleic acid genome can be either DNA or RNA, and can exist in either circular or linear forms. The size of the phage genome varies depending upon the phage. The simplest phages have genomes that are only a few thousand nucleotides in size, while the more complex phages may contain more than 100,000 nucleotides in their genome, and in rare instances more than 1,000,000. The number and amount of individual types of protein in phage particles will vary depending upon the phage.

Optionally, the bacteriophage is selected from the Order Caudovirales consisting of, based on the taxonomy of Krupovic et al, Arch Virol, 2015:
- family Myoviridae (such as, without limitation, genus Cp220virus, Cp8virus, Ea214virus, Felixolvirus, Mooglevirus, Suspvirus, Hp1virus, P2virus, Kayvirus, P100virus, Silviavirus, Spo1virus, Tsarbombavirus, Twortvirus, Cc31virus, Jd18virus, Js98virus, Kp15virus, Moonvirus, Rb49virus, Rb69virus, S16virus, Schizot4virus, Sp18virus, T4virus, Cr3virus, Se1virus, V5virus, Abouovirus, Agatevirus, Agrican357virus, Ap22virus, Arv1virus, B4virus, Bastillevirus, Bc431virus, Bcep78virus, Bcepmuvirus, Biquartavirus, Bxz1virus, Cd119virus, Cp51virus, Cvm10virus, Eah2virus, Elvirus, Hapunavirus, Jimmervirus, Kpp10virus, M12virus, Machinavirus, Marthavirus, Msw3virus, Muvirus, Myohalovirus, Nit1virus, P1virus, Pakpunavirus, Pbunavirus, Phikzvirus, Rheph4virus, Rsl2virus, Rslunavirus, Secunda5virus, Sep1virus, Spn3virus, Svunavirus, Tglvirus, Vhmlvirus and Wphvirus)
- family Podoviridae (such as, without limitation, genus Fri1virus, Kp32virus, Kp34virus, Phikmvvirus, Pradovirus, Sp6virus, T7virus, Cp1virus, P68virus, Phi29virus, Nona33virus, Pocjvirus, TI2011virus, Bcep22virus, Bpp1virus, Cba41virus, Dfl12virus, Ea92virus, Epsilon15virus, F116virus, G7cvirus, Jwalphavirus, Kf1virus, Kpp25virus, Lit1virus, Luz24virus, Luz7virus, N4virus, Nonanavirus, P22virus, Pagevirus, Phieco32virus, Prtbvirus, Sp58virus, Una961virus and Vp5virus)
- family Siphoviridae (such as, without limitation, genus Camvirus, Likavirus, R4virus, Acadianvirus, Coopervirus, Pg1virus, Pipefishvirus, Rosebushvirus, Brujitavirus, Che9cvirus, Hawkeyevirus, Plotvirus, Jerseyvirus, K1gvirus, Sp31virus, Lmd1virus, Una4virus, Bongovirus, Reyvirus, Buttersvirus, Charlievirus, Redivirus, Baxtervirus, Nymphadoravirus, Bignuzvirus, Fishburnevirus, Phayoncevirus, Kp36virus, Rogue1virus, Rtpvirus, T1virus, Tlsvirus, Ab18virus, Amigovirus, Anatolevirus, Andromedavirus, Attisvirus, Barnyardvirus, Bernal13virus, Biseptimavirus, Bronvirus, C2virus, C5virus, Cba181virus, Cbastvirus, Cecivirus, Che8virus, Chivirus, Cjw1virus, Corndogvirus, Cronusvirus, D3112virus, D3virus, Decurrovirus, Demosthenesvirus, Doucettevirus, E125virus, Eiauvirus, Ff47virus, Gaiavirus, Gilesvirus, Gordonvirus, Gordtnkvirus, Harrisonvirus, Hk578virus, Hk97virus, Jenstvirus, Jwxvirus, Kelleziovirus, Korravirus, L5virus, Lambdavirus, Laroyevirus, Liefievirus, Marvinvirus, Mudcatvirus, N15virus, Nonagvirus, Np1virus, Omegavirus, P12002virus, P12024virus, P23virus, P70virus, Pa6virus, Pamx74virus, Patiencevirus, Pbi1virus, Pepy6virus, Pfr1virus, Phic31virus, Phicbkvirus, Phietavirus, Phifelvirus, Phijl1virus, Pis4avirus, Psavirus, Psimunavirus, Rdjlvirus, Rer2virus, Sap6virus, Send513virus, Septima3virus, Seuratvirus, Sextaecvirus, Sfi11virus, Sfi21dt1virus, Sitaravirus, Sk1virus, Slashvirus, Smoothievirus, Soupsvirus, Spbetavirus, Ssp2virus, T5virus, Tankvirus, Tin2virus, Titanvirus, Tm4virus, Tp21virus, Tp84virus, Triavirus, Trigintaduovirus, Vegasvirus, Vendettavirus, Wbetavirus, Wildcatvirus, Wizardvirus, Woesvirus, Xp10virus, Ydn12virus and Yuavirus)
- family Ackermannviridae (such as, without limitation, genus Ag3virus, Limestonevirus, Cba120virus and Vi1virus)

Optionally, the bacteriophage is not part of the Order Caudovirales but from families with Unassigned order such as, without limitation, family Tectiviridae (such as genus Alphatectivirus, Betatectivirus), family Corticoviridae (such as genus Corticovirus), family Inoviridae (such as genus Fibrovirus, Habenivirus, Inovirus, Lineavirus, Plectrovirus, Saetivirus, Vespertiliovirus), family Cystoviridae(such as genus *Cystovirus*)*,* family Leviviridae(such as genus Allolevivirus, Levivirus), family Microviridae (such as genus Alpha3microvirus, G4microvirus, Phix174microvirus, Bdellomicrovirus, Chlamydiamicrovirus, Spiromicrovirus) and family Plasmaviridae (such as genus *Plasmavirus*)*.*

Optionally, the bacteriophage is targeting Archea not part of the Order Caudovirales but from families with Unassigned order such as, without limitation, Ampullaviridae, FuselloViridae, Globuloviridae, *Guttaviridae, Lipothrixviridae,* Pleolipoviridae, *Rudiviridae,* Salterprovirus and *Bicaudaviridae.*

A non-exhaustive listing of bacterial genera and their known host-specific bacteria viruses is presented in the following paragraphs. Synonyms and spelling variants are indicated in parentheses. Homonyms are repeated as often as they occur (e.g., D, D, d). Unnamed phages are indicated by "NN" beside their genus and their numbers are given in parentheses.

Bacteria of the genus Actinomyces can be infected by the following phages: Av-I, Av-2, Av-3, BF307, CTI, CT2, CT3, CT4, CT6, CT7, CT8 and 1281.

Bacteria of the genus *Aeromonas* can be infected by the following phages: AA-I, Aeh2, N, PMI, TP446, 3, 4, 11, 13, 29, 31, 32, 37, 43, 43-10T, 51, 54, 55R.1, 56, 56RR2, 57, 58, 59.1, 60, 63, Aehl, F, PM2, 1, 25, 31, 40RR2.8t, (syn= 44R), (syn= 44RR2.8t), 65, PM3, PM4, PM5 and PM6.

Bacteria of the genus *Bacillus* can be infected by the following phages: A, aizl, Al-K-I, B, BCJAI, BCI, BC2, BLLI, BLI, BP142, BSLI, BSL2, BSI, BS3, BS8, BS15, BS18, BS22, BS26, BS28, BS31, BS104, BS105, BS106, BTB, B1715V1, C, CK-I, Coll, Corl, CP-53, CS-I, CSi, D, D, D, D5, entl, FP8, FP9, FSi, FS2, FS3, FS5, FS8, FS9, G, GH8, GT8, GV-I, GV-2, GT-4, g3, gI2, gI3, gI4, gI6, gI7, g21, g23, g24, g29, H2, kenl, KK-88, Kuml, Kyul, J7W-1, LP52, (syn= LP-52), L7, Mexl, MJ-I, mor2, MP-7, MPIO, MP12, MP14, MP15, Neol, N°2, N5, N6P, PBCI, PBLA, PBPI, P2, S-a, SF2, SF6, Shal, Sill, SP02, (syn= ΦSPP1), SPβ, STI, STi, SU-II, t, Tbl, Tb2, Tb5, TbIO, Tb26, Tb51, Tb53, Tb55, Tb77, Tb97, Tb99, Tb560, Tb595, Td8, Td6, TdI5, Tgl, Tg4, Tg6, Tg7, Tg9, TgIO, TgII, TgI3, TgI5, Tg21, Tinl, Tin7, Tin8, Tinl3, Tm3, Tocl, Togl, toll, TP-I, TP-10vir, TP-15c, TP-16c, TP-17c, TP-19, TP35, TP51, TP-84, Tt4, Tt6, type A, type B, type C, type D, type E, Tϕ3, VA-9, W, wx23, wx26, Yunl, α, γ, pI I,, ϕmed-2, ϕT, ϕµ-4, ϕ3T, ϕ75, ϕIO5, (syn= ϕIO5), IA, IB, 1-97A, 1-97B, 2, 2, 3, 3, 3, 5, 12, 14, 20, 30, 35, 36, 37, 38, 41C, 51, 63, 64, 138D, I, II, IV, NN-Bacillus (13), alel, ARI, AR2, AR3, AR7, AR9, Bace-11, (syn= 11), Bastille, BLI, BL2, BL3, BL4, BL5, BL6, BL8, BL9, BP124, BS28, BS80, Ch, CP-51, CP-54, D-5, darl, denl, DP-7, entl, FoSi, FoS2, FS4, FS6, FS7, G, gall, gamma, GEI, GF-2, GSi, GT-I, GT-2, GT-3, GT-4, GT-5, GT-6, GT-7, GV-6, gI5, 19, 110, ISi, K, MP9, MP13, MP21, MP23, MP24, MP28, MP29, MP30, MP32, MP34, MP36, MP37, MP39, MP40, MP41, MP43, MP44, MP45, MP47, MP50, NLP-I, No.I, N17, N19, PBSI, PKI, PMBI, PMB12, PMJI, S, SPOI, SP3, SP5, SP6, SP7, SP8, SP9, SPIO, SP-15, SP50, (syn= SP-50), SP82, SST, subl, SW, Tg8, TgI2, TgI3, TgI4, thul, thuΛ, thuS, Tin4, Tin23, TP-13, TP33, TP50, TSP-I, type V, type VI, V, Vx, β22, ϕe, ϕNR2, ϕ25, ϕ63, 1, 1, 2, 2C, 3NT, 4, 5, 6, 7, 8, 9, 10, 12, 12, 17, 18, 19, 21, 138, III, 4 (B. megateriwn), 4 (B. sphaericus), AR13, BPP-IO, BS32, BS107, BI, B2, GA-I, GP-IO, GV-3, GV-5, g8, MP20, MP27, MP49, Nf, PP5, PP6, SF5, TgI8, TP-I, Versailles, ϕI5, ϕ29, 1-97, 837/IV, mï-Bacillus (1), BatIO, BSLIO, BSLI I, BS6, BSI I, BS16, BS23, BSIOI, BS102, gI8, morl, PBLI, SN45, thu2, thu3, Tml, Tm2, TP-20, TP21, TP52, type F, type G, type IV, HN-BacMus (3), BLE, (syn= θc), BS2, BS4, BS5, BS7, BIO, B12, BS20, BS21, F, MJ-4, PBA12, AP50, AP50-04, AP50-11, AP50-23, AP50-26, AP50-27 and Bam35. The following Bacillus-specific phages are defective: DLP10716, DLP-11946, DPB5, DPB12, DPB21, DPB22, DPB23, GA-2, M, No. IM, PBLB, PBSH, PBSV, PBSW, PBSX, PBSY, PBSZ, phi, SPa, type 1 and µ.

Bacteria of the genus *Bacteriodes* can be infected by the following phages: crAss-phage, ad 12, Baf-44, Baf-48B, Baf-64, Bf-I, Bf-52, B40-8, FI, βI, ϕAI, ϕBrOI, ϕBrO2, 11, 67.1, 67.3, 68.1, mt-Bacteroides (3), Bf42, Bf71, HN-Bdellovibrio (1) and BF-41.

Bacteria of the genus *Bordetella* can be infected by the following phages: 134 and NN-Bordetella (3).

Bacteria of the genus *Borrellia* can be infected by the following phages: NN-Borrelia (1) and NN-Borrelia (2).

Bacteria of the genus *Brucella* can be infected by the following phages: A422, Bk, (syn= Berkeley), BM29, FOi, (syn= FOI), (syn= FQI), D, FP2, (syn= FP2), (syn= FD2), Fz, (syn= Fz75/13), (syn= Firenze 75/13), (syn= Fi), Fi, (syn= FI), Fim, (syn= Flm), (syn= Fim), FiU, (syn= FIU), (syn= FiU), F2, (syn= F2), F3, (syn= F3), F4, (syn= F4), F5, (syn= F5), F6, F7, (syn= F7), F25, (syn= F25), (syn= £25), F25U, (syn= F25u), (syn= F25U), (syn= F25V), F44, (syn- F44), F45, (syn= F45), F48, (syn= F48), I, Im, M, MC/75, M51, (syn= M85), P, (syn= D), S708, R, Tb, (syn= TB), (syn= Tbilisi), W, (syn= Wb), (syn= Weybridge), X, 3, 6, 7, 10/1, (syn= 10), (syn= F8), (syn= F8), 12m, 24/11, (syn= 24), (syn= F9), (syn= F9), 45/111, (syn= 45), 75, 84, 212/XV, (syn= 212), (syn= Fi0), (syn= FIO), 371/XXIX, (syn= 371), (syn= Fn), (syn= FI I) and 513.

Bacteria of the genus *Burkholderia* can be infected by the following phages: CP75, NN-Burkholderia (1) and 42.

Bacteria of the genus *Campylobacter* can be infected by the following phages: C type, NTCC12669, NTCC12670, NTCC12671, NTCC12672, NTCC12673, NTCC12674, NTCC12675, NTCC12676, NTCC12677, NTCC12678, NTCC12679, NTCC12680, NTCC12681, NTCC12682, NTCC12683, NTCC12684, 32f, 111c, 191, NN-Campylobacter (2), Vfi-6, (syn= V19), VfV-3, V2, V3, V8, V16, (syn= Vfi-1), V19, V20(V45), V45, (syn= V-45) and NN-Campylobacter (1).

Bacteria of the genus *Chlamydia* can be infected by the following phage: Chpl.

Bacteria of the genus *Clostridium* can be infected by the following phages: CAKI, CA5, Ca7, CEβ, (syn= 1C), CEy, Cldl, c-n71, c-203 Tox-, DEβ, (syn= ID), (syn= IDt0X+), HM3, KMI, KT, Ms, NAI, (syn= Naltox+), PA135Oe, Pfó, PL73, PL78, PL81, PI, P50, P5771, P19402, ICt0X+, 2Ct0X\ 2D3 (syn= 2Dt0X+), 3C, (syn= 3Ctox+), 4C, (syn= 4Ct0X+), 56, III-I, NN-Clostridium (61), NBIt0X+, αI, CAI, HMT, HM2, PFI5 P-23, P-46, Q-05, Q-oe, Q-16, Q-21, Q-26, Q-40, Q-46, S111, SA02, WA01, WA03, Wm, W523, 80, C, CA2, CA3, CPTI, CPT4, cl, c4, c5, HM7, H11/A1, H18/Ax, FWS23, Hi58ZA1, K2ZA1, K21ZS23, ML, NA2t0X; Pf2, Pf3, Pf4, S9ZS3, S41ZA1, S44ZS23, α2, 41, 112ZS23, 214/S23, 233/Ai, 234/S23, 235/S23, II-I, II-2, II-3, NN-Clostridium (12), CAI, FI, K, S2, 1, 5 and NN-Clostridium (8).

Bacteria of the genus *Corynebacterium* can be infected by the following phages: CGKI (defective), A, A2, A3, AIOI, A128, A133, A137, A139, A155, A182, B, BF, B17, B18, B51, B271, B275, B276, B277, B279, B282, C, capi, CCI, CGI, CG2, CG33, CL31, Cog, (syn= CG5), D, E, F, H, H-I, hqi, hq2, 11ZH33, Ii/31, J, K, K, (syn= Ktox"), L, L, (syn= Ltox+), M, MC-I, MC-2, MC-3, MC-4, MLMa, N, O, ovi, ov2, ov3, P, P, R, RP6, RS29, S, T, U, UB1, ub2, UH1, UH3, uh3, uh5, uh6, β, (syn= βtox+), βhv64, βvir, γ, (syn= γtoχ-), γI9, δ, (syn= δ'οx+), p, (syn= ptοχ-), Φ9, ϕ984, ω, IA, 1/1180, 2, 2/1180, 5/1180, 5ad/9717, 7/4465, 8/4465, 8ad/10269, 10/9253, 13Z9253, 15/3148, 21/9253, 28, 29, 55, 2747, 2893, 4498 and 5848.

Bacteria of the genus *Enterococcus* are infected by the following phage: DF78, FI, F2, 1, 2, 4, 14, 41, 867, DI, SB24, 2BV, 182, 225, C2, C2F, E3, E62, DS96, H24, M35, P3, P9, SBIOI, S2, 2BII, 5, 182a, 705, 873, 881, 940, 1051, 1057, 21096C, NN-Enterococcus (1), PEI, FI, F3, F4, VD13, 1, 200, 235 and 341.

Bacteria of the genus *Erysipelothrix* can be infected by the following phage: NN-Eiysipelothrix (1).

Bacteria of the genus *Escherichia* can be infected by the following phages: BW73, B278, D6, D108, E, EI, E24, E41, FI-2, FI-4, FI-5, HI8A, FfI8B, i, MM, Mu, (syn= mu), (syn= Mul), (syn= Mu-I), (syn= MU-I), (syn= Mul), (syn= µ), 025, Phl-5, Pk, PSP3, PI, PID, P2, P4 (defective), SI, Wϕ, ϕK13, ϕR73 (defective), ϕI, ϕ2, ϕ7, ϕ92, ψ (defective), 7 A, 8ϕ, 9ϕ, 15 (defective), 18, 28-1, 186,299, HH-Escherichia (2), AB48, CM, C4, C16, DD-VI, (syn= Dd-Vi), (syn= DDVI), (syn= DDVi), E4, E7, E28, FII, FI3, H, HI, H3, H8, K3, M, N, ND-2, ND-3, ND4, ND-5, ND6, ND-7, Ox-I (syn= OXI), (syn= HF), Ox-2 (syn= 0x2), (syn= 0X2), Ox-3, Ox-4, Ox-5, (syn= 0X5), Ox-6, (syn= 66F), (syn= ϕ66t), (syn= ϕ66t-)5 0111, PhI-I, RB42, RB43, RB49, RB69, S, Sal-I, Sal-2, Sal-3, Sal- 4, Sal-5, Sal-6, TC23, TC45, TuII*-6, (syn= TuII*), TuIP-24, TuII*46, TuIP-60, T2, (syn= ganuTia), (syn= γ), (syn= PC), (syn= P.C.), (syn= T-2), (syn= T2), (syn= P4), T4, (syn= T-4), (syn= T4), T6, T35, αI, 1, IA, 3, (syn= Ac3), 3A, 3T+, (syn= 3), (syn= MI), 5ϕ, (syn= ϕ5), 9266Q, CFO103, HK620, J, K, KIF, m59, no. A, no. E, no. 3, no. 9, N4, sd, (syn= Sd), (syn= SD), (syn= Sa)3 (syn= sd), (syn= SD), (syn= CD), T3, (syn= T-3), (syn= T3), T7, (syn= T-7), (syn= T7), WPK, W31, ΔH, ϕC3888, ϕK3, ϕK7, ϕK12, ϕV-1, Φ04-CF, Φ05, Φ06, Φ07, ϕI, ϕI.2, ϕ20, ϕ95, ϕ263, ϕIO92, ϕI, ϕII, (syn=ϕW), Ω8, 1, 3, 7, 8, 26, 27, 28-2, 29, 30, 31, 32, 38, 39, 42, 933W, NN-Escherichia (1), Esc-7-11, AC30, CVX-5, CI, DDUP, ECI, EC2, E21, E29, FI, F26S, F27S, Hi, HK022, HK97, (syn= ΦHK97), HK139, HK253, HK256, K7, ND-I, no.D, PA-2, q, S2, TI, (syn= ααα), (syn= P28), (syn= T-I), (syn= Tx), T3C, T5, (syn= T-5), (syn= T5), UC-I, w, β4, γ2, λ (syn= lambda), (syn= Φλ), ΦD326, ϕγ, Φ06, Φ7, Φ10, ϕ80, χ, (syn= χi), (syn= ϕχ), (syn= ϕχi), 2, 4, 4A, 6, 8A, 102, 150, 168, 174, 3000, AC6, AC7, AC28, AC43, AC50, AC57, AC81, AC95, HK243, KIO, ZG/3A, 5, 5A, 21EL, H19-J, 933H, 0157 typing phages 1 to 16, JES-2013, 121Q, 172-1, 1720a-02, ADB-2, AKFV33, av-05, bV_EcoS_AHP42, bV_EcoS_AHP24, bC_EcoS_AHS24, bV_EcoS_AKS96, CBA120.

Bacteria of the genus *Fusobacterium* are infected by the following phage: NN-Fusobacterium (2), fv83-554/3, fv88-531/2, 227, fv2377, fv2527 and fv8501.

Bacteria of the genus *Haemophilus* are infected by the following phage: HPI, S2 and N3.

Bacteria of the genus *Helicobacter* are infected by the following phage: HPI and ^^-Helicobacter (1).

Bacteria of the genus *Klebsiella* are infected by the following phage: AIO-2, KI4B, KI6B, KI9, (syn= K19), KI14, KI15, KI21, KI28, KI29, KI32, KI33, KI35, KI106B, KI171B, KI181B, KI832B, AIO-I, AO-I, AO-2, AO-3, FC3-10, K, KI1, (syn= KII), KI2, (syn= K12), KI3, (syn= K13), (syn= KI 70/11), KI4, (syn= K14), KI5, (syn= K15), KI6, (syn= K16), KI7, (syn= K17), KI8, (syn= K18), KI19, (syn= K19), KI27, (syn= K127), KI31, (syn= K131), KI35, KI171B, II, VI, IX, CI-I, KI4B, KI8, KI11, KI12, KI13, KI16, KI17, KI18, KI20, KI22, KI23, KI24, KI26, KI30, KI34, KI106B, KIi65B, KI328B, KLXI, K328, P5046, 11, 380, III, IV, VII, VIII, FC3-11, KI2B, (syn= K12B), KI25, (syn= K125), KI42B, (syn= K142), (syn= K142B), KI181B, (syn= KII 81), (syn= K1181B), KI765/!, (syn= K1765/1), KI842B, (syn= K1832B), KI937B, (syn= K1937B), LI, ϕ28, 7, 231, 483, 490, 632 and 864/100.

Bacteria of the genus *Lepitospira* are infected by the following phage: LEI, LE3, LE4 and ∼NN-Leptospira (1).

Bacteria of the genus *Listeria* are infected by the following phage: A511, 01761, 4211, 4286, (syn= BO54), A005, A006, A020, A500, A502, A511, AI 18, A620, A640, B012, B021, B024, B025, B035, B051, B053, B054, B055, B056, BIOI, BI IO, B545, B604, B653, C707, D441, HSO47, HIOG, H8/73, H19, H21, H43, H46, H107, H108, HI IO, H163/84, H312, H340, H387, H391/73, H684/74, H924A, PSA, U153, ϕMLUP5, (syn= P35), 00241, 00611, 02971A, 02971C, 5/476, 5/911, 5/939, 5/11302, 5/11605, 5/11704, 184, 575, 633, 699/694, 744, 900, 1090, 1317, 1444, 1652, 1806, 1807, 1921/959, 1921/11367, 1921/11500, 1921/11566, 1921/12460, 1921/12582, 1967, 2389, 2425, 2671, 2685, 3274, 3550, 3551, 3552, 4276, 4277, 4292, 4477, 5337, 5348/11363, 5348/11646, 5348/12430, 5348/12434, 10072, 11355C, 11711A, 12029, 12981, 13441, 90666, 90816, 93253, 907515, 910716 and NN-Lisferia (15).

Bacteria of the genus *Morganella* are infected by the following phage: 47.

Bacteria of the genus *Mycobacterium* are infected by the following phage: 13, AGI, ALi, ATCC 11759, A2, B.C3, BG2, BKI, BK5, butyricum, B-I, B5, B7, B30, B35, Clark, CI, C2, DNAIII, DSP1, D4, D29, GS4E, (syn= GS4E), GS7, (syn= GS-7), (syn= GS7), IPa, lacticola, Legendre, Leo, L5, (syn= ΦL-5), MC-I, MC-3, MC-4, minetti, MTPHI I, Mx4, MyF3P/59a, phlei, (syn= phlei 1), phlei 4, Polonus II, rabinovitschi, smegmatis, TM4, TM9, TMIO, TM20, Y7, YIO, ϕ630, IB, IF, IH, 1/1, 67, 106, 1430, BI, (syn= Bol), B24, D, D29, F-K, F-S, HP, Polonus I, Roy, RI, (syn= RI-Myb), (syn= Ri), 11, 31, 40, 50, 103a, 103b, 128, 3111-D, 3215-D and NN-Mycobacterium (1).

Bacteria of the genus *Neisseria* are infected by the following phage: Group I, group II and NPI.

Bacteria of the genus *Nocardia* are infected by the following phage: MNP8, NJ-L, NS-8, N5 and TtiN-Nocardia.

Bacteria of the genus *Proteus* are infected by the following phage: Pm5, 13vir, 2/44, 4/545, 6/1004, 13/807, 20/826, 57, 67b, 78, 107/69, 121, 9/0, 22/608, 30/680, Pml, Pm3, Pm4, Pm6, Pm7, Pm9, PmIO, PmI I, Pv2, πI, ϕm, 7/549, 9B/2, 10A/31, 12/55, 14, 15, 16/789, 17/971, 19A/653, 23/532, 25/909, 26/219, 27/953, 32A/909, 33/971, 34/13, 65, 5006M, 7480b, VI, 13/3a, Clichy 12, π2600, ϕχ7, 1/1004, 5/742, 9, 12, 14, 22, 24/860, 2600/D52, Pm8 and 24/2514.

Bacteria of the genus *Providencia* are infected by the following phage: PL25, PL26, PL37, 9211/9295, 9213/921 Ib, 9248, 7/R49, 7476/322, 7478/325, 7479, 7480, 9000/9402 and 9213/921 Ia.

Bacteria of the genus *Pseudomonas* are infected by the following phage: Pfl, (syn= Pf-I), Pf2, Pf3, PP7, PRRI, 7s, im-Pseudomonas (1), AI-I, AI-2, B 17, B89, CB3, Col 2, Col 11, Col 18, Col 21, C154, C163, C167, C2121, E79, F8, ga, gb, H22, K1, M4, N2, Nu, PB-I, (syn= PBI), pfl6, PMN17, PPI, PP8, Psal, PsPI, PsP2, PsP3, PsP4, PsP5, PS3, PS17, PTB80, PX4, PX7, PYOI, PYO2, PYO5, PYO6, PYO9, PYOIO, PYO13, PYO14, PYO16, PYO18, PYO19, PYO20, PYO29, PYO32, PYO33, PYO35, PYO36, PYO37, PYO38, PYO39, PYO41, PYO42, PYO45, PYO47, PYO48, PYO64, PYO69, PYO103, PIK, SLPI, SL2, S2, UNL-I, wy, Yai, Ya4, Yan, ϕBE, ϕCTX, ϕC17, ϕKZ, (syn=ΦKZ), ϕ-LT, Φmu78, ϕNZ, ϕPLS-1, ϕST-1, ϕW-14, ϕ-2, 1/72, 2/79, 3, 3/DO, 4/237, 5/406, 6C, 6/6660, 7, 7v, 7/184, 8/280, 9/95, 10/502, 11/DE, 12/100, 12S, 16, 21, 24, 25F, 27, 31, 44, 68, 71, 95, 109, 188, 337, 352, 1214, HN-Pseudomonas (23), A856, B26, CI-I, CI-2, C5, D, gh-1, FI 16, HF, H90, K5, K6, KI 04, K109, K166, K267, N4, N5, O6N-25P, PE69, Pf, PPN25, PPN35, PPN89, PPN91, PP2, PP3, PP4, PP6, PP7, PP8, PP56, PP87, PPI 14, PP206, PP207, PP306, PP651, Psp231a, Pssy401, Pssy9220, psi, PTB2, PTB20, PTB42, PXI, PX3, PXIO, PX12, PX14, PYO70, PYO71, R, SH6, SH133, tf, Ya5, Ya7, ϕBS, ΦKf77, ϕ-MC, ΦmnF82, ϕPLS27, ϕPLS743, ϕS-1, 1, 2, 2, 3, 4, 5, 6, 7, 7, 8, 9, 10, 11, 12, 12B, 13, 14, 15, 14, 15, 16, 17, 18, 19, 20, 20, 21, 21, 22, 23, 23, 24, 25, 31, 53, 73, 119x, 145, 147, 170, 267, 284, 308, 525, NN-Pseudomonas (5), af, A7, B3, B33, B39, BI-I, C22, D3, D37, D40, D62, D3112, F7, FIO, g, gd, ge, gξ HwI2, Jb 19, KFI, L°, OXN-32P, O6N-52P, PCH-I, PC13-1, PC35-1, PH2, PH51, PH93, PH132, PMW, PM13, PM57, PM61, PM62, PM63, PM69, PM105, PMI 13, PM681, PM682, PO4, PPI, PP4, PP5, PP64, PP65, PP66, PP71, PP86, PP88, PP92, PP401, PP711, PP891, Pssy41, Pssy42, Pssy403, Pssy404, Pssy420, Pssy923, PS4, PS-IO, Pz, SDI, SLI, SL3, SL5, SM, ϕC5, ϕCI I, ϕCI I-1, ϕC13, ϕC15, ϕMO, ϕX, ϕO4, ϕI I, ϕ240, 2, 2F, 5, 7m, 11, 13, 13/441, 14, 20, 24, 40, 45, 49, 61, 73, 148, 160, 198, 218, 222, 236, 242, 246, 249, 258, 269, 295, 297, 309,318, 342, 350, 351, 357-1, 400-1, HN-Pseudomonas (6), GIOI, M6, M6a, LI, PB2, Pssyl5, Pssy4210, Pssy4220, PYO12, PYO34, PYO49, PYO50, PYO51, PYO52, PYO53, PYO57, PYO59, PYO200, PX2, PX5, SL4, ϕO3, ϕO6 and 1214.

Bacteria of the genus *Rickettsia* are infected by the following phage: NN-Rickettsia.

Bacteria of the genus *Salmonella* are infected by the following phage: b, Beccles, CT, d, Dundee, f, Fels 2, GI, GUI, GVI, GVIII, k, K, i, j, L, 01, (syn= 0-1), (syn= O1), (syn= O-I), (syn= 7), 02, 03, P3, P9a, PIO, Sab3, Sab5, SanIS, Sanl7, SI, Taunton, ViI, (syn= ViI), 9, imSalmonella (1), N-I, N-5, N-IO, N-17, N-22, 11, 12, 16-19, 20.2, 36, 449C/C178, 966A/C259, a, B.A.O.R., e, G4, GUI, L, LP7, M, MG40, N-18, PSA68, P4, P9c, P22, (syn= P22), (syn= PLT22), (syn= PLT22), P22al, P22-4, P22-7, P22-11, SNT- I, SNT-2, SP6, Villi, ViIV, ViV, ViVI, ViVII, Worksop, Sj5, ε34, 1,37, 1(40), (syn= ϕI[40]), 1,422, 2, 2.5, 3b, 4, 5, 6,14(18), 8, 14(6,7), 10, 27, 28B, 30, 31, 32, 33, 34, 36, 37, 39, 1412, SNT-3, 7-11, 40.3, c, C236, C557, C625, C966N, g, GV, G5, GI 73, h, IRA, Jersey, MB78, P22-1, P22-3, P22-12, Sabl, Sab2, Sab2, Sab4, Sanl, San2, San3, San4, San6, San7, San8, San9, Sanl3, Sanl4, SanI6, Sanl8, Sanl9, San20, San21, San22, San23, San24, San25, San26, SasLI, SasL2, SasL3, SasL4, SasL5, SIBL, SII, ViII, ϕI, 1, 2, 3a, 3al, 1010, Ym-Salmonella (1), N-4, SasL6 and 27.

Bacteria of the genus *Serratia* are infected by the following phage: A2P, PS20, SMB3, SMP, SMP5, SM2, V40, V56, ic, ΦOPP-3, ΦOPP-6, 3M, 10/la, 20A, 34CC, 34H, 38T, 345G, 345P, 501B, SMB2, SMP2, BC, BT, CW2, CW3, CW4, CW5, Lt232, L2232, L34, L.228, SLP, SMPA, V.43, σ, ϕCWI, ΦCPP6-1, ΦCPP6-2, ΦCP6-5, 3T, 5, 8, 9F, 10/1, 2OE, 32/6, 34B, 34CT, 34P, 37, 41, 56, 56D, 56P, 6OP, 61/6, 74/6, 76/4, 101/8900, 226, 227, 228, 229F, 286, 289, 290F, 512, 764a, 2847/10, 2847/1Oa, L.359 and SMBI.

Bacteria of the genus *Shigella* are infected by the following phage: Fsa, (syn=a), FSD2d, (syn= D2d), (syn= W2d), FSD2E, (syn= W2e), fv, F6, f7.8, H-Sh, PE5, P90, SfII, Sh, SHm, SHrv, (syn= HIV), SHvi, (syn= HVI), SHVvm, (syn= HVIII), SKγ66, (syn= gamma 66), (syn= yββ), (syn= γ66b), SKm, (syn= SIIIb)5 (syn= UI), SKw, (syn= Siva), (syn= IV), SIC^{™}, (syn= SIVA.), (syn= IVA), SKvi, (syn= KVI), (syn= Svi), (syn= VI), SKvm, (syn= Svm), (syn= VIII), SKVΠIA, (syn= SvmA), (syn= VIIIA), STvi, STK, STx1, STxn, S66, W2, (syn= D2c), (syn= D20), ϕI, ϕIVb 3-SO-R, 8368-SO-R, F7, (syn= FS7), (syn= K29), FIO, (syn= FSIO), (syn= K31), I1, (syn= alfa), (syn= FSa), (syn= KI 8), (syn= α), 12, (syn= a), (syn= K19), SG33, (syn= G35), (syn= SO-35/G), SG35, (syn= SO-55/G), SG3201, (syn= SO-3201/G), SHn, (syn= HII), SHv, (syn= SHV), SHx, SHX, SKn, (syn= K2), (syn= KII), (syn= Sn), (syn= Ssll), (syn= II), SKrv, (syn= Sm), (syn= SsIV), (syn= IV), SK1Va, (syn= Swab), (syn= SsIVa), (syn= IVa), SKV, (syn= K4), (syn= KV), (syn= SV), (syn= SsV), (syn= V), SKx, (syn= K9), (syn= KX), (syn= SX), (syn= SsX), (syn= X), STV, (syn= T35), (syn= 35-50-R), STvm, (syn= T8345), (syn= 8345-SO-S-R), W1, (syn= D8), (syn= FSD8), W2a, (syn= D2A), (syn= FS2a), DD-2, Sf6, FSi, (syn= FI), SF6, (syn= F6), SG42, (syn= SO-42/G), SG3203, (syn= SO-3203/G), SKF12, (syn= SsF12), (syn= F12), (syn= F12), STn, (syn= 1881-SO-R), γ66, (syn= gamma 66a), (syn= Ssγ66), ϕ2, BII, DDVII, (syn= DD7), FSD2b, (syn= W2B), FS2, (syn= F2), (syn= F2), FS4, (syn= F4), (syn= F4), FS5, (syn= F5), (syn= F5), FS9, (syn= F9), (syn= F9), FII, P2-S0-S, SG36, (syn= SO-36/G), (syn= G36), SG3204, (syn= SO-3204/G), SG3244, (syn= SO-3244/G), SHi, (syn= HI), SHvπ, (syn= HVII), SHK, (syn= HIX), SHx1, SHxπ, (syn= HXn), SKI, KI, (syn= S1), (syn= Ssl), SKVII, (syn= KVII), (syn= Svπ), (syn= SsVII), SKIX, (syn= KIX), (syn= S1x), (syn= SsIX), SKXII, (syn= KXII), (syn= Sxn), (syn= SsXII), STi, STffl, STrv, STVi, STvπ, S70, S206, U2-S0-S, 3210-SO-S, 3859-SO-S, 4020-SO-S, ϕ3, ϕ5, ϕ7, ϕ8, ϕ9, ϕIO, ϕI I, ϕI3, ϕI4, ϕI8, SHm, (syn=Hπi), SHχi, (syn= HXt) and SKxl, (syn= KXI), (syn= Sχi), (syn= SsXI), (syn= XI).

Bacteria of the genus *Staphylococcus* are infected by the following phage: A, EW, K, Ph5, Ph9, PhIO, PhI3, PI, P2, P3, P4, P8, P9, PIO, RG, SB-i, (syn= Sb-I), S3K, Twort, ΦSK311, ϕ812, 06, 40, 58, 119, 130, 131, 200, 1623, STCI, (syn=stcl), STC2, (syn=stc2), 44AHJD, 68, ACI, AC2, A6"C", A9"C", b581, CA-I, CA-2, CA-3, CA-4, CA-5, DII, L39x35, L54a, M42, NI, N2, N3, N4, N5, N7, N8, NIO, Ni I, N12, N13, N14, N16, Ph6, PhI2, PhI4, UC-18, U4, U15, SI, S2, S3, S4, S5, X2, Z1, ϕB5-2, ϕD, ω, 11, (syn= ϕII), (syn= P11-M15), 15, 28, 28A, 29, 31, 31B, 37, 42D, (syn= P42D), 44A, 48, 51, 52, 52A, (syn= P52A), 52B, 53, 55, 69, 71, (syn= P71), 71A, 72, 75, 76, 77, 79, 80, 80α, 82, 82A, 83 A, 84, 85, 86, 88, 88A, 89, 90, 92, 95, 96, 102, 107, 108, 111, 129-26, 130, 130A, 155, 157, 157A, 165, 187, 275, 275A, 275B, 356, 456, 459, 471, 471A, 489, 581, 676, 898, 1139, 1154A, 1259, 1314, 1380, 1405, 1563, 2148, 2638A, 2638B, 2638C, 2731, 2792A, 2792B, 2818, 2835, 2848A, 3619, 5841, 12100, AC3, A8, AIO, A13, b594n, D, HK2, N9, N15, P52, P87, SI, S6, Z4, ϕRE, 3A, 3B, 3C, 6, 7, 16, 21, 42B, 42C, 42E, 44, 47, 47A5 47C, 51, 54, 54x1, 70, 73, 75, 78, 81, 82, 88, 93, 94, 101, 105, 110, 115, 129/16, 174, 594n, 1363/14, 2460 and mS-Staphylococcus (1).

Bacteria of the genus *Streptococcus* are infected by the following phage: EJ-I, NN-Streptococais (1), a, Cl, FL0Ths, H39, Cp-I, Cp-5, Cp-7, Cp-9, Cp-IO, AT298, A5, aIO/JI, aIO/J2, aIO/J5, aIO/J9, A25, BTII, b6, CAI, c20-I, c20-2, DP-I, Dp-4, DTI, ET42, elO, FA101, FEThs, Fκ, FKKIOI, FKLIO, FKP74, FKH, FLOThs, FyIOI, fI, F10, F20140/76, g, GT-234, HB3, (syn= HB-3), HB-623, HB-746, M102, 01205, ϕO1205, PST, PO, PI, P2, P3, P5, P6, P8, P9, P9, P12, P13, P14, P49, P50, P51, P52, P53, P54, P55, P56, P57, P58, P59, P64, P67, P69, P71, P73, P75, P76, P77, P82, P83, P88, sc, sch, sf, SfII 1, (syn= SFiI I), (syn= ϕSFiII), (syn= ΦSfiI I), (syn= ϕSfiI I), sfil9, (syn= SFiI9), (syn= ϕSFiI9), (syn= ϕSfiI9), Sfi21, (syn= SFi21), (syn= ϕSFi21), (syn= ϕSfi21), ST0, STX, st2, ST2, ST4, S3, (syn= ϕS3), s265, Φ17, ϕ42, Φ57, ϕ80, ϕ81, ϕ82, ϕ83, ϕ84, ϕ85, ϕ86, ϕ87, ϕ88, ϕ89, ϕ90, ϕ91, ϕ92, ϕ93, ϕ94, ϕ95, ϕ96, ϕ97, ϕ98, ϕ99, ϕIOO, ϕIOI, ϕIO2, ϕ227, Φ7201, ωI, ω2, ω3, ω4, ω5, ω6, ω8, ωIO, 1, 6, 9, 1OF, 12/12, 14, 17SR, 19S, 24, 50/33, 50/34, 55/14, 55/15, 70/35, 70/36, 71/ST15, 71/45, 71/46, 74F, 79/37, 79/38, 80/J4, 80/J9, 80/ST16, 80/15, 80/47, 80/48, 101, 103/39, 103/40, 121/41, 121/42, 123/43, 123/44, 124/44, 337/ST17 and mStreptococcus (34).

Bacteria of the genus *Treponema* are infected by the following phage: NN-Treponema (1).

Bacteria of the genus *Vibrio* are infected by the following phage: CTXΦ, fs, (syn= si), fs2, Ivpf5, VfI2, Vf33, VPIΦ, VSK, v6, 493, CP-TI, ET25, kappa, K139, Labol, )XN-69P, OXN-86, O6N-21P, PB-I, P147, rp-1, SE3, VA-I, (syn= VcA-I), VcA-2, VPI, VP2, VP4, VP7, VP8, VP9, VPIO, VP17, VP18, VP19, X29, (syn= 29 d'Herelle), t, ΦHAWI-1, ΦHAWI-2, ΦHAWI-3, ΦHAWI-4, ΦHAWI-5, ΦHAWI-6, ΦHAWI-7, XHAWI-8, ΦHAWI-9, ΦHAWI-10, ΦHCI-1, ΦHC1-2, ΦHC1-3, ΦHC1-4, ΦHC2-1, >HC2-2, ΦHC2-3, ΦHC2-4, ΦHC3-1, ΦHC3-2, ΦHC3-3, ΦHD1S-1, ΦHD1S-2, ΦHD2S-1, ΦHD2S-2, ΦHD2S-3, ΦHD2S-4, ΦHD2S-5, ΦHDO-1, ΦHDO-2, ΦHDO-3, ΦHDO-4, ΦHDO-5, ΦHDO-6, ΦKL-33, ΦKL-34, ΦKL-35, ΦKL-36, ΦKWH-2, ΦKWH-3, ΦKWH-4, ΦMARQ-1, ΦMARQ-2, ΦMARQ-3, ΦMOAT-1, ΦO139, ΦPEL1A-1, ΦPEL1A-2, ΦPEL8A-1, ΦPEL8A-2, ΦPEL8A-3, ΦPEL8C-1, ΦPEL8C-2, ΦPEL13A-1, ΦPEL13B-1, ΦPEL13B-2, ΦPEL13B-3, ΦPEL13B-4, ΦPEL13B-5, ΦPEL13B-6, ΦPEL13B-7, ΦPEL13B-8, ΦPEL13B-9, ΦPEL13B-10, ϕVP143, ϕVP253, Φ16, ϕI38, 1- II, 5, 13, 14, 16, 24, 32, 493, 6214, 7050, 7227, II, (syn= group II), (syn== ϕ2), V, VIII, ∼m-Vibrio (13), KVP20, KVP40, nt-1, O6N-22P, P68, eI, e2, e3, e4, e5, FK, G, I, K, nt-6, NI, N2, N3, N4, N5, O6N-34P, OXN-72P, OXN-85P, OXN-100P, P, Ph-I, PL163/10, Q, S, T, ϕ92, 1-9, 37, 51, 57, 70A-8, 72A-4, 72A-10, 110A-4, 333, 4996, I (syn= group I), III (syn= group III), VI, (syn= A-Saratov), VII, IX, X, HN-Vibrio (6), pAI, 7, 7-8, 70A-2, 71A-6, 72A-5, 72A-8, 108A-10, 109A-6, 109A-8, I IOA-1, 110A-5, 110A-7, hv-1, OXN-52P, P13, P38, P53, P65, P108, Pill, TPI3 VP3, VP6, VP12, VP13, 70A-3, 70A-4, 70A-10, 72A-1, 108A-3, 109-B1, 110A-2, 149, (syn= ϕI49), IV, (syn= group IV), NN-Vibrio (22), VP5, VPII, VP15, VP16, αI, α2, a3a, a3b, 353B and HN-Vibrio (7).

Bacteria of the genus *Yersinia* are infected by the following phage: H, H-I, H-2, H-3, H-4, Lucas 110, Lucas 303, Lucas 404, YerA3, YerA7, YerA20, YerA41, 3/M64-76, 5/G394-76, 6/C753-76, 8/C239-76, 9/F18167, 1701, 1710, PST, 1/F2852-76, D'Herelle, EV, H, Kotljarova, PTB, R, Y, YerA41, ϕYerO3-12, 3, 4/C1324-76, 7/F783-76, 903, 1/M6176 and Yer2AT.

More preferably, the bacteriophage is selected in the group consisting of Salmonella virus SKML39, Shigella virus AG3, Dickeya virus Limestone, Dickeya virus RC2014, Escherichia virus CBA120, Escherichia virus Phaxl, Salmonella virus 38, Salmonella virus Det7, Salmonella virus GG32, Salmonella virus PM10, Salmonella virus SFP10, Salmonella virus SH19, Salmonella virus SJ3, Escherichia virus ECML4, Salmonella virus Marshall, Salmonella virus Maynard, Salmonella virus SJ2, Salmonella virus STML131, Salmonella virus ViI, Erwinia virus Ea2809, Klebsiella virus 0507KN21, Serratia virus IME250, Serratia virus MAM1, Campylobacter virus CP21, Campylobacter virus CP220, Campylobacter virus CPt10, Campylobacter virus IBB35, Campylobacter virus CP81, Campylobacter virus CP30A, Campylobacter virus CPX, Campylobacter virus NCTC12673, Erwinia virus Ea214, Erwinia virus M7, Escherichia virus AYO145A, Escherichia virus EC6, Escherichia virus HY02, Escherichia virus JH2, Escherichia virus TP1, Escherichia virus VpaE1, Escherichia virus wV8, Salmonella virus FelixO1, Salmonella virus HB2014, Salmonella virus Mushroom, Salmonella virus UAB87, Citrobacter virus Moogle, Citrobacter virus Mordin, Escherichia virus SUSP1, Escherichia virus SUSP2, Aeromonas virus phiO18P, Haemophilus virus HP1, Haemophilus virus HP2, Pasteurella virus F108, Vibrio virus K139, Vibrio virus Kappa, Burkholderia virus phi52237, Burkholderia virus phiE122, Burkholderia virus phiE202, Escherichia virus 186, Escherichia virus P4, Escherichia virus P2, Escherichia virus Wphi, Mannheimia virus PHL101, Pseudomonas virus phiCTX, Ralstonia virus RSA1, Salmonella virus Fels2, Salmonella virus PsP3, Salmonella virus SopEphi, Yersinia virus L413C, Staphylococcus virus G1, Staphylococcus virus G15, Staphylococcus virus JD7, Staphylococcus virus K, Staphylococcus virus MCE2014, Staphylococcus virus P108, Staphylococcus virus Rodi, Staphylococcus virus S253, Staphylococcus virus S25-4, Staphylococcus virus SA12, Listeria virus A511, Listeria virus P100, Staphylococcus virus Remus, Staphylococcus virus SA11, Staphylococcus virus Stau2, Bacillus virus Camphawk, Bacillus virus SPO1, Bacillus virus BCP78, Bacillus virus TsarBomba, Staphylococcus virus Twort, Enterococcus virus phiEC24C, Lactobacillus virus Lb338-1, Lactobacillus virus LP65, Enterobacter virus PG7, Escherichia virus CC31, Klebsiella virus JD18, Klebsiella virus PKO111, Escherichia virus Bp7, Escherichia virus IME08, Escherichia virus JS10, Escherichia virus JS98, Escherichia virus QL01, Escherichia virus VR5, Enterobacter virus Eap3, Klebsiella virus KP15, Klebsiella virus KP27, Klebsiella virus Matisse, Klebsiella virus Miro, Citrobacter virus Merlin, Citrobacter virus Moon, Escherichia virus JSE, Escherichia virus phil, Escherichia virus RB49, Escherichia virus HX01, Escherichia virus JS09, Escherichia virus RB69, Shigella virus UTAM, Salmonella virus S16, Salmonella virus STML198, Vibrio virus KVP40, Vibrio virus nt1, Vibrio virus VaIKK3, Escherichia virus VR7, Escherichia virus VR20, Escherichia virus VR25, Escherichia virus VR26, Shigella virus SP18, Escherichia virus AR1, Escherichia virus C40, Escherichia virus E112, Escherichia virus ECML134, Escherichia virus HY01, Escherichia virus Ime09, Escherichia virus RB3, Escherichia virus RB14, Escherichia virus T4, Shigella virus Pss1, Shigella virus Shfl2, Yersinia virus D1, Yersinia virus PST, Acinetobacter virus 133, Aeromonas virus 65, Aeromonas virus Aeh1, Escherichia virus RB16, Escherichia virus RB32, Escherichia virus RB43, Pseudomonas virus 42, Cronobacter virus CR3, Cronobacter virus CR8, Cronobacter virus CR9, Cronobacter virus PBES02, Pectobacterium virus phiTE, Cronobacter virus GAP31, Escherichia virus 4MG, Salmonella virus SE1, Salmonella virus SSE121, Escherichia virus FFH2, Escherichia virus FV3, Escherichia virus JES2013, Escherichia virus V5, Brevibacillus virus Abouo, Brevibacillus virus Davies, Bacillus virus Agate, Bacillus virus Bobb, Bacillus virus Bp8pC, Erwinia virus Deimos, Erwinia virus Ea35-70, Erwinia virus RAY, Erwinia virus Simmy50, Erwinia virus SpecialG, Acinetobacter virus AB1, Acinetobacter virus AB2, Acinetobacter virus AbC62, Acinetobacter virus AP22, Arthrobacter virus ArV1, Arthrobacter virus Trina, Bacillus virus AvesoBmore, Bacillus virus B4, Bacillus virus Bigbertha, Bacillus virus Riley, Bacillus virus Spock, Bacillus virus Troll, Bacillus virus Bastille, Bacillus virus CAM003, Bacillus virus Bc431, Bacillus virus Bcp1, Bacillus virus BCP82, Bacillus virus BM15, Bacillus virus Deepblue, Bacillus virus JBP901, Burkholderia virus Bcep1, Burkholderia virus Bcep43, Burkholderia virus Bcep781, Burkholderia virus BcepNY3, Xanthomonas virus OP2, Burkholderia virus BcepMu, Burkholderia virus phiE255, Aeromonas virus 44RR2, Mycobacterium virus Alice, Mycobacterium virus Bxz1, Mycobacterium virus Dandelion, Mycobacterium virus HyRo, Mycobacterium virus 13, Mycobacterium virus Nappy, Mycobacterium virus Sebata, Clostridium virus phiC2, Clostridium virus phiCD27, Clostridium virus phiCD119, Bacillus virus CP51, Bacillus virus JL, Bacillus virus Shanette, Escherichia virus CVM10, Escherichia virus ep3, Erwinia virus Asesino, Erwinia virus EaH2, Pseudomonas virus EL, Halomonas virus HAP1, Vibrio virus VP882, Brevibacillus virus Jimmer, Brevibacillus virus Osiris, Pseudomonas virus Ab03, Pseudomonas virus KPP10, Pseudomonas virus PAKP3, Sinorhizobium virus M7, Sinorhizobium virus M12, Sinorhizobium virus N3, Erwinia virus Machina, Arthrobacter virus Brent, Arthrobacter virus Jawnski, Arthrobacter virus Martha, Arthrobacter virus Sonny, Edwardsiella virus MSW3, Edwardsiella virus PEi21, Escherichia virus Mu, Shigella virus SfMu, Halobacterium virus phiH, Bacillus virus Grass, Bacillus virus NIT1, Bacillus virus SPG24, Aeromonas virus 43, Escherichia virus P1, Pseudomonas virus CAb1, Pseudomonas virus CAb02, Pseudomonas virus JG004, Pseudomonas virus PAKP1, Pseudomonas virus PAKP4, Pseudomonas virus PaP1, Burkholderia virus BcepF1, Pseudomonas virus 141, Pseudomonas virus Ab28, Pseudomonas virus DL60, Pseudomonas virus DL68, Pseudomonas virus F8, Pseudomonas virus JG024, Pseudomonas virus KPP12, Pseudomonas virus LBL3, Pseudomonas virus LMA2, Pseudomonas virus PB1, Pseudomonas virus SN, Pseudomonas virus PA7, Pseudomonas virus phiKZ, Rhizobium virus RHEph4, Ralstonia virus RSF1, Ralstonia virus RSL2, Ralstonia virus RSL1, Aeromonas virus 25, Aeromonas virus 31, Aeromonas virus Aes12, Aeromonas virus Aes508, Aeromonas virus AS4, Stenotrophomonas virus IME13, Staphylococcus virus IPLAC1C, Staphylococcus virus SEP1, Salmonella virus SPN3US, Bacillus virus 1, Geobacillus virus GBSV1, Yersinia virus R1RT, Yersinia virus TG1, Bacillus virus G, Bacillus virus PBS1, Microcystis virus Ma-LMM01, Vibrio virus MAR, Vibrio virus VHML, Vibrio virus VP585, Bacillus virus BPS13, Bacillus virus Hakuna, Bacillus virus Megatron, Bacillus virus WPh, Acinetobacter virus AB3, Acinetobacter virus Abp1, Acinetobacter virus Fri1, Acinetobacter virus IME200, Acinetobacter virus PD6A3, Acinetobacter virus PDAB9, Acinetobacter virus phiAB1, Escherichia virus K30, Klebsiella virus K5, Klebsiella virus K11, Klebsiella virus Kp1, Klebsiella virus KP32, Klebsiella virus KpV289, Klebsiella virus F19, Klebsiella virus K244, Klebsiella virus Kp2, Klebsiella virus KP34, Klebsiella virus KpV41, Klebsiella virus KpV71, Klebsiella virus KpV475, Klebsiella virus SU503, Klebsiella virus SU552A, Pantoea virus Limelight, Pantoea virus Limezero, Pseudomonas virus LKA1, Pseudomonas virus phiKMV, Xanthomonas virus f20, Xanthomonas virus f30, Xylella virus Prado, Erwinia virus Era103, Escherichia virus K5, Escherichia virus K1-5, Escherichia virus K1E, Salmonella virus SP6, Escherichia virus T7, Kluyvera virus Kvp1, Pseudomonas virus gh1, Prochlorococcus virus PSSP7, Synechococcus virus P60, Synechococcus virus Syn5, Streptococcus virus Cp1, Streptococcus virus Cp7, Staphylococcus virus 44AHJD, Streptococcus virus C1, Bacillus virus B103, Bacillus virus GA1, Bacillus virus phi29, Kurthia virus 6, Actinomyces virus Av1, Mycoplasma virus P1, Escherichia virus 24B, Escherichia virus 933W, Escherichia virus Min27, Escherichia virus PA28, Escherichia virus Stx2 II, Shigella virus 7502Stx, Shigella virus POCJ13, Escherichia virus 191, Escherichia virus PA2, Escherichia virus TL2011, Shigella virus VASD, Burkholderia virus Bcep22, Burkholderia virus BcepiI02, Burkholderia virus Bcepmigl, Burkholderia virus DC1, Bordetella virus BPP1, Burkholderia virus BcepC6B, Cellulophaga virus Cba41, Cellulophaga virus Cba172, Dinoroseobacter virus DFL12, Erwinia virus Ea9-2, Erwinia virus Frozen, Escherichia virus phiV10, Salmonella virus Epsilon15, Salmonella virus SPN1S, Pseudomonas virus F116, Pseudomonas virus H66, Escherichia virus APEC5, Escherichia virus APEC7, Escherichia virus Bp4, Escherichia virus EC1UPM, Escherichia virus ECBP1, Escherichia virus G7C, Escherichia virus IME11, Shigella virus Sb1, Achromobacter virus Axp3, Achromobacter virus JWAIpha, Edwardsiella virus KF1, Pseudomonas virus KPP25, Pseudomonas virus R18, Pseudomonas virus Ab09, Pseudomonas virus LIT1, Pseudomonas virus PA26, Pseudomonas virus Ab22, Pseudomonas virus CHU, Pseudomonas virus LUZ24, Pseudomonas virus PAA2, Pseudomonas virus PaP3, Pseudomonas virus PaP4, Pseudomonas virus TL, Pseudomonas virus KPP21, Pseudomonas virus LUZ7, Escherichia virus N4, Salmonella virus 9NA, Salmonella virus SP069, Salmonella virus BTP1, Salmonella virus HK620, Salmonella virus P22, Salmonella virus ST64T, Shigella virus Sf6, Bacillus virus Page, Bacillus virus Palmer, Bacillus virus Pascal, Bacillus virus Pony, Bacillus virus Pookie, Escherichia virus 172-1, Escherichia virus ECB2, Escherichia virus NJ01, Escherichia virus phiEco32, Escherichia virus Septima11, Escherichia virus SU10, Brucella virus Pr, Brucella virus Tb, Escherichia virus Pollock, Salmonella virus FSL SP-058, Salmonella virus FSL SP-076, Helicobacter virus 1961P, Helicobacter virus KHP30, Helicobacter virus KHP40, Hamiltonella virus APSE1, Lactococcus virus KSY1, Phormidium virus WMP3, Phormidium virus WMP4, Pseudomonas virus 119X, Roseobacter virus SIO1, Vibrio virus VpV262, Vibrio virus VC8, Vibrio virus VP2, Vibrio virus VP5, Streptomyces virus Amela, Streptomyces virus phiCAM, Streptomyces virus Aaronocolus, Streptomyces virus Caliburn, Streptomyces virus Danzina, Streptomyces virus Hydra, Streptomyces virus Izzy, Streptomyces virus Lannister, Streptomyces virus Lika, Streptomyces virus Sujidade, Streptomyces virus Zemlya, Streptomyces virus ELB20, Streptomyces virus R4, Streptomyces virus phiHau3, Mycobacterium virus Acadian, Mycobacterium virus Baee, Mycobacterium virus Reprobate, Mycobacterium virus Adawi, Mycobacterium virus Bane1, Mycobacterium virus BrownCNA, Mycobacterium virus Chrisnmich, Mycobacterium virus Cooper, Mycobacterium virus JAMaL, Mycobacterium virus Nigel, Mycobacterium virus Stinger, Mycobacterium virus Vincenzo, Mycobacterium virus Zemanar, Mycobacterium virus Apizium, Mycobacterium virus Manad, Mycobacterium virus Oline, Mycobacterium virus Osmaximus, Mycobacterium virus Pg1, Mycobacterium virus Soto, Mycobacterium virus Suffolk, Mycobacterium virus Athena, Mycobacterium virus Bernardo, Mycobacterium virus Gadjet, Mycobacterium virus Pipefish, Mycobacterium virus Godines, Mycobacterium virus Rosebush, Mycobacterium virus Babsiella, Mycobacterium virus Brujita, Mycobacterium virus Che9c, Mycobacterium virus Sbash, Mycobacterium virus Hawkeye, Mycobacterium virus Plot, Salmonella virus AG11, Salmonella virus Ent1, Salmonella virus f18SE, Salmonella virus Jersey, Salmonella virus L13, Salmonella virus LSPA1, Salmonella virus SE2, Salmonella virus SETP3, Salmonella virus SETP7, Salmonella virus SETP13, Salmonella virus SP101, Salmonella virus SS3e, Salmonella virus wksl3, Escherichia virus K1G, Escherichia virus K1H, Escherichia virus K1ind1, Escherichia virus K1ind2, Salmonella virus SP31, Leuconostoc virus Lmd1, Leuconostoc virus LN03, Leuconostoc virus LN04, Leuconostoc virus LN12, Leuconostoc virus LN6B, Leuconostoc virus P793, Leuconostoc virus 1A4, Leuconostoc virus Ln8, Leuconostoc virus Ln9, Leuconostoc virus LN25, Leuconostoc virus LN34, Leuconostoc virus LNTR3, Mycobacterium virus Bongo, Mycobacterium virus Rey, Mycobacterium virus Butters, Mycobacterium virus Michelle, Mycobacterium virus Charlie, Mycobacterium virus Pipsqueaks, Mycobacterium virus Xeno, Mycobacterium virus Panchino, Mycobacterium virus Phrann, Mycobacterium virus Redi, Mycobacterium virus Skinnyp, Gordonia virus BaxterFox, Gordonia virus Yeezy, Gordonia virus Kita, Gordonia virus Zirinka, Gorrdonia virus Nymphadora, Mycobacterium virus Bignuz, Mycobacterium virus Brusacoram, Mycobacterium virus Donovan, Mycobacterium virus Fishburne, Mycobacterium virus Jebeks, Mycobacterium virus Malithi, Mycobacterium virus Phayonce, Enterobacter virus F20, Klebsiella virus 1513, Klebsiella virus KLPN1, Klebsiella virus KP36, Klebsiella virus PKP126, Klebsiella virus Sushi, Escherichia virus AHP42, Escherichia virus AHS24, Escherichia virus AKS96, Escherichia virus C119, Escherichia virus E41c, Escherichia virus Eb49, Escherichia virus Jk06, Escherichia virus KP26, Escherichia virus Rogue1, Escherichia virus ACGM12, Escherichia virus Rtp, Escherichia virus ADB2, Escherichia virus JMPW1, Escherichia virus JMPW2, Escherichia virus T1, Shigella virus PSf2, Shigella virus Shfl1, Citrobacter virus Stevie, Escherichia virus TLS, Salmonella virus SP126, Cronobacter virus Esp2949-1, Pseudomonas virus Ab18, Pseudomonas virus Ab19, Pseudomonas virus PaMx11, Arthrobacter virus Amigo, Propionibacterium virus Anatole, Propionibacterium virus B3, Bacillus virus Andromeda, Bacillus virus Blastoid, Bacillus virus Curly, Bacillus virus Eoghan, Bacillus virus Finn, Bacillus virus Glittering, Bacillus virus Riggi, Bacillus virus Taylor, Gordonia virus Attis, Mycobacterium virus Barnyard, Mycobacterium virus Konstantine, Mycobacterium virus Predator, Mycobacterium virus Bernal13, Staphylococcus virus 13, Staphylococcus virus 77, Staphylococcus virus 108PVL, Mycobacterium virus Bron, Mycobacterium virus Faith1, Mycobacterium virus Joedirt, Mycobacterium virus Rumpelstiltskin, Lactococcus virus bIL67, Lactococcus virus c2, Lactobacillus virus c5, Lactobacillus virus Ld3, Lactobacillus virus Ld17, Lactobacillus virus Ld25A, Lactobacillus virus LLKu, Lactobacillus virus phiLdb, Cellulophaga virus Cba121, Cellulophaga virus Cba171, Cellulophaga virus Cba181, Cellulophaga virus ST, Bacillus virus 250, Bacillus virus IEBH, Mycobacterium virus Ardmore, Mycobacterium virus Avani, Mycobacterium virus Boomer, Mycobacterium virus Che8, Mycobacterium virus Che9d, Mycobacterium virus Deadp, Mycobacterium virus DIane, Mycobacterium virus Dorothy, Mycobacterium virus Dotproduct, Mycobacterium virus Drago, Mycobacterium virus Fruitloop, Mycobacterium virus Gumbie, Mycobacterium virus Ibhubesi, Mycobacterium virus Llij, Mycobacterium virus Mozy, Mycobacterium virus Mutaforma13, Mycobacterium virus Pacc40, Mycobacterium virus PMC, Mycobacterium virus Ramsey, Mycobacterium virus Rockyhorror, Mycobacterium virus SG4, Mycobacterium virus Shauna1, Mycobacterium virus Shilan, Mycobacterium virus Spartacus, Mycobacterium virus Taj, Mycobacterium virus Tweety, Mycobacterium virus Wee, Mycobacterium virus Yoshi, Salmonella virus Chi, Salmonella virus FSLSP030, Salmonella virus FSLSP088, Salmonella virus iEPS5, Salmonella virus SPN19, Mycobacterium virus 244, Mycobacterium virus Bask21, Mycobacterium virus CJW1, Mycobacterium virus Eureka, Mycobacterium virus Kostya, Mycobacterium virus Porky, Mycobacterium virus Pumpkin, Mycobacterium virus Sirduracell, Mycobacterium virus Toto, Mycobacterium virus Corndog, Mycobacterium virus Firecracker, Rhodobacter virus RcCronus, Pseudomonas virus D3112, Pseudomonas virus DMS3, Pseudomonas virus FHA0480, Pseudomonas virus LPB1, Pseudomonas virus MP22, Pseudomonas virus MP29, Pseudomonas virus MP38, Pseudomonas virus PA1KOR, Pseudomonas virus D3, Pseudomonas virus PMG1, Arthrobacter virus Decurro, Gordonia virus Demosthenes, Gordonia virus Katyusha, Gordonia virus Kvothe, Propionibacterium virus B22, Propionibacterium virus Doucette, Propionibacterium virus E6, Propionibacterium virus G4, Burkholderia virus phi6442, Burkholderia virus phi1026b, Burkholderia virus phiE125, Edwardsiella virus eiAU, Mycobacterium virus Ff47, Mycobacterium virus Muddy, Mycobacterium virus Gaia, Mycobacterium virus Giles, Arthrobacter virus Captnmurica, Arthrobacter virus Gordon, Gordonia virus GordTnk2, Paenibacillus virus Harrison, Escherichia virus EK99P1, Escherichia virus HK578, Escherichia virus JL1, Escherichia virus SSL2009a, Escherichia virus YD2008s, Shigella virus EP23, Sodalis virus SO1, Escherichia virus HK022, Escherichia virus HK75, Escherichia virus HK97, Escherichia virus HK106, Escherichia virus HK446, Escherichia virus HK542, Escherichia virus HK544, Escherichia virus HK633, Escherichia virus mEp234, Escherichia virus mEp235, Escherichia virus mEpX1, Escherichia virus mEpX2, Escherichia virus mEp043, Escherichia virus mEp213, Escherichia virus mEp237, Escherichia virus mEp390, Escherichia virus mEp460, Escherichia virus mEp505, Escherichia virus mEp506, Brevibacillus virus Jenst, Achromobacter virus 83-24, Achromobacter virus JWX, Arthrobacter virus Kellezzio, Arthrobacter virus Kitkat, Arthrobacter virus Bennie, Arthrobacter virus DrRobert, Arthrobacter virus Glenn, Arthrobacter virus HunterDalle, Arthrobacter virus Joann, Arthrobacter virus Korra, Arthrobacter virus Preamble, Arthrobacter virus Pumancara, Arthrobacter virus Wayne, Mycobacterium virus Alma, Mycobacterium virus Arturo, Mycobacterium virus Astro, Mycobacterium virus Backyardigan, Mycobacterium virus BBPiebs31, Mycobacterium virus Benedict, Mycobacterium virus Bethlehem, Mycobacterium virus Billknuckles, Mycobacterium virus Bruns, Mycobacterium virus Bxb1, Mycobacterium virus Bxz2, Mycobacterium virus Che12, Mycobacterium virus Cuco, Mycobacterium virus D29, Mycobacterium virus Doom, Mycobacterium virus Ericb, Mycobacterium virus Euphoria, Mycobacterium virus George, Mycobacterium virus Gladiator, Mycobacterium virus Goose, Mycobacterium virus Hammer, Mycobacterium virus Heldan, Mycobacterium virus Jasper, Mycobacterium virus JC27, Mycobacterium virus Jeffabunny, Mycobacterium virus JHC117, Mycobacterium virus KBG, Mycobacterium virus Kssjeb, Mycobacterium virus Kugel, Mycobacterium virus L5, Mycobacterium virus Lesedi, Mycobacterium virus LHTSCC, Mycobacterium virus lockley, Mycobacterium virus Marcell, Mycobacterium virus Microwolf, Mycobacterium virus Mrgordo, Mycobacterium virus Museum, Mycobacterium virus Nepal, Mycobacterium virus Packman, Mycobacterium virus Peaches, Mycobacterium virus Perseus, Mycobacterium virus Pukovnik, Mycobacterium virus Rebeuca, Mycobacterium virus Redrock, Mycobacterium virus Ridgecb, Mycobacterium virus Rockstar, Mycobacterium virus Saintus, Mycobacterium virus Skipole, Mycobacterium virus Solon, Mycobacterium virus Switzer, Mycobacterium virus SWU1, Mycobacterium virus Ta17a, Mycobacterium virus Tiger, Mycobacterium virus Timshel, Mycobacterium virus Trixie, Mycobacterium virus Turbido, Mycobacterium virus Twister, Mycobacterium virus U2, Mycobacterium virus Violet, Mycobacterium virus Wonder, Escherichia virus DE3, Escherichia virus HK629, Escherichia virus HK630, Escherichia virus Lambda, Arthrobacter virus Laroye, Mycobacterium virus Halo, Mycobacterium virus Liefie, Mycobacterium virus Marvin, Mycobacterium virus Mosmoris, Arthrobacter virus Circum, Arthrobacter virus Mudcat, Escherichia virus N15, Escherichia virus 9g, Escherichia virus JenK1, Escherichia virus JenP1, Escherichia virus JenP2, Pseudomonas virus NP1, Pseudomonas virus PaMx25, Mycobacterium virus Baka, Mycobacterium virus Courthouse, Mycobacterium virus Littlee, Mycobacterium virus Omega, Mycobacterium virus Optimus, Mycobacterium virus Thibault, Polaribacter virus P12002L, Polaribacter virus P12002S, Nonlabens virus P12024L, Nonlabens virus P12024S, Thermus virus P23-45, Thermus virus P74-26, Listeria virus LP26, Listeria virus LP37, Listeria virus LP110, Listeria virus LP114, Listeria virus P70, Propionibacterium virus ATCC29399BC, Propionibacterium virus ATCC29399BT, Propionibacterium virus Attacne, Propionibacterium virus Keiki, Propionibacterium virus Kubed, Propionibacterium virus Lauchelly, Propionibacterium virus MrAK, Propionibacterium virus Ouroboros, Propionibacterium virus P91, Propionibacterium virus P105, Propionibacterium virus P144, Propionibacterium virus P1001, Propionibacterium virus P1.1, Propionibacterium virus P100A, Propionibacterium virus P100D, Propionibacterium virus P101A, Propionibacterium virus P104A, Propionibacterium virus PA6, Propionibacterium virus Pacnes201215, Propionibacterium virus PAD20, Propionibacterium virus PAS50, Propionibacterium virus PHL009M11, Propionibacterium virus PHL025M00, Propionibacterium virus PHL037M02, Propionibacterium virus PHL041M10, Propionibacterium virus PHL060L00, Propionibacterium virus PHL067M01, Propionibacterium virus PHL070N00, Propionibacterium virus PHL071N05, Propionibacterium virus PHL082M03, Propionibacterium virus PHL092M00, Propionibacterium virus PHL095N00, Propionibacterium virus PHL111M01, Propionibacterium virus PHL112N00, Propionibacterium virus PHL113M01, Propionibacterium virus PHL114L00, Propionibacterium virus PHL116M00, Propionibacterium virus PHL117M00, Propionibacterium virus PHL117M01, Propionibacterium virus PHL132N00, Propionibacterium virus PHL141N00, Propionibacterium virus PHL151M00, Propionibacterium virus PHL151N00, Propionibacterium virus PHL152M00, Propionibacterium virus PHL163M00, Propionibacterium virus PHL171M01, Propionibacterium virus PHL179M00, Propionibacterium virus PHL194M00, Propionibacterium virus PHL199M00, Propionibacterium virus PHL301M00, Propionibacterium virus PHL308M00, Propionibacterium virus Pirate, Propionibacterium virus Procrass1, Propionibacterium virus SKKY, Propionibacterium virus Solid, Propionibacterium virus Stormborn, Propionibacterium virus Wizzo, Pseudomonas virus PaMx28, Pseudomonas virus PaMx74, Mycobacterium virus Patience, Mycobacterium virus PBI1, Rhodococcus virus Pepy6, Rhodococcus virus Poco6, Propionibacterium virus PFR1, Streptomyces virus phiBT1, Streptomyces virus phiC31, Streptomyces virus TG1, Caulobacter virus Karma, Caulobacter virus Magneto, Caulobacter virus phiCbK, Caulobacter virus Rogue, Caulobacter virus Swift, Staphylococcus virus 11, Staphylococcus virus 29, Staphylococcus virus 37, Staphylococcus virus 53, Staphylococcus virus 55, Staphylococcus virus 69, Staphylococcus virus 71, Staphylococcus virus 80, Staphylococcus virus 85, Staphylococcus virus 88, Staphylococcus virus 92, Staphylococcus virus 96, Staphylococcus virus 187, Staphylococcus virus 52a, Staphylococcus virus 80alpha, Staphylococcus virus CNPH82, Staphylococcus virus EW, Staphylococcus virus IPLA5, Staphylococcus virus IPLA7, Staphylococcus virus IPLA88, Staphylococcus virus PH15, Staphylococcus virus phiETA, Staphylococcus virus phiETA2, Staphylococcus virus phiETA3, Staphylococcus virus phiMR11, Staphylococcus virus phiMR25, Staphylococcus virus phiNM1, Staphylococcus virus phiNM2, Staphylococcus virus phiNM4, Staphylococcus virus SAP26, Staphylococcus virus X2, Enterococcus virus FL1, Enterococcus virus FL2, Enterococcus virus FL3, Lactobacillus virus ATCC8014, Lactobacillus virus phiJL1, Pediococcus virus cIP1, Aeromonas virus pIS4A, Listeria virus LP302, Listeria virus PSA, Methanobacterium virus psiM1, Roseobacter virus RDJL1, Roseobacter virus RDJL2, Rhodococcus virus RER2, Enterococcus virus BC611, Enterococcus virus IMEEF1, Enterococcus virus SAP6, Enterococcus virus VD13, Streptococcus virus SPQS1, Mycobacterium virus Papyrus, Mycobacterium virus Send513, Burkholderia virus KL1, Pseudomonas virus 73, Pseudomonas virus Ab26, Pseudomonas virus Kakheti25, Escherichia virus Cajan, Escherichia virus Seurat, Staphylococcus virus SEP9, Staphylococcus virus Sextaec, Streptococcus virus 858, Streptococcus virus 2972, Streptococcus virus ALQ132, Streptococcus virus 01205, Streptococcus virus Sfi11, Streptococcus virus 7201, Streptococcus virus DT1, Streptococcus virus phiAbc2, Streptococcus virus Sfi19, Streptococcus virus Sfi21, Paenibacillus virus Diva, Paenibacillus virus Hb10c2, Paenibacillus virus Rani, Paenibacillus virus Shelly, Paenibacillus virus Sitara, Paenibacillus virus Willow, Lactococcus virus 712, Lactococcus virus ASCC191, Lactococcus virus ASCC273, Lactococcus virus ASCC281, Lactococcus virus ASCC465, Lactococcus virus ASCC532, Lactococcus virus Bibb29, Lactococcus virus bIL170, Lactococcus virus CB13, Lactococcus virus CB14, Lactococcus virus CB19, Lactococcus virus CB20, Lactococcus virus jj50, Lactococcus virus P2, Lactococcus virus P008, Lactococcus virus sk1, Lactococcus virus SI4, Bacillus virus Slash, Bacillus virus Stahl, Bacillus virus Staley, Bacillus virus Stills, Gordonia virus Bachita, Gordonia virus ClubL, Gordonia virus OneUp, Gordonia virus Smoothie, Gordonia virus Soups, Bacillus virus SPbeta, Vibrio virus MAR10, Vibrio virus SSP002, Escherichia virus AKFV33, Escherichia virus BF23, Escherichia virus DT57C, Escherichia virus EPS7, Escherichia virus FFH1, Escherichia virus H8, Escherichia virus slur09, Escherichia virus T5, Salmonella virus 118970saI2, Salmonella virus Shivani, Salmonella virus SPC35, Salmonella virus Stitch, Arthrobacter virus Tank, Tsukamurella virus TIN2, Tsukamurella virus TIN3, Tsukamurella virus TIN4, Rhodobacter virus RcSpartan, Rhodobacter virus RcTitan, Mycobacterium virus Anaya, Mycobacterium virus Angelica, Mycobacterium virus Crimd, Mycobacterium virus Fionnbarth, Mycobacterium virus Jaws, Mycobacterium virus Larva, Mycobacterium virus Macncheese, Mycobacterium virus Pixie, Mycobacterium virus TM4, Bacillus virus BMBtp2, Bacillus virus TP21, Geobacillus virus Tp84, Staphylococcus virus 47, Staphylococcus virus 3a, Staphylococcus virus 42e, Staphylococcus virus IPLA35, Staphylococcus virus phi12, Staphylococcus virus phiSLT, Mycobacterium virus 32HC, Rhodococcus virus RGL3, Paenibacillus virus Vegas, Gordonia virus Vendetta, Bacillus virus Wbeta, Mycobacterium virus Wildcat, Gordonia virus Twister6, Gordonia virus Wizard, Gordonia virus Hotorobo, Gordonia virus Monty, Gordonia virus Woes, Xanthomonas virus CP1, Xanthomonas virus OP1, Xanthomonas virus phil7, Xanthomonas virus Xop411, Xanthomonas virus Xp10, Streptomyces virus TP1604, Streptomyces virus YDN12, Alphaproteobacteria virus phiJI001, Pseudomonas virus LKO4, Pseudomonas virus M6, Pseudomonas virus MP1412, Pseudomonas virus PAE1, Pseudomonas virus Yua, Pseudoalteromonas virus PM2, Pseudomonas virus phi6, Pseudomonas virus phi8, Pseudomonas virus phi12, Pseudomonas virus phi13, Pseudomonas virus phi2954, Pseudomonas virus phiNN, Pseudomonas virus phiYY, Vibrio virus fs1, Vibrio virus VGJ, Ralstonia virus RS603, Ralstonia virus RSM1, Ralstonia virus RSM3, Escherichia virus M13, Escherichia virus I22, Salmonella virus IKe, Acholeplasma virus L51, Vibrio virus fs2, Vibrio virus VFJ, Escherichia virus If1, Propionibacterium virus B5, Pseudomonas virus Pf1, Pseudomonas virus Pf3, Ralstonia virus PE226, Ralstonia virus RSS1, Spiroplasma virus SVTS2, Stenotrophomonas virus PSH1, Stenotrophomonas virus SMA6, Stenotrophomonas virus SMA7, Stenotrophomonas virus SMA9, Vibrio virus CTXphi, Vibrio virus KSF1, Vibrio virus VCY, Vibrio virus Vf33, Vibrio virus VfO3K6, Xanthomonas virus Cf1c, Spiroplasma virus C74, Spiroplasma virus R8A2B, Spiroplasma virus SkV1CR23x, Escherichia virus FI, Escherichia virus Qbeta, Escherichia virus BZ13, Escherichia virus MS2, Escherichia virus alpha3, Escherichia virus ID21, Escherichia virus ID32, Escherichia virus ID62, Escherichia virus NC28, Escherichia virus NC29, Escherichia virus NC35, Escherichia virus phiK, Escherichia virus St1, Escherichia virus WA45, Escherichia virus G4, Escherichia virus ID52, Escherichia virus Talmos, Escherichia virus phiX174, Bdellovibrio virus MAC1, Bdellovibrio virus MH2K, Chlamydia virus Chp1, Chlamydia virus Chp2, Chlamydia virus CPAR39, Chlamydia virus CPG1, Spiroplasma virus SpV4, Acholeplasma virus L2, Pseudomonas virus PR4, Pseudomonas virus PRD1, Bacillus virus AP50, Bacillus virus Bam35, Bacillus virus GIL16, Bacillus virus Wip1, Escherichia virus phi80, Escherichia virus RB42, Escherichia virus T2, Escherichia virus T3, Escherichia virus T6, Escherichia virus VT2-Sa, Escherichia virus VT1-Sakai, Escherichia virus VT2-Sakai, Escherichia virus CP-933V, Escherichia virus P27, Escherichia virus Stx2phi-I, Escherichia virus Stx1phi, Escherichia virus Stx2phi-II, Escherichia virus CP-1639,, based on the Escherichia virus BP-4795, Escherichia virus 86, Escherichia virus Min27, Escherichia virus 2851, Escherichia virus 1717, Escherichia virus YYZ-2008, Escherichia virus EC026_P06, Escherichia virus ECO103_P15, Escherichia virus EC0103_P12, Escherichia virus ECO111_P16, Escherichia virus ECO111_P11, Escherichia virus VT2phi_272, Escherichia virus TL-2011c, Escherichia virus P13374, Escherichia virus Sp5.

In one embodiment, the bacterial virus particles target *E. coli* and includes the capsid of a bacteriophage selected in the group consisting of BW73, B278, D6, D108, E, EI, E24, E41, FI-2, FI-4, FI-5, HI8A, FfI8B, i, MM, Mu, 025, Phl-5, Pk, PSP3, PI, PID, P2, P4, SI, Wϕ, ϕK13, ϕI, ϕ2, ϕ7, ϕ92, 7A, 8ϕ, 9ϕ, 18, 28-1, 186, 299, HH-Escherichia (2), AB48, CM, C4, C16, DD-VI, E4, E7, E28, FII, FI3, H, HI, H3, H8, K3, M, N, ND-2, ND-3, ND4, ND-5, ND6, ND-7, Ox-I, Ox-2, Ox-3, Ox-4, Ox-5, Ox-6, PhI-I, RB42, RB43, RB49, RB69, S, Sal-I, Sal-2, Sal-3, Sal-4, SaI-5, SaI-6, TC23, TC45, TuII*-6, TuIP-24, TuII*46, TuIP-60, T2, T4, T6, T35, αI, 1, IA, 3, 3A, 3T+, 5ϕ, 9266Q, CFO103, HK620, J, K, KIF, m59, no. A, no. E, no. 3, no. 9, N4, sd, T3, T7, WPK, W31, ΔH, ϕC3888, ϕK3, ϕK7, ϕK12, ϕV-1, Φ04-CF, Φ05, Φ06, Φ07, ϕI, ϕI.2, ϕ20, ϕ95, ϕ263, ϕIO92, ϕI, ϕII, Ω8, 1, 3, 7, 8, 26, 27, 28-2, 29, 30, 31, 32, 38, 39, 42, 933W, NN-Escherichia (1), Esc-7-11, AC30, CVX-5, CI, DDUP, ECI, EC2, E21, E29, FI, F26S, F27S, Hi, HK022, HK97, HK139, HK253, HK256, K7, ND-I, PA-2, q, S2, TI, ), T3C, T5, UC-I, w, β4, γ2, λ, ΦD326, ϕγ, Φ06, Φ7, Φ10, ϕ80, χ, 2, 4, 4A, 6, 8A, 102, 150, 168, 174, 3000, AC6, AC7, AC28, AC43, AC50, AC57, AC81, AC95, HK243, KIO, ZG/3A, 5, 5A, 21EL, H19-J and 933H.

In some embodiment the vectors disclosed herein may be used in combination with prebiotics. Prebiotics include, but are not limited to, amino acids, biotin, fructo-oligosaccharide, galacto-oligosaccharides, hemicelluloses (e.g., arabinoxylan, xylan, xyloglucan, and glucomannan), inulin, chitin, lactulose, mannan oligosaccharides, oligofructose-enriched inulin, gums (e.g., guar gum, gum arabic and carrageenan), oligofructose, oligodextrose, tagatose, resistant maltodextrins (e.g., resistant starch), trans- galactooligosaccharide, pectins (e.g., xylogalactouronan, citrus pectin, apple pectin, and rhamnogalacturonan-I), dietary fibers (e.g., soy fiber, sugarbeet fiber, pea fiber, corn bran, and oat fiber) and xylooligosaccharides.

In other embodiment, the vectors disclosed herein may be used in combination with probiotics. Probiotics include, but are not limited to lactobacilli, bifidobacteria, streptococci, enterococci, propionibacteria, saccharomycetes, lactobacilli, bifidobacteria, or proteobacteria.

The probiotic strain can take over the ecological niche left by elimination of the AMR strains from the patient's intestine, further preventing AMR strains from bouncing back.

### Pharmaceutical and Veterinary Compositions and In situ Administration Methods

The invention encompasses pharmaceutical and veterinary compositions comprising at least one, at least two, at least three, at least four, at least five, at least six, at least seven, at least eight, at least nine, at least ten or more vectors of the invention, in particular different vectors of the invention, and, optionally antibiotics.

The invention encompasses pharmaceutical and veterinary compositions comprising at one, at least two, at least three, at least four, at least five, at least six, at least seven, at least eight, at least nine, at least ten or more engineered phages of the invention, in particular different engineered phages of the invention, and optionally antibiotics.

The invention encompasses a pharmaceutical agent which reduces the amount of AMR bacteria in a subject.

In some embodiments, the selective decolonization occurs before the subject or patient undergoes invasive procedures (surgery, intubation, catheterization, etc.) or harsh conditioning procedures (immunosuppression, irradiation, etc.) that typically compromise epithelial barriers and promote dissemination of bacteria and infections in patients. In one embodiment, the selective decolonization occurs before the subject or patient undergoes surgery. In a preferred embodiment, the surgery is a solid organ transplant surgery of the kidney or liver. In some embodiments, the subject or patient is in the ICU and/or is to undergo surgery, dialysis, or a transplant.

Preferably , the subject or patient presents a skin, nasal, or intestinal carriage of AMR bacteria. In some embodiments, the subject or patient presents Methicillin resistant Staphylococcus aureus (MRSA), an Extended Spectrum beta lactamase (ESBL) and/or Carbapenemase-producing (CP) Enterobacteria.

In some embodiments, the subject or patient carries *Klebsiella pneumoniae* of the K-type K2, K1, K62, K64, K23, K28, K54, K16, K5, K27 or combinations thereof. In some embodiments, the subject or patient carries *Klebsiella pneumoniae* of the K-type K2, K1, K62, K64 or combinations thereof. In some embodiments, the subject or patient carries *Klebsiella pneumoniae* of the K-type K23, K28, K54, K16, or combinations thereof. In some embodiments, the subject or patient carries *Klebsiella pneumoniae* of the K-type K5, K27, or combinations thereof. In some embodiments, the subject or patients carries *Klebsiella pneumoniae* of the K-type K24, K2, K23, K71, K62, K60, K41, K28, K22, K37, K17, K16, K64, K1, K15, K50, K51, K58, or combinations thereof. In some embodiments, the subject or patient carries *Klebsiella pneumoniae* of the K-type K24, K2, K23, K71, or combinations thereof. In some embodiments, the subject or patients carries *Klebsiella pneumoniae* of the K-type K62, K60, K41, K28, K22, K37, K17, K16, K64, K1, K15, K50, K51, K58, or combinations thereof. In some embodiments, the subject or patient carries *Klebsiella pneumoniae* of the O-type O1, O2, O3, O5, O4, 012, OL101, OL104 or combinations thereof. In some embodiments, the subject or patient carries *Klebsiella pneumoniae* of the O-type O1, O2, O3, O5, or combinations thereof. In some embodiments, the subject or patient carries *Klebsiella pneumoniae* of the O-type O4, O12, OL101, OL104 or combinations thereof. In some embodiments, the subject or patient carries *Klebsiella pneumoniae* strain MLST258.

In some embodiments, the subject or patient carries *Escherichia coli of* the K type K1, K5 or combinations thereof. In some embodiments, the subject or patient carries *Escherichia coli of* the O-type O1, O2, O6, O25, 018, or combinations thereof. In some embodiments, the subject or patient carries *Escherichia coli of* the O-type O1, O2? O4, O6, O7, O8, O16, O25, O75, or combinations thereof. In some embodiments, the subject or patient carries bacteria selected from the group consisting of *Escherichia coli* strains MLST131, ST73, ST95, ST12, ST10, ST14, ST2278, ST69, and combinations thereof.

The subject treated in the context of the invention is a healthy subject or a patient.

By "healthy subject" is meant herein a subject who does not suffer from a bacterial infection, in particular from an infection caused by antibiotic-resistant bacteria, at the time of administration of the vector of the invention.

By "patient" is meant herein a subject who does not suffer from a bacterial infection, in particular from an infection caused by antibiotic-resistant bacteria, at the time of administration of the vector of the invention, but suffers from another disease such as a disease necessitating invasive or non-invasive medical procedures, for example transplant, in particular solid organ transplant or hematopoietic stem cells transplant, dialysis, immunosuppression or irradiation. In a particular embodiment, said patient suffers from cirrhosis.

In a particular embodiment, said patient suffers from cancer, in particular from a hematologic malignancy. In a particular embodiment, said patient suffers chemotherapy-induced neutropenia and/or chemotherapy-induced gastrointestinal mucositis.

In a particular embodiment, said patient suffers from neutropenia and//or mucositis.

In a particular embodiment, said patient is immunosuppressed. In an alternative embodiment, said patient is not immunosuppressed.

In a particular embodiment, said patient undergoes prolonged hospitalization.

As will be understood by the skilled person, despite the healthy subject or patient treated in the context of the invention is not suffering from a bacterial infection, in particular from an infection caused by antibiotic-resistant bacteria, at the time of administration of the vector of the invention, the healthy subject or patient can carry AMR bacteria.

The invention encompasses *in situ* administration of the pharmaceutical or veterinary composition to the bacteria in a subject. Any method known to the skilled artisan can be used to contact the composition with the bacterial target *in situ.*

In a particular embodiment, the pharmaceutical or veterinary composition of the invention is administered before and/or after the subject or patient undergoes invasive procedures (surgery, intubation, catheterization, etc.) or harsh conditioning procedures (immunosuppression, irradiation, etc.). In one embodiment, the pharmaceutical or veterinary composition of the invention is administered before and/or after the subject or patient undergoes surgery. In a preferred embodiment, the surgery is a solid organ transplant surgery of the kidney or liver. In some embodiments, the subject or patient is in the ICU and/or is to undergo surgery, dialysis, or a transplant. In one embodiment, the pharmaceutical or veterinary composition of the invention is administered before and/or after the subject or patient ungers hematopoietic stem cell transplantation.

In the context of the invention, the pharmaceutical or veterinary composition of the invention is administered before the subject or patient presents any sign of bacterial infection, in particular any sign of AR bacterial infection.

In one embodiment, the composition comprises an effective amount of at least one, 2, 3, 4, 5, 6, 7, 8, 9, 10 or more, antibiotic, phage, recombinant phage, packaged phagemid, plasmid, plasmid inside a carrier bacterium, or combination thereof.

In one embodiment, the antibiotic is selected from methicillin, streptomycin, vancomycin, clindamycin, metronidazole, sulphadoxine, trimethoprim, or any combination of 1, 2, 3, 4, 5 or 6 of these antibiotics.

In various embodiments, the phage, recombinant phage, packaged phagemid or plasmid encodes a nuclease selected from CRISPR-Cas, TALENs and variants, zinc finger nuclease (ZFN) and ZFN variants, natural, evolved or engineered meganuclease or recombinase variants.

In various embodiments, the phage, recombinant phage, packaged phagemid or plasmid encodes a base editor or a prime editor.

The pharmaceutical or veterinary composition according to the invention may further comprise a pharmaceutically acceptable vehicle. A solid pharmaceutically acceptable vehicle may include one or more substances which may also act as flavoring agents, lubricants, solubilizers, suspending agents, dyes, fillers, glidants, compression aids, inert binders, sweeteners, preservatives, dyes, coatings, or tablet- disintegrating agents. Suitable solid vehicles include, for example calcium phosphate, magnesium stearate, talc, sugars, lactose, dextrin, starch, gelatin, cellulose, polyvinylpyrrolidone, low melting waxes and ion exchange resins.

The pharmaceutical or veterinary composition may be prepared as a sterile solid composition that may be suspended at the time of administration using sterile water, saline, or other appropriate sterile injectable medium. The pharmaceutical or veterinary compositions of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The particles according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for enteral administration include sterile solutions, emulsions, and suspensions.

The bacterial delivery vehicles according to the invention, in particular the bacterial virus particles according to the invention, may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavouring agents, suspending agents, thickening agents, colours, viscosity regulators, stabilizers or osmo-regulators. Suitable examples of liquid vehicles for oral and enteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for enteral administration. The liquid vehicle for pressurized compositions can be a halogenated hydrocarbon or other pharmaceutically acceptable propellant.

In some embodiments, the invention encompasses pharmaceutical or veterinary composition formulated for delayed or gradual enteric release. In preferred embodiments, formulations or pharmaceutical preparations of the invention are formulated for delivery of the vector into the distal small bowel and/or the colon. The formulation can allow the vector to pass through stomach acid and pancreatic enzymes and bile, and reach undamaged to be viable in the distal small bowel and colon.

In some embodiments, the pharmaceutical or veterinary composition is micro-encapsulated, formed into tablets and/or placed into capsules, preferably enteric-coated capsules.

In some embodiments, the pharmaceutical or veterinary compositions are formulated for delayed or gradual enteric release, using cellulose acetate (CA) and polyethylene glycol (PEG). In some embodiments, the pharmaceutical or veterinary compositions are formulated for delayed or gradual enteric release using a hydroxypropylmethylcellulose (HPMC), a microcrystalline cellulose (MCC) and magnesium stearate. the pharmaceutical or veterinary compositions are formulated for delayed or gradual enteric release using e.g., a poly(meth)acrylate, e.g. a methacrylic acid copolymer B, a methyl methacrylate and/or a methacrylic acid ester, or a polyvinylpyrrolidone (PVP).

In some embodiments, the pharmaceutical or veterinary compositions are formulated for delayed or gradual enteric release using a release-retarding matrix material such as: an acrylic polymer, a cellulose, a wax, a fatty acid, shellac, zein, hydrogenated vegetable oil, hydrogenated castor oil, polyvinylpyrrolidone, a vinyl acetate copolymer, a vinyl alcohol copolymer, polyethylene oxide, an acrylic acid and methacrylic acid copolymer, a methyl methacrylate copolymer, an ethoxyethyl methacrylate polymer, a cyanoethyl methacrylate polymer, an aminoalkyl methacrylate copolymer, a poly(acrylic acid), a poly(methacrylic acid), a methacrylic acid alkylamide copolymer, a poly(methyl methacrylate), a poly(methacrylic acid anhydride), a methyl methacrylate polymer, a polymethacrylate, a poly(methyl methacrylate) copolymer, a polyacrylamide, an aminoalkyl methacrylate copolymer, a glycidyl methacrylate copolymer, a methyl cellulose, an ethylcellulose, a carboxymethylcellulose, a hydroxypropylmethylcellulose, a hydroxymethyl cellulose, a hydroxyethyl cellulose, a hydroxypropyl cellulose, a crosslinked sodium carboxymethylcellulose, a crosslinked hydroxypropylcellulose, a natural wax, a synthetic wax, a fatty alcohol, a fatty acid, a fatty acid ester, a fatty acid glyceride, a hydrogenated fat, a hydrocarbon wax, stearic acid, stearyl alcohol, beeswax, glycowax, castor wax, carnauba wax, a polylactic acid, polyglycolic acid, a co-polymer of lactic and glycolic acid, carboxymethyl starch, potassium methacrylate/divinylbenzene copolymer, crosslinked polyvinylpyrrolidone, polyvinylalcohols, polyvinylalcohol copolymers, polyethylene glycols, non-crosslinked polyvinylpyrrolidone, polyvinyl acetates, polyvinylacetate copolymers or any combination thereof.

In some embodiments, the pharmaceutical or veterinary compositions are formulated for delayed or gradual enteric release as described in U.S. Pat. App. Pub. 20110218216, which describes an extended release pharmaceutical composition for oral administration, and uses a hydrophilic polymer, a hydrophobic material and a hydrophobic polymer or a mixture thereof, with a microenvironment pH modifier. The hydrophobic polymer can be ethylcellulose, cellulose acetate, cellulose propionate, cellulose butyrate, methacrylic acid-acrylic acid copolymers or a mixture thereof. The hydrophilic polymer can be polyvinylpyrrolidone, hydroxypropylcellulose, methylcellulose, hydroxypropylmethyl cellulose, polyethylene oxide, acrylic acid copolymers or a mixture thereof. The hydrophobic material can be a hydrogenated vegetable oil, hydrogenated castor oil, carnauba wax, candellia wax, beeswax, paraffin wax, stearic acid, glyceryl behenate, cetyl alcohol, cetostearyl alcohol or and a mixture thereof. The microenvironment pH modifier can be an inorganic acid, an amino acid, an organic acid or a mixture thereof. Alternatively, the microenvironment pH modifier can be lauric acid, myristic acid, acetic acid, benzoic acid, palmitic acid, stearic acid, oxalic acid, malonic acid, succinic acid, adipic acid, sebacic acid, fumaric acid, maleic acid; glycolic acid, lactic acid, malic acid, tartaric acid, citric acid, sodium dihydrogen citrate, gluconic acid, a salicylic acid, tosylic acid, mesylic acid or malic acid or a mixture thereof.

In some embodiments, the pharmaceutical or veterinary compositions are a powder that can be included into a tablet or a suppository. In alternative embodiments, a formulation or pharmaceutical preparation of the invention can be a "powder for reconstitution" as a liquid to be drunk or otherwise administered.

In some embodiments, the pharmaceutical or veterinary compositions can be administered in a cream, gel, lotion, liquid, feed, or aerosol spray. In some embodiments, a bacteriophage is immobilized to a solid surface using any substance known in the art and any technology known in the art, for example, but not limited to immobilization of bacteriophages onto polymeric beads using technology as outlined in U.S. Pat. No. 7,482,115, which is incorporated herein by reference. Phages may be immobilized onto appropriately sized polymeric beads so that the coated beads may be added to aerosols, creams, gels or liquids. The size of the polymeric beads may be from about 0.1 µm to 500 µm, for example 50 µm to 100 µm. The coated polymeric beads may be incorporated into animal feed, including pelleted feed and feed in any other format, incorporated into any other edible device used to present phage to the animals, added to water offered to animals in a bowl, presented to animals through water feeding systems. In some embodiments, the compositions are used for treatment of surface wounds and other surface infections using creams, gels, aerosol sprays and the like.

In some embodiments, the pharmaceutical or veterinary compositions can be administered by inhalation, in the form of a suppository or pessary, topically (e.g., in the form of a lotion, solution, cream, ointment or dusting powder), epi- or transdermally (e.g., by use of a skin patch), orally (e.g., as a tablet, which may contain excipients such as starch or lactose), as a capsule, ovule, elixirs, solutions, or suspensions (each optionally containing flavoring, coloring agents and/or excipients), or they can be injected parenterally (e.g., intravenously, intramuscularly or subcutaneously). For parenteral administration, the compositions may be used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner. In a preferred embodiment, a bacteriophage and/or polypeptide of the present invention is administered topically, either as a single agent, or in combination with other antibiotic treatments, as described herein or known in the art.

In some embodiments, the pharmaceutical or veterinary compositions can also be dermally or transdermally administered. For topical application to the skin, the pharmaceutical or veterinary composition can be combined with one or a combination of carriers, which can include but are not limited to, an aqueous liquid, an alcohol base liquid, a water soluble gel, a lotion, an ointment, a nonaqueous liquid base, a mineral oil base, a blend of mineral oil and petrolatum, lanolin, liposomes, proteins carriers such as serum albumin or gelatin, powdered cellulose carmel, and combinations thereof. A topical mode of delivery may include a smear, a spray, a bandage, a time-release patch, a liquid-absorbed wipe, and combinations thereof. The pharmaceutical or veterinary composition can be applied to a patch, wipe, bandage, etc., either directly or in a carrier(s). The patches, wipes, bandages, etc., may be damp or dry, wherein the phage and/or polypeptide (e.g., a lysin) is in a lyophilized form on the patch. The carriers of topical compositions may comprise semi-solid and gel-like vehicles that include a polymer thickener, water, preservatives, active surfactants, or emulsifiers, antioxidants, sun screens, and a solvent or mixed solvent system. U.S. Pat. No. 5,863,560 discloses a number of different carrier combinations that can aid in the exposure of skin to a medicament, and its contents are incorporated herein.

For intranasal or administration by inhalation, the pharmaceutical or veterinary composition is conveniently delivered in the form of a dry powder inhaler or an aerosol spray presentation from a pressurized container, pump, spray, or nebuliser with the use of a suitable propellant, e.g., dichlorodifluoromethane, trichlorofluoromethane, dichlorotetrafluoroethane, a hydrofluoroalkane such as 1,1,1,2-tetrafluoroethane or 1,1,1,2,3,3,3-heptafluoropropane, carbon dioxide, or other suitable gas. In the case of a pressurized aerosol, the dosage unit may be determined by providing a valve to deliver a metered amount. The pressurized container, pump, spray, or nebuliser may contain a solution or suspension of the active compound, e.g., using a mixture of ethanol and the propellant as the solvent, which may additionally contain a lubricant, e.g., sorbitan trioleate. Capsules and cartridges (made, for example, from gelatin) for use in an inhaler or insufflator may be formulated to contain a powder mix of the bacteriophage and/or polypeptide of the invention and a suitable powder base such as lactose or starch.

For administration in the form of a suppository or pessary, the pharmaceutical or veterinary composition can be applied topically in the form of a gel, hydrogel, lotion, solution, cream, ointment, or dusting powder. Compositions of the invention may also be administered by the ocular route. For ophthalmic use, the compositions of the invention can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

Dosages and desired drug concentrations of the pharmaceutical and veterinary composition compositions of the present invention may vary depending on the particular use. The determination of the appropriate dosage or route of administration is within the skill of an ordinary physician. Animal experiments can provide reliable guidance for the determination of effective doses in human therapy.

For transdermal administration, the pharmaceutical or veterinary composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compounds can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

### Subject, regimen and administration

The subject or patient according to the invention is an animal, preferably a mammal, even more preferably a human. However, the term "subject" or "patient" can also refer to non-human animals, in particular mammals such as dogs, cats, horses, cows, pigs, sheep, donkeys, rabbits, ferrets, gerbils, hamsters, chinchillas, rats, mice, guinea pigs and non-human primates, among others, or non-mammals such as poultry, that are in need of treatment.

The human subject or patient according to the invention may be a human at the prenatal stage, a new-born, a child, an infant, an adolescent or an adult at any age.

In a preferred embodiment, the subject or patient is being prepared for an invasive procedure, such as a surgery, intubation, catheterization, etc., or a harsh conditioning procedure, such as immunosuppression, irradiation, etc.

The subject treated in the context of the invention is a healthy subject or a patient.

By "healthy subject" is meant herein a subject who does not suffer from a bacterial infection, in particular from an infection caused by antibiotic-resistant bacteria, at the time of administration of the vector of the invention.

By "patient" is meant herein a subject who does not suffer from a bacterial infection, in particular from an infection caused by antibiotic-resistant bacteria, at the time of administration of the vector of the invention, but suffers from another disease such as a disease necessitating invasive or non-invasive medical procedures, for example transplant, in particular solid organ transplant or hematopoietic stem cells transplant, dialysis, immunosuppression or irradiation.

As will be understood by the skilled person, despite the healthy subject or patient treated in the context of the invention is not suffering from a bacterial infection, in particular from an infection caused by antibiotic-resistant bacteria, at the time of administration of the vector of the invention, the healthy subject or patient can carry AMR bacteria.

In some embodiments, the treatment is administered several times, preferably 2, 3, 4, 5, or 6 times.

The form of the pharmaceutical or veterinary compositions, the route of administration and the dose of administration of delivery vehicles according to the invention, preferably of a payload, vector or engineered phage according to the invention, particularly of a payload packaged into a delivery vehicle according to the invention, preferably of a packaged plasmid or phagemid into a bacterial virus particle according to the invention, or of a pharmaceutical or veterinary composition according to the invention can be adjusted by the man skilled in the art according to the type and level of the colonization (e.g. depending on the bacteria species involved and its localization in the patient's or subject's body), and to the patient or subject, in particular its age, weight, sex, and general physical condition.

Particularly, the amount of delivery vehicles according to the invention, preferably a payload, vector or an engineered phage according to the invention, particularly a payload packaged into a delivery vehicle according to the invention, preferably a packaged plasmid or phagemid into a bacterial virus particle according to the invention, or of a pharmaceutical or veterinary composition according to the invention, to be administered has to be determined by standard procedure well known by those of ordinary skills in the art. Physiological data of the patient or subject (e.g. age, size, and weight) and the routes of administration have to be taken into account to determine the appropriate dosage, so as a therapeutically effective amount will be administered to the patient or subject.

For example, the total amount of delivery vehicles, particularly a payload packaged into a delivery vehicle or an engineered phage according to the invention, preferably a plasmid or phagemid packaged into a bacterial virus particle according to the invention, for each administration is comprised between 10⁴ and 10¹⁵ delivery vehicles.

### DEFINITIONS

### « vector »

As used herein, the term "vector" refers to any construct of sequences that are capable of expression of a polypeptide in a given host cell. If a vector is used then the choice of vector is dependent upon the method that will be used to transform host bacteria as is well known to those skilled in the art. Vectors can include, without limitation, plasmid vectors and recombinant phage vectors, or any other vector known in that art suitable for delivering a nucleic acid sequence of the invention to target bacteria. The skilled artisan is well aware of the genetic elements that must be present on the vector in order to successfully transform, select and optionally propagate host cells comprising any of the isolated nucleotides or nucleic acid sequences of the invention.

### « delivery vehicle »

As used herein, the term « delivery vehicle » refers to any vehicle that allows the transfer of a payload into a bacterium.

There are several types of delivery vehicle encompassed by the present invention including, without limitation, bacteriophage scaffold, virus scaffold, bacterial virus particle, chemical based delivery vehicle (e.g., cyclodextrin, calcium phosphate, cationic polymers, cationic liposomes), protein-based or peptide-based delivery vehicle, lipid-based delivery vehicle, nanoparticle-based delivery vehicles, non-chemical-based delivery vehicles (e.g., transformation, electroporation, sonoporation, optical transfection), particle-based delivery vehicles (e.g., gene gun, magnetofection, impalefection, particle bombardment, cell-penetrating peptides) or donor bacteria (conjugation).

Any combination of delivery vehicles is also encompassed by the present invention.

The delivery vehicle can refer to a bacteriophage derived scaffold and can be obtained from a natural, evolved or engineered capsid.

In some embodiments, the delivery vehicle is the payload as bacteria are naturally competent to take up a payload from the environment on their own.

### « payload »

As used herein, the term « payload » refers to any nucleic acid sequence or amino acid sequence, or a combination of both (such as, without limitation, peptide nucleic acid or peptide-oligonucleotide conjugate) transferred into a bacterium preferably with a delivery vehicle.

The term « payload » may also refer to a plasmid, a vector or a cargo.

The payload can be a phagemid or phasmid obtained from natural, evolved or engineered bacteriophage genome. The payload can also be composed only in part of phagemid or phasmid obtained from natural, evolved or engineered bacteriophage genome.

In some embodiments, the payload is the delivery vehicle as bacteria are naturally competent to take up a payload from the environment on their own.

### « nucleic acid »

As used herein, the term "nucleic acid" refers to a sequence of at least two nucleotides covalently linked together which can be single-stranded or double-stranded or contains portion of both single-stranded and double-stranded sequence. Nucleic acids of the present invention can be naturally occurring, recombinant or synthetic. The nucleic acid can be in the form of a circular sequence or a linear sequence or a combination of both forms. The nucleic acid can be DNA, both genomic or cDNA, or RNA or a combination of both. The nucleic acid may contain any combination of deoxyribonucleotides and ribonucleotides, and any combination of bases, including uracil, adenine, thymine, cytosine, guanine, inosine, xanthine, hypoxanthine, isocytosine, 5-hydroxymethylcytosine and isoguanine. Other examples of modified bases that can be used in the present invention are detailed in Chemical Reviews 2016, 116(20) 12655-12687. The term "nucleic acid" also encompasses any nucleic acid analogs which may contain other backbones comprising, without limitation, phosphoramide, phosphorothioate, phosphorodithioate, O-methylphophoroamidite linkage and/or deoxyribonucleotides and ribonucleotides nucleic acids. Any combination of the above features of a nucleic acid is also encompassed by the present invention.

### "Phagemid " and "packaged phagemid"

As used herein the term "phagemid" or "phasmid" are equivalent and refer to a recombinant DNA vector comprising at least one sequence of a bacteriophage genome and which is preferably not able of producing progeny, more particularly a vector that derives from both a plasmid and a bacteriophage genome. A phagemid of the disclosure comprises a phage packaging site and optionally an origin of replication (ori), in particular a bacterial and/or phage origin of replication. In one embodiment, the phagemid does not comprise an origin of replication and thus cannot replicate by itself once injected into a bacterium. Alternatively, the phagemid comprises a plasmid origin of replication, in particular a bacterial and/or phage origin of replication.

As used herein, the term "packaged phagemid" refers to a phagemid which is encapsidated in a bacteriophage scaffold, bacterial virus particle or capsid. Particularly, it refers to a bacteriophage scaffold, bacterial virus particle or capsid devoid of a bacteriophage genome. The packaged phagemid may be produced with a helper phage strategy, well known from the man skilled in the art. The helper phage comprises all the genes coding for the structural and functional proteins that are indispensable for the phagemid according to the invention to be encapsidated. The packaged phagemid may be produced with a satellite virus strategy, also known from the man skilled in the art. Satellite virus are subviral agent and are composed of nucleic acid that depends on the co-infection of a host cell with a helper virus for all the morphogenetic functions, whereas for all its episomal functions (integration and immunity, multicopy plasmid replication) the satellite is completely autonomous from the helper. In one embodiment, the satellite genes can encode proteins that promote capsid size reduction of the helper phage, as described for the P4 Sid protein that controls the P2 capsid size to fit its smaller genome.

### « peptide »

As used herein, the term "peptide" refers both to a short chain of at least 2 amino acids linked between each other and to a part of, a subset of, or a fragment of a protein which part, subset or fragment being not expressed independently from the rest of the protein. In some instances, a peptide is a protein. In some other instances, a peptide is not a protein and peptide only refers to a part, a subset or a fragment of a protein. Preferably, the peptide is from 4, 5, 6, 7, 8, 9, 10, 11, 12, 13, 14, 15 amino acids to 4, 5, 6, 7, 8, 9, 10, 11, 12, 13, 14, 15, 20, 25, 30, 40, 50, 100, 200 amino acids in size.

The invention further concerns the following clauses:
1. A method of selectively removing antibiotic resistance from an antibiotic resistant bacterial strain(s) in a healthy subject or patient carrying antibiotic resistant bacterial strain(s), wherein the method comprises:
   administering to the subject or patient a vector, said vector encoding a DNA modifying enzyme inactivating the antibiotic resistance gene (s) responsible for antibiotic-resistance of bacterial strain(s),
   thereby selectively removing antibiotic resistance from an antibiotic resistant bacterial strain(s) in the microbiota and preventing antibiotic resistance related infection in the subject.
2. The method of clause 1, wherein the DNA modifying enzyme is a nuclease.
3. The method of clause 1, wherein the DNA modifying enzyme is a base editor.
4. The method of clause 1, wherein the vector is inside a bacterial delivery vehicle.
5. The method of clause 1, wherein the vector is not inside a bacterial delivery vehicle.
6. The method of clause 1, wherein the vector further comprises a conditional origin of replication which is inactive in the targeted antibiotic resistant bacterial strain(s) but is active in a donor bacterial cell.
7. The method of clause 1, wherein antibiotic resistance bacteria are removed from the gut of the healthy subject or patient.
8. The method of clause 1, wherein the selective decolonization occurs before the subject or patient undergoes surgery.
9. The method of clause 8, wherein the surgery is a solid organ transplant surgery of the kidney or liver.
10. The method of clause 1, wherein said microbiota is a gut microbiota.
11. The method of clause 1, wherein said antibiotic is methicillin, vancomycin, or a carbapenem class or beta-lactam class antibiotic.
12. The method of clause 1, wherein the nuclease is a CRISPR associated nuclease.
13. The method of clause 1, further comprising obtaining a microbiota sample from the subject or patient and confirming the presence of said antibiotic resistant bacterial strain(s) in the sample.
14. Composition or combination comprising at least one, in particular at least 2, 3 or 4, recombinant phage(s) for use in a method of preventing antibiotic resistant *Escherichia coli* related infection in a patient carrying antibiotic resistant *Escherichia coli* strain(s),
   wherein said recombinant phage comprises a nucleic acid encoding a DNA modifying enzyme inactivating the antibiotic resistance gene(s) responsible for antibiotic-resistance of E. *coli* strain(s),
   thereby selectively removing antibiotic resistance from antibiotic resistant *E. coli* strain(s) in the microbiota of said patient and preventing antibiotic resistant *E. coli* related infection in the patient.
15. Composition or combination for use according to clause 14, wherein said patient suffers from a hematological malignancy such as hematological cancer.

### Examples

### Example 1 Packaged phagemid construction

A packaged phagemid has been designed consisting of a bacterial delivery vehicle derived from phage lambda and engineered to include host range determinants (stf and gpj) that allow to target clinical isolates of ESBL E.coli involved in hospital-acquired infections in patients. The bacterial delivery vehicle contains a DNA payload encoding for the Fn Cpf1 nuclease and either one or two (multiplexing) crRNAs (also named ctx guide) that target conserved regions of the ESBL gene CTX-M.

To produce the packaged phagemids, the phagemids were transformed in an *E. coli* strain carrying the lambda prophage lacking a packaging site but otherwise possessing all the machinery for the induction of the lambda phage lytic cycle as well as the DNA packaging system. The cl repressor of the lambda prophage carries mutations making it thermosensitive and enabling the induction of the lytic cycle through temperature change. The cells containing the lambda phagemid were grown at 30ºC in liquid LB media. At an OD600 of 0.6, the culture was shifted to 42ºC for 25 minutes to induce the entry into lytic cycle. After that, cells were shifted back to 37ºC for 3 hours to allow for virion assembly containing the lambda phagemid. Cells were then centrifuged and washed in lambda buffer (10 mM Tris pH 7.5, 100 mM NaCl, 10 mM MgSO4). Chloroform was added and the sample was spun down at 17,000g for 5 minutes. Finally, the aqueous phase was collected and filtered through a 0.2 µm pore-size filter. The titer of the packaged phagemids was measured by performing a transduction assay *in* vitro using strain MG-GFP as a recipient. MG-GFP is a derivative of strain MG1655 with a gfp fluorescent reporter gene and an ampicillin resistance gene inserted in the chromosome.

Sequences corresponding to the various features of the packaged phagemid are listed below:
Tip: 1A2 gpJ 
ATGGGTAAAGGAAGCAGTAAGGGGCATACCCCGCGCGAAGCGAAGGACAACCTG
AAGTCCACGCAGTTGCTGAGTGTGATCGATGCCATCAGCGAAGGGCCGATTGAAGGTCCG
GTGGATGGCTTAAAAAGCGTGCTGCTGAACAGTACGCCGGTGCTGGACACTGAGGGGAA
TACCAACATATCCGGTGTCACGGTGGTGTTCCGGGCTGGTGAGCAGGAGCAGACTCCGC
CGGAGGGATTTGAATCCTCCGGCTCCGAGACGGTGCTGGGTACGGAAGTGAAATATGACA
CGCCGATCACCCGCACCATTACGTCTGCAAACATCGACCGTCTGCGCTTTACCTTCGGTG
TACAGGCACTGGTGGAAACCACCTCAAAGGGTGACAGGAATCCGTCGGAAGTCCGCCTG
CTGGTTCAGATACAACGTAACGGTGGCTGGGTGACGGAAAAAGACATCACCATTAAGGGC
AAAACCACCTCGCAGTATCTGGCCTCGGTGGTGATGGGTAACCTGCCGCCGCGCCCGTTT
 
AATATCCGGATGCGCAGGATGACGCCGGACAGCACCACAGACCAGCTGCAGAACAAAAC
GCTCTGGTCGTCATACACTGAAATCATCGATGTGAAACAGTGCTACCCGAACACGGCACT
GGTCGGCGTGCAGGTGGACTCGGAGCAGTTCGGCAGCCAGCAGGTGAGCCGTAATTATC
ATCTGCGCGGGCGTATTCTGCAGGTGCCGTCGAACTATAACCCGCAGACGCGGCAATACA
GCGGTATCTGGGACGGAACGTTTAAACCGGCATACAGCAACAACATGGCCTGGTGTCTGT
GGGATATGCTGACCCATCCGCGCTACGGCATGGGGAAACGTCTTGGTGCGGCGGATGTG
GATAAATGGGCGCTGTATGTCATCGGCCAGTACTGCGACCAGTCAGTGCCGGACGGCTTT
GGCGGCACGGAGCCGCGCATCACCTGTAATGCGTACCTGACCACACAGCGTAAGGCGTG
GGATGTGCTCAGCGATTTCTGCTCGGCGATGCGCTGTATGCCGGTATGGAACGGGCAGA
CGCTGACGTTCGTGCAGGACCGACCGTCGGATAAGACGTGGACCTATAACCGCAGTAATG
TGGTGATGCCGGATGATGGCGCGCCGTTCCGCTACAGCTTCAGCGCCCTGAAGGACCGC
CATAATGCCGTTGAGGTGAACTGGATTGACCCGAACAACGGCTGGGAGACGGCGACAGA
GCTTGTTGAAGATACGCAGGCCATTGCCCGTTACGGTCGTAATGTTACGAAGATGGATGC
CTTTGGCTGTACCAGCCGGGGGCAGGCACACCGCGCCGGGCTGTGGCTGATTAAAACAG
AACTGCTGGAAACGCAGACCGTGGATTTCAGCGTCGGCGCAGAAGGGCTTCGCCATGTA
CCGGGCGATGTTATTGAAATCTGCGATGATGACTATGCCGGTATCAGCACCGGTGGTCGT
GTGCTGGCGGTGAACAGCCAGACCCGGACGCTGACGCTCGACCGTGAAATCACGCTGCC
ATCCTCCGGTACCGCGCTGATAAGCCTGGTTGACGGAAGTGGCAATCCGGTCAGCGTGG
AGGTTCAGTCCGTCACCGACGGCGTGAAGGTAAAAGTGAGCCGTGTTCCTGACGGTGTTG
CTGAATACAGCGTATGGGAGCTGAAGCTGCCGACGCTGCGCCAGCGACTGTTCCGCTGC
GTGAGTATCCGTGAGAACGACGACGGCACGTATGCCATCACCGCCGTGCAGCATGTGCC
GGAAAAAGAGGCCATCGTGGATAACGGGGCGCACTTTGACGGCGAACAGAGTGGCACGG
TGAATGGTGTCACGCCGCCAGCGGTGCAGCACCTGACCGCAGAAGTCACTGCAGACAGC
GGGGAATATCAGGTGCTGGCGCGATGGGACACACCGAAGGTGGTGAAGGGCGTGAGTTT
CCTGCTCCGTCTGACCGTAACAGCGGACGACGGCAGTGAGCGGCTGGTCAGCACGGCCC
GGACGACGGAAACCACATACCGCTTCACGCAACTGGCGCTGGGGAACTACAGGCTGACA
GTCCGGGCGGTAAATGCGTGGGGGCAGCAGGGCGATCCGGCGTCGGTATCGTTCCGGA
TTGCCGCACCGGCAGCACCGTCGAGGATTGAGCTGACGCCGGGCTATTTTCAGATAACCG
CCACGCCGCATCTTGCCGTTTATGACCCGACGGTACAGTTTGAGTTCTGGTTCTCGGAAAA
GCAGATTGCGGATATCAGACAGGTTGAAACCAGCACGCGTTATCTTGGTACGGCGCTGTA
CTGGATAGCCGCCAGTATCAATATCAAACCGGGCCATGATTATTACTTTTATATCCGCAGT
GTGAACACCGTTGGCAAATCGGCATTCGTGGAGGCCGTCGGTCGGGCGAGCGATGATGC
GGAAGGTTACCTGGATTTTTTCAAAGGCAAGATAACCGAATCCCATCTCGGCAAGGAGCT
GCTGGAAAAAGTCGAGCTGACGGAGGATAACGCCAGCAGACTGGAGGAGTTTTCGAAAG
AGTGGAAGGATGCCAGTGATAAGTGGAATGCCATGTGGGCTGTCAAAATTGAGCAGACCA
AAGACGGCAAACATTATGTCGCGGGTATTGGCCTCAGCATGGAGGACACGGAGGAAGGC
AAACTGAGCCAGTTTCTGGTTGCCGCCAATCGTATCGCATTTATTGACCCGGCAAACGGG
AATGAAACGCCGATGTTTGTGGCGCAGGGCAACCAGATATTCATGAACGACGTGTTCCTG
 
AAGCGCCTGACGGCCCCCACCATTACCAGCGGCGGCAATCCTCCGGCCTTTTCCCTGACA
CCGGACGGAAAGCTGACCGCTAAAAATGCGGATATCAGCGGTAACGTGAATGCGAACTCC
GGGACGCTCAACAACGTCACGATTAACGAGAACTGTCGGGTTCTGGAAAATTGTCCGCG
AACCAGATTGAAGGCGATCTCGTTAAAACAGTGGGCAAAGCTTTCCCCCGGGACTCCCGT
GCACCGGAGCGGTGGCCATCAGGAACCATTACCGTCAGGGTTTATGACGATCAGCCGTTT
GACCGGCAGATTGTTATTCCGGCGGTGGCATTCAGCGGCGCTAAACATGAGAAAGAGCAT
ACTGATATTTACTCCTCATGCCGTCTGATAGTGCGGAAAAACGGTGCTGAAATTTATAACC
GTACCGCGCTGGATAATACGCTGATTTACAGTGGCGTTATTGATATGCCTGCCGGTCACG
GTCACATGACACTGGAGTTTTCGGTGTCAGCATGGCTGGTAAATAACTGGTATCCCACAGC
AAGTATCAGCGATTTGCTGGTTGTGGTGATGAAGAAAGCCACTGCAGGCATCACGATTAG
C (SEQ ID NO: 22)
 Aminoacid sequence 
MGKGSSKGHTPREAKDNLKSTQLLSVIDAISEGPIEGPVDGLKSVLLNSTPVLDTEGNT
NISGVTVVFRAGEQEQTPPEGFESSGSETVLGTEVKYDTPITRTITSANIDRLRFTFGVQALVET
TSKGDRNPSEVRLLVQIQRNGGWVTEKDITIKGKTTSQYLASVVMGNLPPRPFNIRMRRMTPD
STTDQLQNKTLWSSYTEIIDVKQCYPNTALVGVQVDSEQFGSQQVSRNYHLRGRILQVPSNYN
PQTRQYSGIWDGTFKPAYSNNMAWCLWDMLTHPRYGMGKRLGAADVDKWALYVIGQYCDQ
SVPDGFGGTEPRITCNAYLTTQRKAWDVLSDFCSAMRCMPVWNGQTLTFVQDRPSDKTWTY
NRSNVVMPDDGAPFRYSFSALKDRHNAVEVNWIDPNNGWETATELVEDTQAIARYGRNVTK
MDAFGCTSRGQAHRAGLWLIKTELLETQTVDFSVGAEGLRHVPGDVIEICDDDYAGISTGGRV
LAVNSQTRTLTLDREITLPSSGTALISLVDGSGNPVSVEVQSVTDGVKVKVSRVPDGVAEYSV
WELKLPTLRQRLFRCVSIRENDDGTYAITAVQHVPEKEAIVDNGAHFDGEQSGTVNGVTPPAV
QHLTAEVTADSGEYQVLARWDTPKVVKGVSFLLRLTVTADDGSERLVSTARTTETTYRFTQLA
LGNYRLTVRAVNAWGQQGDPASVSFRIAAPAAPSRIELTPGYFQITATPHLAVYDPTVQFEFW
FSEKQIADIRQVETSTRYLGTALYWIAASINIKPGHDYYFYIRSVNTVGKSAFVEAVGRASDDAE
GYLDFFKGKITESHLGKELLEKVELTEDNASRLEEFSKEWKDASDKWNAMWAVKIEQTKDGK
HYVAGIGLSMEDTEEGKLSQFLVAANRIAFIDPANGNETPMFVAQGNQIFMNDVFLKRLTAPTIT
SGGNPPAFSLTPDGKLTAKNADISGNVNANSGTLNNVTINENCRVLGKLSANQIEGDLVKTVG
KAFPRDSRAPERWPSGTITVRVYDDQPFDRQIVIPAVAFSGAKHEKEHTDIYSSCRLIVRKNGA
EIYNRTALDNTLIYSGVIDMPAGHGHMTLEFSVSAWLVNNWYPTASISDLLVVVMKKATAGITIS
(SEQ ID NO: 23)
Tail Fiber protein: stf-27
   STF27 - amino acid sequence 
MAVKISGVLKDGTGKPVQNCTIQLKARRNSTTVVVNTVGSENPDEAGRYSMDVEYGQ
YSVILQVDGFPPSHAGTITVYEDSQPGTLNDFLCAMTEDDARPEVLRRLELMVEEVARNASVV
AQSTADAKKSETAAASSKNAAKTSETNAANSAQAAAASQTASANSATAAKKSETSAKNSETAT
 
KASEKNAKSSQTAAKTSETNAKDSEANAKVSETAAANSAKASAASQTAAKASEDAAREYANQ
TAEPYRYVLQPLPDVWIPFNDSLDMITGYSPGYKKVKIGDNVVQVASDKQVNFSRASTATYINK
SGELKTAEINEPRFECDGLLIEGQRTNFFQNSTDPSKWNKSTSLDVTETGTDSFGFNYGRFVV
QDSIVGTSKAHTIIGLYSSTGGVDTSGDEKHVTISCRVKSEVDNIAVRILFEHYDGEVRTSIGAA
NLNLTTRIISKTGQTSRVTARSVKDDATGWIFFEATLKADTTENTVGGFVQYSPDTGQMVTSG
DYLDVTTPQIEAGTGASSFIVTGTAPATRASDMVTVPIKNNLYNLPFTVLCEVHKNWYKTPNVA
PRVFDTGGHQTGAGIVMGFGSSGGYDGFPYCDIGGSDRRINENAGLEKMLIGMRVKSERSTC
VVSNGKLSSETKTKWEYIRSTATIRIGGQTTAGLRHLFGHVRNFRLWHKELTDAQLGEVVE
(SEQ ID NO: 24)
   >STF27 accessory protein - amino acid sequence 
VRDFTLRFSDKADFRAFLRKLNWEEDEELQNAVLVDEIGFTFRETDVSDDGEPEYTRN
EGYFVNIRLLDDGFEDSVFREWVVTPERPLREWF (SEQ ID NO: 25)
   STF27 - DNA sequence 
ATGGCAGTAAAGATTTCAGGAGTCCTGAAAGACGGCACAGGAAAACCGGTACAGA
ACTGCACCATTCAGCTGAAAGCCAGACGTAACAGCACCACGGTGGTGAACACGGTG
GGCTCAGAGAATCCGGATGAAGCCGGGCGTTACAGCATGGATGTGGAGTACGGTCAGTA
CAGTGTCATCCTGCAGGTTGACGGTTTTCCACCATCGCACGCCGGGACCATCACCGTGTA
TGAAGATTCACAACCGGGGACGCTGAATGATTTCTCTGTGCCATGACGGAGGATGATGC
CCGGCCGGAGGTGCTGCGTCGTCTTGAACTGATGGTGGAAGAGGTGGCGCGTAACGCGT
CCGTGGTGGCACAGAGTACGGCAGACGCGAAGAAATCAGAAACGGCAGCAGCCTCATCG
AAGAATGCGGCGAAAACCTCAGAAACGAATGCAGCTAACAGCGCACAGGCGGCAGCGGC
CTCGCAGACTGCATCGGCAAACTCCGCGACAGCAGCCAAAAAATCAGAAACCAGCGCGAA
AAATAGCGAGACAGCCACAAAGGCCAGCGAAAAAACGCAAAATCCAGCCAGACGGCAG
CGAAAACCAGTGAGACGAATGCCAAAGACAGTGAAGCCAACGCAAAGGTGAGCGAAACA
GCGGCGGCGAACTCGGCGAAAGCATCGGCAGCAAGCCAGACGGCAGCAAAAGCAAGTG
AAGATGCTGCCAGAGAATACGCAAACCAGACAGCAGAGCCGTACAGATATGTTTTACAGC
CGCTGCCGGATGTGTGGATACCCTTTAATGATTCGCTGGATATGATTACGGGCTATTCTCC
GGGTTATAAAAAAGTGAAGATTGGTGATAATGTGGTTCAGGTTGCCAGTGATAAACAGGTT
AATTTCAGTCGCGCATCAACGGCAACATATATCAACAAATCTGGCGAACTGAAAACGGCG
GAAATTAATGAGCCGCGATTTGAGTGTGATGGCCTGCTTATTGAGGGACAAAGAACGAACT
TCTTCCAGAACAGTACAGACCCTTCGAAGTGGAATAAGTCAACTTCACTGGACGTTACAGA
AACAGGCACAGATAGTTTCGGGTTTAATTATGGTCGGTTTGTCGTACAGGATTCGATTGTT
GGTACAAGTAAAGCGCATACCATTATCGGACTGTATTCGAGTACCGGAGGGGTTGATACTT
CAGGGGACGAAAAGCATGTAACTATATCCTGTCGGGTAAAAAGTGAAGTTGATAATATCGC
CGTTCGTATTTTATTTGAACATTATGATGGGGAGGTAAGGACATCAATAGGAGCAGCAAAC
CTGAACCTTACCACCCGCATAATTAGCAAGACAGGTCAGACAAGCCGTGTTACAGCAAGG
TCTGTTAAGGATGATGCAACTGGCTGGATATTTTTTGAGGCTACATTAAAAGCAGATACAAC
 
AGAAAATACGGTTGGTGGTTTTGTCCAGTATTCTCCGGATACAGGGCAGATGGTTACATCA
GGGGATTATCTCGATGTAACCACTCCACAGATTGAGGCTGGTACAGGCGCATCATCTTTTA
TTGTTACGGGGACGGCACCGGCAACGCGGGCAAGCGATATGGTGACAGTCCCAATCAAG
AATAACCTTTATAATCTTCCTTTTACGGTTCTTTGTGAGGTACATAAGAACTGGTATAAAAC
GCCAAATGTAGCGCCGCGTGTTTTTGATACCGGCGGTCATCAAACCGGAGCGGGGATCGT
AATGGGGTTTGGTTCATCAGGTGGGTACGACGGTTTTCCGTATTGCGATATAGGTGGTTCA
GACCGACGAATAAATGAAAATGCCGGGCTGGAAAAAATGCTTATTGGTATGCGGGTAAAG
TCCGAACGGTCCACATGTGTAGTCAGTAACGGTAAGTTAAGCAGCGAAACTAAAACCAAAT
GGGAATATATCCGGAGTACAGCAACCATTCGCATTGGTGGACAAACTACAGCAGGATTAC
GCCATTTATTTGGGCATGTGAGGAATTTTCGTCTCTGGCATAAAGAGCTAACAGATGCGCA
GCTTGGGGAGGTTGTGGAG (SEQ ID NO: 26)
   STF27 accessory protein - DNA sequence 
GTGAGAGATTTCACGTTGCGTTTCAGTGATAAAGCAGATTTCAGGGCATTTCTCAG
GAAACTTAACTGGGAAGAGGACGAAGAGCTGCAGAATGCCGTTCTGGTTGATGAGATTGG
TTTTACGTTCAGGGAGACAGATGTTTCTGATGACGGAGAACCAGAATACACGCGAAACGA
AGGGTACTTTGTTAATATCCGTCTTCTTGACGATGGATTTGAGGATTCCGTGTTCCGTGAG
TGGGTGGTTACACCAGAGCGCCCGCTCAGGGAGTGGTTT (SEQ ID NO: 27)
payload
p775: RBS1-AK272-CTX1

Payload encoding a Fn Cpf1 and an associated crRNA targeting a conserved region of CTX-M1 gene variants ("CTX1": GCCAGATCACCGCGATATCGTTGG, SEQ ID NO: 28). 
ACTGTCGGAATGACAAATGGTTCCAATTATTGAACACCCTTCGGGGTGTTTTTTTGT
TTCTGGTTTCCCGAGGCCGGCCTGCGCTAGCGGAGTGTATACTGGCTTACTATGTTGGCA
CTGATGAGGGTGTAAGTGAAGTGCTTCATGTGGCAGGAGAAAAAAGGCTGCATCGGTGCG
TCAGCAGAATATGTGATACAGGATATATTCCGCTTCCTCGCTCACTGACTCGCTACGCTCG
GTCGTTCGACTGTGGCGAGCGGAAATGGCTTACGAACGGGGCGGAGATTTCCTGGAAGA
TGCCAGGAAGATACTTAACAGGGAAGTGAGAGGGTCGCGGCAAAGCCGTTTTTCCATAGG
CTCCGCCCCCCTGACAAGCATCACGAAATCTGACGCTCAAATCAGTGGTGGCGAAACCTG
ACAGGACTATAAAGATACCAGGCGTTTCCCCCTGGCGGCTCCCTCGTGCGCTCTCCTGTT
CCTGCCTTTCGGTTTGCCGGTGTCATTCCTCTGTTACGGCCGAGTTTGTCTCATTCCACGC
CTGACACTCAGTTCCGGGTAGGCAGTTCGCTCCAAGCTGGACTGTATGCACGAACCCCCC
GTTCAGTCCGACCGCTGCGCCTTATCCGGTAACTATCGTCTTGAGTCCAACCCGGAAAGA
CATGCAAAAGCACCACTGGCAGCAGCCACTGGTAATTGATTTAGAGGAGTTAGTCTTGAAG
TCATGCGCCGGATAAGGCTAAACTGAAAGGACAAGTTTTGGCGACTGCGCTCCTCCAAGC
CAGTTACCTCGGTTCAAAGAGTTGGTAGCTCAGAGAACCTTCGAAAAACCGCCCTGCAAG
 
GCGGTTTTTTCGTTTTCAGAGCAAGAGATTACGCGCAGACCAAAACGATCTCAAGAAGATC
ATCTTATTAATCAGATAAAATATTTCTAGATTTCAGTGCAATTTATCTCTTCAAATGTAGCAC
CGGCGCGCCGTGACCAATTATTGAAGGCCGCTAACGCGGCCTTTTTTTGTTTCTGGTATCC
CGAATGGAGCGACTTCTCCCCAAAAAGCCTCGCTTTCAGCACCTGTCGTTTCCTTTCTTTT
CAGAGGGTATTTTAAATAAAAACATTAAGTTATGACGAAGAAGAACGGAAACGCCTTAAAC
CGGAAAATTTTCATAAATAGCGAAAACCCGCGAGGTCGCCGCCCCGTAACCTGTCGGATC
ACCGGAAAGGACCCGTAAAGTGATAATGATTATCATCTACATATCACAACGTGCGTAAAGG
GTAAGTATGAAGGTCGTGTACTCCATCGCTACCAAATTCCAGAAAACAGACGCTTTCGAGC
GTCTTTTTTCGTTTTGGTCACGACGTACGGTGGAAGATTCGTTACCAATTGACAGCTAGCT
CAGTCCTAGGTATATACATACATGCTTGTTTGTTTGTAAACTACTGTTTTCATTAAAGAGGA
GAAAGGAAGCCATGTCCATCTATCAGGAGTTTGTTAACAAGTATTCCCTGTCTAAAACCCT
GCGTTTTGAACTGATCCCGCAGGGCAAAACTTTGGAAAACATTAAAGCGCGTGGCCTGATT
CTGGATGACGAAAAACGTGCAAAGGATTACAAGAAAGCTAAACAGATCATCGACAAATATC
ACCAGTTCTTTATCGAAGAAATTCTGTCGTCGGTGTGCATCAGTGAGGATCTGTTACAGAA
TTATTCTGATGTATACTTTAAACTTAAAAAGTCCGATGACGATAATCTGCAAAAAGATTTCAA
GTCAGCCAAAGATACCATCAAGAAACAGATCTCAGAATATATTAAAGATAGCGAAAAGTTC
AAAAACCTGTTTAACCAAAACCTCATTGATGCTAAGAAAGGCCAAGAATCTGACCTGATCTT
ATGGCTGAAACAGAGCAAAGATAACGGCATTGAACTGTTCAAAGCTAATAGCGACATCACC
GATATTGATGAAGCGCTCGAAATCATCAAGTCTTTCAAAGGCTGGACGACGTATTTCAAAG
GTTTTCATGAAAACCGTAAGAATGTATATTCGAGCAACGATATTCCGACCTCTATTATTTAT
CGTATCGTGGACGACAACCTGCCGAAGTTTCTGGAAAACAAAGCGAAATATGAATCTCTGA
AAGACAAAGCACCGGAAGCTATTAACTATGAACAGATCAAGAAAGATCTGGCGGAAGAAC
TGACCTTCGACATCGACTATAAAACCTCCGAAGTTAACCAGCGTGTTTTCTCACTGGACGA
GGTTTTCGAAATCGCTAATTTCAACAATTACCTGAATCAATCTGGCATCACCAAATTCAACA
CCATTATTGGTGGCAAATTTGTTAACGGCGAAAACACCAAGCGTAAGGGCATCAACGAATA
CATTAACCTGTATAGCCAACAAATCAACGACAAAACCCTGAAAAAGTATAAAATGTCCGTTC
TGTTTAAACAGATTTTATCGGACACCGAATCTAAATCCTTCGTAATTGATAAACTGGAAGAT
GATAGCGACGTTGTCACCACGATGCAGAGCTTTTATGAGCAGATTGCGGCGTTCAAAACC
GTGGAAGAGAAATCTATTAAAGAAACTCTGTCCCTGCTCTTTGACGACCTCAAAGCGCAGA
AACTAGATCTGTCTAAGATTTACTTTAAAAACGACAAATCTCTGACCGATCTCAGTCAACAA
GTTTTCGATGACTATAGCGTGATCGGCACGGCAGTTTTGGAATACATCACCCAACAAATCG
CGCCGAAAAATCTGGACAACCCGTCCAAGAAGGAACAGGAACTGATTGCAAAGAAAACAG
AAAAAGCTAAATACCTGAGCTTAGAAACTATCAAACTGGCACTTGAGGAATTTAATAAACAT
CGTGATATTGATAAACAGTGTCGTTTTGAGGAAATTCTGGCGAACTTTGCGGCAATCCCGA
TGATCTTCGACGAAATTGCTCAAAACAAAGACAATCTGGCGCAGATCTCTATCAAGTACCA
GAATCAGGGTAAGAAAGATCTGCTTCAAGCATCTGCGGAGGACGATGTGAAAGCAATTAA
AGACTTATTAGATCAGACGAATAACTTATTACACAAGCTCAAAATCTTCCACATCAGCCAGA
GCGAGGACAAGGCGAACATTCTGGATAAAGATGAACACTTCTATCTGGTGTTCGAAGAAT
 
GTTACTTCGAACTGGCAAACATCGTCCCTCTCTACAATAAAATCCGCAACTACATCACGCA
GAAGCCTTACTCTGACGAGAAATTCAAACTGAACTTCGAAAACAGCACGCTGGCGAACGG
CTGGGATAAGAACAAAGAGCCGGACAACACCGCAATCCTGTTCATCAAAGACGACAAATA
CTATCTGGGCGTAATGAACAAGAAGAACAACAAGATCTTCGACGATAAAGCGATCAAAGAA
AACAAGGGTGAAGGCTATAAGAAAATCGTGTACAAGCTCCTGCCGGGTGCGAATAAAATG
TTACCGAAAGTGTTCTTTTCCGCGAAAAGCATCAAATTCTACAACCCGTCTGAGGATATTCT
GCGCATCCGCAATCATAGCACGCACACTAAAAACGGTAGCCCGCAGAAAGGGTATGAAAA
ATTCGAATTTAATATAGAGGACTGCCGTAAGTTCATCGACTTCTATAAACAGAGCATTTCCA
AACATCCGGAATGGAAAGACTTCGGCTTCCGTTTCTCTGACACTCAGCGCTATAATAGCAT
CGACGAGTTCTACCGCGAAGTGGAGAATCAGGGCTATAAACTGACCTTCGAGAACATTAG
TGAGTCGTACATCGACTCCGTTGTGAATCAGGGTAAACTGTACCTGTTTCAGATCTATAATA
AAGACTTTAGCGCGTACAGCAAAGGCCGTCCGAATCTGCACACCCTTTACTGGAAAGCATT
ATTTGACGAACGTAACCTGCAAGATGTGGTGTATAAACTGAACGGTGAGGCGGAACTTTTC
TACCGTAAACAGAGTATCCCGAAGAAAATCACGCATCCGGCAAAAGAAGCTATTGCCAACA
AAAACAAAGACAACCCGAAGAAAGAATCAGTATTCGAATATGACCTGATCAAAGATAAACG
TTTCACCGAAGATAAGTTCTTTTTCCACTGTCCGATTACCATCAACTTCAAATCTAGCGGTG
CGAACAAGTTCAACGATGAAATTAACTTATTACTGAAAGAGAAAGCTAATGACGTACACATC
TTATCTATTGATCGCGGTGAACGTCATTTAGCATACTATACACTGGTAGATGGTAAAGGTAA
TATTATTAAACAGGATACTTTCAATATTATCGGTAATGACCGTATGAAAACCAACTATCACG
ATAAGCTGGCGGCGATCGAAAAAGATCGTGATTCTGCGCGTAAAGATTGGAAGAAAATTAA
CAATATCAAAGAAATGAAAGAAGGCTATCTGAGCCAAGTGGTGCACGAGATCGCAAAACT
GGTGATTGAATATAACGCTATCGTGGTTTTCGAAGATCTGAACTTTGGTTTTAAACGTGGTC
GCTTCAAAGTAGAAAAACAGGTGTACCAAAAACTGGAAAAAATGCTGATTGAAAAACTGAA
CTATCTGGTTTTTAAAGACAACGAATTTGACAAAACGGGTGGCGTACTCCGTGCCTATCAG
CTGACCGCTCCGTTCGAAACGTTCAAGAAAATGGGTAAACAAACGGGGATTATCTATTATG
TGCCAGCTGGTTTCACCTCCAAGATTTGTCCAGTTACGGGCTTCGTTAACCAGCTGTACCC
GAAATACGAGAGCGTTAGCAAATCTCAAGAATTTTTCAGCAAATTCGACAAGATCTGCTATA
ATCTGGATAAAGGCTATTTCGAGTTCAGCTTCGATTACAAAAACTTCGGCGATAAAGCGGC
TAAAGGTAAGTGGACTATTGCTAGCTTTGGTAGCCGTCTGATTAACTTTCGCAACTCCGAC
AAAAACCATAATTGGGACACGCGTGAAGTGTATCCGACCAAAGAACTGGAAAAATTACTGA
AAGACTATTCCATCGAATATGGTCATGGGGAGTGCATTAAAGCGGCGATTTGCGGTGAATC
CGATAAGAAATTTTTCGCCAAACTGACCAGCGTGCTTAACACCATTCTGCAAATGCGTAATT
CTAAAACGGGTACGGAGCTGGACTACCTGATTTCTCCGGTAGCCGACGTTAACGGCAACT
TCTTCGATTCTCGTCAAGCACCGAAAAATATGCCACAAGACGCGGATGCCAACGGTGCAT
ACCATATCGGCTTAAAAGGCTTAATGTTATTAGGCCGTATCAAGAATAATCAGGAGGGCAA
GAAATTAAATCTGGTTATCAAAAACGAAGAATACTTCGAGTTCGTTCAGAATCGTAACAATT
AATGTATGCTTAAGCAGCTCGGTACCAAAGACGAACAATAAGACGCTGAAAAGCGTCTTTT
TTCGTTTTGGTCCTGTTGCGGCGCGATAGTGTGAACATGCTATAGACTTCTGGTGCTACCC
 
GACTGACAATTAATCATCCGGCTCGTATAATGCTAGCAATTTCTACTGTTGTAGATGCCAGA
TCACCGCGATATCGTTGGTCGAGACGAACAATAAGGCCTCCCTAACGGGGGGCCTTTTTT
ATTGATAACAAAAGTAACTTCGAGCTTGTCTACCTCCTAGCACCATTATTGCAATTAATAAA
CAACTAACGGACAATTCTACCTAACAGTTTTCATATATGACGAGCAGTTAAGTGATGAGTAA
AGGTGAGGAATTATTTACTGGTGTTGTTCCGATCTTAGTTGAACTGGACGGCGATGTTAAC
GGTCATAAATTCAGTGTTCGTGGTGAAGGTGAAGGTGATGCAACCAACGGTAAGCTGACC
CTGAAATTCATCTGCACTACTGGAAAATTACCAGTACCGTGGCCTACTCTGGTGACTACCC
TGACCTATGGTGTTCAGTGTTTTTCTCGTTACCCTGACCACATGAAGCAACATGATTTCTTC
AAATCTGCAATGCCGGAAGGTTATGTACAGGAGCGCACCATTTCTTTCAAAGACGATGGCA
CGTATAAAACCCGTGCAGAGGTTAAATTTGAAGGTGACACTCTGGTGAATCGTATTGAACT
GAAAGGCATTGATTTCAAAGAGGACGGCAATATTTTAGGCCACAAACTGGAATATAACTTC
AACTCCCATAACGTTTACATCACCGCAGACAAACAGAAGAACGGTATCAAAGCTAACTTCA
AAATTCGCCATAACGTTGAAGATGGTAGCGTACAGCTGGCGGATCATTACCAACAGAACA
CTCCGATTGGAGATGCTCCTGTTTTACTGCCGGATAACCACTACCTGTCCACCCAGTCTAA
ACTGTCGAAGGATCCGAACGAAAAGCGCGACCACATGGTGTTATTAGAGTTCGTTACCGC
TAGTGGTATCACGCACGGTATGGATGAACTCTACAAATAAGACGAACAATAAGGGGAGCG
GGAAACCGCTCCCCTTTTTATTGATAACAAAAGTAAATTGCACGCTGATAGTCTCCCAATT
GCGAAGGACCAAAACGAAAAAACACCCTTTCGGGTGTCTTTTCTGGAATTTGGTACCGAGT
ACTAGGTATCGTGTAAGTAGCGAAGGCCCGTACGCGAGATAAACTGCTAGGCAACCGCGA
CTCTACGACTGGTGCTCGATTTAATTTCGCTGACGTAAAGAAATTATCGGCAGTGCGTCAA
CTGCCGTATCTTTATCTTAATTAGGTAGTTGGACAAGCCCTTGAAAGAAATAGCAAGAGCC
TGCCTCTCTATTGAAGTCACGGCGAAAGTCGGGTAGAAATCAAAGAAAGCAGAAATTAAAT
CGGAGTAACACTAAGGTGGGATAACTCCGTAACTGACTACGCCTTTCTCTAGACTTTACTT
GACCAGATACACTGTCTTTGACACGTTGAAGGATTAGAGCAATCAAATCCAAGACTGGCTA
AGCACGAAGCAACTCTTGAGTGTTAAAAAGTTATCTCCTGTATTCGGGAAGCGGGTACTAG
AAGATTGCAGGGACTCCGACGTTAAGTAAATTACAAAGTAATAAGTATCGTTCAGGATCAC
GTTACCGCAATAAGAAGCGAGAATAATATAATTTCCGAAGTGCTTACCCCAGTAGTGACTA
TTCCTATAACCCTTCTGAGTGTCCGGAGGCGGAAATTTGCCACGAAAGAGAAAGTATTTCC
CCGACAATAATAAAGGGGCGCTCCTCAGCTTTTCCACTTGGTTGGGTAAGCTAGGCAACT
CTGAAAGGAGTTTCGGCGAATTGAAGCCGACAGCTTTGAATTGTTTTAGGGGCGTTATTCG
AGGGCAATCGGAGCTAACTTCAAGACTACTTCTTTGTTGAATACTAAATAGTGCAAAGGTC
GTGTTTCCTCAAGGATACTCCGCTAACAATATAGGATTCCAATCAGATTCAGCACTGGCGG
TACGGGTGTTGCGGTGAGGCGTTCGGGTTTACGGCTCGAAGCTAGCACGGTAGGAAGCC
TGACAATCACCAAGCAAAAGGGCCGTCGAAGGCCCACAAGATACGAAAGCTCTCGAAGCC
TTATCCTTGACCGATCCACCTATTTAGGCAGTTACGCACAAAAGCTACCCAATAATCCGTG
ACAGGCACAATATCACGGAACAAAACCGAAAACTCTCGTACACGGTTAGGTTTTCGCTAGG
AAGAATAAACCTCTATCTTGATTATAAGAAGGCTCCCCAAGCACCCCCAAAACCGAAATAG
CGGTTTGCAATAAGGGACAAGTTACGAGTGTAGACACGCAGAATTATCCAGCCTTTAGTCT
 
TTAGGAAGGCAAAGCTATTGTACGCGGTAGCCGTCGTAGCAATTTACCAACTGTAGAATTA
TTGGACACACGTAGGAAGGGCTTACAGTTGAAGTTTAATAAGGTCACACGCAAAACCGCTA
AGGAATAATCGCACCGTTAGCGAAAGAATATTTCAGAGCGGTTAGTAAAGGTTGAGTAAAG
TGAGATTCCAAAGTGAGCCTTTATAAAAAGTAAAGAGCTATAATAAAACCGTCGAGCAGAA
AACAATCGCCTGAAATCTCAAGCACGTTGCCCTTTCTAACGTCGCTAAGGTTTCGTAAACC
CGTTTGATTAGGAAGAAGAATAAGTAACCCGATTAGGTTTGAGATCGCGGGTTATCGGTTT
GGATTAAAAGTGGATACCAGCGGAGTCAACGCCGACGCAAACGTACAGTGATCCAATCCT
GTTGCACGGTCAAGCACAATCAGCTCGCAAGATCTTGGAATAGTGTGCCCAACAGTTTAGT
TGAGGGCCACGTTCCGACTACAAGTTGCTTCAAGAGGGGAATTTGGATTTGGCAATAGCC
CCCCGTTTCTACCTCAAGAGGCGACGAGTATTAACCGCGCCAGCTGTCGGCACAAGGGC
CAAAGAAGATTCCAATTTCTTATTCCCGAATAACCTCCGAATCCCTGCGGGAAAATCACCG
ACCGAATAGCCTAGAAGCAAGGGGGAACAGATAGGTATAATTAGCTTAAGAGAGTACCAG
CCGTGACAACAGCGTAGTAACCACAAACTTACGCTGGGGCTTCTTTGGCGGATTTTTACAG
ATACTAACAAGGTGATTTGAAGTACCTTAGTTGAGGATTTAAACGCGCTATCCGGTAATCTC
CAAATTGGGAAATACCGTTCAAAGAGGGCTAGAATTACTTAAAAGCCTTCACACCGCCTGC
GCTATACGCGCCCACTCTCCCGTTTATCCGTCCAAGCGGAAGCAGGGCGATCCTCCGCTA
AGATATTCTTACGTGTAACGTAGCTAAGTATCCCAAATAGCTGGCGTACGCGTTGAACACC
GCCTAGAGGATCGTGACTCGCCGGACGAGCGTGTTATTGGGGACTTACGCCAGCGTAGA
CTACAACGCGCCCAGATTAACCCTGCACGTATTGCCTTGAATAACGTACTAATCTCTCCGG
CTCTCGACAATCTATCGAGCGACTCGATTATCAACGGGTGTCTTGCAGTTCTAATCTCTTG
CCCCCGCCCGTAATAGCCTCCAAGAGATTGAAGATAGTAAAGGGCAAGAGCTGATTCGGC
GTTGAAGGATAGCGGACTTTCGGTCAACCACAATTCCCCACTCGACAAAACCAGCCGTGC
GAATAACTCTGAAAGTACAAGCAACCCAAGAGGGCTGAGCCTAAACTCAGCTAATTCCTAA
GTGAGCTAAAGACTCGAAGTGACAGCTCTTAATAAATAGAGCGGGAACGTCGAACGGTCG
TGAAAGTAATAGTACAACGGGTATTAACTTACTGAGGATATTGCTTGAAGCTGTACCGTTTT
ATTGGGTGAACGAATAAGATCCAGCAATTCAGCCAAAGAAGCTACCAATTTTTAGTTTAAG
AGTGTCACGTCTGACCTCGCGGGTAGATTGCCGAACGTAGAGCTTACGAGCCAGCGGAAA
CAGTAGCCGCAGGATAAGTAAGGGGAGTAAGTGATCGAACGAATCAGAAGTGACAATATA
CTTAGGCTGGATCTCGTCCCGTGAATCCCAACCCTCACCAACTACGAGATAAGAGGTAAG
CCAAAAATCGACTTGGTGGCGACCAACGACTGTTCCCCCCCTGTAACTAATCGTTCCGTCA
AAACCTGACTTACTTCAAGGCCAATTCCAAGCGCAAACAATACCGTCCTAGTTCTTCGGTT
AAGTTTCCGAAGTAGGAGTGAGCCTACCTCCGTTTGCGTCTTGTTACCACTGACCCAGCTA
TTTACTTTGTATTGCCTGCAATCGAATTTCTGAACTCTCAGATAGTGGGGATAACGGGAAA
GTTCCTATATTTGCGAACTAACTTAGCCGTCCACCTCGAAGCTACCTACTCACACCCACCC
CGCGCGGGGTAAATAAGGCACTAATCCCAGCTGAGAGCTGGCGTAGCACTTAGCCACAA
GTTAATTAACAGTTGTCTGGTAGTTTGGCGGTATTAGGAAGATCCTAGAAGCAAGGCAGAG
TTAGTTCTAACCTAAAGCCACAAATAAGACAGGTTGCCAAAGCCCGCCGGAAATTAAATCT
TGCTCAGTTCGGTAACGGAGTTTCCCTCCCGCGTACTTAATTCCCAATAAGAAACGCGCCC
 
AAGTCCTATCAGGCAAAATTCAGCCCCTTCCCGTGTTAGAACGAGGGTAAAAATACAAGCC
GATTGAACAAGGGTTGGGGGCTTCAAATCGTCGTTTACCCCACTTTACAACGGAGATTAAG
TAGTTCACCCTATAGTACGAAGCAGAACTATTTCGAGGGGCGTGCAATAATCGAATCTTCT
GCGGTTGACTTAACACGCTAGGGACGTGCCCTCGATTCAATCGAAGGTACTCCTACTCAG
ACTGCCTCACACCCAGCTAGTCACTGAGCGATAAAATTGACCCGCCCTCTAGGGAAGCGA
GTACGTCCCAAAGGGCTCCGGACAGGGCTATATAGGAGAGTTTGATCTCGCCCCGACAAC
TGCAACCCTCAACTCCCTTAGATAATATTGTTAGCCGAAGTTGCACGACCCGCCGTCCACG
GACTGCTCTTAGGGTGTGGCTCCTTAATCTGACAACGTGCAACCCCTATCGAAGTCGATTG
TTTCTGCGAAAGGTGTTGTCCTAATAGTCCCGAAATTTGGCCCTTGTAGGTGTGAAACCAC
TTAGCTTCGCGCCGTAGTCCTAAAGGCCCACCTATTGACTTTGTTTCGGGTAGCACTAGGA
ATCTTAACAATTTGAATTTGGACGTGGAACGCGTACACCTTAATCTCCGAATAATTCTAGGG
ATTTGGAAGTCCTCTACGTTGACACACCTACACTGCTCGAAGTAAATATACGAATAACGCG
GGCCTCGCGGAGCCGTTCCGAATCGTCACGTGTTCGTTTACTGTTAATTGGTGGCAAATAA
GCAATATCGTAGTCCGTCAGGCCCAGCCCTGTTATCCACGGCGTTATTTGTCAAATTGCGT
AGAACTGGATTGACTGCCTGACAATACCTAATTATCGGTACGAAGTCCCCGAATCTGTCGG
GCTATTTCACTAATACTTTCCAAACGCCCCGTATCCAAGAAGAACGAATTTATCCACGCTCC
CGTCTTTGGGACGAATACCGCTACAAGTGGACAGAGGATCGGTACGGGCCTCTAATAAAT
CCAACACTCTACGCCCTCTTCAAGAGCTAGAAGAACAGGGTGCAGTTGGAAAGGAATTA
TTTCGTAAGGCGAGCCAATACCGTAATTAATTCGGAAGAGTTAACACGATTGGAAGTAGGA
ATAGTTTCTAACCACGGTTACTAATCCTAATAACGGAACGCTGTCTGATAGATTAGTGTCAG
CGCTCGGTACCAAAGAAAAATAAAAAGACGCTGAAAAGCGTCTTTTTATTTTTCGGTCCAG
TGTAACTCAGGCAAAAGCACGTAATATTCGTACTTTCTTCCTCCGTAAGCGTCACCCACATT
CCTTAAAGAGTGCATGTGCATATTTTGTTATCAATAAAAAAGGCCGCGATTTGCGGCCTTAT
TGTTCGTCTTGCCGGATTACGCCCCGCCCTGCCACTCATCGCAGTATTGTTGTAATTCATT
AAGCATTCTGCCGACATGGAAGCCATCACAAACGGCATGATGAACTTGGATCGCCAGTGG
CATTAACACCTTGTCGCCTTGCGTATAATATTTTCCCATAGTGAAAACGGGGGCGAAGAAG
TTGTCCATATTTGCTACGTTTAAATCAAAACTGGTGAAACTCACCCACGGATTGGCACTGA
CGAAAAACATATTTTCGATAAACCCTTTAGGGAAATATGCTAAGTTTTCACCGTAACACGCC
ACATCTTGACTATATATGTGTAGAAACTGCCGGAAATCGTCGTGGTATTCTGACCAGAGCG
ATGAAAACGTTTCAGTTTGCTCATGGAAAACGGTGTAACAAGGGTGAACACTATCCCATAT
CACCAGCTCACCGTCTTTCATTGCCATACGAAACTCCGGATGTGCATTCATCAGGCGGGC
AAGAATGTGAATAAAGGCCGGATAAAACTTGTGCTTATTTTTCTTTACGGTTTTTAAAAAGG
CCGTAATATCCAGCTGAACGGTTTGGTTATAGGTGCACTGAGCAACTGACTGGAATGCCTC
AAAATGTTCTTTACGATGCCATTGACTTATATCAACTGTAGTATATCCAGTGATTTTTTTCTC
CATTTTAGCTTCCTTAGCTTGCGAAATCTCGATAACTCAAAAAATAGTAGTGATCTTATTTCA
TTATGGTGAAAGTTGTCTTACGTGCAACATTTTCGCAAAAGTTGGCGCTTTATCAAC (SEQ
ID NO: 29)
- p776: RBS1-AK272-CTX3

Payload encoding a Fn Cpf1 and an associated crRNA targeting a conserved region of CTX-M9 gene variants ("CTX3": TCGCGGTGATGAACGCTTTCCAAT, SEQ ID NO: 30). 
ACTGTCGGAATGACAAATGGTTCCAATTATTGAACACCCTTCGGGGTGTTTTTTGT
TTCTGGTTTCCCGAGGCCGGCCTGCGCTAGCGGAGTGTATACTGGCTTACTATGTTGGCA
CTGATGAGGGTGTAAGTGAAGTGCTTCATGTGGCAGGAGAAAAAGGCTGCATCGGTGCG
TCAGCAGAATATGTGATACAGGATATATTCCGCTTCCTCGCTCACTGACTCGCTACGCTCG
GTCGTTCGACTGTGGCGAGCGGAAATGGCTTACGAACGGGGCGGAGATTTCCTGGAAGA
TGCCAGGAAGATACTTAACAGGGAAGTGAGAGGGTCGCGGCAAAGCCGTTTTTCCATAGG
CTCCGCCCCCCTGACAAGCATCACGAAATCTGACGCTCAAATCAGTGGTGGCGAAACCTG
ACAGGACTATAAAGATACCAGGCGTTTCCCCCTGGCGGCTCCCTCGTGCGCTCTCCTGTT
CCTGCCTTTCGGTTTGCCGGTGTCATTCCTCTGTTACGGCCGAGTTTGTCTCATTCCACGC
CTGACACTCAGTTCCGGGTAGGCAGTTCGCTCCAAGCTGGACTGTATGCACGAACCCCCC
GTTCAGTCCGACCGCTGCGCCTTATCCGGTAACTATCGTCTTGAGTCCAACCCGGAAAGA
CATGCAAAAGCACCACTGGCAGCAGCCACTGGTAATTGATTTAGAGGAGTTAGTCTTGAAG
TCATGCGCCGGATAAGGCTAAACTGAAAGGACAAGTTTTGGCGACTGCGCTCCTCCAAGC
CAGTTACCTCGGTTCAAAGAGTTGGTAGCTCAGAGAACCTTCGAAAAACCGCCCTGCAAG
GCGGTTTTTTCGTTTTCAGAGCAAGAGATTACGCGCAGACCAAAACGATCTCAAGAAGATC
ATCTTATTAATCAGATAAAATATTTCTAGATTTCAGTGCAATTTATCTCTTCAAATGTAGCAC
CGGCGCGCCGTGACCAATTATTGAAGGCCGCTAACGCGGCCTTTTTTTGTTTCTGGTATCC
CGAATGGAGCGACTTCTCCCCAAAAAGCCTCGCTTTCAGCACCTGTCGTTTCCTTTCTTTT
CAGAGGGTATTTTAAATAAAAACATTAAGTTATGACGAAGAAGAACGGAAACGCCTTAAAC
CGGAAAATTTTCATAAATAGCGAAAACCCGCGAGGTCGCCGCCCCGTAACCTGTCGGATC
ACCGGAAAGGACCCGTAAAGTGATAATGATTATCATCTACATATCACAACGTGCGTAAAGG
GTAAGTATGAAGGTCGTGTACTCCATCGCTACCAAATTCCAGAAAACAGACGCTTTCGAGC
GTCTTTTTTCGTTTTGGTCACGACGTACGGTGGAAGATTCGTTACCAATTGACAGCTAGCT
CAGTCCTAGGTATATACATACATGCTTGTTTGTTTGTAAACTACTGTTTTCATTAAAGAGGA
GAAAGGAAGCCATGTCCATCTATCAGGAGTTTGTTAACAAGTATTCCCTGTCTAAAACCCT
GCGTTTTGAACTGATCCCGCAGGGCAAAACTTTGGAAAACATTAAAGCGCGTGGCCTGATT
CTGGATGACGAAAAACGTGCAAAGGATTACAAGAAAGCTAAACAGATCATCGACAAATATC
ACCAGTTCTTTATCGAAGAAATTCTGTCGTCGGTGTGCATCAGTGAGGATCTGTTACAGAA
TTATTCTGATGTATACTTTAAACTTAAAAAGTCCGATGACGATAATCTGCAAAAAGATTTCAA
GTCAGCCAAAGATACCATCAAGAAACAGATCTCAGAATATATTAAAGATAGCGAAAAGTTC
AAAAACCTGTTTAACCAAAACCTCATTGATGCTAAGAAAGGCCAAGAATCTGACCTGATCTT
ATGGCTGAAACAGAGCAAAGATAACGGCATTGAACTGTTCAAAGCTAATAGCGACATCACC
GATATTGATGAAGCGCTCGAAATCATCAAGTCTTTCAAAGGCTGGACGACGTATTTCAAAG
GTTTTCATGAAAACCGTAAGAATGTATATTCGAGCAACGATATTCCGACCTCTATTATTTAT
CGTATCGTGGACGACAACCTGCCGAAGTTTCTGGAAAACAAAGCGAAATATGAATCTCTGA
 
AAGACAAAGCACCGGAAGCTATTAACTATGAACAGATCAAGAAAGATCTGGCGGAAGAAC
TGACCTTCGACATCGACTATAAAACCTCCGAAGTTAACCAGCGTGTTTTCTCACTGGACGA
GGTTTTCGAAATCGCTAATTTCAACAATTACCTGAATCAATCTGGCATCACCAAATTCAACA
CCATTATTGGTGGCAAATTTGTTAACGGCGAAAACACCAAGCGTAAGGGCATCAACGAATA
CATTAACCTGTATAGCCAACAAATCAACGACAAAACCCTGAAAAAGTATAAAATGTCCGTTC
TGTTTAAACAGATTTTATCGGACACCGAATCTAAATCCTTCGTAATTGATAAACTGGAAGAT
GATAGCGACGTTGTCACCACGATGCAGAGCTTTTATGAGCAGATTGCGGCGTTCAAAACC
GTGGAAGAGAAATCTATTAAAGAAACTCTGTCCCTGCTCTTTGACGACCTCAAAGCGCAGA
AACTAGATCTGTCTAAGATTTACTTTAAAAACGACAAATCTCTGACCGATCTCAGTCAACAA
GTTTTCGATGACTATAGCGTGATCGGCACGGCAGTTTTGGAATACATCACCCAACAAATCG
CGCCGAAAAATCTGGACAACCCGTCCAAGAAGGAACAGGAACTGATTGCAAAGAAAACAG
AAAAAGCTAAATACCTGAGCTTAGAAACTATCAAACTGGCACTTGAGGAATTTAATAAACAT
CGTGATATTGATAAACAGTGTCGTTTTGAGGAAATTCTGGCGAACTTTGCGGCAATCCCGA
TGATCTTCGACGAAATTGCTCAAAACAAAGACAATCTGGCGCAGATCTCTATCAAGTACCA
GAATCAGGGTAAGAAAGATCTGCTTCAAGCATCTGCGGAGGACGATGTGAAAGCAATTAA
AGACTTATTAGATCAGACGAATAACTTATTACACAAGCTCAAAATCTTCCACATCAGCCAGA
GCGAGGACAAGGCGAACATTCTGGATAAAGATGAACACTTCTATCTGGTGTTCGAAGAAT
GTTACTTCGAACTGGCAAACATCGTCCCTCTCTACAATAAAATCCGCAACTACATCACGCA
GAAGCCTTACTCTGACGAGAAATTCAAACTGAACTTCGAAAACAGCACGCTGGCGAACGG
CTGGGATAAGAACAAAGAGCCGGACAACACCGCAATCCTGTTCATCAAAGACGACAAATA
CTATCTGGGCGTAATGAACAAGAAGAACAACAAGATCTTCGACGATAAAGCGATCAAAGAA
AACAAGGGTGAAGGCTATAAGAAAATCGTGTACAAGCTCCTGCCGGGTGCGAATAAAATG
TTACCGAAAGTGTTCTTTTCCGCGAAAAGCATCAAATTCTACAACCCGTCTGAGGATATTCT
GCGCATCCGCAATCATAGCACGCACACTAAAAACGGTAGCCCGCAGAAAGGGTATGAAAA
ATTCGAATTTAATATAGAGGACTGCCGTAAGTTCATCGACTTCTATAAACAGAGCATTTCCA
AACATCCGGAATGGAAAGACTTCGGCTTCCGTTTCTCTGACACTCAGCGCTATAATAGCAT
CGACGAGTTCTACCGCGAAGTGGAGAATCAGGGCTATAAACTGACCTTCGAGAACATTAG
TGAGTCGTACATCGACTCCGTTGTGAATCAGGGTAAACTGTACCTGTTTCAGATCTATAATA
AAGACTTTAGCGCGTACAGCAAAGGCCGTCCGAATCTGCACACCCTTTACTGGAAAGCATT
ATTTGACGAACGTAACCTGCAAGATGTGGTGTATAAACTGAACGGTGAGGCGGAACTTTTC
TACCGTAAACAGAGTATCCCGAAGAAAATCACGCATCCGGCAAAAGAAGCTATTGCCAACA
AAAACAAAGACAACCCGAAGAAAGAATCAGTATTCGAATATGACCTGATCAAAGATAAACG
TTTCACCGAAGATAAGTTCTTTTTCCACTGTCCGATTACCATCAACTTCAAATCTAGCGGTG
CGAACAAGTTCAACGATGAAATTAACTTATTACTGAAAGAGAAAGCTAATGACGTACACATC
TTATCTATTGATCGCGGTGAACGTCATTTAGCATACTATACACTGGTAGATGGTAAAGGTAA
TATTATTAAACAGGATACTTTCAATATTATCGGTAATGACCGTATGAAAACCAACTATCACG
ATAAGCTGGCGGCGATCGAAAAAGATCGTGATTCTGCGCGTAAAGATTGGAAGAAAATTAA
CAATATCAAAGAAATGAAAGAAGGCTATCTGAGCCAAGTGGTGCACGAGATCGCAAAACT
 
GGTGATTGAATATAACGCTATCGTGGTTTTCGAAGATCTGAACTTTGGTTTTAAACGTGGTC
GCTTCAAAGTAGAAAAACAGGTGTACCAAAAACTGGAAAAAATGCTGATTGAAAAACTGAA
CTATCTGGTTTTTAAAGACAACGAATTTGACAAAACGGGTGGCGTACTCCGTGCCTATCAG
CTGACCGCTCCGTTCGAAACGTTCAAGAAAATGGGTAAACAAACGGGGATTATCTATTATG
TGCCAGCTGGTTTCACCTCCAAGATTTGTCCAGTTACGGGCTTCGTTAACCAGCTGTACCC
GAAATACGAGAGCGTTAGCAAATCTCAAGAATTTTTCAGCAAATTCGACAAGATCTGCTATA
ATCTGGATAAAGGCTATTTCGAGTTCAGCTTCGATTACAAAAACTTCGGCGATAAAGCGGC
TAAAGGTAAGTGGACTATTGCTAGCTTTGGTAGCCGTCTGATTAACTTTCGCAACTCCGAC
AAAAACCATAATTGGGACACGCGTGAAGTGTATCCGACCAAAGAACTGGAAAAATTACTGA
AAGACTATTCCATCGAATATGGTCATGGGGAGTGCATTAAAGCGGCGATTTGCGGTGAATC
CGATAAGAAATTTTTCGCCAAACTGACCAGCGTGCTTAACACCATTCTGCAAATGCGTAATT
CTAAAACGGGTACGGAGCTGGACTACCTGATTTCTCCGGTAGCCGACGTTAACGGCAACT
TCTTCGATTCTCGTCAAGCACCGAAAAATATGCCACAAGACGCGGATGCCAACGGTGCAT
ACCATATCGGCTTAAAAGGCTTAATGTTATTAGGCCGTATCAAGAATAATCAGGAGGGCAA
GAAATTAAATCTGGTTATCAAAAACGAAGAATACTTCGAGTTCGTTCAGAATCGTAACAATT
AATGTATGCTTAAGCAGCTCGGTACCAAAGACGAACAATAAGACGCTGAAAAGCGTCTTTT
TTCGTTTTGGTCCTGTTGCGGCGCGATAGTGTGAACATGCTATAGACTTCTGGTGCTACCC
GACTGACAATTAATCATCCGGCTCGTATAATGCTAGCAATTTCTACTGTTGTAGATTCGCG
GTGATGAACGCTTTCCAATTCGAGACGAACAATAAGGCCTCCCTAACGGGGGGCCTTTTTT
ATTGATAACAAAAGTAACTTCGAGCTTGTCTACCTCCTAGCACCATTATTGCAATTAATAAA
CAACTAACGGACAATTCTACCTAACAGTTTTCATATATGACGAGCAGTTAAGTGATGAGTAA
AGGTGAGGAATTATTTACTGGTGTTGTTCCGATCTTAGTTGAACTGGACGGCGATGTTAAC
GGTCATAAATTCAGTGTTCGTGGTGAAGGTGAAGGTGATGCAACCAACGGTAAGCTGACC
CTGAAATTCATCTGCACTACTGGAAAATTACCAGTACCGTGGCCTACTCTGGTGACTACCC
TGACCTATGGTGTTCAGTGTTTTTCTCGTTACCCTGACCACATGAAGCAACATGATTTCTTC
AAATCTGCAATGCCGGAAGGTTATGTACAGGAGCGCACCATTTCTTTCAAAGACGATGGCA
CGTATAAAACCCGTGCAGAGGTTAAATTTGAAGGTGACACTCTGGTGAATCGTATTGAACT
GAAAGGCATTGATTTCAAAGAGGACGGCAATATTTTAGGCCACAAACTGGAATATAACTTC
AACTCCCATAACGTTTACATCACCGCAGACAAACAGAAGAACGGTATCAAAGCTAACTTCA
AAATTCGCCATAACGTTGAAGATGGTAGCGTACAGCTGGCGGATCATTACCAACAGAACA
CTCCGATTGGAGATGCTCCTGTTTTACTGCCGGATAACCACTACCTGTCCACCCAGTCTAA
ACTGTCGAAGGATCCGAACGAAAAGCGCGACCACATGGTGTTATTAGAGTTCGTTACCGC
TAGTGGTATCACGCACGGTATGGATGAACTCTACAAATAAGACGAACAATAAGGGGAGCG
GGAAACCGCTCCCCTTTTTATTGATAACAAAAGTAAATTGCACGCTGATAGTCTCCCAATT
GCGAAGGACCAAAACGAAAAAACACCCTTTCGGGTGTCTTTTCTGGAATTTGGTACCGAGT
ACTAGGTATCGTGTAAGTAGCGAAGGCCCGTACGCGAGATAAACTGCTAGGCAACCGCGA
CTCTACGACTGGTGCTCGATTTAATTTCGCTGACGTAAAGAAATTATCGGCAGTGCGTCAA
CTGCCGTATCTTTATCTTAATTAGGTAGTTGGACAAGCCCTTGAAAGAAATAGCAAGAGCC
 
TGCCTCTCTATTGAAGTCACGGCGAAAGTCGGGTAGAAATCAAAGAAAGCAGAAATTAAAT
CGGAGTAACACTAAGGTGGGATAACTCCGTAACTGACTACGCCTTTCTCTAGACTTTACTT
GACCAGATACACTGTCTTTGACACGTTGAAGGATTAGAGCAATCAAATCCAAGACTGGCTA
AGCACGAAGCAACTCTTGAGTGTTAAAAAGTTATCTCCTGTATTCGGGAAGCGGGTACTAG
AAGATTGCAGGGACTCCGACGTTAAGTAAATTACAAAGTAATAAGTATCGTTCAGGATCAC
GTTACCGCAATAAGAAGCGAGAATAATATAATTTCCGAAGTGCTTACCCCAGTAGTGACTA
TTCCTATAACCCTTCTGAGTGTCCGGAGGCGGAAATTTGCCACGAAAGAGAAAGTATTTCC
CCGACAATAATAAAGGGGCGCTCCTCAGCTTTTCCACTTGGTTGGGTAAGCTAGGCAACT
CTGAAAGGAGTTTCGGCGAATTGAAGCCGACAGCTTTGAATTGTTTTAGGGGCGTTATTCG
AGGGCAATCGGAGCTAACTTCAAGACTACTTCTTTGTTGAATACTAAATAGTGCAAAGGTC
GTGTTTCCTCAAGGATACTCCGCTAACAATATAGGATTCCAATCAGATTCAGCACTGGCGG
TACGGGTGTTGCGGTGAGGCGTTCGGGTTTACGGCTCGAAGCTAGCACGGTAGGAAGCC
TGACAATCACCAAGCAAAAGGGCCGTCGAAGGCCCACAAGATACGAAAGCTCTCGAAGCC
TTATCCTTGACCGATCCACCTATTTAGGCAGTTACGCACAAAAGCTACCCAATAATCCGTG
ACAGGCACAATATCACGGAACAAAACCGAAAACTCTCGTACACGGTTAGGTTTTCGCTAGG
AAGAATAAACCTCTATCTTGATTATAAGAAGGCTCCCCAAGCACCCCCAAAACCGAAATAG
CGGTTTGCAATAAGGGACAAGTTACGAGTGTAGACACGCAGAATTATCCAGCCTTTAGTCT
TTAGGAAGGCAAAGCTATTGTACGCGGTAGCCGTCGTAGCAATTTACCAACTGTAGAATTA
TTGGACACACGTAGGAAGGGCTTACAGTTGAAGTTTAATAAGGTCACACGCAAAACCGCTA
AGGAATAATCGCACCGTTAGCGAAAGAATATTTCAGAGCGGTTAGTAAAGGTTGAGTAAAG
TGAGATTCCAAAGTGAGCCTTTATAAAAAGTAAAGAGCTATAATAAAACCGTCGAGCAGAA
AACAATCGCCTGAAATCTCAAGCACGTTGCCCTTTCTAACGTCGCTAAGGTTTCGTAAACC
CGTTTGATTAGGAAGAAGAATAAGTAACCCGATTAGGTTTGAGATCGCGGGTTATCGGTTT
GGATTAAAAGTGGATACCAGCGGAGTCAACGCCGACGCAAACGTACAGTGATCCAATCCT
GTTGCACGGTCAAGCACAATCAGCTCGCAAGATCTTGGAATAGTGTGCCCAACAGTTTAGT
TGAGGGCCACGTTCCGACTACAAGTTGCTTCAAGAGGGGAATTTGGATTTGGCAATAGCC
CCCCGTTTCTACCTCAAGAGGCGACGAGTATTAACCGCGCCAGCTGTCGGCACAAGGGC
CAAAGAAGATTCCAATTTCTTATTCCCGAATAACCTCCGAATCCCTGCGGGAAAATCACCG
ACCGAATAGCCTAGAAGCAAGGGGGAACAGATAGGTATAATTAGCTTAAGAGAGTACCAG
CCGTGACAACAGCGTAGTAACCACAAACTTACGCTGGGGCTTCTTTGGCGGATTTTTACAG
ATACTAACAAGGTGATTTGAAGTACCTTAGTTGAGGATTTAAACGCGCTATCCGGTAATCTC
CAAATTGGGAAATACCGTTCAAAGAGGGCTAGAATTACTTAAAAGCCTTCACACCGCCTGC
GCTATACGCGCCCACTCTCCCGTTTATCCGTCCAAGCGGAAGCAGGGCGATCCTCCGCTA
AGATATTCTTACGTGTAACGTAGCTAAGTATCCCAAATAGCTGGCGTACGCGTTGAACACC
GCCTAGAGGATCGTGACTCGCCGGACGAGCGTGTTATTGGGGACTTACGCCAGCGTAGA
CTACAACGCGCCCAGATTAACCCTGCACGTATTGCCTTGAATAACGTACTAATCTCTCCGG
CTCTCGACAATCTATCGAGCGACTCGATTATCAACGGGTGTCTTGCAGTTCTAATCTCTTG
CCCCCGCCCGTAATAGCCTCCAAGAGATTGAAGATAGTAAAGGGCAAGAGCTGATTCGGC
 
GTTGAAGGATAGCGGACTTTCGGTCAACCACAATTCCCCACTCGACAAAACCAGCCGTGC
GAATAACTCTGAAAGTACAAGCAACCCAAGAGGGCTGAGCCTAAACTCAGCTAATTCCTAA
GTGAGCTAAAGACTCGAAGTGACAGCTCTTAATAAATAGAGCGGGAACGTCGAACGGTCG
TGAAAGTAATAGTACAACGGGTATTAACTTACTGAGGATATTGCTTGAAGCTGTACCGTTTT
ATTGGGTGAACGAATAAGATCCAGCAATTCAGCCAAAGAAGCTACCAATTTTAGTTTAAG
AGTGTCACGTCTGACCTCGCGGGTAGATTGCCGAACGTAGAGCTTACGAGCCAGCGGAAA
CAGTAGCCGCAGGATAAGTAAGGGGAGTAAGTGATCGAACGAATCAGAAGTGACAATATA
CTTAGGCTGGATCTCGTCCCGTGAATCCCAACCCTCACCAACTACGAGATAAGAGGTAAG
CCAAAAATCGACTTGGTGGCGACCAACGACTGTTCCCCCCCTGTAACTAATCGTTCCGTCA
AAACCTGACTTACTTCAAGGCCAATTCCAAGCGCAAACAATACCGTCCTAGTTCTTCGGTT
AAGTTTCCGAAGTAGGAGTGAGCCTACCTCCGTTTGCGTCTTGTTACCACTGACCCAGCTA
TTTACTTTGTATTGCCTGCAATCGAATTTCTGAACTCTCAGATAGTGGGGATAACGGGAAA
GTTCCTATATTTGCGAACTAACTTAGCCGTCCACCTCGAAGCTACCTACTCACACCCACCC
CGCGCGGGGTAAATAAGGCACTAATCCCAGCTGAGAGCTGGCGTAGCACTTAGCCACAA
GTTAATTAACAGTTGTCTGGTAGTTTGGCGGTATTAGGAAGATCCTAGAAGCAAGGCAGAG
TTAGTTCTAACCTAAAGCCACAAATAAGACAGGTTGCCAAAGCCCGCCGGAAATTAAATCT
TGCTCAGTTCGGTAACGGAGTTTCCCTCCCGCGTACTTAATTCCCAATAAGAAACGCGCCC
AAGTCCTATCAGGCAAAATTCAGCCCCTTCCCGTGTTAGAACGAGGGTAAAAATACAAGCC
GATTGAACAAGGGTTGGGGGCTTCAAATCGTCGTTTACCCCACTTTACAACGGAGATTAAG
TAGTTCACCCTATAGTACGAAGCAGAACTATTTCGAGGGGCGTGCAATAATCGAATCTTCT
GCGGTTGACTTAACACGCTAGGGACGTGCCCTCGATTCAATCGAAGGTACTCCTACTCAG
ACTGCCTCACACCCAGCTAGTCACTGAGCGATAAAATTGACCCGCCCTCTAGGGAAGCGA
GTACGTCCCAAAGGGCTCCGGACAGGGCTATATAGGAGAGTTTGATCTCGCCCCGACAAC
TGCAACCCTCAACTCCCTTAGATAATATTGTTAGCCGAAGTTGCACGACCCGCCGTCCACG
GACTGCTCTTAGGGTGTGGCTCCTTAATCTGACAACGTGCAACCCCTATCGAAGTCGATTG
TTTCTGCGAAAGGTGTTGTCCTAATAGTCCCGAAATTTGGCCCTTGTAGGTGTGAAACCAC
TTAGCTTCGCGCCGTAGTCCTAAAGGCCCACCTATTGACTTTGTTTCGGGTAGCACTAGGA
ATCTTAACAATTTGAATTTGGACGTGGAACGCGTACACCTTAATCTCCGAATAATTCTAGGG
ATTTGGAAGTCCTCTACGTTGACACACCTACACTGCTCGAAGTAAATATACGAATAACGCG
GGCCTCGCGGAGCCGTTCCGAATCGTCACGTGTTCGTTTACTGTTAATTGGTGGCAAATAA
GCAATATCGTAGTCCGTCAGGCCCAGCCCTGTTATCCACGGCGTTATTTGTCAAATTGCGT
AGAACTGGATTGACTGCCTGACAATACCTAATTATCGGTACGAAGTCCCCGAATCTGTCGG
GCTATTTCACTAATACTTTCCAAACGCCCCGTATCCAAGAAGAACGAATTTATCCACGCTCC
CGTCTTTGGGACGAATACCGCTACAAGTGGACAGAGGATCGGTACGGGCCTCTAATAAAT
CCAACACTCTACGCCCTCTTCAAGAGCTAGAAGAACAGGGTGCAGTTGGAAAGGAATTA
TTTCGTAAGGCGAGCCAATACCGTAATTAATTCGGAAGAGTTAACACGATTGGAAGTAGGA
ATAGTTTCTAACCACGGTTACTAATCCTAATAACGGAACGCTGTCTGATAGATTAGTGTCAG
CGCTCGGTACCAAAGAAAAATAAAAAGACGCTGAAAAGCGTCTTTTTATTTTTCGGTCCAG
 
TGTAACTCAGGCAAAAGCACGTAATATTCGTACTTTCTTCCTCCGTAAGCGTCACCCACATT
CCTTAAAGAGTGCATGTGCATATTTTGTTATCAATAAAAAGGCCGCGATTTGCGGCCTTAT
TGTTCGTCTTGCCGGATTACGCCCCGCCCTGCCACTCATCGCAGTATTGTTGTAATTCATT
AAGCATTCTGCCGACATGGAAGCCATCACAAACGGCATGATGAACTTGGATCGCCAGTGG
CATTAACACCTTGTCGCCTTGCGTATAATATTTCCCATAGTGAAAACGGGGGCGAAGAAG
TTGTCCATATTTGCTACGTTTAAATCAAAACTGGTGAAACTCACCCACGGATTGGCACTGA
CGAAAAACATATTTTCGATAAACCCTTTAGGGAAATATGCTAAGTTTTCACCGTAACACGCC
ACATCTTGACTATATATGTGTAGAAACTGCCGGAAATCGTCGTGGTATTCTGACCAGAGCG
ATGAAAACGTTTCAGTTTGCTCATGGAAAACGGTGTAACAAGGGTGAACACTATCCCATAT
CACCAGCTCACCGTCTTTCATTGCCATACGAAACTCCGGATGTGCATTCATCAGGCGGGC
AAGAATGTGAATAAAGGCCGGATAAAACTTGTGCTTATTTTTCTTTACGGTTTTTAAAAAGG
CCGTAATATCCAGCTGAACGGTTTGGTTATAGGTGCACTGAGCAACTGACTGGAATGCCTC
AAAATGTTCTTTACGATGCCATTGACTTATATCAACTGTAGTATATCCAGTGATTTTTTTCTC
CATTTTAGCTTCCTTAGCTTGCGAAATCTCGATAACTCAAAAAATAGTAGTGATCTTATTTCA
TTATGGTGAAAGTTGTCTTACGTGCAACATTTTCGCAAAAAGTTGGCGCTTTATCAAC (SEQ
ID NO: 31)
- p844: RBS1-AK272-19-CTX1/3-4nt

Payload encoding a Fn Cpf1 and an associated duplex of crRNA targeting different conserved regions of CTX-M gene variants ("CTX1": GCCAGATCACCGCGATATCGTTGG, SEQ ID NO: 28; "CTX3": TCGCGGTGATGAACGCTTTCCAAT, SEQ ID NO: 30).

ACTGTCGGAATGACAAATGGTTCCAATTATTGAACACCCTTCGGGGTGTTTTTTTGT
TTCTGGTTTCCCGAGGCCGGCCTGCGCTAGCGGAGTGTATACTGGCTTACTATGTTGGCA
CTGATGAGGGTGTAAGTGAAGTGCTTCATGTGGCAGGAGAAAAAAGGCTGCATCGGTGCG
TCAGCAGAATATGTGATACAGGATATATTCCGCTTCCTCGCTCACTGACTCGCTACGCTCG
GTCGTTCGACTGTGGCGAGCGGAAATGGCTTACGAACGGGGCGGAGATTTCCTGGAAGA
TGCCAGGAAGATACTTAACAGGGAAGTGAGAGGGTCGCGGCAAAGCCGTTTTTCCATAGG
CTCCGCCCCCCTGACAAGCATCACGAAATCTGACGCTCAAATCAGTGGTGGCGAAACCTG
ACAGGACTATAAAGATACCAGGCGTTTCCCCCTGGCGGCTCCCTCGTGCGCTCTCCTGTT
CCTGCCTTTCGGTTTGCCGGTGTCATTCCTCTGTTACGGCCGAGTTTGTCTCATTCCACGC
CTGACACTCAGTTCCGGGTAGGCAGTTCGCTCCAAGCTGGACTGTATGCACGAACCCCCC
GTTCAGTCCGACCGCTGCGCCTTATCCGGTAACTATCGTCTTGAGTCCAACCCGGAAAGA
CATGCAAAAGCACCACTGGCAGCAGCCACTGGTAATTGATTTAGAGGAGTTAGTCTTGAAG
TCATGCGCCGGATAAGGCTAAACTGAAAGGACAAGTTTTGGCGACTGCGCTCCTCCAAGC
CAGTTACCTCGGTTCAAAGAGTTGGTAGCTCAGAGAACCTTCGAAAAACCGCCCTGCAAG
GCGGTTTTTTCGTTTTCAGAGCAAGAGATTACGCGCAGACCAAAACGATCTCAAGAAGATC
ATCTTATTAATCAGATAAAATATTTCTAGATTTCAGTGCAATTTATCTCTTCAAATGTAGCAC
CGGCGCGCCGTGACCAATTATTGAAGGCCGCTAACGCGGCCTTTTTTTGTTTCTGGTATCC

CGAATGGAGCGACTTCTCCCCAAAAAGCCTCGCTTTCAGCACCTGTCGTTTCCTTTCTTTT
CAGAGGGTATTTTAAATAAAAACATTAAGTTATGACGAAGAAGAACGGAAACGCCTTAAAC
CGGAAAATTTTCATAAATAGCGAAAACCCGCGAGGTCGCCGCCCCGTAACCTGTCGGATC
ACCGGAAAGGACCCGTAAAGTGATAATGATTATCATCTACATATCACAACGTGCGTAAAGG
GTAAGTATGAAGGTCGTGTACTCCATCGCTACCAAATTCCAGAAAACAGACGCTTTCGAGC
GTCTTTTTTCGTTTTGGTCACGACGTACGGTGGAAGATTCGTTACCAATTGACAGCTAGCT
CAGTCCTAGGTATATACATACATGCTTGTTTGTTTGTAAACTACTGTTTTCATTAAAGAGGA
GAAAGGAAGCCATGTCCATCTATCAGGAGTTTGTTAACAAGTATTCCCTGTCTAAAACCCT
GCGTTTTGAACTGATCCCGCAGGGCAAAACTTTGGAAAACATTAAAGCGCGTGGCCTGATT
CTGGATGACGAAAAACGTGCAAAGGATTACAAGAAAGCTAAACAGATCATCGACAAATATC
ACCAGTTCTTTATCGAAGAAATTCTGTCGTCGGTGTGCATCAGTGAGGATCTGTTACAGAA
TTATTCTGATGTATACTTTAAACTTAAAAAGTCCGATGACGATAATCTGCAAAAAGATTTCAA
GTCAGCCAAAGATACCATCAAGAAACAGATCTCAGAATATATTAAAGATAGCGAAAAGTTC
AAAAACCTGTTTAACCAAAACCTCATTGATGCTAAGAAAGGCCAAGAATCTGACCTGATCTT
ATGGCTGAAACAGAGCAAAGATAACGGCATTGAACTGTTCAAAGCTAATAGCGACATCACC
GATATTGATGAAGCGCTCGAAATCATCAAGTCTTTCAAAGGCTGGACGACGTATTTCAAAG
GTTTTCATGAAAACCGTAAGAATGTATATTCGAGCAACGATATTCCGACCTCTATTATTTAT
CGTATCGTGGACGACAACCTGCCGAAGTTTCTGGAAAACAAAGCGAAATATGAATCTCTGA
AAGACAAAGCACCGGAAGCTATTAACTATGAACAGATCAAGAAAGATCTGGCGGAAGAAC
TGACCTTCGACATCGACTATAAAACCTCCGAAGTTAACCAGCGTGTTTTCTCACTGGACGA
GGTTTTCGAAATCGCTAATTTCAACAATTACCTGAATCAATCTGGCATCACCAAATTCAACA
CCATTATTGGTGGCAAATTTGTTAACGGCGAAAACACCAAGCGTAAGGGCATCAACGAATA
CATTAACCTGTATAGCCAACAAATCAACGACAAAACCCTGAAAAAGTATAAAATGTCCGTTC
TGTTTAAACAGATTTTATCGGACACCGAATCTAAATCCTTCGTAATTGATAAACTGGAAGAT
GATAGCGACGTTGTCACCACGATGCAGAGCTTTTATGAGCAGATTGCGGCGTTCAAAACC
GTGGAAGAGAAATCTATTAAAGAAACTCTGTCCCTGCTCTTTGACGACCTCAAAGCGCAGA
AACTAGATCTGTCTAAGATTTACTTTAAAAACGACAAATCTCTGACCGATCTCAGTCAACAA
GTTTTCGATGACTATAGCGTGATCGGCACGGCAGTTTTGGAATACATCACCCAACAAATCG
CGCCGAAAAATCTGGACAACCCGTCCAAGAAGGAACAGGAACTGATTGCAAAGAAAACAG
AAAAAGCTAAATACCTGAGCTTAGAAACTATCAAACTGGCACTTGAGGAATTTAATAAACAT
CGTGATATTGATAAACAGTGTCGTTTTGAGGAAATTCTGGCGAACTTTGCGGCAATCCCGA
TGATCTTCGACGAAATTGCTCAAAACAAAGACAATCTGGCGCAGATCTCTATCAAGTACCA
GAATCAGGGTAAGAAAGATCTGCTTCAAGCATCTGCGGAGGACGATGTGAAAGCAATTAA
AGACTTATTAGATCAGACGAATAACTTATTACACAAGCTCAAAATCTTCCACATCAGCCAGA
GCGAGGACAAGGCGAACATTCTGGATAAAGATGAACACTTCTATCTGGTGTTCGAAGAAT
GTTACTTCGAACTGGCAAACATCGTCCCTCTCTACAATAAAATCCGCAACTACATCACGCA
GAAGCCTTACTCTGACGAGAAATTCAAACTGAACTTCGAAAACAGCACGCTGGCGAACGG
CTGGGATAAGAACAAAGAGCCGGACAACACCGCAATCCTGTTCATCAAAGACGACAAATA

CTATCTGGGCGTAATGAACAAGAAGAACAACAAGATCTTCGACGATAAAGCGATCAAAGAA
AACAAGGGTGAAGGCTATAAGAAAATCGTGTACAAGCTCCTGCCGGGTGCGAATAAAATG
TTACCGAAAGTGTTCTTTTCCGCGAAAAGCATCAAATTCTACAACCCGTCTGAGGATATTCT
GCGCATCCGCAATCATAGCACGCACACTAAAAACGGTAGCCCGCAGAAAGGGTATGAAAA
ATTCGAATTTAATATAGAGGACTGCCGTAAGTTCATCGACTTCTATAAACAGAGCATTTCCA
AACATCCGGAATGGAAAGACTTCGGCTTCCGTTTCTCTGACACTCAGCGCTATAATAGCAT
CGACGAGTTCTACCGCGAAGTGGAGAATCAGGGCTATAAACTGACCTTCGAGAACATTAG
TGAGTCGTACATCGACTCCGTTGTGAATCAGGGTAAACTGTACCTGTTTCAGATCTATAATA
AAGACTTTAGCGCGTACAGCAAAGGCCGTCCGAATCTGCACACCCTTTACTGGAAAGCATT
ATTTGACGAACGTAACCTGCAAGATGTGGTGTATAAACTGAACGGTGAGGCGGAACTTTTC
TACCGTAAACAGAGTATCCCGAAGAAAATCACGCATCCGGCAAAAGAAGCTATTGCCAACA
AAAACAAAGACAACCCGAAGAAAGAATCAGTATTCGAATATGACCTGATCAAAGATAAACG
TTTCACCGAAGATAAGTTCTTTTTCCACTGTCCGATTACCATCAACTTCAAATCTAGCGGTG
CGAACAAGTTCAACGATGAAATTAACTTATTACTGAAAGAGAAAGCTAATGACGTACACATC
TTATCTATTGATCGCGGTGAACGTCATTTAGCATACTATACACTGGTAGATGGTAAAGGTAA
TATTATTAAACAGGATACTTTCAATATTATCGGTAATGACCGTATGAAAACCAACTATCACG
ATAAGCTGGCGGCGATCGAAAAAGATCGTGATTCTGCGCGTAAAGATTGGAAGAAAATTAA
CAATATCAAAGAAATGAAAGAAGGCTATCTGAGCCAAGTGGTGCACGAGATCGCAAAACT
GGTGATTGAATATAACGCTATCGTGGTTTTCGAAGATCTGAACTTTGGTTTTAAACGTGGTC
GCTTCAAAGTAGAAAAACAGGTGTACCAAAAACTGGAAAAAATGCTGATTGAAAAACTGAA
CTATCTGGTTTTTAAAGACAACGAATTTGACAAAACGGGTGGCGTACTCCGTGCCTATCAG
CTGACCGCTCCGTTCGAAACGTTCAAGAAAATGGGTAAACAAACGGGGATTATCTATTATG
TGCCAGCTGGTTTCACCTCCAAGATTTGTCCAGTTACGGGCTTCGTTAACCAGCTGTACCC
GAAATACGAGAGCGTTAGCAAATCTCAAGAATTTTTCAGCAAATTCGACAAGATCTGCTATA
ATCTGGATAAAGGCTATTTCGAGTTCAGCTTCGATTACAAAAACTTCGGCGATAAAGCGGC
TAAAGGTAAGTGGACTATTGCTAGCTTTGGTAGCCGTCTGATTAACTTTCGCAACTCCGAC
AAAAACCATAATTGGGACACGCGTGAAGTGTATCCGACCAAAGAACTGGAAAAATTACTGA
AAGACTATTCCATCGAATATGGTCATGGGGAGTGCATTAAAGCGGCGATTTGCGGTGAATC
CGATAAGAAATTTTTCGCCAAACTGACCAGCGTGCTTAACACCATTCTGCAAATGCGTAATT
CTAAAACGGGTACGGAGCTGGACTACCTGATTTCTCCGGTAGCCGACGTTAACGGCAACT
TCTTCGATTCTCGTCAAGCACCGAAAAATATGCCACAAGACGCGGATGCCAACGGTGCAT
ACCATATCGGCTTAAAAGGCTTAATGTTATTAGGCCGTATCAAGAATAATCAGGAGGGCAA
GAAATTAAATCTGGTTATCAAAAACGAAGAATACTTCGAGTTCGTTCAGAATCGTAACAATT
AATGTATGCTTAAGCAGCTCGGTACCAAAGACGAACAATAAGACGCTGAAAAGCGTCTTTT
TTCGTTTTGGTCCTGTTGCGGCGCGATAGTGTGAACATGCTATAGACTTCTGGTGCTACCC
GACTGACAATTAATCATCCGGCTCGTATAATGCTAGCAATTTCTACTGTTGTAGATGCCAGA
TCACCGCGATATCGTTGGAATTTCTACTGTTGTAGATTCGCGGTGATGAACGCTTTCCAAT
TCGAGACGAACAATAAGGCCTCCCTAACGGGGGGCCTTTTTTATTGATAACAAAAGTAACT

TCGAGCTTGTCTACCTCCTAGCACCATTATTGCAATTAATAAACAACTAACGGACAATTCTA
CCTAACAGTTTTCATATATGACGAGCAGTTAAGTGATGAGTAAAGGTGAGGAATTATTTACT
GGTGTTGTTCCGATCTTAGTTGAACTGGACGGCGATGTTAACGGTCATAAATTCAGTGTTC
GTGGTGAAGGTGAAGGTGATGCAACCAACGGTAAGCTGACCCTGAAATTCATCTGCACTA
CTGGAAAATTACCAGTACCGTGGCCTACTCTGGTGACTACCCTGACCTATGGTGTTCAGTG
TTTTTCTCGTTACCCTGACCACATGAAGCAACATGATTTCTTCAAATCTGCAATGCCGGAAG
GTTATGTACAGGAGCGCACCATTTCTTTCAAAGACGATGGCACGTATAAAACCCGTGCAGA
GGTTAAATTTGAAGGTGACACTCTGGTGAATCGTATTGAACTGAAAGGCATTGATTTCAAA
GAGGACGGCAATATTTTAGGCCACAAACTGGAATATAACTTCAACTCCCATAACGTTTACAT
CACCGCAGACAAACAGAAGAACGGTATCAAAGCTAACTTCAAAATTCGCCATAACGTTGAA
GATGGTAGCGTACAGCTGGCGGATCATTACCAACAGAACACTCCGATTGGAGATGCTCCT
GTTTTACTGCCGGATAACCACTACCTGTCCACCCAGTCTAAACTGTCGAAGGATCCGAACG
AAAAGCGCGACCACATGGTGTTATTAGAGTTCGTTACCGCTAGTGGTATCACGCACGGTAT
GGATGAACTCTACAAATAAGACGAACAATAAGGGGAGCGGAAACCGCTCCCCTTTTTAT
TGATAACAAAAGTAAATTGCACGCTGATAGTCTCCCAATTGCGAAGGACCAAAACGAAAAA
ACACCCTTTCGGGTGTCTTTTCTGGAATTTGGTACCGAGTACTAGGTATCGTGTAAGTAGC
GAAGGCCCGTACGCGAGATAAACTGCTAGGCAACCGCGACTCTACGACTGGTGCTCGATT
TAATTTCGCTGACGTAAAGAAATTATCGGCAGTGCGTCAACTGCCGTATCTTTATCTTAATT
AGGTAGTTGGACAAGCCCTTGAAAGAAATAGCAAGAGCCTGCCTCTCTATTGAAGTCACG
GCGAAAGTCGGGTAGAAATCAAAGAAAGCAGAAATTAAATCGGAGTAACACTAAGGTGGG
ATAACTCCGTAACTGACTACGCCTTTCTCTAGACTTTACTTGACCAGATACACTGTCTTTGA
CACGTTGAAGGATTAGAGCAATCAAATCCAAGACTGGCTAAGCACGAAGCAACTCTTGAGT
GTTAAAAAGTTATCTCCTGTATTCGGGAAGCGGGTACTAGAAGATTGCAGGACTCCGAC
GTTAAGTAAATTACAAAGTAATAAGTATCGTTCAGGATCACGTTACCGCAATAAGAAGCGA
GAATAATATAATTTCCGAAGTGCTTACCCCAGTAGTGACTATTCCTATAACCCTTCTGAGTG
TCCGGAGGCGGAAATTTGCCACGAAAGAGAAAGTATTTCCCCGACAATAATAAAGGGGCG
CTCCTCAGCTTTTCCACTTGGTTGGGTAAGCTAGGCAACTCTGAAAGGAGTTTCGGCGAAT
TGAAGCCGACAGCTTTGAATTGTTTTAGGGGCGTTATTCGAGGGCAATCGGAGCTAACTTC
AAGACTACTTCTTTGTTGAATACTAAATAGTGCAAAGGTCGTGTTTCCTCAAGGATACTCCG
CTAACAATATAGGATTCCAATCAGATTCAGCACTGGCGGTACGGGTGTTGCGGTGAGGCG
TTCGGGTTTACGGCTCGAAGCTAGCACGGTAGGAAGCCTGACAATCACCAAGCAAAAGGG
CCGTCGAAGGCCCACAAGATACGAAAGCTCTCGAAGCCTTATCCTTGACCGATCCACCTA
TTTAGGCAGTTACGCACAAAAGCTACCCAATAATCCGTGACAGGCACAATATCACGGAACA
AAACCGAAAACTCTCGTACACGGTTAGGTTTTCGCTAGGAAGAATAAACCTCTATCTTGATT
ATAAGAAGGCTCCCCAAGCACCCCCAAAACCGAAATAGCGGTTTGCAATAAGGGACAAGT
TACGAGTGTAGACACGCAGAATTATCCAGCCTTTAGTCTTTAGGAAGGCAAAGCTATTGTA
CGCGGTAGCCGTCGTAGCAATTTACCAACTGTAGAATTATTGGACACACGTAGGAAGGGC
TTACAGTTGAAGTTTAATAAGGTCACACGCAAAACCGCTAAGGAATAATCGCACCGTTAGC

GAAAGAATATTTCAGAGCGGTTAGTAAAGGTTGAGTAAAGTGAGATTCCAAAGTGAGCCTT
TATAAAAAGTAAAGAGCTATAATAAAACCGTCGAGCAGAAAACAATCGCCTGAAATCTCAA
GCACGTTGCCCTTTCTAACGTCGCTAAGGTTTCGTAAACCCGTTTGATTAGGAAGAAGAAT
AAGTAACCCGATTAGGTTTGAGATCGCGGGTTATCGGTTTGGATTAAAAGTGGATACCAGC
GGAGTCAACGCCGACGCAAACGTACAGTGATCCAATCCTGTTGCACGGTCAAGCACAATC
AGCTCGCAAGATCTTGGAATAGTGTGCCCAACAGTTTAGTTGAGGGCCACGTTCCGACTA
CAAGTTGCTTCAAGAGGGGAATTTGGATTTGGCAATAGCCCCCCGTTTCTACCTCAAGAGG
CGACGAGTATTAACCGCGCCAGCTGTCGGCACAAGGGCCAAAGAAGATTCCAATTTCTTA
TTCCCGAATAACCTCCGAATCCCTGCGGGAAAATCACCGACCGAATAGCCTAGAAGCAAG
GGGGAACAGATAGGTATAATTAGCTTAAGAGAGTACCAGCCGTGACAACAGCGTAGTAAC
CACAAACTTACGCTGGGGCTTCTTTGGCGGATTTTTACAGATACTAACAAGGTGATTTGAA
GTACCTTAGTTGAGGATTTAAACGCGCTATCCGGTAATCTCCAAATTGGGAAATACCGTTC
AAAGAGGGCTAGAATTACTTAAAAGCCTTCACACCGCCTGCGCTATACGCGCCCACTCTC
CCGTTTATCCGTCCAAGCGGAAGCAGGGCGATCCTCCGCTAAGATATTCTTACGTGTAAC
GTAGCTAAGTATCCCAAATAGCTGGCGTACGCGTTGAACACCGCCTAGAGGATCGTGACT
CGCCGGACGAGCGTGTTATTGGGGACTTACGCCAGCGTAGACTACAACGCGCCCAGATTA
ACCCTGCACGTATTGCCTTGAATAACGTACTAATCTCTCCGGCTCTCGACAATCTATCGAG
CGACTCGATTATCAACGGGTGTCTTGCAGTTCTAATCTCTTGCCCCCGCCCGTAATAGCCT
CCAAGAGATTGAAGATAGTAAAGGGCAAGAGCTGATTCGGCGTTGAAGGATAGCGGACTT
TCGGTCAACCACAATTCCCCACTCGACAAAACCAGCCGTGCGAATAACTCTGAAAGTACAA
GCAACCCAAGAGGGCTGAGCCTAAACTCAGCTAATTCCTAAGTGAGCTAAAGACTCGAAG
TGACAGCTCTTAATAAATAGAGCGGGAACGTCGAACGGTCGTGAAAGTAATAGTACAACG
GGTATTAACTTACTGAGGATATTGCTTGAAGCTGTACCGTTTTATTGGGTGAACGAATAAGA
TCCAGCAATTCAGCCAAAGAAGCTACCAATTTTTAGTTTAAGAGTGTCACGTCTGACCTCG
CGGGTAGATTGCCGAACGTAGAGCTTACGAGCCAGCGGAAACAGTAGCCGCAGGATAAG
TAAGGGGAGTAAGTGATCGAACGAATCAGAAGTGACAATATACTTAGGCTGGATCTCGTC
CCGTGAATCCCAACCCTCACCAACTACGAGATAAGAGGTAAGCCAAAAATCGACTTGGTG
GCGACCAACGACTGTTCCCCCCCTGTAACTAATCGTTCCGTCAAAACCTGACTTACTTCAA
GGCCAATTCCAAGCGCAAACAATACCGTCCTAGTTCTTCGGTTAAGTTTCCGAAGTAGGAG
TGAGCCTACCTCCGTTTGCGTCTTGTTACCACTGACCCAGCTATTTACTTTGTATTGCCTGC
AATCGAATTTCTGAACTCTCAGATAGTGGGGATAACGGAAAGTTCCTATATTTGCGAACT
AACTTAGCCGTCCACCTCGAAGCTACCTACTCACACCCACCCCGCGCGGGGTAAATAAGG
CACTAATCCCAGCTGAGAGCTGGCGTAGCACTTAGCCACAAGTTAATTAACAGTTGTCTGG
TAGTTTGGCGGTATTAGGAAGATCCTAGAAGCAAGGCAGAGTTAGTTCTAACCTAAAGCCA
CAAATAAGACAGGTTGCCAAAGCCCGCCGGAAATTAAATCTTGCTCAGTTCGGTAACGGA
GTTTCCCTCCCGCGTACTTAATTCCCAATAAGAAACGCGCCCAAGTCCTATCAGGCAAAAT
TCAGCCCCTTCCCGTGTTAGAACGAGGGTAAAAATACAAGCCGATTGAACAAGGGTTGGG
GGCTTCAAATCGTCGTTTACCCCACTTTACAACGGAGATTAAGTAGTTCACCCTATAGTAC

GAAGCAGAACTATTTCGAGGGGCGTGCAATAATCGAATCTTCTGCGGTTGACTTAACACGC
TAGGGACGTGCCCTCGATTCAATCGAAGGTACTCCTACTCAGACTGCCTCACACCCAGCT
AGTCACTGAGCGATAAAATTGACCCGCCCTCTAGGGAAGCGAGTACGTCCCAAAGGGCTC
CGGACAGGGCTATATAGGAGAGTTTGATCTCGCCCCGACAACTGCAACCCTCAACTCCCT
TAGATAATATTGTTAGCCGAAGTTGCACGACCCGCCGTCCACGGACTGCTCTTAGGGTGT
GGCTCCTTAATCTGACAACGTGCAACCCCTATCGAAGTCGATTGTTTCTGCGAAAGGTGTT
GTCCTAATAGTCCCGAAATTTGGCCCTTGTAGGTGTGAAACCACTTAGCTTCGCGCCGTAG
TCCTAAAGGCCCACCTATTGACTTTGTTTCGGGTAGCACTAGGAATCTTAACAATTTGAATT
TGGACGTGGAACGCGTACACCTTAATCTCCGAATAATTCTAGGGATTTGGAAGTCCTCTAC
GTTGACACACCTACACTGCTCGAAGTAAATATACGAATAACGCGGGCCTCGCGGAGCCGT
TCCGAATCGTCACGTGTTCGTTTACTGTTAATTGGTGGCAAATAAGCAATATCGTAGTCCG
TCAGGCCCAGCCCTGTTATCCACGGCGTTATTTGTCAAATTGCGTAGAACTGGATTGACTG
CCTGACAATACCTAATTATCGGTACGAAGTCCCCGAATCTGTCGGGCTATTTCACTAATAC
TTTCCAAACGCCCCGTATCCAAGAAGAACGAATTTATCCACGCTCCCGTCTTTGGGACGAA
TACCGCTACAAGTGGACAGAGGATCGGTACGGGCCTCTAATAAATCCAACACTCTACGCC
CTCTTCAAGAGCTAGAAGAACAGGGTGCAGTTGGAAAGGAATTATTTCGTAAGGCGAGC
CAATACCGTAATTAATTCGGAAGAGTTAACACGATTGGAAGTAGGAATAGTTTCTAACCAC
GGTTACTAATCCTAATAACGGAACGCTGTCTGATAGATTAGTGTCAGCGCTCGGTACCAAA
GAAAAATAAAAAGACGCTGAAAAGCGTCTTTTTATTTTTCGGTCCAGTGTAACTCAGGCAAA
AGCACGTAATATTCGTACTTTCTTCCTCCGTAAGCGTCACCCACATTCCTTAAAGAGTGCAT
GTGCATATTTTGTTATCAATAAAAAAGGCCGCGATTTGCGGCCTTATTGTTCGTCTTGCCG
GATTACGCCCCGCCCTGCCACTCATCGCAGTATTGTTGTAATTCATTAAGCATTCTGCCGA
CATGGAAGCCATCACAAACGGCATGATGAACTTGGATCGCCAGTGGCATTAACACCTTGT
CGCCTTGCGTATAATATTTCCCATAGTGAAAACGGGGGCGAAGAAGTTGTCCATATTTGC
TACGTTTAAATCAAAACTGGTGAAACTCACCCACGGATTGGCACTGACGAAAAACATATTTT
CGATAAACCCTTTAGGGAAATATGCTAAGTTTTCACCGTAACACGCCACATCTTGACTATAT
ATGTGTAGAAACTGCCGGAAATCGTCGTGGTATTCTGACCAGAGCGATGAAAACGTTTCAG
TTTGCTCATGGAAAACGGTGTAACAAGGGTGAACACTATCCCATATCACCAGCTCACCGTC
TTTCATTGCCATACGAAACTCCGGATGTGCATTCATCAGGCGGGCAAGAATGTGAATAAAG
GCCGGATAAAACTTGTGCTTATTTTTCTTTACGGTTTTTAAAAAGGCCGTAATATCCAGCTG
AACGGTTTGGTTATAGGTGCACTGAGCAACTGACTGGAATGCCTCAAAATGTTCTTTACGA
TGCCATTGACTTATATCAACTGTAGTATATCCAGTGATTTTTTTCTCCATTTTAGCTTCCTTA
GCTTGCGAAATCTCGATAACTCAAAAAATAGTAGTGATCTTATTTCATTATGGTGAAAGTTG
TCTTACGTGCAACATTTTCGCAAAAAGTTGGCGCTTTATCAAC. (SEQ ID NO: 32)

### Example 2: In vitro ESBL elimination assay

Sequence specific elimination of clinical strains of ESBL E. coli using packaged phagemids. Two clinical E. coli strains carrying different ESBL gene variants (CTX-M1 and CTX-M9) were transduced with packaged phagemids targeting either CTX-M1 or CTX-M and comprising a chloramphenicol resistance gene. Strains were then plated on LB agar supplemented with chloramphenicol at different Multiplicity Of Infection (MOI). Results of a spot assay and associated cell counts are shown in **Figure 3****.**

### Example 3: In vivo ESBL elimination assay

This example shows the efficient elimination of ESBL *E*. *coli* strains using packaged phagemids of the invention.

### Materials and methods

Groups of streptomycin-treated mice were orally administered with one of four ESBL *E. coli* strains (further made resistant to streptomycin for mice colonization purpose), namely strains s17367 (herein referred to Target strain #1), s20545 (herein referred to Target strain #2), s20546 (herein referred to Target strain #3) and s20547 (herein referred to Target strain #4), all carrying the CTX-M1 gene, to establish a durable intestinal colonization with these bacterial strains for several days.

Packaged phagemids comprising a payload encoding a Fn Cpf1 nuclease and a ctx1 guide (as disclosed in Example 1) were produced as disclosed in Example 1, within a bacterial delivery vehicle comprising an 1A2 gpJ protein, as disclosed in Example 1, and a STF29 protein of sequence SEQ ID NO: 4 (encoded by nucleic acid of sequence SEQ ID NO: 5, and produced with its chaperone protein of sequence SEQ ID NO: 6, encoded by a nucleic acid of sequence SEQ ID NO: 7).

Colonized mice were then given orally 100 µl of packaged phagemids (approximately 2 x 10¹² particles) along with 100 µl of a buffer (sucrose and bicarbonate in water) in order to temporarily neutralize the gastric pH.

The bacterial colonization levels were measured non-invasively by plating dilutions of stool recovered from each animal individually onto agar plates. These levels were compared before treatment was initiated (termed 'T0') and 8 hours after the treatment (termed 'T8h') for each animal, and the median for each group of mice was graphed.

### Results

As shown on **Figure 4****,** the packaged phagemids caused a statistically significant reduction in bacterial burden recovered from the stool 8 hours after oral administration for all four tested ESBL *E. coli* strains. The average reduction in bacterial colonization was between 0.5 and 1.0 log₁₀.

### Example 4: in vitro Sequence-specific killing of Klebsiella pneumoniae using Lambda-derived particles

The *Klebsiella pneumoniae* strain 166A1, belonging to MLST type 45, was selected as a candidate strain for *in vitro* sequence-specific nuclease-mediated killing assays. The strain 166A1 contains a *bla-oxa-48* gene and the nuclease-containing payload was programmed to target a region within this gene (SEQ ID NO: 9). Two Lambda-derived particles engineered to contain a modified gpJ (A8, SEQ ID NO: 10) and either chimeric STF118 (SEQ ID NO: 11, SEQ ID NO: 12, SEQ ID NO: 13 and SEQ ID NO: 14) or chimeric STF SIEA11 (SEQ ID NO: 15, SEQ ID NO: 16, SEQ ID NO: 17 and SEQ ID NO: 18) were packaged with a payload containing a nuclease programmed to target the *bla-oxa-48* gene (SEQ ID NO: 19) following the production method described in Example 1.

The *K. pneumoniae* 166A1 strain was grown overnight in LB supplemented with 5 mM CaCl₂ at 37ºC with shaking. Next day, the culture was diluted 1:100 in fresh LB supplemented with 5 mM CaCl₂ and grown for 2 hours at 37ºC with shaking. The culture OD600 was then measured and adjusted to OD 0.025 using fresh LB supplemented with 5 mM CaCl₂.

The phagemids were serially diluted 3-fold and 10 µL of each dilution added to a 96-well plate horizontally, to which 90 µL of the cell culture adjusted to OD600 0.025 were then added. The plate was then incubated for 30 min at 37ºC. After that, each transduction well was serially diluted 10 fold vertically, 10 µL of each dilution spotted on an LB-agar plate and incubated at 37ºC for 18 h. Next day, the cell count of each dilution was determined by counting the CFUs and plotted against the MOI **(****Figure 5****).**

As can be seen from **Figure 5****,** Lambda particles, originally derived from an E. coli-specific phage, can be engineered to efficiently target *Klebsiella pneumoniae* and mediate 2-logs of sequence-specific killing at MOIs <50.

### References

Bode, L., Kluytmans, J., Wertheim, H., Bogaers, D., Vandenbroucke-Grauls, C., Roosendaal, R., Troelstra, A., Box, A., Voss, A., Tweel, I., Belkum, A., Verbrugh, H., Vos, M. (2010). Preventing Surgical-Site Infections in Nasal Carriers of Staphylococcus aureus New England Journal of Medicine 362(1), 9-17.
Jonge, P., Nobrega, F., Brouns, S., Dutilh, B. (2018). Molecular and Evolutionary Determinants of Bacteriophage Host Range Trends in Microbiology.
Gargiullo, L., Chierico, F., D'Argenio, P., Putignani, L. (2019). Gut Microbiota Modulation for Multidrug-Resistant Organism Decolonization: Present and Future Perspectives Frontiers in Microbiology 10(), 1704.
Kokai-Kun, J., Walsh, S., Chanturiya, T., Mond, J. (2003). Lysostaphin Cream Eradicates Staphylococcus aureus Nasal Colonization in a Cotton Rat Model Antimicrobial Agents and Chemotherapy 47(5), 1589-1597.
Lambelet P, Tascini C, Fortunato S, Stefanelli A, Simonetti F, Vettori C, Leonildi A, Menichetti F. (2017) Oral gentamicin therapy for Carbapenem-resistant Klebsiella pneumoniae gut colonization in hematologic patients: a single center experience. New Microbiol. 40(3):161-164.
Manley, K., Fraenkel, M., Mayall, B., Power, D. (2007). Probiotic treatment of vancomycinresistant enterococci: a randomised controlled trial Medical Journal of Australia 186(9), 454-457.
Millan, A. (2018). Evolution of Plasmid-Mediated Antibiotic Resistance in the Clinical Context Trends in Microbiology.
Pastagia, M., Euler, C., Chahales, P., Fuentes-Duculan, J., Krueger, J., Fischetti, V. (2011). A Novel Chimeric Lysin Shows Superiority to Mupirocin for Skin Decolonization of Methicillin-Resistant and -Sensitive Staphylococcus aureus Strains Antimicrobial Agents and Chemotherapy 55(2), 738-744.
Perl, T., Cullen, J., Wenzel, R., Zimmerman, M., Pfaller, M., Sheppard, D., Twombley, J., French, P., Herwaldt, L., Team, M. (2002). Intranasal Mupirocin to Prevent Postoperative Staphylococcus aureus Infections New England Journal of Medicine 346(24), 1871-1877.
Rieg, S., Küpper, F., With, K., Serr, A., Bohnert, J., Kern, W. (2015). Intestinal decolonization of Enterobacteriaceae producing extended-spectrum β-lactamases (ESBL): a retrospective observational study in patients at risk for infection and a brief review of the literature BMC Infectious Diseases 15(1), 475.
Sakr, A., Brégeon, F., Rolain, J., Blin, O. (2019). Staphylococcus aureus nasal decolonization strategies: a review Expert Review of Anti-infective Therapy
Saha, S., Tariq, R., Tosh, P., Pardi, D., Khanna, S. (2019). Fecal Microbiota Transplantation for Eradicating Carriage of Multidrug-Resistant Organisms: A Systematic Review Clinical Microbiology and Infection 25(8), 958-963.
Salomão, M., Heluany-Filho, M., Menegueti, M., Kraker, M., Martinez, R., Bellissimo-Rodrigues, F. (2016). A randomized clinical trial on the effectiveness of a symbiotic product to decolonize patients harboring multidrug-resistant Gram-negative bacilli. Revista da Sociedade Brasileira de Medicina Tropical 49(5), 559-566.
Satlin MJ, Chavda KD, Baker TM, Chen L, Shashkina E, Soave R, Small CB, Jacobs SE, Shore TB, van Besien K, Westblade LF, Schuetz AN, Fowler VG Jr, Jenkins SG, Walsh TJ, Kreiswirth BN. (2018) Colonization With Levofloxacin-resistant Extended-spectrum β-Lactamase-producing Enterobacteriaceae and Risk of Bacteremia in Hematopoietic Stem Cell Transplant Recipients. Clin Infect Dis. 67(11):1720-1728.
Szachta, P., Ignyś, I., Cichy, W. (2011). An evaluation of the ability of the probiotic strain Lactobacillus rhamnosus GG to eliminate the gastrointestinal carrier state of vancomycinresistant enterococci in colonized children. Journal of clinical gastroenterology 45(10), 872-7.
Tannock, G., Tiong, I., Priest, P., Munro, K., Taylor, C., Richardson, A., Schultz, M. (2010). Testing probiotic strain Escherichia coli Nissle 1917 (Mutaflor) for its ability to reduce carriage of multidrug-resistant E. coli by elderly residents in long-term care facilities. Journal of medical microbiology 60(Pt 3), 366-70.
Walker, E., Vasquez, J., Dula, R., & Bullock, H. (2003). Mupirocin-Resistant, Methicillin-Resistant Staphylococcus aureus: Does Mupirocin Remain Effective? Infection Control & Hospital Epidemiology, Volume 24, Issue 5, May 2003 , pp. 342-346.

## Claims

1. A vector for use in a method of selectively removing antibiotic resistance from an antibiotic resistant bacterial strain(s) in a healthy subject carrying antibiotic resistant bacterial strain(s) or in a patient carrying antibiotic resistant bacterial strain(s),
wherein said vector encoding a DNA modifying enzyme inactivating the antibiotic resistance gene(s) responsible for antibiotic-resistance of bacterial strain(s),
thereby selectively removing antibiotic resistance from an antibiotic resistant bacterial strain(s) in the microbiota of said subject or patient.

2. Vector for use in a method of preventing antibiotic resistance related infection in a healthy subject carrying antibiotic resistant bacterial strain(s) or in a patient carrying antibiotic resistant bacterial strain(s),
wherein said vector encodes a DNA modifying enzyme inactivating the antibiotic resistance gene(s) responsible for antibiotic-resistance of bacterial strain(s),
thereby selectively removing antibiotic resistance from an antibiotic resistant bacterial strain(s) in the microbiota of said subject or patient and preventing antibiotic resistance related infection in the subject or patient.

3. Recombinant phage for use in a method of selectively removing antibiotic resistance from an antibiotic resistant bacterial strain(s) in a healthy subject carrying antibiotic resistant bacterial strain(s) or in a patient carrying antibiotic resistant bacterial strain(s),
wherein said recombinant phage comprises a nucleic acid encoding a DNA modifying enzyme inactivating the antibiotic resistance gene(s) responsible for antibiotic-resistance of bacterial strain(s),
thereby selectively removing antibiotic resistance from an antibiotic resistant bacterial strain(s) in the microbiota of said subject or patient.

4. Recombinant phage for use in a method of preventing antibiotic resistance related infection in a healthy subject carrying antibiotic resistant bacterial strain(s) or in a patient carrying antibiotic resistant bacterial strain(s),
wherein said recombinant phage comprises a nucleic acid encoding a DNA modifying enzyme inactivating the antibiotic resistance gene(s) responsible for antibiotic-resistance of bacterial strain(s),
thereby selectively removing antibiotic resistance from an antibiotic resistant bacterial strain(s) in the microbiota of said subject or patient and preventing antibiotic resistance related infection in the subject or patient.

5. Vector for use according to claim 1 or 2 or recombinant phage for use according to claim 3 or 4, wherein the DNA modifying enzyme is a nuclease, such as a CRISPR associated nuclease, or a base editor.

6. Vector or recombinant phage for use according to any one of claims 1 to 5, wherein the vector or recombinant phage further comprises a conditional origin of replication which is inactive in the targeted antibiotic resistant bacterial strain(s) but is active in a donor bacterial cell.

7. Vector or recombinant phage for use according to any one of claims 1 to 6, wherein antibiotic resistance bacteria are removed from the gut of the healthy subject or patient.

8. Vector or recombinant phage for use according to any one of claims 1 to 7, wherein the selective decolonization occurs before the subject or patient undergoes surgery and/or undergoes cancer treatment and/or immunosuppressive treatment.

9. Vector or recombinant phage for use according to claim 8, wherein the surgery is a solid organ transplant surgery of the kidney or liver.

10. Vector or recombinant phage for use according to any one of claims 1 to 9, wherein said microbiota is a gut microbiota.

11. Vector or reombinant phage for use according to any one of claims 1 to 10, wherein said antibiotic is methicillin, vancomycin, or a carbapenem class or beta-lactam class antibiotic.

12. Vector or recombinant phage for use according to any one of claims 1 to 11, wherein the method further comprises obtaining a microbiota sample from the subject or patient and confirming the presence of said antibiotic resistant bacterial strain(s) in the sample.

13. Vector or recombinant phage for use according to any one of claims 1 to 12, wherein at least two distinct vectors or recombinant phages are used, preferably four distinct vectors or recombinant phages, each vector or recombinant phage targeting a different bacteria, in particular a different *E. coli* bacteria, and/or encoding a DNA modifying enzyme targeting a different nucleic acid sequence inside the targeted bacteria.

14. Vector or recombinant phage for use according to claim 13, wherein said patient suffers from a hematological malignancy such as hematological cancer, and/or wherein said patient undergoes cancer treatment and/or immunosuppressive treatment.

15. Composition or combination comprising at least 4 recombinant phages as defined in any one of claims 3 to 13 for use in a method of preventing antibiotic resistant *Escherichia coli* related infection in a patient carrying antibiotic resistant *Escherichia coli* strain(s),
wherein said recombinant phage comprises a nucleic acid encoding a DNA modifying enzyme inactivating the antibiotic resistance gene(s) responsible for antibiotic-resistance of *E*. *coli* strain(s),
thereby selectively removing antibiotic resistance from antibiotic resistant *E. coli* strain(s) in the microbiota of said patient and preventing antibiotic resistant *E. coli* related infection in the patient,
wherein said patient suffers from a hematological malignancy such as hematological cancer, and optionally undergoes cancer treatment and/or immunosuppressive treatment.


SEQUENCE LISTING

<110>  ELIGO BIOSCIENCE

<120>  Specific decolonization of antibiotic resistant bacteria for
       prophylactic purposes

<130>  EB2019-13

<150>  US63/038,386
<151>  2020-06-12

<150>  US63/148,479
<151>  2021-02-11

<150>  US63/048,034
<151>  2020-07-03

<150>  US63/132,190
<151>  2020-12-30

<150>  US63/137,989
<151>  2021-01-15

<160>  32

<170>  PatentIn version 3.5

<210>  1
<211>  282
<212>  DNA
<213>  Artificial Sequence

<220>
<223>  Primase ori deltaGAAABCC

<400>  1
tttgttgcaa tggctgtcta ccctgtctac ctgagtaaag aaaaatacat ttaattcagt      60

acattaactt gggtagacag ccttttttta ctgtctacct actatctacc ctctctacct      120

gattttacct gaatcagaca gggaggtaga tacggggtag atagtggata aaagcactct      180

accccactga aagcagcgcc attactggca tggtggccag taaggtagat aaggtagaca      240

aggggaggca caactcaaaa ctttttaaac gagggggtaa aa                        282


<210>  2
<211>  282
<212>  DNA
<213>  Artificial Sequence

<220>
<223>   Primase ori devoid of restriction sites

<400>  2
tttgttgcaa tggctgtcta ccctgtctac ctgagtaaag aaaaatacat ttaattcagt      60

atattaactt gggtagacag ccttttttta ctgtctacct tctgtctacc ctctctacct      120

gattttacct gaatcagaca gggaggtaga cacggggtag acagtggata aaagcactct      180

accccactga aagcagtgcc attactggca tggttgccag taaggttgat aaggtagaca 240

aggggaggga caactcaaaa ctttttaaac gagggggtaa aa 282

<210> 3
<211> 282
<212> DNA
<213> Artificial Sequence

<220>
<223> primase ori from the PICI of the Escherichia coli strain CFT073

<400> 3
tttgttgcaa tggctgtcta ccctgtctac ctgagtaaag aaaaatacat ttaattcagt 60

acattaactt gggtagacag ccttttttta ctgtctacct actatctacc ctctctacct 120

gattttacct gaatcagaca gggaggtaga tacggggtag atagtggata aaagcactct 180

accccactga aagccgcgcc attactggca tggtggccag taaggtagat aaggtagaca 240

aggggaggca caactcaaaa ctttttaaac gagggggtaa aa 282

<210> 4
<211> 687
<212> PRT
<213> Artificial Sequence

<220>
<223> STF29

<400> 4
   Met Ala Val Lys Ile Ser Gly Val Leu Lys Asp Gly Thr Gly Lys Pro
   1 5 10 15
   Val Gln Asn Cys Thr Ile Gln Leu Lys Ala Arg Arg Asn Ser Thr Thr
   20 25 30
   Val Val Val Asn Thr Val Gly Ser Glu Asn Pro Asp Glu Ala Gly Arg
   35 40 45
   Tyr Ser Met Asp Val Glu Tyr Gly Gln Tyr Ser Val Ile Leu Gln Val
   50 55 60
   Asp Gly Phe Pro Pro Ser His Ala Gly Thr Ile Thr Val Tyr Glu Asp
   65 70 75 80
   Ser Gln Pro Gly Thr Leu Asn Asp Phe Leu Cys Ala Met Thr Glu Asp
   85 90 95
   Asp Ala Arg Pro Glu Val Leu Arg Arg Leu Glu Leu Met Val Glu Glu
   100 105 110
   Val Ala Arg Asn Ala Ser Val Val Ala Gln Ser Thr Ala Asp Ala Lys
           115                 120                 125
   Lys Ser Glu Thr Ala Ala Ala Ser Ser Lys Asn Ala Ala Lys Thr Ser
       130                 135                 140
   Glu Thr Asn Ala Ala Asn Ser Ala Gln Ala Ala Ala Ala Ser Gln Thr
   145                 150                 155                 160
   Ala Ser Ala Asn Ser Ala Thr Ala Ala Lys Lys Ser Glu Thr Ser Ala
                   165                 170                 175
   Lys Asn Ser Glu Thr Ala Thr Lys Ala Ser Glu Lys Asn Ala Lys Ser
               180                 185                 190
   Ser Gln Thr Ala Ala Lys Thr Ser Glu Thr Asn Ala Lys Asp Ser Glu
           195                 200                 205
   Ala Asn Ala Lys Val Ser Glu Thr Ala Ala Ala Asn Ser Ala Lys Ala
       210                 215                 220
   Ser Ala Ala Ser Gln Thr Ala Ala Lys Ala Ser Glu Asp Ala Ala Arg
   225                 230                 235                 240
   Glu Tyr Ala Asn Gln Thr Ala Glu Pro Tyr Arg Tyr Val Leu Gln Pro
                   245                 250                 255
   Leu Pro Asp Val Trp Ile Pro Phe Asn Asp Ser Leu Asp Met Ile Thr
               260                 265                 270
   Gly Tyr Ser Pro Gly Tyr Lys Lys Val Lys Ile Gly Asp Asn Val Val
           275                 280                 285
   Gln Val Ala Ser Asp Lys Gln Val Asn Phe Ser Arg Ala Ser Thr Ala
       290                 295                 300
   Thr Tyr Ile Asn Lys Ser Gly Glu Leu Lys Thr Ala Glu Ile Asn Glu
   305                 310                 315                 320
   Pro Arg Phe Glu Cys Asp Gly Leu Leu Ile Glu Gly Gln Arg Thr Asn
                   325                 330                 335
   Phe Phe Gln Asn Ser Thr Asp Pro Ser Lys Trp Asn Lys Ser Thr Ser
               340                 345                 350
   Leu Asp Val Thr Glu Thr Gly Thr Asp Ser Phe Gly Phe Asn Tyr Gly
           355                 360                 365
   Arg Phe Val Val Gln Asp Ser Ile Val Gly Thr Ser Lys Ala His Thr
       370                 375                 380
   Ile Ile Gly Leu Tyr Ser Ser Thr Gly Gly Val Asp Thr Ser Gly Asp
   385                 390                 395                 400
   Glu Lys His Val Thr Ile Ser Cys Arg Val Lys Ser Glu Val Asp Asn
                   405                 410                 415
   Ile Ala Val Arg Ile Leu Phe Glu His Tyr Asp Gly Glu Val Arg Thr
               420                 425                 430
   Ser Ile Gly Ala Ala Asn Leu Asn Leu Thr Thr Arg Ile Ile Ser Lys
           435                 440                 445
   Thr Gly Gln Thr Ser Arg Val Thr Ala Arg Ser Val Lys Asp Asp Ala
       450                 455                 460
   Thr Gly Trp Ile Phe Phe Glu Ala Thr Leu Lys Ala Asp Thr Thr Glu
   465                 470                 475                 480
   Asn Thr Val Gly Gly Phe Val Gln Tyr Ser Pro Asp Thr Gly Gln Met
                   485                 490                 495
   Val Thr Ser Gly Asp Tyr Leu Asp Val Thr Thr Pro Gln Ile Glu Ala
               500                 505                 510
   Gly Thr Gly Ala Ser Ser Phe Ile Val Thr Gly Thr Ala Pro Ala Thr
           515                 520                 525
   Arg Ala Ser Asp Met Val Thr Val Pro Ile Lys Asn Asn Leu Tyr Asn
       530                 535                 540
   Leu Pro Phe Thr Val Leu Cys Glu Val His Lys Asn Trp Tyr Lys Thr
   545                 550                 555                 560
   Pro Asn Val Ala Pro Arg Val Phe Asp Thr Gly Gly His Gln Thr Gly
                   565                 570                 575
   Ala Gly Ile Val Met Gly Phe Gly Ser Ser Gly Gly Tyr Asp Gly Phe
               580                 585                 590
   Pro Tyr Cys Asp Ile Gly Gly Ser Asp Arg Arg Ile Asn Glu Asn Ala
           595                 600                 605
   Gly Leu Glu Lys Met Leu Ile Gly Met Arg Val Lys Ser Glu Arg Ser
       610                 615                 620
   Thr Cys Val Val Ser Asn Gly Lys Leu Ser Ser Glu Thr Lys Thr Lys
   625                 630                 635                 640
   Trp Glu Tyr Ile Arg Ser Thr Ala Thr Ile Arg Ile Gly Gly Gln Thr
                   645                 650                 655
   Thr Ala Gly Leu Arg His Leu Phe Gly His Val Arg Asn Phe Arg Leu
               660                 665                 670
   Trp His Lys Glu Leu Thr Asp Ala Gln Leu Gly Glu Val Val Glu
           675                 680                 685

<210>  5
<211>  2061
<212>  DNA
<213>  Artificial Sequence

<220>
<223>  STF29

<400>  5
atggcagtaa agatttcagg agtcctgaaa gacggcacag gaaaaccggt acagaactgc      60

accattcagc tgaaagccag acgtaacagc accacggtgg tggtgaacac ggtgggctca     120

gagaatccgg atgaagccgg gcgttacagc atggatgtgg agtacggtca gtacagtgtc     180

atcctgcagg ttgacggttt tccaccatcg cacgccggga ccatcaccgt gtatgaagat     240

tcacaaccgg ggacgctgaa tgattttctc tgtgccatga cggaggatga tgcccggccg     300

gaggtgctgc gtcgtcttga actgatggtg gaagaggtgg cgcgtaacgc gtccgtggtg     360

gcacagagta cggcagacgc gaagaaatca gaaacggcag cagcctcatc gaagaatgcg     420

gcgaaaacct cagaaacgaa tgcagctaac agcgcacagg cggcagcggc ctcgcagact     480

gcatcggcaa actccgcgac agcagccaaa aaatcagaaa ccagcgcgaa aaatagcgag     540

acagccacaa aggccagcga aaaaaacgca aaatccagcc agacggcagc gaaaaccagt     600

gagacgaatg ccaaagacag tgaagccaac gcaaaggtga gcgaaacagc ggcggcgaac     660

tcggcgaaag catcggcagc aagccagacg gcagcaaaag caagtgaaga tgctgccaga     720

gaatacgcaa accagacagc agagccgtac agatatgttt tacagccgct gccggatgtg     780

tggataccct ttaatgattc gctggatatg attacgggct attctccggg ttataaaaaa     840

gtgaagattg gtgataatgt ggttcaggtt gccagtgata aacaggttaa tttcagtcgc     900

gcatcaacgg caacatatat caacaaatct ggcgaactga aaacggcgga aattaatgag     960

ccgcgatttg agtgtgatgg cctgcttatt gagggacaaa gaacgaactt cttccagaac    1020

agtacagacc cttcgaagtg gaataagtca acttcactgg acgttacaga aacaggcaca    1080

gatagtttcg ggtttaatta tggtcggttt gtcgtacagg attcgattgt tggtacaagt 1140

aaagcgcata ccattatcgg actgtattcg agtaccggag gggttgatac ttcaggggac 1200

gaaaagcatg taactatatc ctgtcgggta aaaagtgaag ttgataatat cgccgttcgt 1260

attttatttg aacattatga tggggaggta aggacatcaa taggagcagc aaacctgaac 1320

cttaccaccc gcataattag caagacaggt cagacaagcc gtgttacagc aaggtctgtt 1380

aaggatgatg caactggctg gatatttttt gaggctacat taaaagcaga tacaacagaa 1440

aatacggttg gtggttttgt ccagtattct ccggatacag ggcagatggt tacatcaggg 1500

gattatctcg atgtaaccac tccacagatt gaggctggta caggcgcatc atcttttatt 1560

gttacgggga cggcaccggc aacgcgggca agcgatatgg tgacagtccc aatcaagaat 1620

aacctttata atcttccttt tacggttctt tgtgaggtac ataagaactg gtataaaacg 1680

ccaaatgtag cgccgcgtgt tttgataccc ggcggtcatc aaaccggagc ggggatcgta 1740

atggggtttg gttcatcagg tgggtacgac ggttttccgt attgcgatat aggtggttca 1800

gaccgacgaa taaatgaaaa tgccgggctg gaaaaaatgc ttattggtat gcgggtaaag 1860

tccgaacggt ccacatgtgt agtcagtaac ggtaagttaa gcagcgaaac taaaaccaaa 1920

tgggaatata tccggagtac agcaaccatt cgcattggtg gacaaactac agcaggatta 1980

cgccatttat ttgggcatgt gaggaatttt cgtctctggc ataaagagct aacagatgcg 2040

cagcttgggg aggttgtgga g 2061

<210> 6
<211> 92
<212> PRT
<213> Artificial Sequence

<220>
<223> STF29 accessory protein

<400> 6

Met Arg Asp Phe Thr Leu Arg Phe Ser Asp Lys Ala Asp Phe Arg Ala
1               5                   10                  15

Phe Leu Arg Lys Leu Asn Trp Glu Glu Asp Glu Glu Leu Gln Asn Ala
            20                  25                  30

Val Leu Val Asp Glu Ile Gly Phe Thr Phe Arg Glu Thr Asp Val Ser
        35                  40                  45

Asp Asp Gly Glu Pro Glu Tyr Thr Arg Asn Glu Gly Tyr Phe Val Asn
    50                  55                  60

Ile Arg Leu Leu Asp Asp Gly Phe Glu Asp Ser Val Phe Arg Glu Trp
65                  70                  75                  80

Val Val Thr Pro Glu Arg Pro Leu Arg Glu Trp Phe
                85                  90

<210>  7
<211>  276
<212>  DNA
<213>  Artificial Sequence

<220>
<223>  STF29 accessory protein-encoding nucleic acid

<400>  7
gtgagagatt tcacgttgcg tttcagtgat aaagcagatt tcagggcatt tctcaggaaa      60

cttaactggg aagaggacga agagctgcag aatgccgttc tggttgatga gattggtttt     120

acgttcaggg agacagatgt ttctgatgac ggagaaccag aatacacgcg aaacgaaggg     180

tactttgtta atatccgtct tcttgacgat ggatttgagg attccgtgtt ccgtgagtgg     240

gtggttacac cagagcgccc gctcagggag tggttt                               276

<210>  8
<211>  13
<212>  DNA
<213>  Artificial Sequence

<220>
<223>  restriction site

<220>
<221>  misc_feature
<222>  (5)..(10)
<223>  n is a, c, g, or t

<400>  8
twcannnnnn tgg                                                         13

<210>  9
<211>  12299
<212>  DNA
<213>  Artificial Sequence

<220>
<223>  nuclease-containing payload targeting bla-oxa-48 gene

<400>  9
actgtcggaa tgacaaatgg ttccaattat tgaacaccct tcggggtgtt tttttgtttc      60

tggtttcccg aggccggcct gcgctagcgg agtgtatact ggcttactat gttggcactg     120

atgagggtgt aagtgaagtg cttcatgtgg caggagaaaa aaggctgcat cggtgcgtca     180

gcagaatatg tgatacagga tatattccgc ttcctcgctc actgactcgc tacgctcggt     240

cgttcgactg tggcgagcgg aaatggctta cgaacggggc ggagatttcc tggaagatgc     300

caggaagata cttaacaggg aagtgagagg gtcgcggcaa agccgttttt ccataggctc 360

cgcccccctg acaagcatca cgaaatctga cgctcaaatc agtggtggcg aaacctgaca 420

ggactataaa gataccaggc gtttccccct ggcggctccc tcgtgcgctc tcctgttcct 480

gcctttcggt ttgccggtgt cattcctctg ttacggccga gtttgtctca ttccacgcct 540

gacactcagt tccgggtagg cagttcgctc caagctggac tgtatgcacg aaccccccgt 600

tcagtccgac cgctgcgcct tatccggtaa ctatcgtctt gagtccaacc cggaaagaca 660

tgcaaaagca ccactggcag cagccactgg taattgattt agaggagtta gtcttgaagt 720

catgcgccgg ataaggctaa actgaaagga caagttttgg cgactgcgct cctccaagcc 780

agttacctcg gttcaaagag ttggtagctc agagaacctt cgaaaaaccg ccctgcaagg 840

cggttttttc gttttcagag caagagatta cgcgcagacc aaaacgatct caagaagatc 900

atcttattaa tcagataaaa tatttctaga tttcagtgca atttatctct tcaaatgtag 960

caccggcgcg ccgtgaccaa ttattgaagg ccgctaacgc ggcctttttt tgtttctggt 1020

atcccgaatg gagcgacttc tccccaaaaa gcctcgcttt cagcacctgt cgtttccttt 1080

cttttcagag ggtattttaa ataaaaacat taagttatga cgaagaagaa cggaaacgcc 1140

ttaaaccgga aaattttcat aaatagcgaa aacccgcgag gtcgccgccc cgtaacctgt 1200

cggatcaccg gaaaggaccc gtaaagtgat aatgattatc atctacatat cacaacgtgc 1260

gtaaagggta agtatgaagg tcgtgtactc catcgctacc aaattccaga aaacagacgc 1320

tttcgagcgt cttttttcgt tttggtcacg acgtacggtg gaagattcgt taccaattga 1380

cagctagctc agtcctaggt atatacatac atgcttgttt gtttgtaaac tactgttttc 1440

attaaagagg agaaaggaag ccatgtccat ctatcaggag tttgttaaca agtattccct 1500

gtctaaaacc ctgcgttttg aactgatccc gcagggcaaa actttggaaa acattaaagc 1560

gcgtggcctg attctggatg acgaaaaacg tgcaaaggat tacaagaaag ctaaacagat 1620

catcgacaaa tatcaccagt tctttatcga agaaattctg tcgtcggtgt gcatcagtga 1680

ggatctgtta cagaattatt ctgatgtata ctttaaactt aaaaagtccg atgacgataa 1740

tctgcaaaaa gatttcaagt cagccaaaga taccatcaag aaacagatct cagaatatat 1800

taaagatagc gaaaagttca aaaacctgtt taaccaaaac ctcattgatg ctaagaaagg 1860

ccaagaatct gacctgatct tatggctgaa acagagcaaa gataacggca ttgaactgtt 1920

caaagctaat agcgacatca ccgatattga tgaagcgctc gaaatcatca agtctttcaa 1980

aggctggacg acgtatttca aaggttttca tgaaaaccgt aagaatgtat attcgagcaa 2040

cgatattccg acctctatta tttatcgtat cgtggacgac aacctgccga agtttctgga 2100

aaacaaagcg aaatatgaat ctctgaaaga caaagcaccg gaagctatta actatgaaca 2160

gatcaagaaa gatctggcgg aagaactgac cttcgacatc gactataaaa cctccgaagt 2220

taaccagcgt gttttctcac tggacgaggt tttcgaaatc gctaatttca acaattacct 2280

gaatcaatct ggcatcacca aattcaacac cattattggt ggcaaatttg ttaacggcga 2340

aaacaccaag cgtaagggca tcaacgaata cattaacctg tatagccaac aaatcaacga 2400

caaaaccctg aaaaagtata aaatgtccgt tctgtttaaa cagattttat cggacaccga 2460

atctaaatcc ttcgtaattg ataaactgga agatgatagc gacgttgtca ccacgatgca 2520

gagcttttat gagcagattg cggcgttcaa aaccgtggaa gagaaatcta ttaaagaaac 2580

tctgtccctg ctctttgacg acctcaaagc gcagaaacta gatctgtcta agatttactt 2640

taaaaacgac aaatctctga ccgatctcag tcaacaagtt ttcgatgact atagcgtgat 2700

cggcacggca gttttggaat acatcaccca acaaatcgcg ccgaaaaatc tggacaaccc 2760

gtccaagaag gaacaggaac tgattgcaaa gaaaacagaa aaagctaaat acctgagctt 2820

agaaactatc aaactggcac ttgaggaatt taataaacat cgtgatattg ataaacagtg 2880

tcgttttgag gaaattctgg cgaactttgc ggcaatcccg atgatcttcg acgaaattgc 2940

tcaaaacaaa gacaatctgg cgcagatctc tatcaagtac cagaatcagg gtaagaaaga 3000

tctgcttcaa gcatctgcgg aggacgatgt gaaagcaatt aaagacttat tagatcagac 3060

gaataactta ttacacaagc tcaaaatctt ccacatcagc cagagcgagg acaaggcgaa 3120

cattctggat aaagatgaac acttctatct ggtgttcgaa gaatgttact tcgaactggc 3180

aaacatcgtc cctctctaca ataaaatccg caactacatc acgcagaagc cttactctga 3240

cgagaaattc aaactgaact tcgaaaacag cacgctggcg aacggctggg ataagaacaa 3300

agagccggac aacaccgcaa tcctgttcat caaagacgac aaatactatc tgggcgtaat 3360

gaacaagaag aacaacaaga tcttcgacga taaagcgatc aaagaaaaca agggtgaagg 3420

ctataagaaa atcgtgtaca agctcctgcc gggtgcgaat aaaatgttac cgaaagtgtt 3480

cttttccgcg aaaagcatca aattctacaa cccgtctgag gatattctgc gcatccgcaa 3540

tcatagcacg cacactaaaa acggtagccc gcagaaaggg tatgaaaaat tcgaatttaa 3600

tatagaggac tgccgtaagt tcatcgactt ctataaacag agcatttcca aacatccgga 3660

atggaaagac ttcggcttcc gtttctctga cactcagcgc tataatagca tcgacgagtt 3720

ctaccgcgaa gtggagaatc agggctataa actgaccttc gagaacatta gtgagtcgta 3780

catcgactcc gttgtgaatc agggtaaact gtacctgttt cagatctata ataaagactt 3840

tagcgcgtac agcaaaggcc gtccgaatct gcacaccctt tactggaaag cattatttga 3900

cgaacgtaac ctgcaagatg tggtgtataa actgaacggt gaggcggaac ttttctaccg 3960

taaacagagt atcccgaaga aaatcacgca tccggcaaaa gaagctattg ccaacaaaaa 4020

caaagacaac ccgaagaaag aatcagtatt cgaatatgac ctgatcaaag ataaacgttt 4080

caccgaagat aagttctttt tccactgtcc gattaccatc aacttcaaat ctagcggtgc 4140

gaacaagttc aacgatgaaa ttaacttatt actgaaagag aaagctaatg acgtacacat 4200

cttatctatt gatcgcggtg aacgtcattt agcatactat acactggtag atggtaaagg 4260

taatattatt aaacaggata ctttcaatat tatcggtaat gaccgtatga aaaccaacta 4320

tcacgataag ctggcggcga tcgaaaaaga tcgtgattct gcgcgtaaag attggaagaa 4380

aattaacaat atcaaagaaa tgaaagaagg ctatctgagc caagtggtgc acgagatcgc 4440

aaaactggtg attgaatata acgctatcgt ggttttcgaa gatctgaact ttggttttaa 4500

acgtggtcgc ttcaaagtag aaaaacaggt gtaccaaaaa ctggaaaaaa tgctgattga 4560

aaaactgaac tatctggttt ttaaagacaa cgaatttgac aaaacgggtg gcgtactccg 4620

tgcctatcag ctgaccgctc cgttcgaaac gttcaagaaa atgggtaaac aaacggggat 4680

tatctattat gtgccagctg gtttcacctc caagatttgt ccagttacgg gcttcgttaa 4740

ccagctgtac ccgaaatacg agagcgttag caaatctcaa gaatttttca gcaaattcga 4800

caagatctgc tataatctgg ataaaggcta tttcgagttc agcttcgatt acaaaaactt 4860

cggcgataaa gcggctaaag gtaagtggac tattgctagc tttggtagcc gtctgattaa 4920

ctttcgcaac tccgacaaaa accataattg ggacacgcgt gaagtgtatc cgaccaaaga 4980

actggaaaaa ttactgaaag actattccat cgaatatggt catggggagt gcattaaagc 5040

ggcgatttgc ggtgaatccg ataagaaatt tttcgccaaa ctgaccagcg tgcttaacac 5100

cattctgcaa atgcgtaatt ctaaaacggg tacggagctg gactacctga tttctccggt 5160

agccgacgtt aacggcaact tcttcgattc tcgtcaagca ccgaaaaata tgccacaaga 5220

cgcggatgcc aacggtgcat accatatcgg cttaaaaggc ttaatgttat taggccgtat 5280

caagaataat caggagggca agaaattaaa tctggttatc aaaaacgaag aatacttcga 5340

gttcgttcag aatcgtaaca attaatgtat gcttaagcag ctcggtacca aagacgaaca 5400

ataagacgct gaaaagcgtc ttttttcgtt ttggtcctgt tgcggcgcga tagtgtgaac 5460

atgctataga cttctggtgc tacccgactg acaattaatc atccggctcg tataatgcta 5520

gcaatttcta ctgttgtaga tggcgtggtt aaggatgaac accaatcgag acgaacaata 5580

aggcctccct aacggggggc cttttttatt gataacaaaa gtaacttcga gcttgtctac 5640

ctcctagcac cattattgca attaataaac aactaacgga caattctacc taacagtttt 5700

catatatgac gagcagttaa gtgatgagta aaggtgagga attatttact ggtgttgttc 5760

cgatcttagt tgaactggac ggcgatgtta acggtcataa attcagtgtt cgtggtgaag 5820

gtgaaggtga tgcaaccaac ggtaagctga ccctgaaatt catctgcact actggaaaat 5880

taccagtacc gtggcctact ctggtgacta ccctgaccta tggtgttcag tgtttttctc 5940

gttaccctga ccacatgaag caacatgatt tcttcaaatc tgcaatgccg gaaggttatg 6000

tacaggagcg caccatttct ttcaaagacg atggcacgta taaaacccgt gcagaggtta 6060

aatttgaagg tgacactctg gtgaatcgta ttgaactgaa aggcattgat ttcaaagagg 6120

acggcaatat tttaggccac aaactggaat ataacttcaa ctcccataac gtttacatca 6180

ccgcagacaa acagaagaac ggtatcaaag ctaacttcaa aattcgccat aacgttgaag 6240

atggtagcgt acagctggcg gatcattacc aacagaacac tccgattgga gatgctcctg 6300

ttttactgcc ggataaccac tacctgtcca cccagtctaa actgtcgaag gatccgaacg 6360

aaaagcgcga ccacatggtg ttattagagt tcgttaccgc tagtggtatc acgcacggta 6420

tggatgaact ctacaaataa gacgaacaat aaggggagcg ggaaaccgct cccctttttt 6480

attgataaca aaagtaaatt gcacgctgat agtctcccaa ttgcgaagga ccaaaacgaa 6540

aaaacaccct ttcgggtgtc ttttctggaa tttggtaccg agtactaggt atcgtgtaag 6600

tagcgaaggc ccgtacgcga gataaactgc taggcaaccg cgactctacg actggtgctc 6660

gatttaattt cgctgacgta aagaaattat cggcagtgcg tcaactgccg tatctttatc 6720

ttaattaggt agttggacaa gcccttgaaa gaaatagcaa gagcctgcct ctctattgaa 6780

gtcacggcga aagtcgggta gaaatcaaag aaagcagaaa ttaaatcgga gtaacactaa 6840

ggtgggataa ctccgtaact gactacgcct ttctctagac tttacttgac cagatacact 6900

gtctttgaca cgttgaagga ttagagcaat caaatccaag actggctaag cacgaagcaa 6960

ctcttgagtg ttaaaaagtt atctcctgta ttcgggaagc gggtactaga agattgcagg 7020

gactccgacg ttaagtaaat tacaaagtaa taagtatcgt tcaggatcac gttaccgcaa 7080

taagaagcga gaataatata atttccgaag tgcttacccc agtagtgact attcctataa 7140

cccttctgag tgtccggagg cggaaatttg ccacgaaaga gaaagtattt ccccgacaat 7200

aataaagggg cgctcctcag cttttccact tggttgggta agctaggcaa ctctgaaagg 7260

agtttcggcg aattgaagcc gacagctttg aattgtttta ggggcgttat tcgagggcaa 7320

tcggagctaa cttcaagact acttctttgt tgaatactaa atagtgcaaa ggtcgtgttt 7380

cctcaaggat actccgctaa caatatagga ttccaatcag attcagcact ggcggtacgg 7440

gtgttgcggt gaggcgttcg ggtttacggc tcgaagctag cacggtagga agcctgacaa 7500

tcaccaagca aaagggccgt cgaaggccca caagatacga aagctctcga agccttatcc 7560

ttgaccgatc cacctattta ggcagttacg cacaaaagct acccaataat ccgtgacagg 7620

cacaatatca cggaacaaaa ccgaaaactc tcgtacacgg ttaggttttc gctaggaaga 7680

ataaacctct atcttgatta taagaaggct ccccaagcac ccccaaaacc gaaatagcgg 7740

tttgcaataa gggacaagtt acgagtgtag acacgcagaa ttatccagcc tttagtcttt 7800

aggaaggcaa agctattgta cgcggtagcc gtcgtagcaa tttaccaact gtagaattat 7860

tggacacacg taggaagggc ttacagttga agtttaataa ggtcacacgc aaaaccgcta 7920

aggaataatc gcaccgttag cgaaagaata tttcagagcg gttagtaaag gttgagtaaa 7980

gtgagattcc aaagtgagcc tttataaaaa gtaaagagct ataataaaac cgtcgagcag 8040

aaaacaatcg cctgaaatct caagcacgtt gccctttcta acgtcgctaa ggtttcgtaa 8100

acccgtttga ttaggaagaa gaataagtaa cccgattagg tttgagatcg cgggttatcg 8160

gtttggatta aaagtggata ccagcggagt caacgccgac gcaaacgtac agtgatccaa 8220

tcctgttgca cggtcaagca caatcagctc gcaagatctt ggaatagtgt gcccaacagt 8280

ttagttgagg gccacgttcc gactacaagt tgcttcaaga ggggaatttg gatttggcaa 8340

tagccccccg tttctacctc aagaggcgac gagtattaac cgcgccagct gtcggcacaa 8400

gggccaaaga agattccaat ttcttattcc cgaataacct ccgaatccct gcgggaaaat 8460

caccgaccga atagcctaga agcaaggggg aacagatagg tataattagc ttaagagagt 8520

accagccgtg acaacagcgt agtaaccaca aacttacgct ggggcttctt tggcggattt 8580

ttacagatac taacaaggtg atttgaagta ccttagttga ggatttaaac gcgctatccg 8640

gtaatctcca aattgggaaa taccgttcaa agagggctag aattacttaa aagccttcac 8700

accgcctgcg ctatacgcgc ccactctccc gtttatccgt ccaagcggaa gcagggcgat 8760

cctccgctaa gatattctta cgtgtaacgt agctaagtat cccaaatagc tggcgtacgc 8820

gttgaacacc gcctagagga tcgtgactcg ccggacgagc gtgttattgg ggacttacgc 8880

cagcgtagac tacaacgcgc ccagattaac cctgcacgta ttgccttgaa taacgtacta 8940

atctctccgg ctctcgacaa tctatcgagc gactcgatta tcaacgggtg tcttgcagtt 9000

ctaatctctt gcccccgccc gtaatagcct ccaagagatt gaagatagta aagggcaaga 9060

gctgattcgg cgttgaagga tagcggactt tcggtcaacc acaattcccc actcgacaaa 9120

accagccgtg cgaataactc tgaaagtaca agcaacccaa gagggctgag cctaaactca 9180

gctaattcct aagtgagcta aagactcgaa gtgacagctc ttaataaata gagcgggaac 9240

gtcgaacggt cgtgaaagta atagtacaac gggtattaac ttactgagga tattgcttga 9300

agctgtaccg ttttattggg tgaacgaata agatccagca attcagccaa agaagctacc 9360

aatttttagt ttaagagtgt cacgtctgac ctcgcgggta gattgccgaa cgtagagctt 9420

acgagccagc ggaaacagta gccgcaggat aagtaagggg agtaagtgat cgaacgaatc 9480

agaagtgaca atatacttag gctggatctc gtcccgtgaa tcccaaccct caccaactac 9540

gagataagag gtaagccaaa aatcgacttg gtggcgacca acgactgttc cccccctgta 9600

actaatcgtt ccgtcaaaac ctgacttact tcaaggccaa ttccaagcgc aaacaatacc 9660

gtcctagttc ttcggttaag tttccgaagt aggagtgagc ctacctccgt ttgcgtcttg 9720

ttaccactga cccagctatt tactttgtat tgcctgcaat cgaatttctg aactctcaga 9780

tagtggggat aacgggaaag ttcctatatt tgcgaactaa cttagccgtc cacctcgaag 9840

ctacctactc acacccaccc cgcgcggggt aaataaggca ctaatcccag ctgagagctg 9900

gcgtagcact tagccacaag ttaattaaca gttgtctggt agtttggcgg tattaggaag 9960

atcctagaag caaggcagag ttagttctaa cctaaagcca caaataagac aggttgccaa 10020

agcccgccgg aaattaaatc ttgctcagtt cggtaacgga gtttccctcc cgcgtactta 10080

attcccaata agaaacgcgc ccaagtccta tcaggcaaaa ttcagcccct tcccgtgtta 10140

gaacgagggt aaaaatacaa gccgattgaa caagggttgg gggcttcaaa tcgtcgttta 10200

ccccacttta caacggagat taagtagttc accctatagt acgaagcaga actatttcga 10260

ggggcgtgca ataatcgaat cttctgcggt tgacttaaca cgctagggac gtgccctcga 10320

ttcaatcgaa ggtactccta ctcagactgc ctcacaccca gctagtcact gagcgataaa 10380

attgacccgc cctctaggga agcgagtacg tcccaaaggg ctccggacag ggctatatag 10440

gagagtttga tctcgccccg acaactgcaa ccctcaactc ccttagataa tattgttagc 10500

cgaagttgca cgacccgccg tccacggact gctcttaggg tgtggctcct taatctgaca 10560

acgtgcaacc cctatcgaag tcgattgttt ctgcgaaagg tgttgtccta atagtcccga 10620

aatttggccc ttgtaggtgt gaaaccactt agcttcgcgc cgtagtccta aaggcccacc 10680

tattgacttt gtttcgggta gcactaggaa tcttaacaat ttgaatttgg acgtggaacg 10740

cgtacacctt aatctccgaa taattctagg gatttggaag tcctctacgt tgacacacct 10800

acactgctcg aagtaaatat acgaataacg cgggcctcgc ggagccgttc cgaatcgtca 10860

cgtgttcgtt tactgttaat tggtggcaaa taagcaatat cgtagtccgt caggcccagc 10920

cctgttatcc acggcgttat ttgtcaaatt gcgtagaact ggattgactg cctgacaata 10980

cctaattatc ggtacgaagt ccccgaatct gtcgggctat ttcactaata ctttccaaac 11040

gccccgtatc caagaagaac gaatttatcc acgctcccgt ctttgggacg aataccgcta 11100

caagtggaca gaggatcggt acgggcctct aataaatcca acactctacg ccctcttcaa 11160

gagctagaag aacagggtgc agttggaaag ggaattattt cgtaaggcga gccaataccg 11220

taattaattc ggaagagtta acacgattgg aagtaggaat agtttctaac cacggttact 11280

aatcctaata acggaacgct gtctgataga ttagtgtcag cgctcggtac caaagaaaaa 11340

taaaaagacg ctgaaaagcg tctttttatt tttcggtcca gtgtaactca ggcaaaagca 11400

cgtaatattc gtactttctt cctccgtaag cgtcacccac attccttaaa gagtgcatgt 11460

gcatattttg ttatcaataa aaaaggccgc gatttgcggc cttattgttc gtcttgccgg 11520

attacgcccc gccctgccac tcatcgcagt attgttgtaa ttcattaagc attctgccga 11580

catggaagcc atcacaaacg gcatgatgaa cttggatcgc cagtggcatt aacaccttgt 11640

cgccttgcgt ataatatttt cccatagtga aaacgggggc gaagaagttg tccatatttg 11700

ctacgtttaa atcaaaactg gtgaaactca cccacggatt ggcactgacg aaaaacatat 11760

tttcgataaa ccctttaggg aaatatgcta agttttcacc gtaacacgcc acatcttgac 11820

tatatatgtg tagaaactgc cggaaatcgt cgtggtattc tgaccagagc gatgaaaacg 11880

tttcagtttg ctcatggaaa acggtgtaac aagggtgaac actatcccat atcaccagct 11940

caccgtcttt cattgccata cgaaactccg gatgtgcatt catcaggcgg gcaagaatgt 12000

gaataaaggc cggataaaac ttgtgcttat ttttctttac ggtttttaaa aaggccgtaa 12060

tatccagctg aacggtttgg ttataggtgc actgagcaac tgactggaat gcctcaaaat 12120

gttctttacg atgccattga cttatatcaa ctgtagtata tccagtgatt tttttctcca 12180

ttttagcttc cttagcttgc gaaatctcga taactcaaaa aatagtagtg atcttatttc 12240

attatggtga aagttgtctt acgtgcaaca ttttcgcaaa aagttggcgc tttatcaac 12299

<210> 10
<211> 1132
<212> PRT
<213> Artificial Sequence

<220>
<223> A8

<400> 10

Met Gly Lys Gly Ser Ser Lys Gly His Thr Pro Arg Glu Ala Lys Asp
1               5                   10                  15

Asn Leu Lys Ser Thr Gln Leu Leu Ser Val Ile Asp Ala Ile Ser Glu
            20                  25                  30

Gly Pro Ile Glu Gly Pro Val Asp Gly Leu Lys Ser Val Leu Leu Asn
        35                  40                  45

Ser Thr Pro Val Leu Asp Thr Glu Gly Asn Thr Asn Ile Ser Gly Val
    50                  55                  60

Thr Val Val Phe Arg Ala Gly Glu Gln Glu Gln Thr Pro Pro Glu Gly
65                  70                  75                  80

Phe Glu Ser Ser Gly Ser Glu Thr Val Leu Gly Thr Glu Val Lys Tyr
                85                  90                  95

Asp Thr Pro Ile Thr Arg Thr Ile Thr Ser Ala Asn Ile Asp Arg Leu
            100                 105                 110

Arg Phe Thr Phe Gly Val Gln Ala Leu Val Glu Thr Thr Ser Lys Gly
        115                 120                 125

Asp Arg Asn Pro Ser Glu Val Arg Leu Leu Val Gln Ile Gln Arg Asn
    130                 135                 140

Gly Gly Trp Val Thr Glu Lys Asp Ile Thr Ile Lys Gly Lys Thr Thr
145                 150                 155                 160

Ser Gln Tyr Leu Ala Ser Val Val Met Gly Asn Leu Pro Pro Arg Pro
                165                 170                 175

Phe Asn Ile Arg Met Arg Arg Met Thr Pro Asp Ser Thr Thr Asp Gln
            180                 185                 190

Leu Gln Asn Lys Thr Leu Trp Ser Ser Tyr Thr Glu Ile Ile Asp Val
        195                 200                 205

Lys Gln Cys Tyr Pro Asn Thr Ala Leu Val Gly Val Gln Val Asp Ser
    210                 215                 220

Glu Gln Phe Gly Ser Gln Gln Val Ser Arg Asn Tyr His Leu Arg Gly
225                 230                 235                 240

Arg Ile Leu Gln Val Pro Ser Asn Tyr Asn Pro Gln Thr Arg Gln Tyr
                245                 250                 255

Ser Gly Ile Trp Asp Gly Thr Phe Lys Pro Ala Tyr Ser Asn Asn Met
            260                 265                 270

Ala Trp Cys Leu Trp Asp Met Leu Thr His Pro Arg Tyr Gly Met Gly
        275                 280                 285

Lys Arg Leu Gly Ala Ala Asp Val Asp Lys Trp Ala Leu Tyr Val Ile
    290                 295                 300

Gly Gln Tyr Cys Asp Gln Ser Val Pro Asp Gly Phe Gly Gly Thr Glu
305                 310                 315                 320

Pro Arg Ile Thr Cys Asn Ala Tyr Leu Thr Thr Gln Arg Lys Ala Trp
                325                 330                 335

Asp Val Leu Ser Asp Phe Cys Ser Ala Met Arg Cys Met Pro Val Trp
            340                 345                 350

Asn Gly Gln Thr Leu Thr Phe Val Gln Asp Arg Pro Ser Asp Lys Thr
        355                 360                 365

Trp Thr Tyr Asn Arg Ser Asn Val Val Met Pro Asp Asp Gly Ala Pro

    370                 375                 380

Phe Arg Tyr Ser Phe Ser Ala Leu Lys Asp Arg His Asn Ala Val Glu
385                 390                 395                 400

Val Asn Trp Ile Asp Pro Asn Asn Gly Trp Glu Thr Ala Thr Glu Leu
                405                 410                 415

Val Glu Asp Thr Gln Ala Ile Ala Arg Tyr Gly Arg Asn Val Thr Lys
            420                 425                 430

Met Asp Ala Phe Gly Cys Thr Ser Arg Gly Gln Ala His Arg Ala Gly
        435                 440                 445

Leu Trp Leu Ile Lys Thr Glu Leu Leu Glu Thr Gln Thr Val Asp Phe
    450                 455                 460

Ser Val Gly Ala Glu Gly Leu Arg His Val Pro Gly Asp Val Ile Glu
465                 470                 475                 480

Ile Cys Asp Asp Asp Tyr Ala Gly Ile Ser Thr Gly Gly Arg Val Leu
                485                 490                 495

Ala Val Asn Ser Gln Thr Arg Thr Leu Thr Leu Asp Arg Glu Ile Thr
            500                 505                 510

Leu Pro Ser Ser Gly Thr Ala Leu Ile Ser Leu Val Asp Gly Ser Gly
        515                 520                 525

Asn Pro Val Ser Val Glu Val Gln Ser Val Thr Asp Gly Val Lys Val
    530                 535                 540

Lys Val Ser Arg Val Pro Asp Gly Val Ala Glu Tyr Ser Val Trp Glu
545                 550                 555                 560

Leu Lys Leu Pro Thr Leu Arg Gln Arg Leu Phe Arg Cys Val Ser Ile
                565                 570                 575

Arg Glu Asn Asp Asp Gly Thr Tyr Ala Ile Thr Ala Val Gln His Val
            580                 585                 590

Pro Glu Lys Glu Ala Ile Val Asp Asn Gly Ala His Phe Asp Gly Glu
        595                 600                 605

Gln Ser Gly Thr Val Asn Gly Val Thr Pro Pro Ala Val Gln His Leu
    610                 615                 620

Thr Ala Glu Val Thr Ala Asp Ser Gly Glu Tyr Gln Val Leu Ala Arg
625                 630                 635                 640

Trp Asp Thr Pro Lys Val Val Lys Gly Val Ser Phe Leu Leu Arg Leu
                645                 650                 655

Thr Val Thr Ala Asp Asp Gly Ser Glu Arg Leu Val Ser Thr Ala Arg
            660                 665                 670

Thr Thr Glu Thr Thr Tyr Arg Phe Thr Gln Leu Ala Leu Gly Asn Tyr
        675                 680                 685

Arg Leu Thr Val Arg Ala Val Asn Ala Trp Gly Gln Gln Gly Asp Pro
    690                 695                 700

Ala Ser Val Ser Phe Arg Ile Ala Ala Pro Ala Ala Pro Ser Arg Ile
705                 710                 715                 720

Glu Leu Thr Pro Gly Tyr Phe Gln Ile Thr Ala Thr Pro His Leu Ala
                725                 730                 735

Val Tyr Asp Pro Thr Val Gln Phe Glu Phe Trp Phe Ser Glu Lys Gln
            740                 745                 750

Ile Ala Asp Ile Arg Gln Val Glu Thr Ser Thr Arg Tyr Leu Gly Thr
        755                 760                 765

Ala Leu Tyr Trp Ile Ala Ala Ser Ile Asn Ile Lys Pro Gly His Asp
    770                 775                 780

Tyr Tyr Phe Tyr Ile Arg Ser Val Asn Thr Val Gly Lys Ser Ala Phe
785                 790                 795                 800

Val Glu Ala Val Gly Arg Ala Ser Asp Asp Ala Glu Gly Tyr Leu Asp
                805                 810                 815

Phe Phe Lys Gly Lys Ile Thr Glu Ser His Leu Gly Lys Glu Leu Leu
            820                 825                 830

Glu Lys Val Glu Leu Thr Glu Asp Asn Ala Ser Arg Leu Glu Glu Phe
        835                 840                 845

Ser Lys Glu Trp Lys Asp Ala Ser Asp Lys Trp Asn Ala Met Trp Ala
    850                 855                 860

Val Lys Ile Glu Gln Thr Lys Asp Gly Lys His Tyr Val Ala Gly Ile
865                 870                 875                 880

Gly Leu Ser Met Glu Asp Thr Glu Glu Gly Lys Leu Ser Gln Phe Leu
                885                 890                 895

Val Ala Ala Asn Arg Ile Ala Phe Ile Asp Pro Ala Asn Gly Asn Glu
            900                 905                 910

Thr Pro Met Phe Val Ala Gln Gly Asn Gln Ile Phe Met Asn Asp Val
        915                 920                 925

Phe Leu Lys Arg Leu Thr Ala Pro Thr Ile Thr Ser Gly Gly Asn Pro
    930                 935                 940

Pro Ala Phe Ser Leu Thr Pro Asp Gly Lys Leu Thr Ala Lys Asn Ala
945                 950                 955                 960

Asp Ile Ser Gly Ser Val Asn Ala Asn Ser Gly Thr Leu Asn Asn Val
                965                 970                 975

Thr Ile Asn Glu Asn Cys Gln Ile Lys Gly Lys Leu Ser Ala Asn Gln
            980                 985                 990

Ile Glu Gly Asp Ile Val Lys Thr  Val Ser Lys Ser Phe  Pro Arg Thr
        995                 1000                1005

Asn Ser  Tyr Ala Ser Gly Thr  Ile Thr Val Arg Ile  Ser Asp Asp
    1010                1015                1020

Gln Lys  Phe Asp Arg Gln Val  Met Ile Pro Pro Val  Leu Phe Arg
    1025                1030                1035

Gly Gly  Lys His Glu Asn Phe  Asn Ser Asn Asn Gln  Gln Ser Tyr
    1040                1045                1050

Trp Tyr  Ser Thr Cys Arg Leu  Arg Val Thr Arg Asn  Gly Gln Glu
    1055                1060                1065

Ile Phe  Asn Gln Ser Thr Thr  Asp Ala Gln Gly Val  Phe Ser Ser
    1070                1075                1080

Val Ile  Asp Met Pro Ala Gly  Gln Gly Thr Leu Thr  Leu Thr Phe
    1085                1090                1095

Thr Val  Ser Ser Ser Gly Ala  Asn Asn Trp Thr Pro  Thr Thr Ser
    1100                1105                1110

Ile Ser  Asp Leu Leu Val Val  Val Met Lys Lys Ser  Thr Ala Gly
    1115                1120                1125

Ile Ser Ile Ser
    1130


<210>  11
<211>  2475
<212>  DNA
<213>  Artificial Sequence

<220>
<223>  nucleic acid sequence encoding chimeric STF118

<400>  11
atggcagtaa agatttcagg agtcctgaaa gacggcacag gaaaaccggt acagaactgc      60

accattcagc tgaaagccag acgtaacagc accacggtgg tggtgaacac ggtgggctca     120

gagaatccgg atgaagccgg gcgttacagc atggatgtgg agtacggtca gtacagtgtc     180

atcctgcagg ttgacggttt tccaccatcg cacgccggga ccatcaccgt gtatgaagat     240

tcacaaccgg ggacgctgaa tgattttctc tgtgccatga cggaggatga tgcccggccg     300

gaggtgctgc gtcgtcttga actgatggtg gaagaggtgg cgcgtaacgc gtccgtggtg     360

gcacagagta cggcagacgc gaagaaatca gccggcgatg ccagtgcatc agctgctcag     420

gtcgcggccc ttgtgactga tgcaactgac tcagcacgcg ccgccagcac gtccgccgga     480

caggctgcat cgtcagctca ggaagcgtcc tccggcgcag aagcggcatc agcaaaggcc     540

actgaagcgg aaaaaagtgc cgcagccgca gagtcctcaa aaaacgcggc ggccaccagt     600

gccggtgcgg cgaaaacgtc agaaacgaat gctgcagcgt cacaacaatc agccgccacg     660

tctgcctcca ccgcggccac gaaagcgtca gaggccgcca cttcagcacg agatgcggtg     720

gcctcaaaag aggcagcaaa atcatcagaa acgaacgcat catcaagtgc cggtcgtgca     780

gcttcctcgg caacggcggc agaaaattct gccagggcgg caaaaacgtc cgagacgaat     840

gccaggtcat ctgaaacagc agcggaacgg agcgcctctg ccgcggcaga cgcaaaaaca     900

gcggcggcgg ggagtgcgtc aacggcatcc acgaaggcga cagaggctgc gggaagtgcg     960

gtatcagcat cgcagagcaa aagtgcggca gaagcggcgg caatacgtgc aaaaaattcg    1020

gcaaaacgtg cagaagatat agcttcagct gtcgcgcttg aggatgcgga cacaacgaga    1080

aaggggatag tgcagctcag cagtgcaacc aacagcacgt ctgaaacgct tgctgcaacg    1140

ccaaaggcgg ttaaggtggt aatggatgag actaatcgta aagcgccatt aaacagccct    1200

gcactgaccg gaacgccaac gacgccaact gcgcgacagg gaacgaataa tactcagatc    1260

gcaaacacgg ctttcgttat ggccgcgatt gccgcccttg tagactcgtc gcctgacgca    1320

ctgaatacgc tgaacgagct ggcagcggcg ctgggcaacg acccgaattt tgctaccact    1380

atgactaatg cgcttgcggg taagcaaccg aaagatgcta ccctggcggc gctggcgggg    1440

cttgctactg cggcagacag gtttccgtat tttacgggga atgatgttgc cagtctggca 1500

actctgacaa aagtcgggcg ggatattctt gcgaaatcga ccgtttccgc cgttatcgaa 1560

tatctcggtt tacaggaaac ggtaaaccga gccgggaacg ccgtgcaaaa aaatggcgat 1620

accttgtccg gtggacttac ttttgaaaac gactcaatcc ttgcctggat tcgaaatact 1680

gactgggcga agattggatt taaaaatgat gccgatggtg acactgattc atatatgtgg 1740

tttgaaacag gtgacaacgg caatgaatac ttcaaatgga gaagtcgcca gagcaccaca 1800

acaaaagacc tgatgaatct taaatgggat gctctgtatg ttcttgttaa agcccttttc 1860

agcagtgaag taaaaatatc tacagtcaat gcactgagga tatttaattc atcttttggt 1920

gctatttttc gccgttctga agaaaacctg tatatcatcc ctacacgaga aaatgagggt 1980

gaaaatggag atattgggcc attaaggcca ttcggcatca acttaagaac aggagttgtg 2040

tctgttggta atggtgccag gattgatggc gggctggcac ttggcacgaa taacgcgttg 2100

ggtgggaact ctattgttct tggtgataac gacaccggat ttaaacaaaa tggcgatggt 2160

aatctggatg tttatgctaa taacgtccat gttatgcgct ttgtttccgg aagcattcaa 2220

agtaataaga ccataaatat tacggggcgt gttaatccct cggattacgg taactttgat 2280

tcccgctatg tgagagatat cagacttggc acacgtgttg tccagaccat gcagaaaggg 2340

gtgatgtatg agaaagcagg gcacgtaatt accgggcttg gtattgtcgg tgaagtcgat 2400

ggtgatgacc ccgcagtatt caggccaata caaaaataca tcaatggcac atggtataac 2460

gtcgcacagg tgtaa 2475

<210> 12
<211> 824
<212> PRT
<213> Artificial Sequence

<220>
<223> chimeric STF118

<400> 12

Met Ala Val Lys Ile Ser Gly Val Leu Lys Asp Gly Thr Gly Lys Pro
1               5                   10                  15

Val Gln Asn Cys Thr Ile Gln Leu Lys Ala Arg Arg Asn Ser Thr Thr
            20                  25                  30

Val Val Val Asn Thr Val Gly Ser Glu Asn Pro Asp Glu Ala Gly Arg
        35                  40                  45

Tyr Ser Met Asp Val Glu Tyr Gly Gln Tyr Ser Val Ile Leu Gln Val
    50                  55                  60

Asp Gly Phe Pro Pro Ser His Ala Gly Thr Ile Thr Val Tyr Glu Asp
65                  70                  75                  80

Ser Gln Pro Gly Thr Leu Asn Asp Phe Leu Cys Ala Met Thr Glu Asp
                85                  90                  95

Asp Ala Arg Pro Glu Val Leu Arg Arg Leu Glu Leu Met Val Glu Glu
            100                 105                 110

Val Ala Arg Asn Ala Ser Val Val Ala Gln Ser Thr Ala Asp Ala Lys
        115                 120                 125

Lys Ser Ala Gly Asp Ala Ser Ala Ser Ala Ala Gln Val Ala Ala Leu
    130                 135                 140

Val Thr Asp Ala Thr Asp Ser Ala Arg Ala Ala Ser Thr Ser Ala Gly
145                 150                 155                 160

Gln Ala Ala Ser Ser Ala Gln Glu Ala Ser Ser Gly Ala Glu Ala Ala
                165                 170                 175

Ser Ala Lys Ala Thr Glu Ala Glu Lys Ser Ala Ala Ala Ala Glu Ser
            180                 185                 190

Ser Lys Asn Ala Ala Ala Thr Ser Ala Gly Ala Ala Lys Thr Ser Glu
        195                 200                 205

Thr Asn Ala Ala Ala Ser Gln Gln Ser Ala Ala Thr Ser Ala Ser Thr
    210                 215                 220

Ala Ala Thr Lys Ala Ser Glu Ala Ala Thr Ser Ala Arg Asp Ala Val
225                 230                 235                 240

Ala Ser Lys Glu Ala Ala Lys Ser Ser Glu Thr Asn Ala Ser Ser Ser
                245                 250                 255

Ala Gly Arg Ala Ala Ser Ser Ala Thr Ala Ala Glu Asn Ser Ala Arg
            260                 265                 270

Ala Ala Lys Thr Ser Glu Thr Asn Ala Arg Ser Ser Glu Thr Ala Ala
        275                 280                 285

Glu Arg Ser Ala Ser Ala Ala Ala Asp Ala Lys Thr Ala Ala Ala Gly
    290                 295                 300

Ser Ala Ser Thr Ala Ser Thr Lys Ala Thr Glu Ala Ala Gly Ser Ala
305                 310                 315                 320

Val Ser Ala Ser Gln Ser Lys Ser Ala Ala Glu Ala Ala Ala Ile Arg
                325                 330                 335

Ala Lys Asn Ser Ala Lys Arg Ala Glu Asp Ile Ala Ser Ala Val Ala
            340                 345                 350

Leu Glu Asp Ala Asp Thr Thr Arg Lys Gly Ile Val Gln Leu Ser Ser
        355                 360                 365

Ala Thr Asn Ser Thr Ser Glu Thr Leu Ala Ala Thr Pro Lys Ala Val
    370                 375                 380

Lys Val Val Met Asp Glu Thr Asn Arg Lys Ala Pro Leu Asn Ser Pro
385                 390                 395                 400

Ala Leu Thr Gly Thr Pro Thr Thr Pro Thr Ala Arg Gln Gly Thr Asn
                405                 410                 415

Asn Thr Gln Ile Ala Asn Thr Ala Phe Val Met Ala Ala Ile Ala Ala
            420                 425                 430

Leu Val Asp Ser Ser Pro Asp Ala Leu Asn Thr Leu Asn Glu Leu Ala
        435                 440                 445

Ala Ala Leu Gly Asn Asp Pro Asn Phe Ala Thr Thr Met Thr Asn Ala
    450                 455                 460

Leu Ala Gly Lys Gln Pro Lys Asp Ala Thr Leu Ala Ala Leu Ala Gly
465                 470                 475                 480

Leu Ala Thr Ala Ala Asp Arg Phe Pro Tyr Phe Thr Gly Asn Asp Val
                485                 490                 495

Ala Ser Leu Ala Thr Leu Thr Lys Val Gly Arg Asp Ile Leu Ala Lys
            500                 505                 510

Ser Thr Val Ser Ala Val Ile Glu Tyr Leu Gly Leu Gln Glu Thr Val
        515                 520                 525

Asn Arg Ala Gly Asn Ala Val Gln Lys Asn Gly Asp Thr Leu Ser Gly
    530                 535                 540

Gly Leu Thr Phe Glu Asn Asp Ser Ile Leu Ala Trp Ile Arg Asn Thr
545                 550                 555                 560

Asp Trp Ala Lys Ile Gly Phe Lys Asn Asp Ala Asp Gly Asp Thr Asp
                565                 570                 575

Ser Tyr Met Trp Phe Glu Thr Gly Asp Asn Gly Asn Glu Tyr Phe Lys
            580                 585                 590

Trp Arg Ser Arg Gln Ser Thr Thr Thr Lys Asp Leu Met Asn Leu Lys
        595                 600                 605

Trp Asp Ala Leu Tyr Val Leu Val Lys Ala Leu Phe Ser Ser Glu Val
    610                 615                 620

Lys Ile Ser Thr Val Asn Ala Leu Arg Ile Phe Asn Ser Ser Phe Gly
625                 630                 635                 640

Ala Ile Phe Arg Arg Ser Glu Glu Asn Leu Tyr Ile Ile Pro Thr Arg
                645                 650                 655

Glu Asn Glu Gly Glu Asn Gly Asp Ile Gly Pro Leu Arg Pro Phe Gly
            660                 665                 670

Ile Asn Leu Arg Thr Gly Val Val Ser Val Gly Asn Gly Ala Arg Ile
        675                 680                 685

Asp Gly Gly Leu Ala Leu Gly Thr Asn Asn Ala Leu Gly Gly Asn Ser
    690                 695                 700

Ile Val Leu Gly Asp Asn Asp Thr Gly Phe Lys Gln Asn Gly Asp Gly
705                 710                 715                 720

Asn Leu Asp Val Tyr Ala Asn Asn Val His Val Met Arg Phe Val Ser
                725                 730                 735

Gly Ser Ile Gln Ser Asn Lys Thr Ile Asn Ile Thr Gly Arg Val Asn
            740                 745                 750

Pro Ser Asp Tyr Gly Asn Phe Asp Ser Arg Tyr Val Arg Asp Ile Arg
        755                 760                 765

Leu Gly Thr Arg Val Val Gln Thr Met Gln Lys Gly Val Met Tyr Glu
    770                 775                 780

Lys Ala Gly His Val Ile Thr Gly Leu Gly Ile Val Gly Glu Val Asp
785                 790                 795                 800

Gly Asp Asp Pro Ala Val Phe Arg Pro Ile Gln Lys Tyr Ile Asn Gly
                805                 810                 815

Thr Trp Tyr Asn Val Ala Gln Val

            820

<210>  13
<211>  528
<212>  DNA
<213>  Artificial Sequence

<220>
<223>  nucleic acid sequence encoding accessory protein for STF118

<400>  13
atgcagcatt taaaaaatat tactgcgggt aatccaaaaa ctgttgccca atatcaactg      60

acaaaaaatt ttgatgttat ctggttatgg tccgaagagg gaaaaaactg gtatgaggaa      120

gtaagtaatt ttcaggaaga cacgataaag attgtttacg acgagaataa tataattgtc      180

ggcatcacca gagatgcttc aacgcttaac cctgaaggtt tcagcgttgt cgaggttcct      240

gatattacct ccaaccgacg tgctgatgac tcaggtaaat ggatgtttaa ggatggtgcc      300

gtgattaagc ggatttatac ggcagatgaa caggagcaac aggcagaatc acaaaaggca      360

gctttacttt ccgaagctga atccgtgatt ttgccgctgg aacgcgctgt caggctgaat      420

atggcgacgg atgaggaacg cagccgactg gaagcatggg aacgctacag cgttctggtc      480

agtcgtgtgg atcctgcaaa tcccgaatgg ccggaaatgc cgcaataa                  528

<210>  14
<211>  175
<212>  PRT
<213>  Artificial Sequence

<220>
<223>  accessory protein for STF118

<400>  14

Met Gln His Leu Lys Asn Ile Thr Ala Gly Asn Pro Lys Thr Val Ala
1               5                   10                  15

Gln Tyr Gln Leu Thr Lys Asn Phe Asp Val Ile Trp Leu Trp Ser Glu
            20                  25                  30

Glu Gly Lys Asn Trp Tyr Glu Glu Val Ser Asn Phe Gln Glu Asp Thr
        35                  40                  45

Ile Lys Ile Val Tyr Asp Glu Asn Asn Ile Ile Val Gly Ile Thr Arg
    50                  55                  60

Asp Ala Ser Thr Leu Asn Pro Glu Gly Phe Ser Val Val Glu Val Pro
65                  70                  75                  80

Asp Ile Thr Ser Asn Arg Arg Ala Asp Asp Ser Gly Lys Trp Met Phe
                85                  90                  95

Lys Asp Gly Ala Val Ile Lys Arg Ile Tyr Thr Ala Asp Glu Gln Glu
            100                 105                 110

Gln Gln Ala Glu Ser Gln Lys Ala Ala Leu Leu Ser Glu Ala Glu Ser
        115                 120                 125

Val Ile Leu Pro Leu Glu Arg Ala Val Arg Leu Asn Met Ala Thr Asp
    130                 135                 140

Glu Glu Arg Ser Arg Leu Glu Ala Trp Glu Arg Tyr Ser Val Leu Val
145                 150                 155                 160

Ser Arg Val Asp Pro Ala Asn Pro Glu Trp Pro Glu Met Pro Gln
                165                 170                 175

<210>  15
<211>  3291
<212>  DNA
<213>  Artificial Sequence

<220>
<223>  nucleic acid encoding chimeric STF SIEA11

<400>  15
atggcagtaa agatttcagg agtcctgaaa gacggcacag gaaaaccggt acagaactgc      60

accattcagc tgaaagccag acgtaacagc accacggtgg tggtgaacac ggtgggctca     120

gagaatccgg atgaagccgg gcgttacagc atggatgtgg agtacggtca gtacagtgtc     180

atcctgcagg ttgacggttt tccaccatcg cacgccggga ccatcaccgt gtatgaagat     240

tcacaaccgg ggacgctgaa tgattttctc tgtgccatga cggaggatga tgcccggccg     300

gaggtgctgc gtcgtcttga actgatggtg gaagaggtgg cgcgtaacgc gtccgtggtg     360

gcacagagta cggcagacgc gaagaaatca gccggcgatg ccagtgcatc agctgctcag     420

gtcgcggccc ttgtgactga tgcaactgac tcagcacgcg ccgccagcac gtccgccgga     480

caggctgcat cgtcagctca ggaagcgtcc tccggcgcag aagcggcatc agcaaaggcc     540

actgaagcgg aaaaaagtgc cgcagccgca gagtcctcaa aaaacgcggc ggccaccagt     600

gccggtgcgg cgaaaacgtc agaaacgaat gctgcagcgt cacaacaatc agccgccacg     660

tctgcctcca ccgcggccac gaaagcgtca gaggccgcca cttcagcacg agatgcggtg     720

gcctcaaaag aggcagcaaa atcatcagaa acgaacgcat catcaagtgc cggtcgtgca     780

gcttcctcgg caacggcggc agaaaattct gccagggcgg caaaaacgtc cgagacgaat     840

gccaggtcat ctgaaacagc agcggaacgg agcgcctctg ccgcggcaga cgcaaaaaca     900

gcggcggcgg ggagtgcgtc aacggcatcc acgaaggcga cagaggctgc gggaagtgcg     960

gtatcagcat cgcagagcaa aagtgcggca gaagcggcgg caatacgtgc aaaaaattcg 1020

gcaaaacgtg cagaagatat agcttcagct gtcgcgcttg aggatgcgga cacaacgaga 1080

aaggggatag tgcagctcag cagtgcaacc aacagcacgt ctgaaacgct tgctgcaacg 1140

ccaaaggcgg ttaaggcagc aaatgacaac gcaaattcac gtctggcgaa aaatcagaat 1200

ggtgcagata tccaggataa atcagctttt ctggacaatg ttggcgttac cagcctgacg 1260

tttatgaaaa acaatggcga aatgccggtt gatgctgatc tgaatacgtt tggttctgtt 1320

aaggcttatt caggtatctg gtctaaagca acgtccacca acgcaacact ggagaaaaac 1380

ttccctgaag ataatgctgt cggtgtgctt gaggttttta ctggcggcaa ttttgcaggc 1440

acgcaacgct ataccacacg tgacggaaat ttgtatatcc gcaaactcat tggaacatgg 1500

aatggtaatg atggaccatg gggagcatgg cgccatgttc aggctgtaac gcgagctcta 1560

agtacgacca ttgaccttaa ctctctcggt ggcgcagaac atttaggtct atggagaaac 1620

agcagttcag caatagcttc ttttgaacga cattaccccg agcagggagg agacgcgcag 1680

ggcattctgg aaattttcga aggtgggcta tatggacgca cacagcgtta tacaacccgt 1740

aacgggacta tgtatattcg cggcctgaca gccaaatggg atgcagaaaa tccacagtgg 1800

gaagactgga accaaattgg ttatcagacc agtagtacct tctatgagga tgacctggat 1860

gatttgatgt ctccaggtat ttacagtgtg acaggcaaag cgacccacac cccaatccag 1920

gggcagtctg gttttctgga agtcatcagg cgcaaggatg gtgtctatgt tttgcaacgt 1980

tacacgacca caggaaccag cgcagctaca aaagaccgtt tatatgagcg agtgtttctt 2040

ggtggctcat ttaacgcgtg gggggagtgg cgacagattt ataactcaaa ctctttgccg 2100

ttagagttgg gtatcggtgg cgcagtggca aaactcacca gcctggactg gcagacatac 2160

gattttgtgc cgggcagtct gataaccgtt cggctggata acatgaccaa tattcccgac 2220

ggtatggact ggggcgtcat tgatggcaac ctgataaaca tctcagtcgg tccgagtgat 2280

gattctggtt cgggacgctc aatgcatgta tggcgcagca ctgtaagtaa agccaactac 2340

cgctttttta tggtgcgtat ttcaggaaat ccgggaagcc gcacgatcac gacaagacgt 2400

gtgccaatta tcgacgaagc ccagacatgg ggcgcgaaac agacattcag tgctggcctt 2460

tctggtgaac tgtccggcaa tgcggcgaca gcaacaaagc tgaaaacagc ccgtaaaatt 2520

aataacgttt cgtttgatgg aacatcagat attaacctga cgccgaaaaa tattggtgca 2580

tttgcttcag gaaaaacagg agacaccgtt gcgaatgata aagccgttgg atggaactgg 2640

agtagcggag cctataacgc aactattggt ggggcatcaa cgttaattct tcattttaat 2700

atcggggaag gaagttgtcc cgccgcccag tttcgcgtta attataagaa cggtggtatt 2760

ttttatcgtt ctgctcgtga cggttacgga ttcgaggctg actggtctga gttttatacc 2820

acaacgcgaa aacctacagc gggagatgtc ggtgcactgc cgttatctgg tggtcaattg 2880

aatggtgctc tgggtatagg aacatccagt gctcttggcg gtaattcgat tgttttgggt 2940

gataatgaca cgggctttaa acaaaatggt gatggtaatc tggatgttta tgctaatagc 3000

gtccatgtta tgcgctttgt ctccggaagc gttcaaagta ataaaaccat aaatattacg 3060

gggcgtgtta atccctcgga ttacggtaac tttgattccc gctatgtgag agatgtcaga 3120

cttggcacac gtgttgtcca gaccatgcag aaaggggtga tgtatgagaa agcagggcac 3180

gtaattaccg ggcttggtat tgtcggtgaa gtcgatggtg atgaccccgc agtattcaga 3240

ccaatacaaa aatacatcaa tggcacatgg tataacgtcg cacaggtgta a 3291

<210> 16
<211> 1096
<212> PRT
<213> Artificial Sequence

<220>
<223> chimeric STF SIEA11

<400> 16

Met Ala Val Lys Ile Ser Gly Val Leu Lys Asp Gly Thr Gly Lys Pro
1               5                   10                  15

Val Gln Asn Cys Thr Ile Gln Leu Lys Ala Arg Arg Asn Ser Thr Thr
            20                  25                  30

Val Val Val Asn Thr Val Gly Ser Glu Asn Pro Asp Glu Ala Gly Arg
        35                  40                  45

Tyr Ser Met Asp Val Glu Tyr Gly Gln Tyr Ser Val Ile Leu Gln Val
    50                  55                  60

Asp Gly Phe Pro Pro Ser His Ala Gly Thr Ile Thr Val Tyr Glu Asp
65                  70                  75                  80

Ser Gln Pro Gly Thr Leu Asn Asp Phe Leu Cys Ala Met Thr Glu Asp
                85                  90                  95

Asp Ala Arg Pro Glu Val Leu Arg Arg Leu Glu Leu Met Val Glu Glu
            100                 105                 110

Val Ala Arg Asn Ala Ser Val Val Ala Gln Ser Thr Ala Asp Ala Lys
        115                 120                 125

Lys Ser Ala Gly Asp Ala Ser Ala Ser Ala Ala Gln Val Ala Ala Leu
    130                 135                 140

Val Thr Asp Ala Thr Asp Ser Ala Arg Ala Ala Ser Thr Ser Ala Gly

145                 150                 155                 160

Gln Ala Ala Ser Ser Ala Gln Glu Ala Ser Ser Gly Ala Glu Ala Ala
                165                 170                 175

Ser Ala Lys Ala Thr Glu Ala Glu Lys Ser Ala Ala Ala Ala Glu Ser
            180                 185                 190

Ser Lys Asn Ala Ala Ala Thr Ser Ala Gly Ala Ala Lys Thr Ser Glu
        195                 200                 205

Thr Asn Ala Ala Ala Ser Gln Gln Ser Ala Ala Thr Ser Ala Ser Thr
    210                 215                 220

Ala Ala Thr Lys Ala Ser Glu Ala Ala Thr Ser Ala Arg Asp Ala Val
225                 230                 235                 240

Ala Ser Lys Glu Ala Ala Lys Ser Ser Glu Thr Asn Ala Ser Ser Ser
                245                 250                 255

Ala Gly Arg Ala Ala Ser Ser Ala Thr Ala Ala Glu Asn Ser Ala Arg
            260                 265                 270

Ala Ala Lys Thr Ser Glu Thr Asn Ala Arg Ser Ser Glu Thr Ala Ala
        275                 280                 285

Glu Arg Ser Ala Ser Ala Ala Ala Asp Ala Lys Thr Ala Ala Ala Gly
    290                 295                 300

Ser Ala Ser Thr Ala Ser Thr Lys Ala Thr Glu Ala Ala Gly Ser Ala
305                 310                 315                 320

Val Ser Ala Ser Gln Ser Lys Ser Ala Ala Glu Ala Ala Ala Ile Arg
                325                 330                 335

Ala Lys Asn Ser Ala Lys Arg Ala Glu Asp Ile Ala Ser Ala Val Ala
            340                 345                 350

Leu Glu Asp Ala Asp Thr Thr Arg Lys Gly Ile Val Gln Leu Ser Ser
        355                 360                 365

Ala Thr Asn Ser Thr Ser Glu Thr Leu Ala Ala Thr Pro Lys Ala Val
    370                 375                 380

Lys Ala Ala Asn Asp Asn Ala Asn Ser Arg Leu Ala Lys Asn Gln Asn
385                 390                 395                 400

Gly Ala Asp Ile Gln Asp Lys Ser Ala Phe Leu Asp Asn Val Gly Val
                405                 410                 415

Thr Ser Leu Thr Phe Met Lys Asn Asn Gly Glu Met Pro Val Asp Ala
            420                 425                 430

Asp Leu Asn Thr Phe Gly Ser Val Lys Ala Tyr Ser Gly Ile Trp Ser
        435                 440                 445

Lys Ala Thr Ser Thr Asn Ala Thr Leu Glu Lys Asn Phe Pro Glu Asp
    450                 455                 460

Asn Ala Val Gly Val Leu Glu Val Phe Thr Gly Gly Asn Phe Ala Gly
465                 470                 475                 480

Thr Gln Arg Tyr Thr Thr Arg Asp Gly Asn Leu Tyr Ile Arg Lys Leu
                485                 490                 495

Ile Gly Thr Trp Asn Gly Asn Asp Gly Pro Trp Gly Ala Trp Arg His
            500                 505                 510

Val Gln Ala Val Thr Arg Ala Leu Ser Thr Thr Ile Asp Leu Asn Ser
        515                 520                 525

Leu Gly Gly Ala Glu His Leu Gly Leu Trp Arg Asn Ser Ser Ser Ala
    530                 535                 540

Ile Ala Ser Phe Glu Arg His Tyr Pro Glu Gln Gly Gly Asp Ala Gln
545                 550                 555                 560

Gly Ile Leu Glu Ile Phe Glu Gly Gly Leu Tyr Gly Arg Thr Gln Arg
                565                 570                 575

Tyr Thr Thr Arg Asn Gly Thr Met Tyr Ile Arg Gly Leu Thr Ala Lys
            580                 585                 590

Trp Asp Ala Glu Asn Pro Gln Trp Glu Asp Trp Asn Gln Ile Gly Tyr
        595                 600                 605

Gln Thr Ser Ser Thr Phe Tyr Glu Asp Asp Leu Asp Asp Leu Met Ser
    610                 615                 620

Pro Gly Ile Tyr Ser Val Thr Gly Lys Ala Thr His Thr Pro Ile Gln
625                 630                 635                 640

Gly Gln Ser Gly Phe Leu Glu Val Ile Arg Arg Lys Asp Gly Val Tyr
                645                 650                 655

Val Leu Gln Arg Tyr Thr Thr Thr Gly Thr Ser Ala Ala Thr Lys Asp
            660                 665                 670

Arg Leu Tyr Glu Arg Val Phe Leu Gly Gly Ser Phe Asn Ala Trp Gly
        675                 680                 685

Glu Trp Arg Gln Ile Tyr Asn Ser Asn Ser Leu Pro Leu Glu Leu Gly
    690                 695                 700

Ile Gly Gly Ala Val Ala Lys Leu Thr Ser Leu Asp Trp Gln Thr Tyr
705                 710                 715                 720

Asp Phe Val Pro Gly Ser Leu Ile Thr Val Arg Leu Asp Asn Met Thr
                725                 730                 735

Asn Ile Pro Asp Gly Met Asp Trp Gly Val Ile Asp Gly Asn Leu Ile
            740                 745                 750

Asn Ile Ser Val Gly Pro Ser Asp Asp Ser Gly Ser Gly Arg Ser Met
        755                 760                 765

His Val Trp Arg Ser Thr Val Ser Lys Ala Asn Tyr Arg Phe Phe Met
    770                 775                 780

Val Arg Ile Ser Gly Asn Pro Gly Ser Arg Thr Ile Thr Thr Arg Arg
785                 790                 795                 800

Val Pro Ile Ile Asp Glu Ala Gln Thr Trp Gly Ala Lys Gln Thr Phe
                805                 810                 815

Ser Ala Gly Leu Ser Gly Glu Leu Ser Gly Asn Ala Ala Thr Ala Thr
            820                 825                 830

Lys Leu Lys Thr Ala Arg Lys Ile Asn Asn Val Ser Phe Asp Gly Thr
        835                 840                 845

Ser Asp Ile Asn Leu Thr Pro Lys Asn Ile Gly Ala Phe Ala Ser Gly
    850                 855                 860

Lys Thr Gly Asp Thr Val Ala Asn Asp Lys Ala Val Gly Trp Asn Trp
865                 870                 875                 880

Ser Ser Gly Ala Tyr Asn Ala Thr Ile Gly Gly Ala Ser Thr Leu Ile
                885                 890                 895

Leu His Phe Asn Ile Gly Glu Gly Ser Cys Pro Ala Ala Gln Phe Arg
            900                 905                 910

Val Asn Tyr Lys Asn Gly Gly Ile Phe Tyr Arg Ser Ala Arg Asp Gly
        915                 920                 925

Tyr Gly Phe Glu Ala Asp Trp Ser Glu Phe Tyr Thr Thr Thr Arg Lys
    930                 935                 940

Pro Thr Ala Gly Asp Val Gly Ala Leu Pro Leu Ser Gly Gly Gln Leu
945                 950                 955                 960

Asn Gly Ala Leu Gly Ile Gly Thr Ser Ser Ala Leu Gly Gly Asn Ser
                965                 970                 975

Ile Val Leu Gly Asp Asn Asp Thr Gly Phe Lys Gln Asn Gly Asp Gly
            980                 985                 990

Asn Leu Asp Val Tyr Ala Asn Ser  Val His Val Met Arg  Phe Val Ser
        995                 1000                1005

Gly Ser  Val Gln Ser Asn Lys  Thr Ile Asn Ile Thr  Gly Arg Val
    1010                1015                1020

Asn Pro  Ser Asp Tyr Gly Asn  Phe Asp Ser Arg Tyr  Val Arg Asp
    1025                1030                1035

Val Arg  Leu Gly Thr Arg Val  Val Gln Thr Met Gln  Lys Gly Val
    1040                1045                1050

Met Tyr  Glu Lys Ala Gly His  Val Ile Thr Gly Leu  Gly Ile Val
    1055                1060                1065

Gly Glu  Val Asp Gly Asp Asp  Pro Ala Val Phe Arg  Pro Ile Gln
    1070                1075                1080

Lys Tyr  Ile Asn Gly Thr Trp  Tyr Asn Val Ala Gln  Val
    1085                1090                1095

<210>  17
<211>  528
<212>  DNA
<213>  Artificial Sequence

<220>
<223>  nucleic acid encoding accessory protein for STF SIEA11

<400>  17
atgcagcatt taaaaaatat tactgcgggt aatccaaaaa ctgttgccca atatcaactg      60

acaaaaaatt ttgatgttat ctggttatgg tccgaagagg gaaaaaactg gtatgaggaa     120

gtaagtaatt ttcaggaaga cacgataaag attgtttacg atgagaataa tataattgtc 180

ggcatcacca gagatgcttc aacgctcaac cctgaaggtt ttagcgttgt cgaggttcct 240

gatattaccg ccaaccgacg tgctgatgac tcaggtaaat ggatgtttaa ggatggtgcc 300

gtgattaagc ggatttatac ggcagacgaa cagctgcaac tggcggaatt acagaagtca 360

gctttgcttt ccgaagctga aactatcatt cagccactgg aacgctctgt cagactgaat 420

atggcaacag atgaggagcg tagccgactg gaagcatggg aacgctacag tgttctggtc 480

agccgtgtgg atcctgcaaa tcctgaatgg ccggaaatgc cgcaataa 528

<210> 18
<211> 175
<212> PRT
<213> Artificial Sequence

<220>
<223> accessory protein for STF SIEA11

<400> 18

Met Gln His Leu Lys Asn Ile Thr Ala Gly Asn Pro Lys Thr Val Ala
1               5                   10                  15

Gln Tyr Gln Leu Thr Lys Asn Phe Asp Val Ile Trp Leu Trp Ser Glu
            20                  25                  30

Glu Gly Lys Asn Trp Tyr Glu Glu Val Ser Asn Phe Gln Glu Asp Thr
        35                  40                  45

Ile Lys Ile Val Tyr Asp Glu Asn Asn Ile Ile Val Gly Ile Thr Arg
    50                  55                  60

Asp Ala Ser Thr Leu Asn Pro Glu Gly Phe Ser Val Val Glu Val Pro
65                  70                  75                  80

Asp Ile Thr Ala Asn Arg Arg Ala Asp Asp Ser Gly Lys Trp Met Phe
                85                  90                  95

Lys Asp Gly Ala Val Ile Lys Arg Ile Tyr Thr Ala Asp Glu Gln Leu
            100                 105                 110

Gln Leu Ala Glu Leu Gln Lys Ser Ala Leu Leu Ser Glu Ala Glu Thr
        115                 120                 125

Ile Ile Gln Pro Leu Glu Arg Ser Val Arg Leu Asn Met Ala Thr Asp
    130                 135                 140

Glu Glu Arg Ser Arg Leu Glu Ala Trp Glu Arg Tyr Ser Val Leu Val
145                 150                 155                 160

Ser Arg Val Asp Pro Ala Asn Pro Glu Trp Pro Glu Met Pro Gln
                165                 170                 175

<210>  19
<211>  747
<212>  DNA
<213>  Artificial Sequence

<220>
<223>  bla-oxa-48  gene

<400>  19
atgcctgcgg tagcaaagga atggcaagaa aacaaaagtt ggaatgctca ctttactgaa      60

cataaatcac agggcgtagt tgtgctctgg aatgagaata agcagcaagg atttaccaat     120

aatcttaaac gggcgaacca agcattttta cccgcatcta cctttaaaat tcccaatagc     180

ttgatcgccc tcgatttggg cgtggttaag gatgaacacc aagtctttaa gtgggatgga     240

cagacgcgcg atatcgccac ttggaatcgc gatcataatc taatcaccgc gatgaaatat     300

tcagttgtgc ctgtttatca agaatttgcc cgccaaattg gcgaggcacg tatgagcaag     360

atgctacatg ctttcgatta tggtaatgag gacatttcgg gcaatgtaga cagtttctgg     420

ctcgacggtg gtattcgaat ttcggccacg gagcaaatca gctttttaag aaagctgtat     480

cacaataagt tacacgtatc ggagcgcagc cagcgtattg tcaaacaagc catgctgacc     540

gaagccaatg gtgactatat tattcgggct aaaactggat actcgactag aatcgaacct     600

aagattggct ggtgggtcgg ttgggttgaa cttgatgata atgtgtggtt ttttgcgatg     660

aatatggata tgcccacatc ggatggttta gggctgcgcc aagccatcac aaaagaagtg     720

ctcaaacagg aaaaaattat tccctag                                         747

<210>  20
<211>  584
<212>  PRT
<213>  Artificial Sequence

<220>
<223>  PICI primase-helicase

<400>  20

Met Lys Leu Ala Pro Asn Val Lys Gln Gln Ser Arg Gly Ile Lys His
1               5                   10                  15

Lys Glu Thr Glu Val Ile Ile Phe Ala Gly Ser Asp Ala Trp Ser His
            20                  25                  30

Ala Lys Gln Trp Gln Glu His Asp Ala Arg Met Ala Gly Asp Asn Glu
        35                  40                  45

Pro Pro Val Trp Leu Gly Glu Gln Gln Leu Ser Glu Leu Asp Lys Leu
    50                  55                  60

Gln Ile Val Pro Glu Gly Arg Lys Ser Val Arg Ile Phe Arg Ala Gly
65                  70                  75                  80

Tyr Leu Ala Pro Val Met Ile Lys Ala Ile Gly Gln Lys Leu Ala Ala
                85                  90                  95

Ala Gly Val Gln Asp Ala Asn Phe Tyr Pro Asp Gly Met His Gly Gln
            100                 105                 110

Lys Val Glu Asn Trp Arg Glu Tyr Leu Ala Arg Glu Arg Gln Asn Leu
        115                 120                 125

Ser Asp Gly Leu Val Ile Glu Leu Pro Val Lys Gln Lys Ala Gln Leu
    130                 135                 140

Ser Gln Met Ala Asp Ser Glu Arg Ala Gln Leu Leu Ala Asp Arg Phe
145                 150                 155                 160

Asp Gly Val Cys Val His Pro Glu Ser Glu Ile Val His Val Trp Cys
                165                 170                 175

Gly Gly Val Trp Cys Pro Val Ser Thr Met Glu Leu Ser Arg Glu Met
            180                 185                 190

Val Ala Ile Tyr Ser Glu His Arg Ala Thr Phe Ser Lys Arg Val Ile
        195                 200                 205

Asn Asn Ala Val Glu Ala Leu Lys Val Ile Ala Glu Pro Met Gly Glu
    210                 215                 220

Pro Ser Gly Asp Leu Leu Pro Phe Ala Asn Gly Ala Leu Asp Leu Lys
225                 230                 235                 240

Thr Gly Glu Phe Ser Pro His Thr Pro Glu Asn Trp Ile Thr Thr His
                245                 250                 255

Asn Gly Ile Glu Tyr Thr Pro Pro Ala Pro Gly Glu Asn Ile Arg Asp
            260                 265                 270

Asn Ala Pro Asn Phe His Lys Trp Leu Glu His Ala Ala Gly Lys Asp
        275                 280                 285

Pro Arg Lys Met Met Arg Ile Cys Ala Ala Leu Tyr Met Ile Met Ala
    290                 295                 300

Asn Arg Tyr Asp Trp Gln Met Phe Ile Glu Ala Thr Gly Asp Gly Gly
305                 310                 315                 320

Ser Gly Lys Ser Thr Phe Thr His Ile Ala Ser Leu Leu Ala Gly Lys
                325                 330                 335

Gln Asn Thr Val Ser Ala Glu Met Thr Ser Leu Asp Asp Ala Gly Gly
            340                 345                 350

Arg Ala Gln Val Val Gly Ser Arg Leu Ile Val Leu Ala Asp Gln Pro
        355                 360                 365

Lys Tyr Thr Gly Glu Gly Thr Gly Ile Lys Lys Ile Thr Gly Gly Asp
    370                 375                 380

Pro Val Glu Ile Asn Pro Lys Tyr Glu Lys Arg Phe Thr Ala Val Ile
385                 390                 395                 400

Arg Ala Val Val Leu Ala Thr Asn Asn Asn Pro Met Ile Phe Thr Glu
                405                 410                 415

Arg Ala Gly Gly Val Ala Arg Arg Arg Val Ile Phe Arg Phe Asp Asn
            420                 425                 430

Ile Val Ser Glu Ala Glu Lys Asp Arg Glu Leu Pro Glu Lys Ile Ala
        435                 440                 445

Ala Glu Ile Pro Val Ile Ile Arg Arg Leu Leu Ala Asn Phe Ala Asp
    450                 455                 460

Pro Glu Lys Ala Arg Ala Leu Leu Ile Glu Gln Arg Asp Gly Asp Glu
465                 470                 475                 480

Ala Leu Ala Ile Lys Gln Gln Thr Asp Pro Val Ile Glu Phe Cys Gln
                485                 490                 495

Phe Leu Asn Phe Leu Glu Glu Ala Arg Gly Leu Met Met Gly Gly Gly
            500                 505                 510

Gly Asp Ser Val Lys Tyr Thr Thr Arg Asn Ser Leu Tyr Arg Val Tyr
        515                 520                 525

Leu Ala Phe Met Ala Tyr Ala Gly Arg Ser Lys Pro Leu Asn Val Asn
    530                 535                 540

Asp Phe Gly Lys Ala Met Lys Pro Ala Ala Lys Val Tyr Gly His Glu

545                 550                 555                 560

Tyr Ile Thr Arg Lys Val Lys Gly Val Thr Gln Thr Asn Ala Ile Thr
                565                 570                 575

Thr Asp Asp Cys Asp Ala Phe Leu
            580

<210>  21
<211>  1752
<212>  DNA
<213>  Artificial Sequence

<220>
<223>  PICI primase-helicase

<400>  21
atgaaactgg caccgaacgt aaaacagcag tcacgcggca taaaacacaa agaaacagaa      60

gtcattattt ttgcgggtag tgatgcctgg tcacacgcaa aacaatggca ggaacatgac     120

gcgcgtatgg ccggagataa tgagcctcct gtgtggcttg gggagcagca gttatccgaa      180

ctggataagc tgcaaattgt gccggaaggc agaaaatccg tgcgcatatt cagggccgga      240

tatcttgcgc cagtaatgat aaaggcgatt ggtcagaagc tggcggcggc aggcgtacag     300

gatgcaaatt tttaccctga tggtatgcac ggtcagaagg tggagaactg gcgcgaatat     360

ctggcccgtg agcgccagaa tctttctgat ggtctggtca ttgagcttcc ggtaaagcaa     420

aaggcgcaac tttcgcagat ggcggacagt gagcgcgcgc agctgcttgc cgatcgcttt     480

gatggcgttt gcgtacatcc tgaaagtgaa atcgttcacg tatggtgcgg cggggtatgg     540

tgtccggtca gcacaatgga gctgagccgc gaaatggtgg cgatctattc agagcacagg     600

gccactttca gcaagcgcgt aatcaataac gccgtggaag cgttaaaagt tattgccgaa     660

ccaatgggcg agccgtccgg cgatttgctg ccgttcgcca atggtgcgct tgacctgaaa     720

acgggggaat tttccccgca cacgccggag aactggatca ccacgcacaa cggcattgag      780

tacacgccac cagcacccgg ggagaacatc cgcgataacg cgccaaactt tcataaatgg     840

cttgagcacg cagccggaaa agacccgcgc aagatgatgc gtatatgtgc cgcgctgtac     900

atgattatgg cgaaccggta cgactggcag atgtttattg aggccaccgg agacggcggg     960

agcggtaaaa gtacattcac acacatagcc agccttctgg cagggaaaca aaacacggta    1020

agcgctgaaa tgacatcgct tgatgatgct ggtgggcgtg cgcaggttgt cgggagtcgt    1080

cttatcgtcc tggcagacca gccgaaatat acaggcgaag gaacgggcat caagaaaatc     1140

acgggcggcg accccgtgga aattaacccg aaatatgaaa agcgttttac ggcggtaatc    1200

agggcggtgg tgctggcaac caataacaat ccgatgatat tcaccgaacg ggccggaggt    1260

gtggcacgtc gtcgggtgat attccggttc gataacatcg taagcgaggc agaaaaagac    1320

agggagctac cggaaaagat cgcggctgaa atccctgtca ttatccgccg cttgctggcg 1380

aactttgccg accctgaaaa ggcacgggct ttactcattg aacagcgtga cggtgatgaa 1440

gcactggcaa taaagcaaca gacggatccg gttattgagt tttgccagtt cctgaatttt 1500

ctggaggaag cacgcggcct gatgatgggc ggcggtggcg attcagtgaa gtacacgacc 1560

agaaacagcc tttaccgcgt ctatctggcg tttatggcgt acgcaggcag gagcaaaccg 1620

ctaaacgtaa atgactttgg caaggctatg aagccagccg cgaaagttta cggacatgaa 1680

tatattacgc ggaaagttaa aggagtaacg cagactaacg caataacaac agacgattgc 1740

gacgcgtttt ta 1752

<210> 22
<211> 3393
<212> DNA
<213> Artificial Sequence

<220>
<223> nucleic acid encoding 1A2 gpJ

<400> 22
atgggtaaag gaagcagtaa ggggcatacc ccgcgcgaag cgaaggacaa cctgaagtcc 60

acgcagttgc tgagtgtgat cgatgccatc agcgaagggc cgattgaagg tccggtggat 120

ggcttaaaaa gcgtgctgct gaacagtacg ccggtgctgg acactgaggg gaataccaac 180

atatccggtg tcacggtggt gttccgggct ggtgagcagg agcagactcc gccggaggga 240

tttgaatcct ccggctccga gacggtgctg ggtacggaag tgaaatatga cacgccgatc 300

acccgcacca ttacgtctgc aaacatcgac cgtctgcgct ttaccttcgg tgtacaggca 360

ctggtggaaa ccacctcaaa gggtgacagg aatccgtcgg aagtccgcct gctggttcag 420

atacaacgta acggtggctg ggtgacggaa aaagacatca ccattaaggg caaaaccacc 480

tcgcagtatc tggcctcggt ggtgatgggt aacctgccgc cgcgcccgtt taatatccgg 540

atgcgcagga tgacgccgga cagcaccaca gaccagctgc agaacaaaac gctctggtcg 600

tcatacactg aaatcatcga tgtgaaacag tgctacccga acacggcact ggtcggcgtg 660

caggtggact cggagcagtt cggcagccag caggtgagcc gtaattatca tctgcgcggg 720

cgtattctgc aggtgccgtc gaactataac ccgcagacgc ggcaatacag cggtatctgg 780

gacggaacgt ttaaaccggc atacagcaac aacatggcct ggtgtctgtg ggatatgctg 840

acccatccgc gctacggcat ggggaaacgt cttggtgcgg cggatgtgga taaatgggcg 900

ctgtatgtca tcggccagta ctgcgaccag tcagtgccgg acggctttgg cggcacggag 960

ccgcgcatca cctgtaatgc gtacctgacc acacagcgta aggcgtggga tgtgctcagc 1020

gatttctgct cggcgatgcg ctgtatgccg gtatggaacg ggcagacgct gacgttcgtg 1080

caggaccgac cgtcggataa gacgtggacc tataaccgca gtaatgtggt gatgccggat 1140

gatggcgcgc cgttccgcta cagcttcagc gccctgaagg accgccataa tgccgttgag 1200

gtgaactgga ttgacccgaa caacggctgg gagacggcga cagagcttgt tgaagatacg 1260

caggccattg cccgttacgg tcgtaatgtt acgaagatgg atgcctttgg ctgtaccagc 1320

cgggggcagg cacaccgcgc cgggctgtgg ctgattaaaa cagaactgct ggaaacgcag 1380

accgtggatt tcagcgtcgg cgcagaaggg cttcgccatg taccgggcga tgttattgaa 1440

atctgcgatg atgactatgc cggtatcagc accggtggtc gtgtgctggc ggtgaacagc 1500

cagacccgga cgctgacgct cgaccgtgaa atcacgctgc catcctccgg taccgcgctg 1560

ataagcctgg ttgacggaag tggcaatccg gtcagcgtgg aggttcagtc cgtcaccgac 1620

ggcgtgaagg taaaagtgag ccgtgttcct gacggtgttg ctgaatacag cgtatgggag 1680

ctgaagctgc cgacgctgcg ccagcgactg ttccgctgcg tgagtatccg tgagaacgac 1740

gacggcacgt atgccatcac cgccgtgcag catgtgccgg aaaaagaggc catcgtggat 1800

aacggggcgc actttgacgg cgaacagagt ggcacggtga atggtgtcac gccgccagcg 1860

gtgcagcacc tgaccgcaga agtcactgca gacagcgggg aatatcaggt gctggcgcga 1920

tgggacacac cgaaggtggt gaagggcgtg agtttcctgc tccgtctgac cgtaacagcg 1980

gacgacggca gtgagcggct ggtcagcacg gcccggacga cggaaaccac ataccgcttc 2040

acgcaactgg cgctggggaa ctacaggctg acagtccggg cggtaaatgc gtgggggcag 2100

cagggcgatc cggcgtcggt atcgttccgg attgccgcac cggcagcacc gtcgaggatt 2160

gagctgacgc cgggctattt tcagataacc gccacgccgc atcttgccgt ttatgacccg 2220

acggtacagt ttgagttctg gttctcggaa aagcagattg cggatatcag acaggttgaa 2280

accagcacgc gttatcttgg tacggcgctg tactggatag ccgccagtat caatatcaaa 2340

ccgggccatg attattactt ttatatccgc agtgtgaaca ccgttggcaa atcggcattc 2400

gtggaggccg tcggtcgggc gagcgatgat gcggaaggtt acctggattt tttcaaaggc 2460

aagataaccg aatcccatct cggcaaggag ctgctggaaa aagtcgagct gacggaggat 2520

aacgccagca gactggagga gttttcgaaa gagtggaagg atgccagtga taagtggaat 2580

gccatgtggg ctgtcaaaat tgagcagacc aaagacggca aacattatgt cgcgggtatt 2640

ggcctcagca tggaggacac ggaggaaggc aaactgagcc agtttctggt tgccgccaat 2700

cgtatcgcat ttattgaccc ggcaaacggg aatgaaacgc cgatgtttgt ggcgcagggc 2760

aaccagatat tcatgaacga cgtgttcctg aagcgcctga cggcccccac cattaccagc 2820

ggcggcaatc ctccggcctt ttccctgaca ccggacggaa agctgaccgc taaaaatgcg 2880

gatatcagcg gtaacgtgaa tgcgaactcc gggacgctca acaacgtcac gattaacgag 2940

aactgtcggg ttctgggaaa attgtccgcg aaccagattg aaggcgatct cgttaaaaca 3000

gtgggcaaag ctttcccccg ggactcccgt gcaccggagc ggtggccatc aggaaccatt 3060

accgtcaggg tttatgacga tcagccgttt gaccggcaga ttgttattcc ggcggtggca 3120

ttcagcggcg ctaaacatga gaaagagcat actgatattt actcctcatg ccgtctgata 3180

gtgcggaaaa acggtgctga aatttataac cgtaccgcgc tggataatac gctgatttac 3240

agtggcgtta ttgatatgcc tgccggtcac ggtcacatga cactggagtt ttcggtgtca 3300

gcatggctgg taaataactg gtatcccaca gcaagtatca gcgatttgct ggttgtggtg 3360

atgaagaaag ccactgcagg catcacgatt agc 3393

<210> 23
<211> 1131
<212> PRT
<213> Artificial Sequence

<220>
<223> 1A2 gpJ

<400> 23

Met Gly Lys Gly Ser Ser Lys Gly His Thr Pro Arg Glu Ala Lys Asp
1               5                   10                  15

Asn Leu Lys Ser Thr Gln Leu Leu Ser Val Ile Asp Ala Ile Ser Glu
            20                  25                  30

Gly Pro Ile Glu Gly Pro Val Asp Gly Leu Lys Ser Val Leu Leu Asn
        35                  40                  45

Ser Thr Pro Val Leu Asp Thr Glu Gly Asn Thr Asn Ile Ser Gly Val
    50                  55                  60

Thr Val Val Phe Arg Ala Gly Glu Gln Glu Gln Thr Pro Pro Glu Gly
65                  70                  75                  80

Phe Glu Ser Ser Gly Ser Glu Thr Val Leu Gly Thr Glu Val Lys Tyr
                85                  90                  95

Asp Thr Pro Ile Thr Arg Thr Ile Thr Ser Ala Asn Ile Asp Arg Leu
            100                 105                 110

Arg Phe Thr Phe Gly Val Gln Ala Leu Val Glu Thr Thr Ser Lys Gly
        115                 120                 125

Asp Arg Asn Pro Ser Glu Val Arg Leu Leu Val Gln Ile Gln Arg Asn
    130                 135                 140

Gly Gly Trp Val Thr Glu Lys Asp Ile Thr Ile Lys Gly Lys Thr Thr

145                 150                 155                 160

Ser Gln Tyr Leu Ala Ser Val Val Met Gly Asn Leu Pro Pro Arg Pro
                165                 170                 175

Phe Asn Ile Arg Met Arg Arg Met Thr Pro Asp Ser Thr Thr Asp Gln
            180                 185                 190

Leu Gln Asn Lys Thr Leu Trp Ser Ser Tyr Thr Glu Ile Ile Asp Val
        195                 200                 205

Lys Gln Cys Tyr Pro Asn Thr Ala Leu Val Gly Val Gln Val Asp Ser
    210                 215                 220

Glu Gln Phe Gly Ser Gln Gln Val Ser Arg Asn Tyr His Leu Arg Gly
225                 230                 235                 240

Arg Ile Leu Gln Val Pro Ser Asn Tyr Asn Pro Gln Thr Arg Gln Tyr
                245                 250                 255

Ser Gly Ile Trp Asp Gly Thr Phe Lys Pro Ala Tyr Ser Asn Asn Met
            260                 265                 270

Ala Trp Cys Leu Trp Asp Met Leu Thr His Pro Arg Tyr Gly Met Gly
        275                 280                 285

Lys Arg Leu Gly Ala Ala Asp Val Asp Lys Trp Ala Leu Tyr Val Ile
    290                 295                 300

Gly Gln Tyr Cys Asp Gln Ser Val Pro Asp Gly Phe Gly Gly Thr Glu
305                 310                 315                 320

Pro Arg Ile Thr Cys Asn Ala Tyr Leu Thr Thr Gln Arg Lys Ala Trp
                325                 330                 335

Asp Val Leu Ser Asp Phe Cys Ser Ala Met Arg Cys Met Pro Val Trp
            340                 345                 350

Asn Gly Gln Thr Leu Thr Phe Val Gln Asp Arg Pro Ser Asp Lys Thr
        355                 360                 365

Trp Thr Tyr Asn Arg Ser Asn Val Val Met Pro Asp Asp Gly Ala Pro
    370                 375                 380

Phe Arg Tyr Ser Phe Ser Ala Leu Lys Asp Arg His Asn Ala Val Glu
385                 390                 395                 400

Val Asn Trp Ile Asp Pro Asn Asn Gly Trp Glu Thr Ala Thr Glu Leu
                405                 410                 415

Val Glu Asp Thr Gln Ala Ile Ala Arg Tyr Gly Arg Asn Val Thr Lys
            420                 425                 430

Met Asp Ala Phe Gly Cys Thr Ser Arg Gly Gln Ala His Arg Ala Gly
        435                 440                 445

Leu Trp Leu Ile Lys Thr Glu Leu Leu Glu Thr Gln Thr Val Asp Phe
    450                 455                 460

Ser Val Gly Ala Glu Gly Leu Arg His Val Pro Gly Asp Val Ile Glu
465                 470                 475                 480

Ile Cys Asp Asp Asp Tyr Ala Gly Ile Ser Thr Gly Gly Arg Val Leu
                485                 490                 495

Ala Val Asn Ser Gln Thr Arg Thr Leu Thr Leu Asp Arg Glu Ile Thr
            500                 505                 510

Leu Pro Ser Ser Gly Thr Ala Leu Ile Ser Leu Val Asp Gly Ser Gly
        515                 520                 525

Asn Pro Val Ser Val Glu Val Gln Ser Val Thr Asp Gly Val Lys Val
    530                 535                 540

Lys Val Ser Arg Val Pro Asp Gly Val Ala Glu Tyr Ser Val Trp Glu
545                 550                 555                 560

Leu Lys Leu Pro Thr Leu Arg Gln Arg Leu Phe Arg Cys Val Ser Ile
                565                 570                 575

Arg Glu Asn Asp Asp Gly Thr Tyr Ala Ile Thr Ala Val Gln His Val
            580                 585                 590

Pro Glu Lys Glu Ala Ile Val Asp Asn Gly Ala His Phe Asp Gly Glu
        595                 600                 605

Gln Ser Gly Thr Val Asn Gly Val Thr Pro Pro Ala Val Gln His Leu
    610                 615                 620

Thr Ala Glu Val Thr Ala Asp Ser Gly Glu Tyr Gln Val Leu Ala Arg
625                 630                 635                 640

Trp Asp Thr Pro Lys Val Val Lys Gly Val Ser Phe Leu Leu Arg Leu
                645                 650                 655

Thr Val Thr Ala Asp Asp Gly Ser Glu Arg Leu Val Ser Thr Ala Arg
            660                 665                 670

Thr Thr Glu Thr Thr Tyr Arg Phe Thr Gln Leu Ala Leu Gly Asn Tyr
        675                 680                 685

Arg Leu Thr Val Arg Ala Val Asn Ala Trp Gly Gln Gln Gly Asp Pro
    690                 695                 700

Ala Ser Val Ser Phe Arg Ile Ala Ala Pro Ala Ala Pro Ser Arg Ile
705                 710                 715                 720

Glu Leu Thr Pro Gly Tyr Phe Gln Ile Thr Ala Thr Pro His Leu Ala
                725                 730                 735

Val Tyr Asp Pro Thr Val Gln Phe Glu Phe Trp Phe Ser Glu Lys Gln
            740                 745                 750

Ile Ala Asp Ile Arg Gln Val Glu Thr Ser Thr Arg Tyr Leu Gly Thr
        755                 760                 765

Ala Leu Tyr Trp Ile Ala Ala Ser Ile Asn Ile Lys Pro Gly His Asp
    770                 775                 780

Tyr Tyr Phe Tyr Ile Arg Ser Val Asn Thr Val Gly Lys Ser Ala Phe
785                 790                 795                 800

Val Glu Ala Val Gly Arg Ala Ser Asp Asp Ala Glu Gly Tyr Leu Asp
                805                 810                 815

Phe Phe Lys Gly Lys Ile Thr Glu Ser His Leu Gly Lys Glu Leu Leu
            820                 825                 830

Glu Lys Val Glu Leu Thr Glu Asp Asn Ala Ser Arg Leu Glu Glu Phe
        835                 840                 845

Ser Lys Glu Trp Lys Asp Ala Ser Asp Lys Trp Asn Ala Met Trp Ala
    850                 855                 860

Val Lys Ile Glu Gln Thr Lys Asp Gly Lys His Tyr Val Ala Gly Ile
865                 870                 875                 880

Gly Leu Ser Met Glu Asp Thr Glu Glu Gly Lys Leu Ser Gln Phe Leu
                885                 890                 895

Val Ala Ala Asn Arg Ile Ala Phe Ile Asp Pro Ala Asn Gly Asn Glu
            900                 905                 910

Thr Pro Met Phe Val Ala Gln Gly Asn Gln Ile Phe Met Asn Asp Val
        915                 920                 925

Phe Leu Lys Arg Leu Thr Ala Pro Thr Ile Thr Ser Gly Gly Asn Pro
    930                 935                 940

Pro Ala Phe Ser Leu Thr Pro Asp Gly Lys Leu Thr Ala Lys Asn Ala
945                 950                 955                 960

Asp Ile Ser Gly Asn Val Asn Ala Asn Ser Gly Thr Leu Asn Asn Val
                965                 970                 975

Thr Ile Asn Glu Asn Cys Arg Val Leu Gly Lys Leu Ser Ala Asn Gln
            980                 985                 990

Ile Glu Gly Asp Leu Val Lys Thr  Val Gly Lys Ala Phe  Pro Arg Asp
        995                 1000                 1005

Ser Arg  Ala Pro Glu Arg Trp  Pro Ser Gly Thr Ile  Thr Val Arg
    1010                 1015                 1020

Val Tyr  Asp Asp Gln Pro Phe  Asp Arg Gln Ile Val  Ile Pro Ala
    1025                 1030                 1035

Val Ala  Phe Ser Gly Ala Lys  His Glu Lys Glu His  Thr Asp Ile
    1040                 1045                 1050

Tyr Ser  Ser Cys Arg Leu Ile  Val Arg Lys Asn Gly  Ala Glu Ile
    1055                 1060                 1065

Tyr Asn  Arg Thr Ala Leu Asp  Asn Thr Leu Ile Tyr  Ser Gly Val
    1070                 1075                 1080

Ile Asp  Met Pro Ala Gly His  Gly His Met Thr Leu  Glu Phe Ser
    1085                 1090                 1095

Val Ser  Ala Trp Leu Val Asn  Asn Trp Tyr Pro Thr  Ala Ser Ile
    1100                 1105                 1110

Ser Asp  Leu Leu Val Val Val  Met Lys Lys Ala Thr  Ala Gly Ile
    1115                 1120                 1125

Thr Ile  Ser
    1130

<210>  24

<211>  687
<212>  PRT
<213>  Artificial Sequence

<220>
<223>  STF27

<400>  24

Met Ala Val Lys Ile Ser Gly Val Leu Lys Asp Gly Thr Gly Lys Pro
1               5                   10                  15

Val Gln Asn Cys Thr Ile Gln Leu Lys Ala Arg Arg Asn Ser Thr Thr
            20                  25                  30

Val Val Val Asn Thr Val Gly Ser Glu Asn Pro Asp Glu Ala Gly Arg
        35                  40                  45

Tyr Ser Met Asp Val Glu Tyr Gly Gln Tyr Ser Val Ile Leu Gln Val
    50                  55                  60

Asp Gly Phe Pro Pro Ser His Ala Gly Thr Ile Thr Val Tyr Glu Asp
65                  70                  75                  80

Ser Gln Pro Gly Thr Leu Asn Asp Phe Leu Cys Ala Met Thr Glu Asp
                85                  90                  95

Asp Ala Arg Pro Glu Val Leu Arg Arg Leu Glu Leu Met Val Glu Glu
            100                 105                 110

Val Ala Arg Asn Ala Ser Val Val Ala Gln Ser Thr Ala Asp Ala Lys
        115                 120                 125

Lys Ser Glu Thr Ala Ala Ala Ser Ser Lys Asn Ala Ala Lys Thr Ser
    130                 135                 140

Glu Thr Asn Ala Ala Asn Ser Ala Gln Ala Ala Ala Ala Ser Gln Thr
145                 150                 155                 160

Ala Ser Ala Asn Ser Ala Thr Ala Ala Lys Lys Ser Glu Thr Ser Ala
                165                 170                 175

Lys Asn Ser Glu Thr Ala Thr Lys Ala Ser Glu Lys Asn Ala Lys Ser
            180                 185                 190

Ser Gln Thr Ala Ala Lys Thr Ser Glu Thr Asn Ala Lys Asp Ser Glu
        195                 200                 205

Ala Asn Ala Lys Val Ser Glu Thr Ala Ala Ala Asn Ser Ala Lys Ala
    210                 215                 220

Ser Ala Ala Ser Gln Thr Ala Ala Lys Ala Ser Glu Asp Ala Ala Arg
225                 230                 235                 240

Glu Tyr Ala Asn Gln Thr Ala Glu Pro Tyr Arg Tyr Val Leu Gln Pro
                245                 250                 255

Leu Pro Asp Val Trp Ile Pro Phe Asn Asp Ser Leu Asp Met Ile Thr
            260                 265                 270

Gly Tyr Ser Pro Gly Tyr Lys Lys Val Lys Ile Gly Asp Asn Val Val
        275                 280                 285

Gln Val Ala Ser Asp Lys Gln Val Asn Phe Ser Arg Ala Ser Thr Ala
    290                 295                 300

Thr Tyr Ile Asn Lys Ser Gly Glu Leu Lys Thr Ala Glu Ile Asn Glu
305                 310                 315                 320

Pro Arg Phe Glu Cys Asp Gly Leu Leu Ile Glu Gly Gln Arg Thr Asn
                325                 330                 335

Phe Phe Gln Asn Ser Thr Asp Pro Ser Lys Trp Asn Lys Ser Thr Ser
            340                 345                 350

Leu Asp Val Thr Glu Thr Gly Thr Asp Ser Phe Gly Phe Asn Tyr Gly
        355                 360                 365

Arg Phe Val Val Gln Asp Ser Ile Val Gly Thr Ser Lys Ala His Thr
    370                 375                 380

Ile Ile Gly Leu Tyr Ser Ser Thr Gly Gly Val Asp Thr Ser Gly Asp
385                 390                 395                 400

Glu Lys His Val Thr Ile Ser Cys Arg Val Lys Ser Glu Val Asp Asn
                405                 410                 415

Ile Ala Val Arg Ile Leu Phe Glu His Tyr Asp Gly Glu Val Arg Thr
            420                 425                 430

Ser Ile Gly Ala Ala Asn Leu Asn Leu Thr Thr Arg Ile Ile Ser Lys
        435                 440                 445

Thr Gly Gln Thr Ser Arg Val Thr Ala Arg Ser Val Lys Asp Asp Ala
    450                 455                 460

Thr Gly Trp Ile Phe Phe Glu Ala Thr Leu Lys Ala Asp Thr Thr Glu

465                 470                 475                 480

Asn Thr Val Gly Gly Phe Val Gln Tyr Ser Pro Asp Thr Gly Gln Met
                485                 490                 495

Val Thr Ser Gly Asp Tyr Leu Asp Val Thr Thr Pro Gln Ile Glu Ala
            500                 505                 510

Gly Thr Gly Ala Ser Ser Phe Ile Val Thr Gly Thr Ala Pro Ala Thr
        515                 520                 525

Arg Ala Ser Asp Met Val Thr Val Pro Ile Lys Asn Asn Leu Tyr Asn
    530                 535                 540

Leu Pro Phe Thr Val Leu Cys Glu Val His Lys Asn Trp Tyr Lys Thr
545                 550                 555                 560

Pro Asn Val Ala Pro Arg Val Phe Asp Thr Gly Gly His Gln Thr Gly
                565                 570                 575

Ala Gly Ile Val Met Gly Phe Gly Ser Ser Gly Gly Tyr Asp Gly Phe
            580                 585                 590

Pro Tyr Cys Asp Ile Gly Gly Ser Asp Arg Arg Ile Asn Glu Asn Ala
        595                 600                 605

Gly Leu Glu Lys Met Leu Ile Gly Met Arg Val Lys Ser Glu Arg Ser
    610                 615                 620

Thr Cys Val Val Ser Asn Gly Lys Leu Ser Ser Glu Thr Lys Thr Lys
625                 630                 635                 640

Trp Glu Tyr Ile Arg Ser Thr Ala Thr Ile Arg Ile Gly Gly Gln Thr
                645                 650                 655

Thr Ala Gly Leu Arg His Leu Phe Gly His Val Arg Asn Phe Arg Leu
            660                 665                 670

Trp His Lys Glu Leu Thr Asp Ala Gln Leu Gly Glu Val Val Glu
        675                 680                 685

<210>  25
<211>  92
<212>  PRT
<213>  Artificial Sequence

<220>
<223>  STF27 accessory protein

<400>  25

Val Arg Asp Phe Thr Leu Arg Phe Ser Asp Lys Ala Asp Phe Arg Ala
1               5                   10                  15

Phe Leu Arg Lys Leu Asn Trp Glu Glu Asp Glu Glu Leu Gln Asn Ala
            20                  25                  30

Val Leu Val Asp Glu Ile Gly Phe Thr Phe Arg Glu Thr Asp Val Ser
        35                  40                  45

Asp Asp Gly Glu Pro Glu Tyr Thr Arg Asn Glu Gly Tyr Phe Val Asn
    50                  55                  60

Ile Arg Leu Leu Asp Asp Gly Phe Glu Asp Ser Val Phe Arg Glu Trp
65                  70                  75                  80

Val Val Thr Pro Glu Arg Pro Leu Arg Glu Trp Phe
                85                  90

<210>  26
<211>  2061
<212>  DNA
<213>  Artificial Sequence

<220>
<223>  nucleic acid encoding STF27

<400>  26
atggcagtaa agatttcagg agtcctgaaa gacggcacag gaaaaccggt acagaactgc      60

accattcagc tgaaagccag acgtaacagc accacggtgg tggtgaacac ggtgggctca     120

gagaatccgg atgaagccgg gcgttacagc atggatgtgg agtacggtca gtacagtgtc     180

atcctgcagg ttgacggttt tccaccatcg cacgccggga ccatcaccgt gtatgaagat     240

tcacaaccgg ggacgctgaa tgattttctc tgtgccatga cggaggatga tgcccggccg     300

gaggtgctgc gtcgtcttga actgatggtg gaagaggtgg cgcgtaacgc gtccgtggtg     360

gcacagagta cggcagacgc gaagaaatca gaaacggcag cagcctcatc gaagaatgcg     420

gcgaaaacct cagaaacgaa tgcagctaac agcgcacagg cggcagcggc ctcgcagact     480

gcatcggcaa actccgcgac agcagccaaa aaatcagaaa ccagcgcgaa aaatagcgag     540

acagccacaa aggccagcga aaaaaacgca aaatccagcc agacggcagc gaaaaccagt     600

gagacgaatg ccaaagacag tgaagccaac gcaaaggtga gcgaaacagc ggcggcgaac     660

tcggcgaaag catcggcagc aagccagacg gcagcaaaag caagtgaaga tgctgccaga     720

gaatacgcaa accagacagc agagccgtac agatatgttt tacagccgct gccggatgtg     780

tggataccct ttaatgattc gctggatatg attacgggct attctccggg ttataaaaaa     840

gtgaagattg gtgataatgt ggttcaggtt gccagtgata aacaggttaa tttcagtcgc 900

gcatcaacgg caacatatat caacaaatct ggcgaactga aaacggcgga aattaatgag 960

ccgcgatttg agtgtgatgg cctgcttatt gagggacaaa gaacgaactt cttccagaac 1020

agtacagacc cttcgaagtg gaataagtca acttcactgg acgttacaga aacaggcaca 1080

gatagtttcg ggtttaatta tggtcggttt gtcgtacagg attcgattgt tggtacaagt 1140

aaagcgcata ccattatcgg actgtattcg agtaccggag gggttgatac ttcaggggac 1200

gaaaagcatg taactatatc ctgtcgggta aaaagtgaag ttgataatat cgccgttcgt 1260

attttatttg aacattatga tggggaggta aggacatcaa taggagcagc aaacctgaac 1320

cttaccaccc gcataattag caagacaggt cagacaagcc gtgttacagc aaggtctgtt 1380

aaggatgatg caactggctg gatatttttt gaggctacat taaaagcaga tacaacagaa 1440

aatacggttg gtggttttgt ccagtattct ccggatacag ggcagatggt tacatcaggg 1500

gattatctcg atgtaaccac tccacagatt gaggctggta caggcgcatc atcttttatt 1560

gttacgggga cggcaccggc aacgcgggca agcgatatgg tgacagtccc aatcaagaat 1620

aacctttata atcttccttt tacggttctt tgtgaggtac ataagaactg gtataaaacg 1680

ccaaatgtag cgccgcgtgt ttttgatacc ggcggtcatc aaaccggagc ggggatcgta 1740

atggggtttg gttcatcagg tgggtacgac ggttttccgt attgcgatat aggtggttca 1800

gaccgacgaa taaatgaaaa tgccgggctg gaaaaaatgc ttattggtat gcgggtaaag 1860

tccgaacggt ccacatgtgt agtcagtaac ggtaagttaa gcagcgaaac taaaaccaaa 1920

tgggaatata tccggagtac agcaaccatt cgcattggtg gacaaactac agcaggatta 1980

cgccatttat ttgggcatgt gaggaatttt cgtctctggc ataaagagct aacagatgcg 2040

cagcttgggg aggttgtgga g 2061

<210> 27
<211> 276
<212> DNA
<213> Artificial Sequence

<220>
<223> nucleic acid encoding STF27 accessory protein

<400> 27
gtgagagatt tcacgttgcg tttcagtgat aaagcagatt tcagggcatt tctcaggaaa 60

cttaactggg aagaggacga agagctgcag aatgccgttc tggttgatga gattggtttt 120

acgttcaggg agacagatgt ttctgatgac ggagaaccag aatacacgcg aaacgaaggg 180

tactttgtta atatccgtct tcttgacgat ggatttgagg attccgtgtt ccgtgagtgg 240

gtggttacac cagagcgccc gctcagggag tggttt 276

<210>  28
<211>  24
<212>  DNA
<213>  Artificial Sequence

<220>
<223>  CTX1 crRNA

<400>  28
gccagatcac cgcgatatcg ttgg                                              24


<210>  29
<211>  12299
<212>  DNA
<213>  Artificial Sequence

<220>
<223>  p775 payload

<400>  29
actgtcggaa tgacaaatgg ttccaattat tgaacaccct tcggggtgtt tttttgtttc        60

tggtttcccg aggccggcct gcgctagcgg agtgtatact ggcttactat gttggcactg       120

atgagggtgt aagtgaagtg cttcatgtgg caggagaaaa aaggctgcat cggtgcgtca       180

gcagaatatg tgatacagga tatattccgc ttcctcgctc actgactcgc tacgctcggt       240

cgttcgactg tggcgagcgg aaatggctta cgaacggggc ggagatttcc tggaagatgc       300

caggaagata cttaacaggg aagtgagagg gtcgcggcaa agccgttttt ccataggctc       360

cgcccccctg acaagcatca cgaaatctga cgctcaaatc agtggtggcg aaacctgaca       420

ggactataaa gataccaggc gtttccccct ggcggctccc tcgtgcgctc tcctgttcct       480

gcctttcggt ttgccggtgt cattcctctg ttacggccga gtttgtctca ttccacgcct       540

gacactcagt tccgggtagg cagttcgctc caagctggac tgtatgcacg aaccccccgt       600

tcagtccgac cgctgcgcct tatccggtaa ctatcgtctt gagtccaacc cggaaagaca       660

tgcaaaagca ccactggcag cagccactgg taattgattt agaggagtta gtcttgaagt       720

catgcgccgg ataaggctaa actgaaagga caagttttgg cgactgcgct cctccaagcc       780

agttacctcg gttcaaagag ttggtagctc agagaacctt cgaaaaaccg ccctgcaagg       840

cggttttttc gttttcagag caagagatta cgcgcagacc aaaacgatct caagaagatc       900

atcttattaa tcagataaaa tatttctaga tttcagtgca atttatctct tcaaatgtag       960

caccggcgcg ccgtgaccaa ttattgaagg ccgctaacgc ggcctttttt tgtttctggt      1020

atcccgaatg gagcgacttc tccccaaaaa gcctcgcttt cagcacctgt cgtttccttt      1080

cttttcagag ggtattttaa ataaaaacat taagttatga cgaagaagaa cggaaacgcc      1140

ttaaaccgga aaattttcat aaatagcgaa aacccgcgag gtcgccgccc cgtaacctgt      1200

cggatcaccg gaaaggaccc gtaaagtgat aatgattatc atctacatat cacaacgtgc      1260

gtaaagggta agtatgaagg tcgtgtactc catcgctacc aaattccaga aaacagacgc 1320

tttcgagcgt cttttttcgt tttggtcacg acgtacggtg gaagattcgt taccaattga 1380

cagctagctc agtcctaggt atatacatac atgcttgttt gtttgtaaac tactgttttc 1440

attaaagagg agaaaggaag ccatgtccat ctatcaggag tttgttaaca agtattccct 1500

gtctaaaacc ctgcgttttg aactgatccc gcagggcaaa actttggaaa acattaaagc 1560

gcgtggcctg attctggatg acgaaaaacg tgcaaaggat tacaagaaag ctaaacagat 1620

catcgacaaa tatcaccagt tctttatcga agaaattctg tcgtcggtgt gcatcagtga 1680

ggatctgtta cagaattatt ctgatgtata ctttaaactt aaaaagtccg atgacgataa 1740

tctgcaaaaa gatttcaagt cagccaaaga taccatcaag aaacagatct cagaatatat 1800

taaagatagc gaaaagttca aaaacctgtt taaccaaaac ctcattgatg ctaagaaagg 1860

ccaagaatct gacctgatct tatggctgaa acagagcaaa gataacggca ttgaactgtt 1920

caaagctaat agcgacatca ccgatattga tgaagcgctc gaaatcatca agtctttcaa 1980

aggctggacg acgtatttca aaggttttca tgaaaaccgt aagaatgtat attcgagcaa 2040

cgatattccg acctctatta tttatcgtat cgtggacgac aacctgccga agtttctgga 2100

aaacaaagcg aaatatgaat ctctgaaaga caaagcaccg gaagctatta actatgaaca 2160

gatcaagaaa gatctggcgg aagaactgac cttcgacatc gactataaaa cctccgaagt 2220

taaccagcgt gttttctcac tggacgaggt tttcgaaatc gctaatttca acaattacct 2280

gaatcaatct ggcatcacca aattcaacac cattattggt ggcaaatttg ttaacggcga 2340

aaacaccaag cgtaagggca tcaacgaata cattaacctg tatagccaac aaatcaacga 2400

caaaaccctg aaaaagtata aaatgtccgt tctgtttaaa cagattttat cggacaccga 2460

atctaaatcc ttcgtaattg ataaactgga agatgatagc gacgttgtca ccacgatgca 2520

gagcttttat gagcagattg cggcgttcaa aaccgtggaa gagaaatcta ttaaagaaac 2580

tctgtccctg ctctttgacg acctcaaagc gcagaaacta gatctgtcta agatttactt 2640

taaaaacgac aaatctctga ccgatctcag tcaacaagtt ttcgatgact atagcgtgat 2700

cggcacggca gttttggaat acatcaccca acaaatcgcg ccgaaaaatc tggacaaccc 2760

gtccaagaag gaacaggaac tgattgcaaa gaaaacagaa aaagctaaat acctgagctt 2820

agaaactatc aaactggcac ttgaggaatt taataaacat cgtgatattg ataaacagtg 2880

tcgttttgag gaaattctgg cgaactttgc ggcaatcccg atgatcttcg acgaaattgc 2940

tcaaaacaaa gacaatctgg cgcagatctc tatcaagtac cagaatcagg gtaagaaaga 3000

tctgcttcaa gcatctgcgg aggacgatgt gaaagcaatt aaagacttat tagatcagac 3060

gaataactta ttacacaagc tcaaaatctt ccacatcagc cagagcgagg acaaggcgaa 3120

cattctggat aaagatgaac acttctatct ggtgttcgaa gaatgttact tcgaactggc 3180

aaacatcgtc cctctctaca ataaaatccg caactacatc acgcagaagc cttactctga 3240

cgagaaattc aaactgaact tcgaaaacag cacgctggcg aacggctggg ataagaacaa 3300

agagccggac aacaccgcaa tcctgttcat caaagacgac aaatactatc tgggcgtaat 3360

gaacaagaag aacaacaaga tcttcgacga taaagcgatc aaagaaaaca agggtgaagg 3420

ctataagaaa atcgtgtaca agctcctgcc gggtgcgaat aaaatgttac cgaaagtgtt 3480

cttttccgcg aaaagcatca aattctacaa cccgtctgag gatattctgc gcatccgcaa 3540

tcatagcacg cacactaaaa acggtagccc gcagaaaggg tatgaaaaat tcgaatttaa 3600

tatagaggac tgccgtaagt tcatcgactt ctataaacag agcatttcca aacatccgga 3660

atggaaagac ttcggcttcc gtttctctga cactcagcgc tataatagca tcgacgagtt 3720

ctaccgcgaa gtggagaatc agggctataa actgaccttc gagaacatta gtgagtcgta 3780

catcgactcc gttgtgaatc agggtaaact gtacctgttt cagatctata ataaagactt 3840

tagcgcgtac agcaaaggcc gtccgaatct gcacaccctt tactggaaag cattatttga 3900

cgaacgtaac ctgcaagatg tggtgtataa actgaacggt gaggcggaac ttttctaccg 3960

taaacagagt atcccgaaga aaatcacgca tccggcaaaa gaagctattg ccaacaaaaa 4020

caaagacaac ccgaagaaag aatcagtatt cgaatatgac ctgatcaaag ataaacgttt 4080

caccgaagat aagttctttt tccactgtcc gattaccatc aacttcaaat ctagcggtgc 4140

gaacaagttc aacgatgaaa ttaacttatt actgaaagag aaagctaatg acgtacacat 4200

cttatctatt gatcgcggtg aacgtcattt agcatactat acactggtag atggtaaagg 4260

taatattatt aaacaggata ctttcaatat tatcggtaat gaccgtatga aaaccaacta 4320

tcacgataag ctggcggcga tcgaaaaaga tcgtgattct gcgcgtaaag attggaagaa 4380

aattaacaat atcaaagaaa tgaaagaagg ctatctgagc caagtggtgc acgagatcgc 4440

aaaactggtg attgaatata acgctatcgt ggttttcgaa gatctgaact ttggttttaa 4500

acgtggtcgc ttcaaagtag aaaaacaggt gtaccaaaaa ctggaaaaaa tgctgattga 4560

aaaactgaac tatctggttt ttaaagacaa cgaatttgac aaaacgggtg gcgtactccg 4620

tgcctatcag ctgaccgctc cgttcgaaac gttcaagaaa atgggtaaac aaacggggat 4680

tatctattat gtgccagctg gtttcacctc caagatttgt ccagttacgg gcttcgttaa 4740

ccagctgtac ccgaaatacg agagcgttag caaatctcaa gaatttttca gcaaattcga 4800

caagatctgc tataatctgg ataaaggcta tttcgagttc agcttcgatt acaaaaactt 4860

cggcgataaa gcggctaaag gtaagtggac tattgctagc tttggtagcc gtctgattaa 4920

ctttcgcaac tccgacaaaa accataattg ggacacgcgt gaagtgtatc cgaccaaaga 4980

actggaaaaa ttactgaaag actattccat cgaatatggt catggggagt gcattaaagc 5040

ggcgatttgc ggtgaatccg ataagaaatt tttcgccaaa ctgaccagcg tgcttaacac 5100

cattctgcaa atgcgtaatt ctaaaacggg tacggagctg gactacctga tttctccggt 5160

agccgacgtt aacggcaact tcttcgattc tcgtcaagca ccgaaaaata tgccacaaga 5220

cgcggatgcc aacggtgcat accatatcgg cttaaaaggc ttaatgttat taggccgtat 5280

caagaataat caggagggca agaaattaaa tctggttatc aaaaacgaag aatacttcga 5340

gttcgttcag aatcgtaaca attaatgtat gcttaagcag ctcggtacca aagacgaaca 5400

ataagacgct gaaaagcgtc ttttttcgtt ttggtcctgt tgcggcgcga tagtgtgaac 5460

atgctataga cttctggtgc tacccgactg acaattaatc atccggctcg tataatgcta 5520

gcaatttcta ctgttgtaga tgccagatca ccgcgatatc gttggtcgag acgaacaata 5580

aggcctccct aacggggggc cttttttatt gataacaaaa gtaacttcga gcttgtctac 5640

ctcctagcac cattattgca attaataaac aactaacgga caattctacc taacagtttt 5700

catatatgac gagcagttaa gtgatgagta aaggtgagga attatttact ggtgttgttc 5760

cgatcttagt tgaactggac ggcgatgtta acggtcataa attcagtgtt cgtggtgaag 5820

gtgaaggtga tgcaaccaac ggtaagctga ccctgaaatt catctgcact actggaaaat 5880

taccagtacc gtggcctact ctggtgacta ccctgaccta tggtgttcag tgtttttctc 5940

gttaccctga ccacatgaag caacatgatt tcttcaaatc tgcaatgccg gaaggttatg 6000

tacaggagcg caccatttct ttcaaagacg atggcacgta taaaacccgt gcagaggtta 6060

aatttgaagg tgacactctg gtgaatcgta ttgaactgaa aggcattgat ttcaaagagg 6120

acggcaatat tttaggccac aaactggaat ataacttcaa ctcccataac gtttacatca 6180

ccgcagacaa acagaagaac ggtatcaaag ctaacttcaa aattcgccat aacgttgaag 6240

atggtagcgt acagctggcg gatcattacc aacagaacac tccgattgga gatgctcctg 6300

ttttactgcc ggataaccac tacctgtcca cccagtctaa actgtcgaag gatccgaacg 6360

aaaagcgcga ccacatggtg ttattagagt tcgttaccgc tagtggtatc acgcacggta 6420

tggatgaact ctacaaataa gacgaacaat aaggggagcg ggaaaccgct cccctttttt 6480

attgataaca aaagtaaatt gcacgctgat agtctcccaa ttgcgaagga ccaaaacgaa 6540

aaaacaccct ttcgggtgtc ttttctggaa tttggtaccg agtactaggt atcgtgtaag 6600

tagcgaaggc ccgtacgcga gataaactgc taggcaaccg cgactctacg actggtgctc 6660

gatttaattt cgctgacgta aagaaattat cggcagtgcg tcaactgccg tatctttatc 6720

ttaattaggt agttggacaa gcccttgaaa gaaatagcaa gagcctgcct ctctattgaa 6780

gtcacggcga aagtcgggta gaaatcaaag aaagcagaaa ttaaatcgga gtaacactaa 6840

ggtgggataa ctccgtaact gactacgcct ttctctagac tttacttgac cagatacact 6900

gtctttgaca cgttgaagga ttagagcaat caaatccaag actggctaag cacgaagcaa 6960

ctcttgagtg ttaaaaagtt atctcctgta ttcgggaagc gggtactaga agattgcagg 7020

gactccgacg ttaagtaaat tacaaagtaa taagtatcgt tcaggatcac gttaccgcaa 7080

taagaagcga gaataatata atttccgaag tgcttacccc agtagtgact attcctataa 7140

cccttctgag tgtccggagg cggaaatttg ccacgaaaga gaaagtattt ccccgacaat 7200

aataaagggg cgctcctcag cttttccact tggttgggta agctaggcaa ctctgaaagg 7260

agtttcggcg aattgaagcc gacagctttg aattgtttta ggggcgttat tcgagggcaa 7320

tcggagctaa cttcaagact acttctttgt tgaatactaa atagtgcaaa ggtcgtgttt 7380

cctcaaggat actccgctaa caatatagga ttccaatcag attcagcact ggcggtacgg 7440

gtgttgcggt gaggcgttcg ggtttacggc tcgaagctag cacggtagga agcctgacaa 7500

tcaccaagca aaagggccgt cgaaggccca caagatacga aagctctcga agccttatcc 7560

ttgaccgatc cacctattta ggcagttacg cacaaaagct acccaataat ccgtgacagg 7620

cacaatatca cggaacaaaa ccgaaaactc tcgtacacgg ttaggttttc gctaggaaga 7680

ataaacctct atcttgatta taagaaggct ccccaagcac ccccaaaacc gaaatagcgg 7740

tttgcaataa gggacaagtt acgagtgtag acacgcagaa ttatccagcc tttagtcttt 7800

aggaaggcaa agctattgta cgcggtagcc gtcgtagcaa tttaccaact gtagaattat 7860

tggacacacg taggaagggc ttacagttga agtttaataa ggtcacacgc aaaaccgcta 7920

aggaataatc gcaccgttag cgaaagaata tttcagagcg gttagtaaag gttgagtaaa 7980

gtgagattcc aaagtgagcc tttataaaaa gtaaagagct ataataaaac cgtcgagcag 8040

aaaacaatcg cctgaaatct caagcacgtt gccctttcta acgtcgctaa ggtttcgtaa 8100

acccgtttga ttaggaagaa gaataagtaa cccgattagg tttgagatcg cgggttatcg 8160

gtttggatta aaagtggata ccagcggagt caacgccgac gcaaacgtac agtgatccaa 8220

tcctgttgca cggtcaagca caatcagctc gcaagatctt ggaatagtgt gcccaacagt 8280

ttagttgagg gccacgttcc gactacaagt tgcttcaaga ggggaatttg gatttggcaa 8340

tagccccccg tttctacctc aagaggcgac gagtattaac cgcgccagct gtcggcacaa 8400

gggccaaaga agattccaat ttcttattcc cgaataacct ccgaatccct gcgggaaaat 8460

caccgaccga atagcctaga agcaaggggg aacagatagg tataattagc ttaagagagt 8520

accagccgtg acaacagcgt agtaaccaca aacttacgct ggggcttctt tggcggattt 8580

ttacagatac taacaaggtg atttgaagta ccttagttga ggatttaaac gcgctatccg 8640

gtaatctcca aattgggaaa taccgttcaa agagggctag aattacttaa aagccttcac 8700

accgcctgcg ctatacgcgc ccactctccc gtttatccgt ccaagcggaa gcagggcgat 8760

cctccgctaa gatattctta cgtgtaacgt agctaagtat cccaaatagc tggcgtacgc 8820

gttgaacacc gcctagagga tcgtgactcg ccggacgagc gtgttattgg ggacttacgc 8880

cagcgtagac tacaacgcgc ccagattaac cctgcacgta ttgccttgaa taacgtacta 8940

atctctccgg ctctcgacaa tctatcgagc gactcgatta tcaacgggtg tcttgcagtt 9000

ctaatctctt gcccccgccc gtaatagcct ccaagagatt gaagatagta aagggcaaga 9060

gctgattcgg cgttgaagga tagcggactt tcggtcaacc acaattcccc actcgacaaa 9120

accagccgtg cgaataactc tgaaagtaca agcaacccaa gagggctgag cctaaactca 9180

gctaattcct aagtgagcta aagactcgaa gtgacagctc ttaataaata gagcgggaac 9240

gtcgaacggt cgtgaaagta atagtacaac gggtattaac ttactgagga tattgcttga 9300

agctgtaccg ttttattggg tgaacgaata agatccagca attcagccaa agaagctacc 9360

aatttttagt ttaagagtgt cacgtctgac ctcgcgggta gattgccgaa cgtagagctt 9420

acgagccagc ggaaacagta gccgcaggat aagtaagggg agtaagtgat cgaacgaatc 9480

agaagtgaca atatacttag gctggatctc gtcccgtgaa tcccaaccct caccaactac 9540

gagataagag gtaagccaaa aatcgacttg gtggcgacca acgactgttc cccccctgta 9600

actaatcgtt ccgtcaaaac ctgacttact tcaaggccaa ttccaagcgc aaacaatacc 9660

gtcctagttc ttcggttaag tttccgaagt aggagtgagc ctacctccgt ttgcgtcttg 9720

ttaccactga cccagctatt tactttgtat tgcctgcaat cgaatttctg aactctcaga 9780

tagtggggat aacgggaaag ttcctatatt tgcgaactaa cttagccgtc cacctcgaag 9840

ctacctactc acacccaccc cgcgcggggt aaataaggca ctaatcccag ctgagagctg 9900

gcgtagcact tagccacaag ttaattaaca gttgtctggt agtttggcgg tattaggaag 9960

atcctagaag caaggcagag ttagttctaa cctaaagcca caaataagac aggttgccaa 10020

agcccgccgg aaattaaatc ttgctcagtt cggtaacgga gtttccctcc cgcgtactta 10080

attcccaata agaaacgcgc ccaagtccta tcaggcaaaa ttcagcccct tcccgtgtta 10140

gaacgagggt aaaaatacaa gccgattgaa caagggttgg gggcttcaaa tcgtcgttta 10200

ccccacttta caacggagat taagtagttc accctatagt acgaagcaga actatttcga 10260

ggggcgtgca ataatcgaat cttctgcggt tgacttaaca cgctagggac gtgccctcga 10320

ttcaatcgaa ggtactccta ctcagactgc ctcacaccca gctagtcact gagcgataaa 10380

attgacccgc cctctaggga agcgagtacg tcccaaaggg ctccggacag ggctatatag 10440

gagagtttga tctcgccccg acaactgcaa ccctcaactc ccttagataa tattgttagc 10500

cgaagttgca cgacccgccg tccacggact gctcttaggg tgtggctcct taatctgaca 10560

acgtgcaacc cctatcgaag tcgattgttt ctgcgaaagg tgttgtccta atagtcccga 10620

aatttggccc ttgtaggtgt gaaaccactt agcttcgcgc cgtagtccta aaggcccacc 10680

tattgacttt gtttcgggta gcactaggaa tcttaacaat ttgaatttgg acgtggaacg 10740

cgtacacctt aatctccgaa taattctagg gatttggaag tcctctacgt tgacacacct 10800

acactgctcg aagtaaatat acgaataacg cgggcctcgc ggagccgttc cgaatcgtca 10860

cgtgttcgtt tactgttaat tggtggcaaa taagcaatat cgtagtccgt caggcccagc 10920

cctgttatcc acggcgttat ttgtcaaatt gcgtagaact ggattgactg cctgacaata 10980

cctaattatc ggtacgaagt ccccgaatct gtcgggctat ttcactaata ctttccaaac 11040

gccccgtatc caagaagaac gaatttatcc acgctcccgt ctttgggacg aataccgcta 11100

caagtggaca gaggatcggt acgggcctct aataaatcca acactctacg ccctcttcaa 11160

gagctagaag aacagggtgc agttggaaag ggaattattt cgtaaggcga gccaataccg 11220

taattaattc ggaagagtta acacgattgg aagtaggaat agtttctaac cacggttact 11280

aatcctaata acggaacgct gtctgataga ttagtgtcag cgctcggtac caaagaaaaa 11340

taaaaagacg ctgaaaagcg tctttttatt tttcggtcca gtgtaactca ggcaaaagca 11400

cgtaatattc gtactttctt cctccgtaag cgtcacccac attccttaaa gagtgcatgt 11460

gcatattttg ttatcaataa aaaaggccgc gatttgcggc cttattgttc gtcttgccgg 11520

attacgcccc gccctgccac tcatcgcagt attgttgtaa ttcattaagc attctgccga 11580

catggaagcc atcacaaacg gcatgatgaa cttggatcgc cagtggcatt aacaccttgt 11640

cgccttgcgt ataatatttt cccatagtga aaacgggggc gaagaagttg tccatatttg 11700

ctacgtttaa atcaaaactg gtgaaactca cccacggatt ggcactgacg aaaaacatat 11760

tttcgataaa ccctttaggg aaatatgcta agttttcacc gtaacacgcc acatcttgac 11820

tatatatgtg tagaaactgc cggaaatcgt cgtggtattc tgaccagagc gatgaaaacg 11880

tttcagtttg ctcatggaaa acggtgtaac aagggtgaac actatcccat atcaccagct 11940

caccgtcttt cattgccata cgaaactccg gatgtgcatt catcaggcgg gcaagaatgt 12000

gaataaaggc cggataaaac ttgtgcttat ttttctttac ggtttttaaa aaggccgtaa 12060

tatccagctg aacggtttgg ttataggtgc actgagcaac tgactggaat gcctcaaaat 12120

gttctttacg atgccattga cttatatcaa ctgtagtata tccagtgatt tttttctcca 12180

ttttagcttc cttagcttgc gaaatctcga taactcaaaa aatagtagtg atcttatttc 12240

attatggtga aagttgtctt acgtgcaaca ttttcgcaaa aagttggcgc tttatcaac 12299

<210> 30
<211> 24
<212> DNA
<213> Artificial Sequence

<220>
<223> CTX3 crRNA

<400> 30

tcgcggtgat gaacgctttc caat                                          24

<210>  31
<211>  12299
<212>  DNA
<213>  Artificial Sequence

<220>
<223>  p776 payload

<400>  31
actgtcggaa tgacaaatgg ttccaattat tgaacaccct tcggggtgtt tttttgtttc      60

tggtttcccg aggccggcct gcgctagcgg agtgtatact ggcttactat gttggcactg     120

atgagggtgt aagtgaagtg cttcatgtgg caggagaaaa aaggctgcat cggtgcgtca     180

gcagaatatg tgatacagga tatattccgc ttcctcgctc actgactcgc tacgctcggt     240

cgttcgactg tggcgagcgg aaatggctta cgaacggggc ggagatttcc tggaagatgc     300

caggaagata cttaacaggg aagtgagagg gtcgcggcaa agccgttttt ccataggctc     360

cgcccccctg acaagcatca cgaaatctga cgctcaaatc agtggtggcg aaacctgaca     420

ggactataaa gataccaggc gtttccccct ggcggctccc tcgtgcgctc tcctgttcct     480

gcctttcggt ttgccggtgt cattcctctg ttacggccga gtttgtctca ttccacgcct     540

gacactcagt tccgggtagg cagttcgctc caagctggac tgtatgcacg aaccccccgt     600

tcagtccgac cgctgcgcct tatccggtaa ctatcgtctt gagtccaacc cggaaagaca     660

tgcaaaagca ccactggcag cagccactgg taattgattt agaggagtta gtcttgaagt     720

catgcgccgg ataaggctaa actgaaagga caagttttgg cgactgcgct cctccaagcc     780

agttacctcg gttcaaagag ttggtagctc agagaacctt cgaaaaaccg ccctgcaagg     840

cggttttttc gttttcagag caagagatta cgcgcagacc aaaacgatct caagaagatc     900

atcttattaa tcagataaaa tatttctaga tttcagtgca atttatctct tcaaatgtag     960

caccggcgcg ccgtgaccaa ttattgaagg ccgctaacgc ggcctttttt tgtttctggt    1020

atcccgaatg gagcgacttc tccccaaaaa gcctcgcttt cagcacctgt cgtttccttt    1080

cttttcagag ggtattttaa ataaaaacat taagttatga cgaagaagaa cggaaacgcc    1140

ttaaaccgga aaattttcat aaatagcgaa aacccgcgag gtcgccgccc cgtaacctgt    1200

cggatcaccg gaaaggaccc gtaaagtgat aatgattatc atctacatat cacaacgtgc    1260

gtaaagggta agtatgaagg tcgtgtactc catcgctacc aaattccaga aaacagacgc    1320

tttcgagcgt cttttttcgt tttggtcacg acgtacggtg gaagattcgt taccaattga    1380

cagctagctc agtcctaggt atatacatac atgcttgttt gtttgtaaac tactgttttc    1440

attaaagagg agaaaggaag ccatgtccat ctatcaggag tttgttaaca agtattccct    1500

gtctaaaacc ctgcgttttg aactgatccc gcagggcaaa actttggaaa acattaaagc    1560

gcgtggcctg attctggatg acgaaaaacg tgcaaaggat tacaagaaag ctaaacagat 1620

catcgacaaa tatcaccagt tctttatcga agaaattctg tcgtcggtgt gcatcagtga 1680

ggatctgtta cagaattatt ctgatgtata ctttaaactt aaaaagtccg atgacgataa 1740

tctgcaaaaa gatttcaagt cagccaaaga taccatcaag aaacagatct cagaatatat 1800

taaagatagc gaaaagttca aaaacctgtt taaccaaaac ctcattgatg ctaagaaagg 1860

ccaagaatct gacctgatct tatggctgaa acagagcaaa gataacggca ttgaactgtt 1920

caaagctaat agcgacatca ccgatattga tgaagcgctc gaaatcatca agtctttcaa 1980

aggctggacg acgtatttca aaggttttca tgaaaaccgt aagaatgtat attcgagcaa 2040

cgatattccg acctctatta tttatcgtat cgtggacgac aacctgccga agtttctgga 2100

aaacaaagcg aaatatgaat ctctgaaaga caaagcaccg gaagctatta actatgaaca 2160

gatcaagaaa gatctggcgg aagaactgac cttcgacatc gactataaaa cctccgaagt 2220

taaccagcgt gttttctcac tggacgaggt tttcgaaatc gctaatttca acaattacct 2280

gaatcaatct ggcatcacca aattcaacac cattattggt ggcaaatttg ttaacggcga 2340

aaacaccaag cgtaagggca tcaacgaata cattaacctg tatagccaac aaatcaacga 2400

caaaaccctg aaaaagtata aaatgtccgt tctgtttaaa cagattttat cggacaccga 2460

atctaaatcc ttcgtaattg ataaactgga agatgatagc gacgttgtca ccacgatgca 2520

gagcttttat gagcagattg cggcgttcaa aaccgtggaa gagaaatcta ttaaagaaac 2580

tctgtccctg ctctttgacg acctcaaagc gcagaaacta gatctgtcta agatttactt 2640

taaaaacgac aaatctctga ccgatctcag tcaacaagtt ttcgatgact atagcgtgat 2700

cggcacggca gttttggaat acatcaccca acaaatcgcg ccgaaaaatc tggacaaccc 2760

gtccaagaag gaacaggaac tgattgcaaa gaaaacagaa aaagctaaat acctgagctt 2820

agaaactatc aaactggcac ttgaggaatt taataaacat cgtgatattg ataaacagtg 2880

tcgttttgag gaaattctgg cgaactttgc ggcaatcccg atgatcttcg acgaaattgc 2940

tcaaaacaaa gacaatctgg cgcagatctc tatcaagtac cagaatcagg gtaagaaaga 3000

tctgcttcaa gcatctgcgg aggacgatgt gaaagcaatt aaagacttat tagatcagac 3060

gaataactta ttacacaagc tcaaaatctt ccacatcagc cagagcgagg acaaggcgaa 3120

cattctggat aaagatgaac acttctatct ggtgttcgaa gaatgttact tcgaactggc 3180

aaacatcgtc cctctctaca ataaaatccg caactacatc acgcagaagc cttactctga 3240

cgagaaattc aaactgaact tcgaaaacag cacgctggcg aacggctggg ataagaacaa 3300

agagccggac aacaccgcaa tcctgttcat caaagacgac aaatactatc tgggcgtaat 3360

gaacaagaag aacaacaaga tcttcgacga taaagcgatc aaagaaaaca agggtgaagg 3420

ctataagaaa atcgtgtaca agctcctgcc gggtgcgaat aaaatgttac cgaaagtgtt 3480

cttttccgcg aaaagcatca aattctacaa cccgtctgag gatattctgc gcatccgcaa 3540

tcatagcacg cacactaaaa acggtagccc gcagaaaggg tatgaaaaat tcgaatttaa 3600

tatagaggac tgccgtaagt tcatcgactt ctataaacag agcatttcca aacatccgga 3660

atggaaagac ttcggcttcc gtttctctga cactcagcgc tataatagca tcgacgagtt 3720

ctaccgcgaa gtggagaatc agggctataa actgaccttc gagaacatta gtgagtcgta 3780

catcgactcc gttgtgaatc agggtaaact gtacctgttt cagatctata ataaagactt 3840

tagcgcgtac agcaaaggcc gtccgaatct gcacaccctt tactggaaag cattatttga 3900

cgaacgtaac ctgcaagatg tggtgtataa actgaacggt gaggcggaac ttttctaccg 3960

taaacagagt atcccgaaga aaatcacgca tccggcaaaa gaagctattg ccaacaaaaa 4020

caaagacaac ccgaagaaag aatcagtatt cgaatatgac ctgatcaaag ataaacgttt 4080

caccgaagat aagttctttt tccactgtcc gattaccatc aacttcaaat ctagcggtgc 4140

gaacaagttc aacgatgaaa ttaacttatt actgaaagag aaagctaatg acgtacacat 4200

cttatctatt gatcgcggtg aacgtcattt agcatactat acactggtag atggtaaagg 4260

taatattatt aaacaggata ctttcaatat tatcggtaat gaccgtatga aaaccaacta 4320

tcacgataag ctggcggcga tcgaaaaaga tcgtgattct gcgcgtaaag attggaagaa 4380

aattaacaat atcaaagaaa tgaaagaagg ctatctgagc caagtggtgc acgagatcgc 4440

aaaactggtg attgaatata acgctatcgt ggttttcgaa gatctgaact ttggttttaa 4500

acgtggtcgc ttcaaagtag aaaaacaggt gtaccaaaaa ctggaaaaaa tgctgattga 4560

aaaactgaac tatctggttt ttaaagacaa cgaatttgac aaaacgggtg gcgtactccg 4620

tgcctatcag ctgaccgctc cgttcgaaac gttcaagaaa atgggtaaac aaacggggat 4680

tatctattat gtgccagctg gtttcacctc caagatttgt ccagttacgg gcttcgttaa 4740

ccagctgtac ccgaaatacg agagcgttag caaatctcaa gaatttttca gcaaattcga 4800

caagatctgc tataatctgg ataaaggcta tttcgagttc agcttcgatt acaaaaactt 4860

cggcgataaa gcggctaaag gtaagtggac tattgctagc tttggtagcc gtctgattaa 4920

ctttcgcaac tccgacaaaa accataattg ggacacgcgt gaagtgtatc cgaccaaaga 4980

actggaaaaa ttactgaaag actattccat cgaatatggt catggggagt gcattaaagc 5040

ggcgatttgc ggtgaatccg ataagaaatt tttcgccaaa ctgaccagcg tgcttaacac 5100

cattctgcaa atgcgtaatt ctaaaacggg tacggagctg gactacctga tttctccggt 5160

agccgacgtt aacggcaact tcttcgattc tcgtcaagca ccgaaaaata tgccacaaga 5220

cgcggatgcc aacggtgcat accatatcgg cttaaaaggc ttaatgttat taggccgtat 5280

caagaataat caggagggca agaaattaaa tctggttatc aaaaacgaag aatacttcga 5340

gttcgttcag aatcgtaaca attaatgtat gcttaagcag ctcggtacca aagacgaaca 5400

ataagacgct gaaaagcgtc ttttttcgtt ttggtcctgt tgcggcgcga tagtgtgaac 5460

atgctataga cttctggtgc tacccgactg acaattaatc atccggctcg tataatgcta 5520

gcaatttcta ctgttgtaga ttcgcggtga tgaacgcttt ccaattcgag acgaacaata 5580

aggcctccct aacggggggc cttttttatt gataacaaaa gtaacttcga gcttgtctac 5640

ctcctagcac cattattgca attaataaac aactaacgga caattctacc taacagtttt 5700

catatatgac gagcagttaa gtgatgagta aaggtgagga attatttact ggtgttgttc 5760

cgatcttagt tgaactggac ggcgatgtta acggtcataa attcagtgtt cgtggtgaag 5820

gtgaaggtga tgcaaccaac ggtaagctga ccctgaaatt catctgcact actggaaaat 5880

taccagtacc gtggcctact ctggtgacta ccctgaccta tggtgttcag tgtttttctc 5940

gttaccctga ccacatgaag caacatgatt tcttcaaatc tgcaatgccg gaaggttatg 6000

tacaggagcg caccatttct ttcaaagacg atggcacgta taaaacccgt gcagaggtta 6060

aatttgaagg tgacactctg gtgaatcgta ttgaactgaa aggcattgat ttcaaagagg 6120

acggcaatat tttaggccac aaactggaat ataacttcaa ctcccataac gtttacatca 6180

ccgcagacaa acagaagaac ggtatcaaag ctaacttcaa aattcgccat aacgttgaag 6240

atggtagcgt acagctggcg gatcattacc aacagaacac tccgattgga gatgctcctg 6300

ttttactgcc ggataaccac tacctgtcca cccagtctaa actgtcgaag gatccgaacg 6360

aaaagcgcga ccacatggtg ttattagagt tcgttaccgc tagtggtatc acgcacggta 6420

tggatgaact ctacaaataa gacgaacaat aaggggagcg ggaaaccgct cccctttttt 6480

attgataaca aaagtaaatt gcacgctgat agtctcccaa ttgcgaagga ccaaaacgaa 6540

aaaacaccct ttcgggtgtc ttttctggaa tttggtaccg agtactaggt atcgtgtaag 6600

tagcgaaggc ccgtacgcga gataaactgc taggcaaccg cgactctacg actggtgctc 6660

gatttaattt cgctgacgta aagaaattat cggcagtgcg tcaactgccg tatctttatc 6720

ttaattaggt agttggacaa gcccttgaaa gaaatagcaa gagcctgcct ctctattgaa 6780

gtcacggcga aagtcgggta gaaatcaaag aaagcagaaa ttaaatcgga gtaacactaa 6840

ggtgggataa ctccgtaact gactacgcct ttctctagac tttacttgac cagatacact 6900

gtctttgaca cgttgaagga ttagagcaat caaatccaag actggctaag cacgaagcaa 6960

ctcttgagtg ttaaaaagtt atctcctgta ttcgggaagc gggtactaga agattgcagg 7020

gactccgacg ttaagtaaat tacaaagtaa taagtatcgt tcaggatcac gttaccgcaa 7080

taagaagcga gaataatata atttccgaag tgcttacccc agtagtgact attcctataa 7140

cccttctgag tgtccggagg cggaaatttg ccacgaaaga gaaagtattt ccccgacaat 7200

aataaagggg cgctcctcag cttttccact tggttgggta agctaggcaa ctctgaaagg 7260

agtttcggcg aattgaagcc gacagctttg aattgtttta ggggcgttat tcgagggcaa 7320

tcggagctaa cttcaagact acttctttgt tgaatactaa atagtgcaaa ggtcgtgttt 7380

cctcaaggat actccgctaa caatatagga ttccaatcag attcagcact ggcggtacgg 7440

gtgttgcggt gaggcgttcg ggtttacggc tcgaagctag cacggtagga agcctgacaa 7500

tcaccaagca aaagggccgt cgaaggccca caagatacga aagctctcga agccttatcc 7560

ttgaccgatc cacctattta ggcagttacg cacaaaagct acccaataat ccgtgacagg 7620

cacaatatca cggaacaaaa ccgaaaactc tcgtacacgg ttaggttttc gctaggaaga 7680

ataaacctct atcttgatta taagaaggct ccccaagcac ccccaaaacc gaaatagcgg 7740

tttgcaataa gggacaagtt acgagtgtag acacgcagaa ttatccagcc tttagtcttt 7800

aggaaggcaa agctattgta cgcggtagcc gtcgtagcaa tttaccaact gtagaattat 7860

tggacacacg taggaagggc ttacagttga agtttaataa ggtcacacgc aaaaccgcta 7920

aggaataatc gcaccgttag cgaaagaata tttcagagcg gttagtaaag gttgagtaaa 7980

gtgagattcc aaagtgagcc tttataaaaa gtaaagagct ataataaaac cgtcgagcag 8040

aaaacaatcg cctgaaatct caagcacgtt gccctttcta acgtcgctaa ggtttcgtaa 8100

acccgtttga ttaggaagaa gaataagtaa cccgattagg tttgagatcg cgggttatcg 8160

gtttggatta aaagtggata ccagcggagt caacgccgac gcaaacgtac agtgatccaa 8220

tcctgttgca cggtcaagca caatcagctc gcaagatctt ggaatagtgt gcccaacagt 8280

ttagttgagg gccacgttcc gactacaagt tgcttcaaga ggggaatttg gatttggcaa 8340

tagccccccg tttctacctc aagaggcgac gagtattaac cgcgccagct gtcggcacaa 8400

gggccaaaga agattccaat ttcttattcc cgaataacct ccgaatccct gcgggaaaat 8460

caccgaccga atagcctaga agcaaggggg aacagatagg tataattagc ttaagagagt 8520

accagccgtg acaacagcgt agtaaccaca aacttacgct ggggcttctt tggcggattt 8580

ttacagatac taacaaggtg atttgaagta ccttagttga ggatttaaac gcgctatccg 8640

gtaatctcca aattgggaaa taccgttcaa agagggctag aattacttaa aagccttcac 8700

accgcctgcg ctatacgcgc ccactctccc gtttatccgt ccaagcggaa gcagggcgat 8760

cctccgctaa gatattctta cgtgtaacgt agctaagtat cccaaatagc tggcgtacgc 8820

gttgaacacc gcctagagga tcgtgactcg ccggacgagc gtgttattgg ggacttacgc 8880

cagcgtagac tacaacgcgc ccagattaac cctgcacgta ttgccttgaa taacgtacta 8940

atctctccgg ctctcgacaa tctatcgagc gactcgatta tcaacgggtg tcttgcagtt 9000

ctaatctctt gcccccgccc gtaatagcct ccaagagatt gaagatagta aagggcaaga 9060

gctgattcgg cgttgaagga tagcggactt tcggtcaacc acaattcccc actcgacaaa 9120

accagccgtg cgaataactc tgaaagtaca agcaacccaa gagggctgag cctaaactca 9180

gctaattcct aagtgagcta aagactcgaa gtgacagctc ttaataaata gagcgggaac 9240

gtcgaacggt cgtgaaagta atagtacaac gggtattaac ttactgagga tattgcttga 9300

agctgtaccg ttttattggg tgaacgaata agatccagca attcagccaa agaagctacc 9360

aatttttagt ttaagagtgt cacgtctgac ctcgcgggta gattgccgaa cgtagagctt 9420

acgagccagc ggaaacagta gccgcaggat aagtaagggg agtaagtgat cgaacgaatc 9480

agaagtgaca atatacttag gctggatctc gtcccgtgaa tcccaaccct caccaactac 9540

gagataagag gtaagccaaa aatcgacttg gtggcgacca acgactgttc cccccctgta 9600

actaatcgtt ccgtcaaaac ctgacttact tcaaggccaa ttccaagcgc aaacaatacc 9660

gtcctagttc ttcggttaag tttccgaagt aggagtgagc ctacctccgt ttgcgtcttg 9720

ttaccactga cccagctatt tactttgtat tgcctgcaat cgaatttctg aactctcaga 9780

tagtggggat aacgggaaag ttcctatatt tgcgaactaa cttagccgtc cacctcgaag 9840

ctacctactc acacccaccc cgcgcggggt aaataaggca ctaatcccag ctgagagctg 9900

gcgtagcact tagccacaag ttaattaaca gttgtctggt agtttggcgg tattaggaag 9960

atcctagaag caaggcagag ttagttctaa cctaaagcca caaataagac aggttgccaa 10020

agcccgccgg aaattaaatc ttgctcagtt cggtaacgga gtttccctcc cgcgtactta 10080

attcccaata agaaacgcgc ccaagtccta tcaggcaaaa ttcagcccct tcccgtgtta 10140

gaacgagggt aaaaatacaa gccgattgaa caagggttgg gggcttcaaa tcgtcgttta 10200

ccccacttta caacggagat taagtagttc accctatagt acgaagcaga actatttcga 10260

ggggcgtgca ataatcgaat cttctgcggt tgacttaaca cgctagggac gtgccctcga 10320

ttcaatcgaa ggtactccta ctcagactgc ctcacaccca gctagtcact gagcgataaa 10380

attgacccgc cctctaggga agcgagtacg tcccaaaggg ctccggacag ggctatatag 10440

gagagtttga tctcgccccg acaactgcaa ccctcaactc ccttagataa tattgttagc 10500

cgaagttgca cgacccgccg tccacggact gctcttaggg tgtggctcct taatctgaca 10560

acgtgcaacc cctatcgaag tcgattgttt ctgcgaaagg tgttgtccta atagtcccga 10620

aatttggccc ttgtaggtgt gaaaccactt agcttcgcgc cgtagtccta aaggcccacc 10680

tattgacttt gtttcgggta gcactaggaa tcttaacaat ttgaatttgg acgtggaacg 10740

cgtacacctt aatctccgaa taattctagg gatttggaag tcctctacgt tgacacacct 10800

acactgctcg aagtaaatat acgaataacg cgggcctcgc ggagccgttc cgaatcgtca 10860

cgtgttcgtt tactgttaat tggtggcaaa taagcaatat cgtagtccgt caggcccagc 10920

cctgttatcc acggcgttat ttgtcaaatt gcgtagaact ggattgactg cctgacaata 10980

cctaattatc ggtacgaagt ccccgaatct gtcgggctat ttcactaata ctttccaaac 11040

gccccgtatc caagaagaac gaatttatcc acgctcccgt ctttgggacg aataccgcta 11100

caagtggaca gaggatcggt acgggcctct aataaatcca acactctacg ccctcttcaa 11160

gagctagaag aacagggtgc agttggaaag ggaattattt cgtaaggcga gccaataccg 11220

taattaattc ggaagagtta acacgattgg aagtaggaat agtttctaac cacggttact 11280

aatcctaata acggaacgct gtctgataga ttagtgtcag cgctcggtac caaagaaaaa 11340

taaaaagacg ctgaaaagcg tctttttatt tttcggtcca gtgtaactca ggcaaaagca 11400

cgtaatattc gtactttctt cctccgtaag cgtcacccac attccttaaa gagtgcatgt 11460

gcatattttg ttatcaataa aaaaggccgc gatttgcggc cttattgttc gtcttgccgg 11520

attacgcccc gccctgccac tcatcgcagt attgttgtaa ttcattaagc attctgccga 11580

catggaagcc atcacaaacg gcatgatgaa cttggatcgc cagtggcatt aacaccttgt 11640

cgccttgcgt ataatatttt cccatagtga aaacgggggc gaagaagttg tccatatttg 11700

ctacgtttaa atcaaaactg gtgaaactca cccacggatt ggcactgacg aaaaacatat 11760

tttcgataaa ccctttaggg aaatatgcta agttttcacc gtaacacgcc acatcttgac 11820

tatatatgtg tagaaactgc cggaaatcgt cgtggtattc tgaccagagc gatgaaaacg 11880

tttcagtttg ctcatggaaa acggtgtaac aagggtgaac actatcccat atcaccagct 11940

caccgtcttt cattgccata cgaaactccg gatgtgcatt catcaggcgg gcaagaatgt 12000

gaataaaggc cggataaaac ttgtgcttat ttttctttac ggtttttaaa aaggccgtaa 12060

tatccagctg aacggtttgg ttataggtgc actgagcaac tgactggaat gcctcaaaat 12120

gttctttacg atgccattga cttatatcaa ctgtagtata tccagtgatt tttttctcca 12180

ttttagcttc cttagcttgc gaaatctcga taactcaaaa aatagtagtg atcttatttc 12240

attatggtga aagttgtctt acgtgcaaca ttttcgcaaa aagttggcgc tttatcaac 12299

<210> 32
<211> 12342
<212> DNA
<213> Artificial Sequence

<220>
<223> p844 payload

<400> 32
actgtcggaa tgacaaatgg ttccaattat tgaacaccct tcggggtgtt tttttgtttc 60

tggtttcccg aggccggcct gcgctagcgg agtgtatact ggcttactat gttggcactg 120

atgagggtgt aagtgaagtg cttcatgtgg caggagaaaa aaggctgcat cggtgcgtca 180

gcagaatatg tgatacagga tatattccgc ttcctcgctc actgactcgc tacgctcggt 240

cgttcgactg tggcgagcgg aaatggctta cgaacggggc ggagatttcc tggaagatgc 300

caggaagata cttaacaggg aagtgagagg gtcgcggcaa agccgttttt ccataggctc 360

cgcccccctg acaagcatca cgaaatctga cgctcaaatc agtggtggcg aaacctgaca 420

ggactataaa gataccaggc gtttccccct ggcggctccc tcgtgcgctc tcctgttcct 480

gcctttcggt ttgccggtgt cattcctctg ttacggccga gtttgtctca ttccacgcct 540

gacactcagt tccgggtagg cagttcgctc caagctggac tgtatgcacg aaccccccgt 600

tcagtccgac cgctgcgcct tatccggtaa ctatcgtctt gagtccaacc cggaaagaca 660

tgcaaaagca ccactggcag cagccactgg taattgattt agaggagtta gtcttgaagt 720

catgcgccgg ataaggctaa actgaaagga caagttttgg cgactgcgct cctccaagcc 780

agttacctcg gttcaaagag ttggtagctc agagaacctt cgaaaaaccg ccctgcaagg 840

cggttttttc gttttcagag caagagatta cgcgcagacc aaaacgatct caagaagatc 900

atcttattaa tcagataaaa tatttctaga tttcagtgca atttatctct tcaaatgtag 960

caccggcgcg ccgtgaccaa ttattgaagg ccgctaacgc ggcctttttt tgtttctggt 1020

atcccgaatg gagcgacttc tccccaaaaa gcctcgcttt cagcacctgt cgtttccttt 1080

cttttcagag ggtattttaa ataaaaacat taagttatga cgaagaagaa cggaaacgcc 1140

ttaaaccgga aaattttcat aaatagcgaa aacccgcgag gtcgccgccc cgtaacctgt 1200

cggatcaccg gaaaggaccc gtaaagtgat aatgattatc atctacatat cacaacgtgc 1260

gtaaagggta agtatgaagg tcgtgtactc catcgctacc aaattccaga aaacagacgc 1320

tttcgagcgt cttttttcgt tttggtcacg acgtacggtg gaagattcgt taccaattga 1380

cagctagctc agtcctaggt atatacatac atgcttgttt gtttgtaaac tactgttttc 1440

attaaagagg agaaaggaag ccatgtccat ctatcaggag tttgttaaca agtattccct 1500

gtctaaaacc ctgcgttttg aactgatccc gcagggcaaa actttggaaa acattaaagc 1560

gcgtggcctg attctggatg acgaaaaacg tgcaaaggat tacaagaaag ctaaacagat 1620

catcgacaaa tatcaccagt tctttatcga agaaattctg tcgtcggtgt gcatcagtga 1680

ggatctgtta cagaattatt ctgatgtata ctttaaactt aaaaagtccg atgacgataa 1740

tctgcaaaaa gatttcaagt cagccaaaga taccatcaag aaacagatct cagaatatat 1800

taaagatagc gaaaagttca aaaacctgtt taaccaaaac ctcattgatg ctaagaaagg 1860

ccaagaatct gacctgatct tatggctgaa acagagcaaa gataacggca ttgaactgtt 1920

caaagctaat agcgacatca ccgatattga tgaagcgctc gaaatcatca agtctttcaa 1980

aggctggacg acgtatttca aaggttttca tgaaaaccgt aagaatgtat attcgagcaa 2040

cgatattccg acctctatta tttatcgtat cgtggacgac aacctgccga agtttctgga 2100

aaacaaagcg aaatatgaat ctctgaaaga caaagcaccg gaagctatta actatgaaca 2160

gatcaagaaa gatctggcgg aagaactgac cttcgacatc gactataaaa cctccgaagt 2220

taaccagcgt gttttctcac tggacgaggt tttcgaaatc gctaatttca acaattacct 2280

gaatcaatct ggcatcacca aattcaacac cattattggt ggcaaatttg ttaacggcga 2340

aaacaccaag cgtaagggca tcaacgaata cattaacctg tatagccaac aaatcaacga 2400

caaaaccctg aaaaagtata aaatgtccgt tctgtttaaa cagattttat cggacaccga 2460

atctaaatcc ttcgtaattg ataaactgga agatgatagc gacgttgtca ccacgatgca 2520

gagcttttat gagcagattg cggcgttcaa aaccgtggaa gagaaatcta ttaaagaaac 2580

tctgtccctg ctctttgacg acctcaaagc gcagaaacta gatctgtcta agatttactt 2640

taaaaacgac aaatctctga ccgatctcag tcaacaagtt ttcgatgact atagcgtgat 2700

cggcacggca gttttggaat acatcaccca acaaatcgcg ccgaaaaatc tggacaaccc 2760

gtccaagaag gaacaggaac tgattgcaaa gaaaacagaa aaagctaaat acctgagctt 2820

agaaactatc aaactggcac ttgaggaatt taataaacat cgtgatattg ataaacagtg 2880

tcgttttgag gaaattctgg cgaactttgc ggcaatcccg atgatcttcg acgaaattgc 2940

tcaaaacaaa gacaatctgg cgcagatctc tatcaagtac cagaatcagg gtaagaaaga 3000

tctgcttcaa gcatctgcgg aggacgatgt gaaagcaatt aaagacttat tagatcagac 3060

gaataactta ttacacaagc tcaaaatctt ccacatcagc cagagcgagg acaaggcgaa 3120

cattctggat aaagatgaac acttctatct ggtgttcgaa gaatgttact tcgaactggc 3180

aaacatcgtc cctctctaca ataaaatccg caactacatc acgcagaagc cttactctga 3240

cgagaaattc aaactgaact tcgaaaacag cacgctggcg aacggctggg ataagaacaa 3300

agagccggac aacaccgcaa tcctgttcat caaagacgac aaatactatc tgggcgtaat 3360

gaacaagaag aacaacaaga tcttcgacga taaagcgatc aaagaaaaca agggtgaagg 3420

ctataagaaa atcgtgtaca agctcctgcc gggtgcgaat aaaatgttac cgaaagtgtt 3480

cttttccgcg aaaagcatca aattctacaa cccgtctgag gatattctgc gcatccgcaa 3540

tcatagcacg cacactaaaa acggtagccc gcagaaaggg tatgaaaaat tcgaatttaa 3600

tatagaggac tgccgtaagt tcatcgactt ctataaacag agcatttcca aacatccgga 3660

atggaaagac ttcggcttcc gtttctctga cactcagcgc tataatagca tcgacgagtt 3720

ctaccgcgaa gtggagaatc agggctataa actgaccttc gagaacatta gtgagtcgta 3780

catcgactcc gttgtgaatc agggtaaact gtacctgttt cagatctata ataaagactt 3840

tagcgcgtac agcaaaggcc gtccgaatct gcacaccctt tactggaaag cattatttga 3900

cgaacgtaac ctgcaagatg tggtgtataa actgaacggt gaggcggaac ttttctaccg 3960

taaacagagt atcccgaaga aaatcacgca tccggcaaaa gaagctattg ccaacaaaaa 4020

caaagacaac ccgaagaaag aatcagtatt cgaatatgac ctgatcaaag ataaacgttt 4080

caccgaagat aagttctttt tccactgtcc gattaccatc aacttcaaat ctagcggtgc 4140

gaacaagttc aacgatgaaa ttaacttatt actgaaagag aaagctaatg acgtacacat 4200

cttatctatt gatcgcggtg aacgtcattt agcatactat acactggtag atggtaaagg 4260

taatattatt aaacaggata ctttcaatat tatcggtaat gaccgtatga aaaccaacta 4320

tcacgataag ctggcggcga tcgaaaaaga tcgtgattct gcgcgtaaag attggaagaa 4380

aattaacaat atcaaagaaa tgaaagaagg ctatctgagc caagtggtgc acgagatcgc 4440

aaaactggtg attgaatata acgctatcgt ggttttcgaa gatctgaact ttggttttaa 4500

acgtggtcgc ttcaaagtag aaaaacaggt gtaccaaaaa ctggaaaaaa tgctgattga 4560

aaaactgaac tatctggttt ttaaagacaa cgaatttgac aaaacgggtg gcgtactccg 4620

tgcctatcag ctgaccgctc cgttcgaaac gttcaagaaa atgggtaaac aaacggggat 4680

tatctattat gtgccagctg gtttcacctc caagatttgt ccagttacgg gcttcgttaa 4740

ccagctgtac ccgaaatacg agagcgttag caaatctcaa gaatttttca gcaaattcga 4800

caagatctgc tataatctgg ataaaggcta tttcgagttc agcttcgatt acaaaaactt 4860

cggcgataaa gcggctaaag gtaagtggac tattgctagc tttggtagcc gtctgattaa 4920

ctttcgcaac tccgacaaaa accataattg ggacacgcgt gaagtgtatc cgaccaaaga 4980

actggaaaaa ttactgaaag actattccat cgaatatggt catggggagt gcattaaagc 5040

ggcgatttgc ggtgaatccg ataagaaatt tttcgccaaa ctgaccagcg tgcttaacac 5100

cattctgcaa atgcgtaatt ctaaaacggg tacggagctg gactacctga tttctccggt 5160

agccgacgtt aacggcaact tcttcgattc tcgtcaagca ccgaaaaata tgccacaaga 5220

cgcggatgcc aacggtgcat accatatcgg cttaaaaggc ttaatgttat taggccgtat 5280

caagaataat caggagggca agaaattaaa tctggttatc aaaaacgaag aatacttcga 5340

gttcgttcag aatcgtaaca attaatgtat gcttaagcag ctcggtacca aagacgaaca 5400

ataagacgct gaaaagcgtc ttttttcgtt ttggtcctgt tgcggcgcga tagtgtgaac 5460

atgctataga cttctggtgc tacccgactg acaattaatc atccggctcg tataatgcta 5520

gcaatttcta ctgttgtaga tgccagatca ccgcgatatc gttggaattt ctactgttgt 5580

agattcgcgg tgatgaacgc tttccaattc gagacgaaca ataaggcctc cctaacgggg 5640

ggcctttttt attgataaca aaagtaactt cgagcttgtc tacctcctag caccattatt 5700

gcaattaata aacaactaac ggacaattct acctaacagt tttcatatat gacgagcagt 5760

taagtgatga gtaaaggtga ggaattattt actggtgttg ttccgatctt agttgaactg 5820

gacggcgatg ttaacggtca taaattcagt gttcgtggtg aaggtgaagg tgatgcaacc 5880

aacggtaagc tgaccctgaa attcatctgc actactggaa aattaccagt accgtggcct 5940

actctggtga ctaccctgac ctatggtgtt cagtgttttt ctcgttaccc tgaccacatg 6000

aagcaacatg atttcttcaa atctgcaatg ccggaaggtt atgtacagga gcgcaccatt 6060

tctttcaaag acgatggcac gtataaaacc cgtgcagagg ttaaatttga aggtgacact 6120

ctggtgaatc gtattgaact gaaaggcatt gatttcaaag aggacggcaa tattttaggc 6180

cacaaactgg aatataactt caactcccat aacgtttaca tcaccgcaga caaacagaag 6240

aacggtatca aagctaactt caaaattcgc cataacgttg aagatggtag cgtacagctg 6300

gcggatcatt accaacagaa cactccgatt ggagatgctc ctgttttact gccggataac 6360

cactacctgt ccacccagtc taaactgtcg aaggatccga acgaaaagcg cgaccacatg 6420

gtgttattag agttcgttac cgctagtggt atcacgcacg gtatggatga actctacaaa 6480

taagacgaac aataagggga gcgggaaacc gctccccttt tttattgata acaaaagtaa 6540

attgcacgct gatagtctcc caattgcgaa ggaccaaaac gaaaaaacac cctttcgggt 6600

gtcttttctg gaatttggta ccgagtacta ggtatcgtgt aagtagcgaa ggcccgtacg 6660

cgagataaac tgctaggcaa ccgcgactct acgactggtg ctcgatttaa tttcgctgac 6720

gtaaagaaat tatcggcagt gcgtcaactg ccgtatcttt atcttaatta ggtagttgga 6780

caagcccttg aaagaaatag caagagcctg cctctctatt gaagtcacgg cgaaagtcgg 6840

gtagaaatca aagaaagcag aaattaaatc ggagtaacac taaggtggga taactccgta 6900

actgactacg cctttctcta gactttactt gaccagatac actgtctttg acacgttgaa 6960

ggattagagc aatcaaatcc aagactggct aagcacgaag caactcttga gtgttaaaaa 7020

gttatctcct gtattcggga agcgggtact agaagattgc agggactccg acgttaagta 7080

aattacaaag taataagtat cgttcaggat cacgttaccg caataagaag cgagaataat 7140

ataatttccg aagtgcttac cccagtagtg actattccta taacccttct gagtgtccgg 7200

aggcggaaat ttgccacgaa agagaaagta tttccccgac aataataaag gggcgctcct 7260

cagcttttcc acttggttgg gtaagctagg caactctgaa aggagtttcg gcgaattgaa 7320

gccgacagct ttgaattgtt ttaggggcgt tattcgaggg caatcggagc taacttcaag 7380

actacttctt tgttgaatac taaatagtgc aaaggtcgtg tttcctcaag gatactccgc 7440

taacaatata ggattccaat cagattcagc actggcggta cgggtgttgc ggtgaggcgt 7500

tcgggtttac ggctcgaagc tagcacggta ggaagcctga caatcaccaa gcaaaagggc 7560

cgtcgaaggc ccacaagata cgaaagctct cgaagcctta tccttgaccg atccacctat 7620

ttaggcagtt acgcacaaaa gctacccaat aatccgtgac aggcacaata tcacggaaca 7680

aaaccgaaaa ctctcgtaca cggttaggtt ttcgctagga agaataaacc tctatcttga 7740

ttataagaag gctccccaag cacccccaaa accgaaatag cggtttgcaa taagggacaa 7800

gttacgagtg tagacacgca gaattatcca gcctttagtc tttaggaagg caaagctatt 7860

gtacgcggta gccgtcgtag caatttacca actgtagaat tattggacac acgtaggaag 7920

ggcttacagt tgaagtttaa taaggtcaca cgcaaaaccg ctaaggaata atcgcaccgt 7980

tagcgaaaga atatttcaga gcggttagta aaggttgagt aaagtgagat tccaaagtga 8040

gcctttataa aaagtaaaga gctataataa aaccgtcgag cagaaaacaa tcgcctgaaa 8100

tctcaagcac gttgcccttt ctaacgtcgc taaggtttcg taaacccgtt tgattaggaa 8160

gaagaataag taacccgatt aggtttgaga tcgcgggtta tcggtttgga ttaaaagtgg 8220

ataccagcgg agtcaacgcc gacgcaaacg tacagtgatc caatcctgtt gcacggtcaa 8280

gcacaatcag ctcgcaagat cttggaatag tgtgcccaac agtttagttg agggccacgt 8340

tccgactaca agttgcttca agaggggaat ttggatttgg caatagcccc ccgtttctac 8400

ctcaagaggc gacgagtatt aaccgcgcca gctgtcggca caagggccaa agaagattcc 8460

aatttcttat tcccgaataa cctccgaatc cctgcgggaa aatcaccgac cgaatagcct 8520

agaagcaagg gggaacagat aggtataatt agcttaagag agtaccagcc gtgacaacag 8580

cgtagtaacc acaaacttac gctggggctt ctttggcgga tttttacaga tactaacaag 8640

gtgatttgaa gtaccttagt tgaggattta aacgcgctat ccggtaatct ccaaattggg 8700

aaataccgtt caaagagggc tagaattact taaaagcctt cacaccgcct gcgctatacg 8760

cgcccactct cccgtttatc cgtccaagcg gaagcagggc gatcctccgc taagatattc 8820

ttacgtgtaa cgtagctaag tatcccaaat agctggcgta cgcgttgaac accgcctaga 8880

ggatcgtgac tcgccggacg agcgtgttat tggggactta cgccagcgta gactacaacg 8940

cgcccagatt aaccctgcac gtattgcctt gaataacgta ctaatctctc cggctctcga 9000

caatctatcg agcgactcga ttatcaacgg gtgtcttgca gttctaatct cttgcccccg 9060

cccgtaatag cctccaagag attgaagata gtaaagggca agagctgatt cggcgttgaa 9120

ggatagcgga ctttcggtca accacaattc cccactcgac aaaaccagcc gtgcgaataa 9180

ctctgaaagt acaagcaacc caagagggct gagcctaaac tcagctaatt cctaagtgag 9240

ctaaagactc gaagtgacag ctcttaataa atagagcggg aacgtcgaac ggtcgtgaaa 9300

gtaatagtac aacgggtatt aacttactga ggatattgct tgaagctgta ccgttttatt 9360

gggtgaacga ataagatcca gcaattcagc caaagaagct accaattttt agtttaagag 9420

tgtcacgtct gacctcgcgg gtagattgcc gaacgtagag cttacgagcc agcggaaaca 9480

gtagccgcag gataagtaag gggagtaagt gatcgaacga atcagaagtg acaatatact 9540

taggctggat ctcgtcccgt gaatcccaac cctcaccaac tacgagataa gaggtaagcc 9600

aaaaatcgac ttggtggcga ccaacgactg ttccccccct gtaactaatc gttccgtcaa 9660

aacctgactt acttcaaggc caattccaag cgcaaacaat accgtcctag ttcttcggtt 9720

aagtttccga agtaggagtg agcctacctc cgtttgcgtc ttgttaccac tgacccagct 9780

atttactttg tattgcctgc aatcgaattt ctgaactctc agatagtggg gataacggga 9840

aagttcctat atttgcgaac taacttagcc gtccacctcg aagctaccta ctcacaccca 9900

ccccgcgcgg ggtaaataag gcactaatcc cagctgagag ctggcgtagc acttagccac 9960

aagttaatta acagttgtct ggtagtttgg cggtattagg aagatcctag aagcaaggca 10020

gagttagttc taacctaaag ccacaaataa gacaggttgc caaagcccgc cggaaattaa 10080

atcttgctca gttcggtaac ggagtttccc tcccgcgtac ttaattccca ataagaaacg 10140

cgcccaagtc ctatcaggca aaattcagcc ccttcccgtg ttagaacgag ggtaaaaata 10200

caagccgatt gaacaagggt tgggggcttc aaatcgtcgt ttaccccact ttacaacgga 10260

gattaagtag ttcaccctat agtacgaagc agaactattt cgaggggcgt gcaataatcg 10320

aatcttctgc ggttgactta acacgctagg gacgtgccct cgattcaatc gaaggtactc 10380

ctactcagac tgcctcacac ccagctagtc actgagcgat aaaattgacc cgccctctag 10440

ggaagcgagt acgtcccaaa gggctccgga cagggctata taggagagtt tgatctcgcc 10500

ccgacaactg caaccctcaa ctcccttaga taatattgtt agccgaagtt gcacgacccg 10560

ccgtccacgg actgctctta gggtgtggct ccttaatctg acaacgtgca acccctatcg 10620

aagtcgattg tttctgcgaa aggtgttgtc ctaatagtcc cgaaatttgg cccttgtagg 10680

tgtgaaacca cttagcttcg cgccgtagtc ctaaaggccc acctattgac tttgtttcgg 10740

gtagcactag gaatcttaac aatttgaatt tggacgtgga acgcgtacac cttaatctcc 10800

gaataattct agggatttgg aagtcctcta cgttgacaca cctacactgc tcgaagtaaa 10860

tatacgaata acgcgggcct cgcggagccg ttccgaatcg tcacgtgttc gtttactgtt 10920

aattggtggc aaataagcaa tatcgtagtc cgtcaggccc agccctgtta tccacggcgt 10980

tatttgtcaa attgcgtaga actggattga ctgcctgaca atacctaatt atcggtacga 11040

agtccccgaa tctgtcgggc tatttcacta atactttcca aacgccccgt atccaagaag 11100

aacgaattta tccacgctcc cgtctttggg acgaataccg ctacaagtgg acagaggatc 11160

ggtacgggcc tctaataaat ccaacactct acgccctctt caagagctag aagaacaggg 11220

tgcagttgga aagggaatta tttcgtaagg cgagccaata ccgtaattaa ttcggaagag 11280

ttaacacgat tggaagtagg aatagtttct aaccacggtt actaatccta ataacggaac 11340

gctgtctgat agattagtgt cagcgctcgg taccaaagaa aaataaaaag acgctgaaaa 11400

gcgtcttttt atttttcggt ccagtgtaac tcaggcaaaa gcacgtaata ttcgtacttt 11460

cttcctccgt aagcgtcacc cacattcctt aaagagtgca tgtgcatatt ttgttatcaa 11520

taaaaaaggc cgcgatttgc ggccttattg ttcgtcttgc cggattacgc cccgccctgc 11580

cactcatcgc agtattgttg taattcatta agcattctgc cgacatggaa gccatcacaa 11640

acggcatgat gaacttggat cgccagtggc attaacacct tgtcgccttg cgtataatat 11700

tttcccatag tgaaaacggg ggcgaagaag ttgtccatat ttgctacgtt taaatcaaaa 11760

ctggtgaaac tcacccacgg attggcactg acgaaaaaca tattttcgat aaacccttta 11820

gggaaatatg ctaagttttc accgtaacac gccacatctt gactatatat gtgtagaaac 11880

tgccggaaat cgtcgtggta ttctgaccag agcgatgaaa acgtttcagt ttgctcatgg 11940

aaaacggtgt aacaagggtg aacactatcc catatcacca gctcaccgtc tttcattgcc 12000

atacgaaact ccggatgtgc attcatcagg cgggcaagaa tgtgaataaa ggccggataa 12060

aacttgtgct tatttttctt tacggttttt aaaaaggccg taatatccag ctgaacggtt 12120

tggttatagg tgcactgagc aactgactgg aatgcctcaa aatgttcttt acgatgccat 12180

tgacttatat caactgtagt atatccagtg atttttttct ccattttagc ttccttagct 12240

tgcgaaatct cgataactca aaaaatagta gtgatcttat ttcattatgg tgaaagttgt 12300

cttacgtgca acattttcgc aaaaagttgg cgctttatca ac 12342